# EUROPEAN PATENT APPLICATION

(11) **EP 3 571 925 A1**
(43) Date of publication of application: **27.11.2019**
(21) Application number: 18174057.2
(22) Date of filing: 24.05.2018
(51) Int. Cl.: A01H 1/04, C12Q 1/6895, C12N 15/82

(54) **ARTIFICIAL MARKER ALLELE**

(71) Applicant: KWS SAAT SE & Co. KGaA, 37574 Einbeck (DE)
(72) Inventor: BORCHARDT, Dietrich, 37574 Einbeck (DE)

(57) **Abstract**

This invention relates to a method for making an artificial marker allele for the identification of a nucleic acid of interest in an organism. The invention also relates to determining the presence of a nucleic acid of interest in a mixed population and a method for introgressing a nucleic acid of interest into a population. The invention also relates to organisms, particularly plants and seeds, comprising such a marker allele and to various uses for the artificial marker allele.

SEQUENCE LISTING

<110>  KWS SAAT SE

<120>  ARTIFICIAL MARKER ALLELE

<130>  KWS0260EP

<160>  80

<170>  PatentIn version 3.5

<210>  1
<211>  28
<212>  DNA
<213>  Artificial Sequence

<220>
<223>  crRNA_ALS_G/T

<400>  1
tttaggtatg gttgtccaat gggaagat                                          28


<210>  2
<211>  28
<212>  DNA
<213>  Artificial Sequence

<220>
<223>  crRNA_ALS_In1

<400>  2
tttcggctat ggatgttgtg ttgcatca                                          28


<210>  3
<211>  28
<212>  DNA
<213>  Artificial Sequence

<220>
<223>  crRNA_ALS_In2

<400>  3
tttctacgtg gttcggttga tcatatag                                          28


<210>  4
<211>  28
<212>  DNA
<213>  Artificial Sequence

<220>
<223>  crRNA_CYP_Del

<400>  4
tttgtacgtg gttcggttga tcatatag                                          28


<210>  5
<211>  27
<212>  DNA

<213>  Artificial Sequence

<220>
<223>  Mutagenesis primer 1

<400>  5
cagtgcagcc gtcgtctgaa aacgaca                                          27


<210>  6
<211>  26
<212>  DNA
<213>  Artificial Sequence

<220>
<223>  Mutagenesis primer 2

<400>  6
aacgtcagaa gccgactgca ctatag                                           26


<210>  7
<211>  20
<212>  DNA
<213>  Artificial Sequence

<220>
<223>  pSeq_LbCpf1_F1

<400>  7
agcgcaacgc aattaatgtg                                                  20


<210>  8
<211>  20
<212>  DNA
<213>  Artificial Sequence

<220>
<223>  pSeq_LbCpf1_R1

<400>  8
gatgaagctg aggtagtacc                                                  20


<210>  9
<211>  21
<212>  DNA
<213>  Artificial Sequence

<220>
<223>  pSeq_LbCpf1_F2

<400>  9
aggaaggtta gcaagctcga g                                                21


<210>  10
<211>  20
<212>  DNA
<213>  Artificial Sequence

<220>

<223>  pSeq_LbCpf1_R2

<400>  10
tctcgtcgac cttctggatg                                                    20


<210>  11
<211>  21
<212>  DNA
<213>  Artificial Sequence

<220>
<223>  pSeq_LbCpf1_F3

<400>  11
atgctgagta cgatgacatc c                                                  21


<210>  12
<211>  22
<212>  DNA
<213>  Artificial Sequence

<220>
<223>  pSeq_LbCpf1_R3

<400>  12
tagacctgct tctcaacctt ca                                                 22


<210>  13
<211>  20
<212>  DNA
<213>  Artificial Sequence

<220>
<223>  pSeq_LbCpf1_F4

<400>  13
accactcact cctcgataag                                                    20


<210>  14
<211>  21
<212>  DNA
<213>  Artificial Sequence

<220>
<223>  pSeq_LbCpf1_R4

<400>  14
aacgacaatc tgatcgggta c                                                  21


<210>  15
<211>  28
<212>  DNA
<213>  Artificial Sequence

<220>
<223>  crRNA_ALS_GT/TG protospacer oligonucleotide

<400>  15

agatggtatg gttgtccaat gggaagat                                          28

<210>  16
<211>  28
<212>  DNA
<213>  Artificial Sequence

<220>
<223>  crRNA_ALS_G/T protospacer oligonucleotide

<400>  16
ggccatcttc ccattggaca accatacc                                          28

<210>  17
<211>  28
<212>  DNA
<213>  Artificial Sequence

<220>
<223>  crRNA_ALS_In1 protospacer oligonucleotide

<400>  17
agatggctat ggatgttgtg ttgcatca                                          28

<210>  18
<211>  28
<212>  DNA
<213>  Artificial Sequence

<220>
<223>  crRNA_ALS_In1 protospacer oligonucleotide

<400>  18
ggcctgatgc aacacaacat ccatagcc                                          28

<210>  19
<211>  28
<212>  DNA
<213>  Artificial Sequence

<220>
<223>  crRNA_ALS_In2 protospacer oligonulceotide

<400>  19
agattacgtg gttcggttga tcatatag                                          28

<210>  20
<211>  28
<212>  DNA
<213>  Artificial Sequence

<220>
<223>  crRNA_ALS_In2 protospacer oligonucleotide

<400>  20
ggccctatat gatcaaccga accacgta                                          28

<210>  21
<211>  28
<212>  DNA
<213>  Artificial Sequence

<220>
<223>  crRNA_CYP_Del protospacer oligonucleotide

<400>  21
agattacgtg gttcggttga tcatatag                                          28


<210>  22
<211>  28
<212>  DNA
<213>  Artificial Sequence

<220>
<223>  crRNA_CYP_Del protospacer oligonucleotide

<400>  22
ggccctatat gatcaaccga accacgta                                          28


<210>  23
<211>  16862
<212>  DNA
<213>  Artificial Sequence

<220>
<223>  pZFNnptII_LbCpf1_crRNA cassette

<400>  23
ccggattgaa agaaatatag tttaaatatt tattgataaa ataacaagtc aggtattata      60

gtccaagcaa aaacataaat ttattgatgc aagtttaaat tcagaaatat ttcaataact     120

gattatatca gctggtacat tgccgtagat gaaagactga gtgcgatatt atgtgtaata     180

cataaattga tgatatagct agcttagctc atcgggtcag aagaactcgt caagaaggcg     240

atagaaggcg atgcgctgcg aatcgggagc ggcgataccg taaagcacga ggaagcggtc     300

agcccattcg ccgccaagct cttcagcaat atcacgggta gccaacgcta tgtcctgata     360

gcggtccgcc acacccagcc ggccacagtc gatgaatcca gaaaagcggc cattttccac     420

catgatattc ggcaagcagg catcgccatg agtcacgacg agatcctcgc cgtcgggcat     480

gcgcgccttg agcctggcga acagttcggc tggcgcgagc ccctgatgct cttcgtccag     540

atcatcctga tcgacaagac cggcttccat ccgagtacgt gctcgctcga tgcgatgttt     600

cgcttggtgg tcgaatgggc aggtagccgg atcaagcgta tgcagccgcc gcattgcatc     660

agccatgatg gatactttct cggcaggagc aaggtgagat gacaggagat cctgccccgg     720

cacttcgccc aatagcagcc agtcccttcc cgcttcagtg acaacgtcga gcacagctgc     780

gcaaggaacg cccgtcgtgg ccagccacga tagccgcgct gcctcgtcct gaagttcatt     840

cagggcaccg gacaggtcgg tcttgacaaa aagaaccggg cgcccctgcg ctgacagccg     900

gaacacggcg gcatcagagc agccgattgt ctgttgtgcc cagtcatagc cgaatagcct 960
ctccacccaa gcggccggag aacctgcgtg caatccatct tgttcaatcc aagctcccat 1020
tttctgtgat tttggatgtg tttttgtatg gattgagagt gaatatgaga ctctaattgg 1080
ataccgaggg gaatttatgg aacgtcagtg gagcattttt gacaagaaat atttgctagc 1140
tgatagtgac cttaggcgac ttttgaacgc gcaataatgg tttctgacgt atgtgcttag 1200
ctcattaaac tccagaaacc cgcggctgag tggctccttc aacgttgcgg ttctgtcagt 1260
tccaaacgta aaacggcttg tcccgcgtca tcggcggggg tcattccgga ctgtggggcc 1320
atatcccaga actggttgag tcggtccaac acctgggtgc caatcatgtc gatggtgggg 1380
tatggccaat tttttttcaa ttcaaaaatg tagatgtccg cagcgttatt ataaaatgaa 1440
agtacatttt gataaaacga caaattacga tccgtcgtat ttataggcga aagcaataaa 1500
caaattattc taattcggaa atctttattt cgacgtgtct acattcacgt ccaaatgggg 1560
gcttagatga gaaacttcac gatcggctct agaggccatg gcggccgcac tagtggatcc 1620
cccgggatgc atgaattcaa gcttatcgat gtcgacaggc cttaagggcc agatcccccg 1680
ggctgcagaa gcctatactg tacttaactt gattgcataa ttacttgatc atagactcat 1740
agtaaacttg attacacaga taagtgaaga aacaaaccaa ttcaagacat aaccaaagag 1800
aggtgaaaga ctgttttata tgtctaacat tgcaccttaa tatcacactg ttagttcctt 1860
tcttacttaa attcaaccca ttaaagtaaa aacaacagat aataataatt tgagaatgaa 1920
caaaaggacc atatcattta ttaactctta tccatccatt tgcattttga tgtccgaaaa 1980
caaaaactga aagaacacag taaattacaa gcagaacaaa tgatagaaga aaacagcttt 2040
tccaatgcca taatactcaa acttagtagg attctggtgt gtgggcaatg aaactgatgc 2100
attgaacttg acgaacgttg tcgaaaccga tgatacggac gaaagctggg aggcctggtc 2160
ccattcgcca tgccgaagca tgttgcccag ccggcgccag cgaggaggct gggaccatgc 2220
cggccaggtc ttccgcggtc agactgagcg aagactcatc tacacttagt agaaattaga 2280
cgagcttact cgtttcgtcc tcacggactc atcagtaatt tggatccgct gcacatacat 2340
aacatatcaa gatcagaaca cacatataca cacacaaata caatcaagtc aacaactcca 2400
aaaagtccag atctacatat atacatacgt aaataacaaa atcatgtaaa taatcacaat 2460
catgtaatcc agatctatgc acatatatat atacacaatt aataaaaaaa atgatataac 2520
agatctatat ctatgtatgt aacaacacaa tcagatgaga gaagtgatgt tttcagatct 2580
gtatacatac aaacacaaac agatgaacaa ttgatacgta gatccatatg tatacgtaca 2640
attagctaca cgattaaatg aaaaaaatca acgatttcgg attggtacac acaaacgcaa 2700
caatatgaag aaattcatat ctgattagat ataaacataa ccacgtgtag atacacagtc 2760
aaatcaacaa atttatagct tctaaacgga tgagatgaac aagataaaga tattcacata 2820

aggcatacat aagataagca gattaacaaa ctagcaataa tacataccta attaaaacaa 2880

ggaataacag agagagagag agagagagag atttaccttg aaaatgaaga ggagaagaga 2940

ggatttctta aaattggggg tagagaaaga aagatgatga attgtgagaa aggagagata 3000

gaaggggggg ttgtatatat aggctgtaga agattatttt tgtgtttgag gcggtgaagg 3060

aagaggggat ctgactatga cacgtttgcg gttacgtatt tcgataggag tctttcaacg 3120

cttaacgccg ttactctata tgaccgtttg ggccgtaacg gggccgtttg ttaacgctga 3180

tgttgattct tttctttctt tctttcttcc ttttttaaag aagcaattgt acaatcgttg 3240

ctagctgtca aacggataat tcggatacgg atatgcctat attcatatcc gtaatttttc 3300

tgcaggaatt cgatctggca cgacaggttt cccgactgga aagcgggcag tgagcgcaac 3360

gcaattaatg tgagttagct cactcattag gcaccccagg ctttacactt tatgcttccg 3420

gctcgtatgt tgtgtggaat tgtgagcgga taacaatttc acacaggaaa cagctatgac 3480

atgattacga attcaaaaat tacggatatg aatataggca tatccgtatc cgaattatcc 3540

gtttgacagc tagcaacgat tgtacaattg cttctttaaa aaaggaagaa agaaagaaag 3600

aaaagaatca acatcagcgt taacaaacgg ccccgttacg gcccaaacgg tcatatagag 3660

taacggcgtt aagcgttgaa agactcctat cgaaatacgt aaccgcaaac gtgtcatagt 3720

cagatcccct cttccttcac cgcctcaaac acaaaaataa tcttctacag cctatatata 3780

caaccccccc ttctatctct cctttctcac aattcatcat ctttctttct ctacccccaa 3840

ttttaagaaa tcctctcttc tcctcttcat tttcaaggta aatctctctc tctctctctc 3900

tctctgttat tccttgtttt aattaggtat gtattattgc tagtttgtta atctgcttat 3960

cttatgtatg ccttatgtga atatctttat cttgttcatc tcatccgttt agaagctata 4020

aatttgttga tttgactgtg tatctacacg tggttatgtt tatatctaat cagatatgaa 4080

tttcttcata ttgttgcgtt tgtgtgtacc aatccgaaat cgttgatttt tttcatttaa 4140

tcgtgtagct aattgtacgt atacatatgg atctacgtat caattgttca tctgtttgtg 4200

tttgtatgta tacagatctg aaaacatcac ttctctcatc tgattgtgtt gttacataca 4260

tagatataga tctgttatat catttttttt attaattgtg tatatatata tgtgcataga 4320

tctggattac atgattgtga ttatttacat gattttgtta tttacgtatg tatatatgta 4380

gatctggact ttttggagtt gttgacttga ttgtatttgt gtgtgtatat gtgtgttctg 4440

atcttgatat gttatgtatg tgcagcgaat tcggcgcgcc atggctccta agaagaagag 4500

gaaggttagc aagctcgaga agtttaccaa ctgctacagc ctctctaaga ccctcaggtt 4560

caaggctatc cctgtgggaa agacccaaga gaatatcgac aacaagaggc tcctcgtcga 4620

ggatgagaag agagctgaag attacaaggg cgtgaagaag ctcctcgaca ggtactacct 4680

cagcttcatc aacgatgtgc tccacagcat caagctcaag aacctcaaca actacatcag 4740
cctcttccgt aagaaaacca ggaccgagaa agagaacaaa gagcttgaga acctcgagat 4800
caacctccgt aaagagatcg ccaaggcttt caagggaaac gagggataca agagcctctt 4860
caagaaggat attatcgaga caatcctgcc tgagttcctg gacgataagg atgagatcgc 4920
tctcgtgaac agcttcaacg gattcactac tgccttcacc ggattcttcg acaacaggga 4980
aaacatgttc agcgaagagg ccaagagcac ctctatcgct ttcagatgca tcaacgagaa 5040
cctcacgcgt tacatcagca acatggacat cttcgagaag gtggacgcca tcttcgataa 5100
gcacgaggtg caagaaatca aagagaagat cctcaacagc gactacgacg tcgaggactt 5160
ttttgaaggg gagttcttca acttcgttct cacccaagag ggcatcgacg tgtacaacgc 5220
tattatcgga ggattcgtga ccgagtctgg ggagaagatt aagggactca acgagtacat 5280
caacctgtac aaccagaaaa cgaagcagaa gctcccgaag ttcaagccgc tctacaagca 5340
ggttctctct gatcgtgaga gcctctcatt ttacggtgag ggttacacct ctgacgagga 5400
agtgcttgag gttttccgta acaccctcaa caagaacagc gagatcttct cgtccatcaa 5460
gaagttggag aaacttttca agaacttcga cgagtacagc agcgctggga tcttcgttaa 5520
gaacggacct gctatcagca ccatcagcaa ggatattttc ggcgagtgga acgtgatcag 5580
ggacaagtgg aatgctgagt acgatgacat ccacctcaag aagaaggctg tcgtcactga 5640
gaagtacgag gatgacaggc gtaagtcgtt caagaagatc ggctctttca gcctcgagca 5700
gcttcaagaa tacgctgatg ctgatctcag cgtggtcgag aagctcaaag agatcatcat 5760
ccagaaggtc gacgagatct acaaggtgta cgggtcctct gagaagttgt tcgatgctga 5820
tttcgtcctc gagaagagtc tgaagaagaa cgacgctgtc gtcgcgatca tgaaggattt 5880
gctcgacagc gtgaagtcct tcgagaacta tatcaaggcc ttcttcggag agggcaaaga 5940
gactaatagg gacgagtctt tctacgggga tttcgtgctc gcttacgata tcctcctcaa 6000
ggtggaccat atctacgacg ccatcagaaa ctacgtgacc cagaagcctt acagcaagga 6060
caagttcaag ttgtactttc agaacccgca gttcatgggc ggatgggaca aagacaaaga 6120
gacagattac agggccacca tcctcaggta cgggtctaag tactacctgg ccatcatgga 6180
caagaaatac gccaagtgcc tccaaaagat cgacaaggat gacgtgaacg ggaactatga 6240
gaagatcaac tacaagctcc ttccgggacc gaacaagatg cttcctaagg tgttcttcag 6300
caagaaatgg atggcctact acaacccgtc tgaggacatc cagaaaatct acaagaacgg 6360
gaccttcaag aaaggcgaca tgttcaacct caacgactgc cacaagctca tcgatttctt 6420
caaggacagc atctcgcgtt acccgaagtg gtctaacgct tacgacttta acttcagcga 6480
gacagaaaag tacaaggata tcgccgggtt ctaccgtgag gttgaggaac agggttacaa 6540
ggttagcttc gagagcgcct ccaagaaaga ggttgacaag ttggtcgaag agggcaagct 6600

ctacatgttc cagatctata acaaggactt ctccgacaag agccacggaa ctcctaacct 6660
ccatacgatg tacttcaagc tgcttttcga cgagaacaac cacgggcaga tcagactttc 6720
tggtggtgct gaactcttca tgcgtagggc ctcactcaag aaagaagagt tggttgttca 6780
cccggccaac tctccaatcg ctaacaagaa tcctgacaac ccgaaaaaga ccaccacgct 6840
gtcttacgac gtctacaagg acaaaaggtt cagcgaggac cagtacgagc ttcatatccc 6900
gatcgctatc aacaagtgcc cgaagaacat cttcaagatc aataccgagg tgagggtgct 6960
gctcaagcac gatgataacc cttacgtgat cggaatcgat cgtggtgaga gaaacctcct 7020
ctacatcgtt gtggtggacg gaaagggaaa catcgtcgag cagtacagcc tgaacgagat 7080
tatcaacaat ttcaacggca tcaggatcaa gaccgactac cactcactcc tcgataagaa 7140
agaaaaagag cgtttcgagg ccaggcagaa ctggacttct atcgaaaaca tcaaagagtt 7200
gaaggccggc tacatctctc aggtggtgca taagatctgc gagctggtgg aaaagtacga 7260
tgctgtgatc gctcttgagg acctcaactc tgggttcaag aacagtagag tgaaggttga 7320
gaagcaggtc taccaaaagt tcgagaagat gctcatcgac aagctcaact acatggtgga 7380
caaaaagagc aacccttgcg ctaccggtgg tgctcttaag ggataccaga tcacgaacaa 7440
gttcgagtcc ttcaagagca tgagcaccca gaacggcttc atcttctata tccctgcttg 7500
gctcaccagc aagatcgatc cttctactgg tttcgtgaac ctgctcaaga ccaagtacac 7560
ctcgatcgcc gacagcaaga agttcatctc gtctttcgac aggatcatgt acgtgccgga 7620
agaggatctt ttcgagttcg ctctcgacta taagaacttc agcaggaccg acgccgacta 7680
cattaagaag tggaagctct actcctacgg gaaccgtatc aggatcttcc gaaatccgaa 7740
gaaaaacaac gtgttcgact gggaagaagt gtgcctcacc tctgcctaca aagaactgtt 7800
caacaagtac ggcatcaact accagcaggg tgatatcagg gctcttttgt gcgagcagag 7860
cgacaaggca ttctacagct cattcatggc cctcatgtct ctcatgctcc agatgaggaa 7920
ctctatcacc ggaaggaccg atgtggactt ccttatctct ccggtcaaga actctgacgg 7980
gatcttctac gacagccgta actatgaggc tcaagagaac gctatcctgc cgaagaatgc 8040
tgatgcaaac ggggcttaca acattgcgag aaaggttctc tgggctatcg ggcagtttaa 8100
gaaagcggaa gatgagaagc tcgacaaggt gaagatcgcc atctccaaca aagagtggct 8160
tgagtacgct cagacctccg ttaagcacaa gaggcctgct gctactaaga aagctggcca 8220
ggccaaaaag aagaagtgag gcgcgccgag ctccaggcct cccagctttc gtccgtatca 8280
tcggtttcga caacgttcgt caagttcaat gcatcagttt cattgcccac acaccagaat 8340
cctactaagt ttgagtatta tggcattgga aaagctgttt tcttctatca tttgttctgc 8400
ttgtaattta ctgtgttctt tcagtttttg ttttcggaca tcaaaatgca aatggatgga 8460

taagagttaa taaatgatat ggtccttttg ttcattctca aattattatt atctgttgtt 8520

tttactttaa tgggttgaat ttaagtaaga aaggaactaa cagtgtgata ttaaggtgca 8580

atgttagaca tataaaacag tctttcacct ctctttggtt atgtcttgaa ttggtttgtt 8640

tcttcactta tctgtgtaat caagtttact atgagtctat gatcaagtaa ttatgcaatc 8700

aagttaagta cagtataggc ttgagctccc taggatcaag cttatcgatg tcgacaggcc 8760

ttaagggcca gatcttgggc ccggtacccg atcagattgt cgtttcccgc cttcggttta 8820

aactatcagt gtttgacagg atatattggc gggtaaacct aagagaaaag agcgtttatt 8880

agaataatcg gatatttaaa agggcgtgaa aaggtttatc cgttcgtcca tttgtatgtg 8940

catgccaacc acagggttcc cctcgggagt gcttggcatt ccgtgcgata atgacttctg 9000

ttcaaccacc caaacgtcgg aaagcctgac gacggagcag cattccaaaa agatcccttg 9060

gctcgtctgg gtcggctaga aggtcgagtg ggctgctgtg gcttgatccc tcaacgcggt 9120

cgcggacgta gcgcagcgcc gaaaaatcct cgatcgcaaa tccgacgctg tcgaaaagcg 9180

tgatctgctt gtcgctcttt cggccgacgt cctggccagt catcacgcgc caaagttccg 9240

tcacaggatg atctggcgcg agttgctgga tctcgccttc aatccgggtc tgtggcggga 9300

actccacgaa aatatccgaa cgcagcaaga tatcgcggtg catctcggtc ttgcctgggc 9360

agtcgccgcc gacgccgttg atgtggacgc cgaaaaggat ctaggtgaag atcctttttg 9420

ataatctcat gaccaaaatc ccttaacgtg agttttcgtt ccactgagcg tcagaccccg 9480

tagaaaagat caaaggatct tcttgagatc ctttttttct gcgcgtaatc tgctgcttgc 9540

aaacaaaaaa accaccgcta ccagcggtgg tttgtttgcc ggatcaagag ctaccaactc 9600

tttttccgaa ggtaactggc ttcagcagag cgcagatacc aaatactgtt cttctagtgt 9660

agccgtagtt aggccaccac ttcaagaact ctgtagcacc gcctacatac ctcgctctgc 9720

taatcctgtt accagtggct gctgccagtg gcgataagtc gtgtcttacc gggttggact 9780

caagacgata gttaccggat aaggcgcagc ggtcgggctg aacggggggt tcgtgcacac 9840

agcccagctt ggagcgaacg acctacaccg aactgagata cctacagcgt gagctatgag 9900

aaagcgccac gcttcccgaa gggagaaagg cggacaggta tccggtaagc ggcagggtcg 9960

gaacaggaga gcgcacgagg gagcttccag ggggaaacgc ctggtatctt tatagtcctg 10020

tcgggtttcg ccacctctga cttgagcgtc gatttttgtg atgctcgtca ggggggcgga 10080

gcctatggaa aaacgccagc aacgcggcct ttttacggtt cctggccttt tgctggcctt 10140

ttgctcacat gttctttcct gcgttatccc ctgattctgt ggataaccga ttaccgcctt 10200

tgagtgagct gataccgctc gccgcagccg aacgaccgag cgcagcgagt cagtgagcga 10260

ggaagcggaa gagcgcctga tgcggtattt tctccttacg catctgtgcg gtatttcaca 10320

ccgcatatgg tgcactctca gtacaatctg ctctgatgcc gcatagttaa gccagtatac 10380

actccgctat cgctacgtga ctgggtcatg gctgcgcccc gacacccgcc aacacccgct 10440

gacgcgccct gacgggcttg tctgctcccg gcatccgctt acagacaagc tgtgaccgtc 10500

tccgggagct gcatgtgtca gaggttttca ccgtcatcac cgaaacgcgc gaggcagggg 10560

tacgtcgagg tcgatccaac ccctccgctg ctatagtgca gtcggcttct gacgttcagt 10620

gcagccgtcg tctgaaaacg acatgtcgca caagtcctaa gttacgcgac aggctgccgc 10680

cctgcccttt tcctggcgtt ttcttgtcgc gtgttttagt cgcataaagt agaatacttg 10740

cgactagaac cggagacatt acgccatgaa caagagcgcc gccgctggcc tgctgggcta 10800

tgcccgcgtc agcaccgacg accaggactt gaccaaccaa cgggccgaac tgcacgcggc 10860

cggctgcacc aagctgtttt ccgagaagat caccggcacc aggcgcgacc gcccggagct 10920

ggccaggatg cttgaccacc tacgccctgg cgacgttgtg acagtgacca ggctagaccg 10980

cctggcccgc agcacccgcg acctactgga cattgccgag cgcatccagg aggccggcgc 11040

gggcctgcgt agcctggcag agccgtgggc cgacaccacc acgccggccg gccgcatggt 11100

gttgaccgtg ttcgccggca ttgccgagtt cgagcgttcc ctaatcatcg accgcacccg 11160

gagcgggcgc gaggccgcca aggcgcgagg cgtgaagttt ggcccccgcc ctaccctcac 11220

cccggcacag atcgcgcacg cccgcgagct gatcgaccag gaaggccgca ccgtgaaaga 11280

ggcggctgca ctgcttggcg tgcatcgctc gaccctgtac cgcgcacttg agcgcagcga 11340

ggaagtgacg cccaccgagg ccaggcggcg cggtgccttc cgtgaggacg cattgaccga 11400

ggccgacgcc ctggcggccg ccgagaatga acgccaagag gaacaagcat gaaaccgcac 11460

caggacggcc aggacgaacc gtttttcatt accgaagaga tcgaggcgga gatgatcgcg 11520

gccgggtacg tgttcgagcc gcccgcgcac gtctcaaccg tgcggctgca tgaaatcctg 11580

gccggtttgt ctgatgccaa gctcgcggcc tggccggcga gcttggccgc tgaagaaacc 11640

gagcgccgcc gtctaaaaag gtgatgtgta tttgagtaaa acagcttgcg tcatgcggtc 11700

gctgcgtata tgatgcgatg agtaaataaa caaatacgca aggggaacgc atgaaggtta 11760

tcgctgtact taaccagaaa ggcgggtcag gcaagacgac catcgcaacc catctagccc 11820

gcgccctgca actcgccggg gccgatgttc tgttagtcga ttccgatccc cagggcagtg 11880

cccgcgattg ggcggccgtg cgggaagatc aaccgctaac cgttgtcggc atcgaccgcc 11940

cgacgattga ccgcgacgtg aaggccatcg gccggcgcga cttcgtagtg atcgacggag 12000

cgccccaggc ggcggacttg gctgtgtccg cgatcaaggc agccgacttc gtgctgattc 12060

cggtgcagcc aagcccttac gacatatggg ccaccgccga cctggtggag ctggttaagc 12120

agcgcattga ggtcacggat ggaaggctac aagcggcctt tgtcgtgtcg cgggcgatca 12180

aaggcacgcg catcggcggt gaggttgccg aggcgctggc cgggtacgag ctgcccattc 12240

ttgagtcccg tatcacgcag cgcgtgagct acccaggcac tgccgccgcc ggcacaaccg 12300

ttcttgaatc agaacccgag ggcgacgctg cccgcgaggt ccaggcgctg gccgctgaaa 12360

ttaaatcaaa actcatttga gttaatgagg taaagagaaa atgagcaaaa gcacaaacac 12420

gctaagtgcc ggccgtccga gcgcacgcag cagcaaggct gcaacgttgg ccagcctggc 12480

agacacgcca gccatgaagc gggtcaactt tcagttgccg gcggaggatc acaccaagct 12540

gaagatgtac gcggtacgcc aaggcaagac cattaccgag ctgctatctg aatacatcgc 12600

gcagctacca gagtaaatga gcaaatgaat aaatgagtag atgaatttta gcggctaaag 12660

gaggcggcat ggaaaatcaa gaacaaccag gcaccgacgc cgtggaatgc cccatgtgtg 12720

gaggaacggg cggttggcca ggcgtaagcg gctgggttgt ctgccggccc tgcaatggca 12780

ctggaacccc caagcccgag gaatcggcgt gagcggtcgc aaaccatccg gcccggtaca 12840

aatcggcgcg gcgctgggtg atgacctggt ggagaagttg aaggcggcgc aggccgccca 12900

gcggcaacgc atcgaggcag aagcacgccc cggtgaatcg tggcaagcgg ccgctgatcg 12960

aatccgcaaa gaatcccggc aaccgccggc agccggtgcg ccgtcgatta ggaagccgcc 13020

caagggcgac gagcaaccag attttttcgt tccgatgctc tatgacgtgg gcacccgcga 13080

tagtcgcagc atcatggacg tggccgtttt ccgtctgtcg aagcgtgacc gacgagctgg 13140

cgaggtgatc cgctacgagc ttccagacgg gcacgtagag gtttccgcag ggccggccgg 13200

catggcgagt gtgtgggatt acgacctggt actgatggcg gtttcccatc taaccgaatc 13260

catgaaccga taccgggaag ggaagggaga caagcccggc cgcgtgttcc gtccacacgt 13320

tgcggacgta ctcaagttct gccggcgagc cgatggcgga aagcagaaag acgacctggt 13380

agaaacctgc attcggttaa acaccacgca cgttgccatg cagcgtacga agaaggccaa 13440

gaacggccgc ctggtgacgg tatccgaggg tgaagccttg attagccgct acaagatcgt 13500

aaagagcgaa accgggcggc cggagtacat cgagatcgag ctagctgatt ggatgtaccg 13560

cgagatcaca gaaggcaaga acccggacgt gctgacggtt caccccgatt actttttgat 13620

cgatcccggc atcggccgtt ttctctaccg cctggcacgc cgcgccgcag gcaaggcaga 13680

agccagatgg ttgttcaaga cgatctacga acgcagtggc agcgccggag agttcaagaa 13740

gttctgtttc accgtgcgca agctgatcgg gtcaaatgac ctgccggagt acgatttgaa 13800

ggaggaggcg gggcaggctg gcccgatcct agtcatgcgc taccgcaacc tgatcgaggg 13860

cgaagcatcc gccggttcct aatgtacgga gcagatgcta gggcaaattg ccctagcagg 13920

ggaaaaaggt cgaaaaggtc tctttcctgt ggatagcacg tacattggga acccaaagcc 13980

gtacattggg aaccggaacc cgtacattgg gaacccaaag ccgtacattg ggaaccggtc 14040

acacatgtaa gtgactgata taaaagagaa aaaaggcgat ttttccgcct aaaactcttt 14100

aaaacttatt aaaactctta aaacccgcct ggcctgtgca taactgtctg gccagcgcac 14160

agccgaagag ctgcaaaaag cgcctaccct tcggtcgctg cgctccctac gccccgccgc 14220

ttcgcgtcgg cctatcgcgg ccgctggccg ctcaaaaatg gctggcctac ggccaggcaa 14280

tctaccaggg cgcggacaag ccgcgccgtc gccactcgac cgccggcgcc cacatcaagg 14340

caccggtggg tatgcctgac gatgcgtgga gaccgaaacc ttgcgctcgt tcgccagcca 14400

ggacagaaat gcctcgactt cgctgctgcc caaggttgcc gggtgacgca caccgtggaa 14460

acggatgaag gcacgaaccc agtggacata agcctgttcg gttcgtaagc tgtaatgcaa 14520

gtagcgtatg cgctcacgca actggtccag aaccttgacc gaacgcagcg gtggtaacgg 14580

cgcagtggcg gttttcatgg cttgttatga ctgttttttt ggggtacagt ctatgcctcg 14640

ggcatccaag cagcaagcgc gttacgccgt gggtcgatgt ttgatgttat ggagcagcaa 14700

cgatgttacg cagcagggca gtcgccctaa aacaaagtta aacatcatga gggaagcggt 14760

gatcgccgaa gtatcgactc aactatcaga ggtagttggc gtcatcgagc gccatctcga 14820

accgacgttg ctggccgtac atttgtacgg ctccgcagtg gatggcggcc tgaagccaca 14880

cagtgatatt gatttgctgg ttacggtgac cgtaaggctt gatgaaacaa cgcggcgagc 14940

tttgatcaac gaccttttgg aaacttcggc ttcccctgga gagagcgaga ttctccgcgc 15000

tgtagaagtc accattgttg tgcacgacga catcattccg tggcgttatc cagctaagcg 15060

cgaactgcaa tttggagaat ggcagcgcaa tgacattctt gcaggtatct tcgagccagc 15120

cacgatcgac attgatctgg ctatcttgct gacaaaagca agagaacata gcgttgcctt 15180

ggtaggtcca gcggcggagg aactctttga tccggttcct gaacaggatc tatttgaggc 15240

gctaaatgaa accttaacgc tatggaactc gccgcccgac tgggctggcg atgagcgaaa 15300

tgtagtgctt acgttgtccc gcatttggta cagcgcagta accggcaaaa tcgcgccgaa 15360

ggatgtcgct gccgactggg caatggagcg cctgccggcc cagtatcagc ccgtcatact 15420

tgaagctaga caggcttatc ttggacaaga agaagatcgc ttggcctcgc gcgcagatca 15480

gttggaagaa tttgtccact acgtgaaagg cgagatcacc aaggtagtcg gcaaataatg 15540

tctaacaatt cgttcaagcc gacgccgctt cgcggcgcgg cttaactcaa gcgttagatg 15600

cactaagcac ataattgctc acagccaaac tatcaggtca agtctgcttt tattattttt 15660

aagcgtgcat aataagccct acacaaattg ggagatatat catgaaaggc tggctttttc 15720

ttgttatcgc aatagttggc gaagtaatcg caacatagct tgcttggtcg ttccgcgtga 15780

acgtcggctc gattgtacct gcgttcaaat actttgcgat cgtgttgcgc gcctgcccgg 15840

tgcgtcggct gatctcacgg atcgactgct tctctcgcaa cgccatccga cggatgatgt 15900

ttaaaagtcc catgtggatc actccgttgc cccgtcgctc accgtgttgg ggggaaggtg 15960

cacatggctc agttctcaat ggaaattatc tgcctaaccg gctcagttct gcgtagaaac 16020

caacatgcaa gctccaccgg gtgcaaagcg gcagcggcgg caggatatat tcaattgtaa 16080

atggcttcat gtccgggaaa tctacatgga tcagcaatga gtatgatggt caatatggag 16140

aaaaagaaag agtaattacc aatttttttt caattcaaaa atgtagatgt ccgcagcgtt 16200

attataaaat gaaagtacat tttgataaaa cgacaaatta cgatccgtcg tatttatagg 16260

cgaaagcaat aaacaaatta ttctaattcg gaaatcttta tttcgacgtg tctacattca 16320

cgtccaaatg ggggcttaga tgagaaactt cacgatcggc tctagttcga gcgatctagt 16380

aacatagatg acaccgcgcg cgataattta tcctagtttg cgcgctatat tttgttttct 16440

atcgcgtatt aaatgtataa ttgcgggact ctaatcataa aaacccatct cataaataac 16500

gtcatgcatt acatgttaat tattacatgc ttaacgtaat tcaacagaaa ttatatgata 16560

atcatcgcaa gaccggcaac aggattcaat cttaagaaac tttattgcca aatgtttgaa 16620

cgatcgggga aattcgtttc tatgcttcaa ttctgctcta tagatgttct tctagtctta 16680

acttttagaa ggcacaacat ttgaaatgtt ggtaattttt ctgctgggta gtgaaccatt 16740

gtaatttgtg tttgcttata ttatccgttg ggtttctcta tgttatctgt ggggccatat 16800

cccagaactg gttgagtcgg tccaacacct gggtgccaat catgtcgatg gtggggtatg 16860

gt 16862

<210> 24
<211> 2001
<212> DNA
<213> Artificial Sequence

<220>
<223> Repair-Template ALS G/T

<400> 24
ggtaggcctg gacctgtttt gattgatctt cctaaagata ttcagcagca attggttgtt 60

cctgattggg ataggccttt taagttgggt gggtatatgt ctaggctgcc aaagtccaag 120

ttttcgacga atgaggttgg acttcttgag cagattgtga ggttgatgag tgagtcgaag 180

aagcctgtct tgtatgtggg aggtgggtgt ttgaattcta gtgaggagtt gaggagattt 240

gttgagttga cagggattcc ggtggctagt actttgatgg ggttggggtc ttacccttgt 300

aatgatgaac tgtctcttca tatgttgggg atgcacggga ctgtttatgc caattatgcg 360

gtggataagg cggatttgtt gcttgctttc ggggttaggt ttgatgatcg tgtgaccggg 420

aagctcgagg cgtttgctag ccgtgctaag attgtgcata ttgatattga ctctgctgag 480

attgggaaga acaagcagcc ccatgtgtcc atttgtgctg atgttaaatt ggcattgcgg 540

ggtatgaata agattctgga gtctagaata gggaagctga atttggattt ctccaagtgg 600

agagaagaat taggtgagca gaagaaggaa ttcccactga gttttaagac atttggggat 660

gcaattcctc cacaatatgc cattcaggtg cttgatgagt tgaccaatgg taatgctatt 720

ataagtactg gtgttgggca gcaccaaatg tgggctgcgc agcattacaa gtacagaaac 780

cctcgccaat ggctgacctc tggtgggttg ggggctatgg ggtttgggct accagccgcc 840

attggagctg cagttgctcg accagatgca gtggttgtcg atattgatgg ggatggcagt 900

tttattatga atgttcaaga gttggctaca attagggtgg aaaatctccc agttaagata 960

atgctgctaa acaatcaaca tttaggtatg gttgtccaat tggaagatag gttctataaa 1020

gctaaccggg cacatacata ccttggaaac ccttccaaat ctgctgatat cttccctgat 1080

atgctcaaat tcgctgaggc atgtgatatt ccttctgccc gtgttagcaa cgtggctgat 1140

ttgagggccg ccattcaaac aatgttggat actccagggc cgtacctgct cgatgtgatt 1200

gtaccgcatc aagagcatgt gttgcctatg attccaagtg gtgccggttt caaggatacc 1260

attacagagg gtgatggaag aacctcttat tgatcggttt aatgacggtt ggaaccattt 1320

aaagagggta agctatatta ctgtatgtat attagtatgt tcctggataa tttagaagct 1380

tttgtctgtt gtcttttgca gtttatgaag ttagtttgct gttgtcatgt tacttgttac 1440

tttaaaaagc tttttgtagt ttttgagcaa ctagtatgga atgctcttcc tgtattgctt 1500

ggaaaattca caaaagtggt ttttcggcta tggatgttgt gttgcatcat gcatatatag 1560

cttgatatac tagttggctt ggtgcatctt taacatatac taatgagact acgacagcaa 1620

ttgccaatta gttggcttga cataattctt agtctgccac tagaaatctt gcttcttttt 1680

tttccctcat ttgttgaaag tccctgttgc gacctgacat gggagcattg gagattgttt 1740

agcaatagca gttgcagtta ggtgactaca gtcatcttcc aaatatgaat actctctgga 1800

ggggaggagg ttttacaaaa tatgagtttt taacacatga gaaacttata ttaaacaagg 1860

ttgagtcacc ccatattttt caaagttgac ttttgtctga tttgggtgac tatgcctgtt 1920

gtatgcaata aactcgatgt acataagact tgtataatcc aatctaaccc ttcctggctg 1980

attatgaaac cgagtcggct a 2001

<210> 25
<211> 27
<212> DNA
<213> Artificial Sequence

<220>
<223> 5'ALS_G/T primer

<400> 25
gttttggatg tagaggatat tcctaga 27

<210> 26
<211> 23
<212> DNA
<213> Artificial Sequence

<220>
<223>  ALS_G/T_Rv primer

<400>  26
cagggaagat atcagcagat ttg                                              23


<210>  27
<211>  23
<212>  DNA
<213>  Artificial Sequence

<220>
<223>  ALS_G/T_Fw primer

<400>  27
ctacaattag ggtggaaaat ctc                                              23


<210>  28
<211>  21
<212>  DNA
<213>  Artificial Sequence

<220>
<223>  3'ALS_G/T primer

<400>  28
ctctagtggt cacctggcat c                                                21


<210>  29
<211>  20
<212>  DNA
<213>  Artificial Sequence

<220>
<223>  pSeq_LbCpf1_F4 primer

<400>  29
accactcact cctcgataag                                                  20


<210>  30
<211>  22
<212>  DNA
<213>  Artificial Sequence

<220>
<223>  pSeq_LbCpf1_R3 primer

<400>  30
tagacctgct tctcaacctt ca                                               22


<210>  31
<211>  21
<212>  DNA
<213>  Artificial Sequence

<220>
<223>  pSeq_Ribozyme_F primer

<400>  31
tgcagcggat ccaaattact g                                                21


<210>  32
<211>  18
<212>  DNA
<213>  Artificial Sequence

<220>
<223>  pSeq_Ribozyme_R primer

<400>  32
cctggtccca ttcgccat                                                    18


<210>  33
<211>  20
<212>  DNA
<213>  Artificial Sequence

<220>
<223>  pSeq_tDT_F primer

<400>  33
ttacaagaag ctgtccttcc                                                  20


<210>  34
<211>  20
<212>  DNA
<213>  Artificial Sequence

<220>
<223>  primer

<400>  34
gtactgttcc acgatggtgt                                                  20


<210>  35
<211>  2010
<212>  DNA
<213>  Artificial Sequence

<220>
<223>  Repair-Template ALS 9bp Insertion

<400>  35
   gaatttggat ttctccaagt ggagagaaga attaggtgag cagaagaagg aattcccact      60
   gagttttaag acatttgggg atgcaattcc tccacaatat gccattcagg tgcttgatga     120
   gttgaccaat ggtaatgcta ttataagtac tggtgttggg cagcaccaaa tgtgggctgc     180
   gcagcattac aagtacagaa accctcgcca atggctgacc tctggtgggt tgggggctat     240
   ggggtttggg ctaccagccg ccattggagc tgcagttgct cgaccagatg cagtggttgt     300
   cgatattgat ggggatggca gttttattat gaatgttcaa gagttggcta caattagggt     360
   ggaaaatctc ccagttaaga taatgctgct aaacaatcaa catttaggta tggttgtcca     420
   atgggaagat aggttctata aagctaaccg ggcacataca taccttggaa acccttccaa 480
   atctgctgat atcttccctg atatgctcaa attcgctgag gcatgtgata ttccttctgc 540
   ccgtgttagc aacgtggctg atttgagggc cgccattcaa acaatgttgg atactccagg 600
   gccgtacctg ctcgatgtga ttgtaccgca tcaagagcat gtgttgccta tgattccaag 660
   tggtgccggt ttcaaggata ccattacaga gggtgatgga agaacctctt attgatcggt 720
   ttaatgacgg ttggaaccat ttaaagaggg taagctatat tactgtatgt atattagtat 780
   gttcctggat aatttagaag cttttgtctg ttgtcttttg cagtttatga agttagtttg 840
   ctgttgtcat gttacttgtt actttaaaaa gctttttgta gtttttgagc aactagtatg 900
   gaatgctctt cctgtattgc ttggaaaatt cacaaaagtg gtttttcggc tatggatgtt 960
   gtgttgcatc atgcatatat agcttgatat actagttggc tgcactatcg tggtgcatct 1020
   ttaacatata ctaatgagac tacgacagca attgccaatt agttggcttg acataattct 1080
   tagtctgcca ctagaaatct tgcttctttt ttttccctca tttgttgaaa gtccctgttg 1140
   cgacctgaca tgggagcatt ggagattgtt tagcaatagc agttgcagtt aggtgactac 1200
   agtcatcttc caaatatgaa tactctctgg aggggaggag gttttacaaa atatgagttt 1260
   ttaacacatg agaaacttat attaaacaag gttgagtcac cccatatttt tcaaagttga 1320
   cttttgtctg atttgggtga ctatgcctgt tgtatgcaat aaactcgatg tacataagac 1380
   ttgtataatc caatctaacc cttcctggct gattatgaaa ccgagtcggc taatttgttg 1440
   cttgatcttc atgtgtgagc ctgatgccag gtgaccacta gaggagtacc tttcattgag 1500
   atatcgattc ggttaattgg ttcctcatat gggtctcaaa actgaatttt tctcaggctc 1560
   tcttctaacc agttgttgaa ttttatgaaa cttcagtcca gttaaaactt tgatcccgga 1620
   tcagaatttc tctgagttgt tcttccgcct ctcagttcca ctgttccagt gttcttggtc 1680
   ctcaacctgt actctgagct atttctgcaa acacctgaag ttcctgctgc ccttggacaa 1740
   atacagaatg cagcattagc atttatttaa gacaagatga atacttgctc ccctgttagt 1800
   tatgctaatt ctgcattagt attctttaat tttaattaga tactgcaatc cgtgaactct 1860
   gcagtttctt gatctctctc tgtttcaatt tctcctatct tatcgcccat ttctttaggc 1920
   tttcgttatc tatcgtctaa ttcaagagta ggtaactagg tacgggataa aactttttca 1980
   tgaagaacct atgttcccgt attatgctgt 2010

<210> 36
<211> 24
<212> DNA
<213> Artificial Sequence

<220>
<223> 5'ALS_Insertion primer

<400>  36
gtgctgatgt taaattggca ttgc                                              24


<210>  37
<211>  23
<212>  DNA
<213>  Artificial Sequence

<220>
<223>  ALS_Insertion_Rv primer

<400>  37
ctagtggcag actaagaatt atg                                               23


<210>  38
<211>  23
<212>  DNA
<213>  Artificial Sequence

<220>
<223>  ALS_Insertion_Fw primer

<400>  38
gaatgctctt cctgtattgc ttg                                               23


<210>  39
<211>  24
<212>  DNA
<213>  Artificial Sequence

<220>
<223>  3'ALS_Insertion primer

<400>  39
cagttcaaca caaaagaagt tgtc                                              24


<210>  40
<211>  2000
<212>  DNA
<213>  Artificial Sequence

<220>
<223>  Repair-Template CYP Deletionsmarker

<400>  40
tagagttttt cgatttacga ttgtggtaaa ttaattgtaa ttgatcggtt gtcttgtagt      60

caagaaatga cgtatgaatc aatttagggc atgttctctt cggcataaaa cagctgaact     120

gaattgaact gaactgaaat gaatagtgat atgtgagagt aaaagtattg tcaagagctg     180

aactgagctg aactgaacgg atctgaactg aactgatcta atctgaacta atctgaactg     240

aactgaactg aattgaactg aaaataagct agggaaaaca gacccttact actattatat     300

aacctcgttt aaatattagg aaattaaaaa aataattata tttctttata ctttattaac     360

ctattgaggt ttttatattg actcccaaat actattttat agatcatgcc atgttaatga     420

gcaaactact ttctcacatc tttataggag aaaaagtaga tcactcacta gcatatcatg 480

accagcgaaa ccaaccaacg ctaatagttt tattgcttcc attagagata agagttaact 540

aataatacca tctttgtgaa attttgatgg attttggaac tttagcaata tatttactgt 600

gtgcactttt tgctaccaaa attttatacc aatggctcaa gtcctactta tacacaacat 660

acaaactccc tcccggccca ccaaggtggc ccttatttgg aaacctcctt caactagggc 720

cacttcccca ccgcgatttc gcctcatttt gtgaaaaata tgggccttta gtctacataa 780

ggcttggtaa tgtggatgcc ataaccacta atgatccaga aatcatacgt gagatcttag 840

ttcgacaaga tgatgtattt gcgtctcgtc cacatacctt agccgcaacc cacttggctt 900

acaatagtgg tgatgtggcc ttggctccaa tgggaccaaa atggaaaaga atgagaagga 960

tatgcatgga gcacttgctc acaactagac gacttgaact tcatagggct gatgaggcac 1020

gacatttggt ccaagacgta ttaactcgtt cccacaaaga taaagttgtt aatttgaggg 1080

aagtgttagg tgcattttct atgaataacg tgactagaat gttgctaggg aagcaatact 1140

ttggggccgg gacggcgggc ccacaagagg ctctagagtt tatgcatata acacatgagt 1200

tgttttggtt actaggcttg atttacttgg gtgattattt gcctttttgg aggtgggttg 1260

atccatatgg atgtgaaaag aaaatgaggg aagttgaaaa aagggtagat gatttccatc 1320

gcaaaattat agaggaacat aggaaggaga agaaaaggaa agaagaaatg ggagtgaatg 1380

agggtgaaat ggattttgta gatattttgt tggctttgcc tggtgaaaat ggaaatgagc 1440

atatggatga tgcagatatt aaagctctaa ttcaggtaat tcatgtataa tttgaatgtg 1500

atcgatacaa agtttgatag aaaacatatt tgcataaata tatggttgcc ctactagacc 1560

caataaaata cataattatt gccttactag ttgaaagttg aaacaaccta gctaccattt 1620

tgttgtgatt atcattagcc aaccaaaatt atttcttgca tccatatatt aatgttgaga 1680

tcagagtcgg catatttaca attacttgta acattttaag caaacaaatt aaaatatttt 1740

ttggcaagtc cattttattg aataatacct atatcttaaa atgaattctt ggtcatgtac 1800

acttgccttt caaggtacca atatttgacc atatgtaatt actattaaca aatttgataa 1860

aatctaataa tatgtaaata tacattcacg cacatattag aaacaaagat cacaaatgat 1920

aatgcaaaat aacttatttg aactaatgtt gtgaagttaa atttggaaca aaaggtatat 1980

ttgtattgcc gaattttaat 2000

<210> 41
<211> 28
<212> DNA
<213> Artificial Sequence

<220>
<223> 5'CYP_Deletion primer

<400>  41
gtctttacat agcaaaacaa tattgaag                                          28


<210>  42
<211>  25
<212>  DNA
<213>  Artificial Sequence

<220>
<223>  CYP_Deletion_Rv primer

<400>  42
ctaacacttc cctcaaatta acaac                                             25


<210>  43
<211>  23
<212>  DNA
<213>  Artificial Sequence

<220>
<223>  CYP_Deletion_Fw primer

<400>  43
caatagtggt gatgtggcct tgg                                               23


<210>  44
<211>  25
<212>  DNA
<213>  Artificial Sequence

<220>
<223>  3'CYP_Deletion primer

<400>  44
ggtaactagt aaaagtatac tcatc                                             25


<210>  45
<211>  24
<212>  DNA
<213>  Artificial Sequence

<220>
<223>  BvCYP703A2_WT_fwd primer

<400>  45
tagacgactt gaactatttg tgag                                              24


<210>  46
<211>  24
<212>  DNA
<213>  Artificial Sequence

<220>
<223>  BvCYP703A2_gst_fwd primer

<400>  46
tagacgactt gaacttcata gggc                                              24

<210>  47
<211>  23
<212>  DNA
<213>  Artificial Sequence

<220>
<223>  BvCYP703A2_rev primer

<400>  47
aaagtattgc ttccctagca aca                                              23


<210>  48
<211>  21
<212>  DNA
<213>  Artificial Sequence

<220>
<223>  BvALS_WT_fwd primer

<400>  48
actagttggc ttggtgcatc t                                                21


<210>  49
<211>  23
<212>  DNA
<213>  Artificial Sequence

<220>
<223>  BvALS_SU_res_fwd primer

<400>  49
actagttggc tgcactatcg tgc                                              23


<210>  50
<211>  20
<212>  DNA
<213>  Artificial Sequence

<220>
<223>  BvALS_rev primer

<400>  50
ccaatgctcc catgtcaggt                                                  20


<210>  51
<211>  3759
<212>  DNA
<213>  Lachnospiraceae bacterium

<400>  51
atggctccta agaagaagag gaaggttagc aagctcgaga agtttaccaa ctgctacagc      60

ctctctaaga ccctcaggtt caaggctatc cctgtgggaa agacccaaga gaatatcgac     120

aacaagaggc tcctcgtcga ggatgagaag agagctgaag attacaaggg cgtgaagaag     180

ctcctcgaca ggtactacct cagcttcatc aacgatgtgc tccacagcat caagctcaag     240

aacctcaaca actacatcag cctcttccgt aagaaaacca ggaccgagaa agagaacaaa 300

gagcttgaga acctcgagat caacctccgt aaagagatcg ccaaggcttt caagggaaac 360

gagggataca agagcctctt caagaaggat attatcgaga caatcctgcc tgagttcctg 420

gacgataagg atgagatcgc tctcgtgaac agcttcaacg gattcactac tgccttcacc 480

ggattcttcg acaacaggga aaacatgttc agcgaagagg ccaagagcac ctctatcgct 540

ttcagatgca tcaacgagaa cctcacgcgt tacatcagca acatggacat cttcgagaag 600

gtggacgcca tcttcgataa gcacgaggtg caagaaatca aagagaagat cctcaacagc 660

gactacgacg tcgaggactt ttttgaaggg gagttcttca acttcgttct cacccaagag 720

ggcatcgacg tgtacaacgc tattatcgga ggattcgtga ccgagtctgg ggagaagatt 780

aagggactca acgagtacat caacctgtac aaccagaaaa cgaagcagaa gctcccgaag 840

ttcaagccgc tctacaagca ggttctctct gatcgtgaga gcctctcatt ttacggtgag 900

ggttacacct ctgacgagga agtgcttgag gttttccgta acaccctcaa caagaacagc 960

gagatcttct cgtccatcaa gaagttggag aaacttttca agaacttcga cgagtacagc 1020

agcgctggga tcttcgttaa gaacggacct gctatcagca ccatcagcaa ggatattttc 1080

ggcgagtgga acgtgatcag ggacaagtgg aatgctgagt acgatgacat ccacctcaag 1140

aagaaggctg tcgtcactga gaagtacgag gatgacaggc gtaagtcgtt caagaagatc 1200

ggctctttca gcctcgagca gcttcaagaa tacgctgatg ctgatctcag cgtggtcgag 1260

aagctcaaag agatcatcat ccagaaggtc gacgagatct acaaggtgta cgggtcctct 1320

gagaagttgt tcgatgctga tttcgtcctc gagaagagtc tgaagaagaa cgacgctgtc 1380

gtcgcgatca tgaaggattt gctcgacagc gtgaagtcct tcgagaacta tatcaaggcc 1440

ttcttcggag agggcaaaga gactaatagg gacgagtctt tctacgggga tttcgtgctc 1500

gcttacgata tcctcctcaa ggtggaccat atctacgacg ccatcagaaa ctacgtgacc 1560

cagaagcctt acagcaagga caagttcaag ttgtactttc agaacccgca gttcatgggc 1620

ggatgggaca aagacaaaga gacagattac agggccacca tcctcaggta cgggtctaag 1680

tactacctgg ccatcatgga caagaaatac gccaagtgcc tccaaaagat cgacaaggat 1740

gacgtgaacg ggaactatga gaagatcaac tacaagctcc ttccgggacc gaacaagatg 1800

cttcctaagg tgttcttcag caagaaatgg atggcctact acaacccgtc tgaggacatc 1860

cagaaaatct acaagaacgg gaccttcaag aaaggcgaca tgttcaacct caacgactgc 1920

cacaagctca tcgatttctt caaggacagc atctcgcgtt acccgaagtg gtctaacgct 1980

tacgacttta acttcagcga gacagaaaag tacaaggata tcgccgggtt ctaccgtgag 2040

gttgaggaac agggttacaa ggttagcttc gagagcgcct ccaagaaaga ggttgacaag 2100

ttggtcgaag agggcaagct ctacatgttc cagatctata acaaggactt ctccgacaag 2160

agccacggaa ctcctaacct ccatacgatg tacttcaagc tgcttttcga cgagaacaac 2220

cacgggcaga tcagactttc tggtggtgct gaactcttca tgcgtagggc ctcactcaag 2280

aaagaagagt tggttgttca cccggccaac tctccaatcg ctaacaagaa tcctgacaac 2340

ccgaaaaaga ccaccacgct gtcttacgac gtctacaagg acaaaaggtt cagcgaggac 2400

cagtacgagc ttcatatccc gatcgctatc aacaagtgcc cgaagaacat cttcaagatc 2460

aataccgagg tgagggtgct gctcaagcac gatgataacc cttacgtgat cggaatcgat 2520

cgtggtgaga gaaacctcct ctacatcgtt gtggtggacg gaaagggaaa catcgtcgag 2580

cagtacagcc tgaacgagat tatcaacaat ttcaacggca tcaggatcaa gaccgactac 2640

cactcactcc tcgataagaa agaaaaagag cgtttcgagg ccaggcagaa ctggacttct 2700

atcgaaaaca tcaaagagtt gaaggccggc tacatctctc aggtggtgca taagatctgc 2760

gagctggtgg aaaagtacga tgctgtgatc gctcttgagg acctcaactc tgggttcaag 2820

aacagtagag tgaaggttga gaagcaggtc taccaaaagt tcgagaagat gctcatcgac 2880

aagctcaact acatggtgga caaaaagagc aacccttgcg ctaccggtgg tgctcttaag 2940

ggataccaga tcacgaacaa gttcgagtcc ttcaagagca tgagcaccca gaacggcttc 3000

atcttctata tccctgcttg gctcaccagc aagatcgatc cttctactgg tttcgtgaac 3060

ctgctcaaga ccaagtacac ctcgatcgcc gacagcaaga agttcatctc gtctttcgac 3120

aggatcatgt acgtgccgga agaggatctt ttcgagttcg ctctcgacta taagaacttc 3180

agcaggaccg acgccgacta cattaagaag tggaagctct actcctacgg gaaccgtatc 3240

aggatcttcc gaaatccgaa gaaaaacaac gtgttcgact gggaagaagt gtgcctcacc 3300

tctgcctaca aagaactgtt caacaagtac ggcatcaact accagcaggg tgatatcagg 3360

gctcttttgt gcgagcagag cgacaaggca ttctacagct cattcatggc cctcatgtct 3420

ctcatgctcc agatgaggaa ctctatcacc ggaaggaccg atgtggactt ccttatctct 3480

ccggtcaaga actctgacgg gatcttctac gacagccgta actatgaggc tcaagagaac 3540

gctatcctgc cgaagaatgc tgatgcaaac ggggcttaca acattgcgag aaaggttctc 3600

tgggctatcg ggcagtttaa gaaagcggaa gatgagaagc tcgacaaggt gaagatcgcc 3660

atctccaaca aagagtggct tgagtacgct cagacctccg ttaagcacaa gaggcctgct 3720

gctactaaga aagctggcca ggccaaaaag aagaagtga 3759

<210> 52
<211> 5228
<212> DNA
<213> Artificial Sequence

<220>
<223> PcUbi:Cpf1:TPea expression cassette

<400> 52
aaaaattacg gatatgaata taggcatatc cgtatccgaa ttatccgttt gacagctagc 60
aacgattgta caattgcttc tttaaaaaag gaagaaagaa agaaagaaaa gaatcaacat 120
cagcgttaac aaacggcccc gttacggccc aaacggtcat atagagtaac ggcgttaagc 180
gttgaaagac tcctatcgaa atacgtaacc gcaaacgtgt catagtcaga tcccctcttc 240
cttcaccgcc tcaaacacaa aaataatctt ctacagccta tatatacaac ccccccttct 300
atctctcctt tctcacaatt catcatcttt ctttctctac ccccaatttt aagaaatcct 360
ctcttctcct cttcattttc aaggtaaatc tctctctctc tctctctctc tgttattcct 420
tgttttaatt aggtatgtat tattgctagt ttgttaatct gcttatctta tgtatgcctt 480
atgtgaatat ctttatcttg ttcatctcat ccgtttagaa gctataaatt tgttgatttg 540
actgtgtatc tacacgtggt tatgtttata tctaatcaga tatgaatttc ttcatattgt 600
tgcgtttgtg tgtaccaatc cgaaatcgtt gatttttttc atttaatcgt gtagctaatt 660
gtacgtatac atatggatct acgtatcaat tgttcatctg tttgtgtttg tatgtataca 720
gatctgaaaa catcacttct ctcatctgat tgtgttgtta catacataga tatagatctg 780
ttatatcatt ttttttatta attgtgtata tatatatgtg catagatctg gattacatga 840
ttgtgattat ttacatgatt ttgttattta cgtatgtata tatgtagatc tggacttttt 900
ggagttgttg acttgattgt atttgtgtgt gtatatgtgt gttctgatct tgatatgtta 960
tgtatgtgca gcgaattcgg cgcgccatgg ctcctaagaa gaagaggaag gttagcaagc 1020
tcgagaagtt taccaactgc tacagcctct ctaagaccct caggttcaag gctatccctg 1080
tgggaaagac ccaagagaat atcgacaaca agaggctcct cgtcgaggat gagaagagag 1140
ctgaagatta caagggcgtg aagaagctcc tcgacaggta ctacctcagc ttcatcaacg 1200
atgtgctcca cagcatcaag ctcaagaacc tcaacaacta catcagcctc ttccgtaaga 1260
aaaccaggac cgagaaagag aacaaagagc ttgagaacct cgagatcaac ctccgtaaag 1320
agatcgccaa ggctttcaag ggaaacgagg gatacaagag cctcttcaag aaggatatta 1380
tcgagacaat cctgcctgag ttcctggacg ataaggatga gatcgctctc gtgaacagct 1440
tcaacggatt cactactgcc ttcaccggat tcttcgacaa cagggaaaac atgttcagcg 1500
aagaggccaa gagcacctct atcgctttca gatgcatcaa cgagaacctc acgcgttaca 1560
tcagcaacat ggacatcttc gagaaggtgg acgccatctt cgataagcac gaggtgcaag 1620
aaatcaaaga gaagatcctc aacagcgact acgacgtcga ggactttttt gaaggggagt 1680
tcttcaactt cgttctcacc caagagggca tcgacgtgta caacgctatt atcggaggat 1740
tcgtgaccga gtctggggag aagattaagg gactcaacga gtacatcaac ctgtacaacc 1800
agaaaacgaa gcagaagctc ccgaagttca agccgctcta caagcaggtt ctctctgatc 1860

gtgagagcct ctcattttac ggtgagggtt acacctctga cgaggaagtg cttgaggttt 1920

tccgtaacac cctcaacaag aacagcgaga tcttctcgtc catcaagaag ttggagaaac 1980

ttttcaagaa cttcgacgag tacagcagcg ctgggatctt cgttaagaac ggacctgcta 2040

tcagcaccat cagcaaggat attttcggcg agtggaacgt gatcagggac aagtggaatg 2100

ctgagtacga tgacatccac ctcaagaaga aggctgtcgt cactgagaag tacgaggatg 2160

acaggcgtaa gtcgttcaag aagatcggct ctttcagcct cgagcagctt caagaatacg 2220

ctgatgctga tctcagcgtg gtcgagaagc tcaaagagat catcatccag aaggtcgacg 2280

agatctacaa ggtgtacggg tcctctgaga agttgttcga tgctgatttc gtcctcgaga 2340

agagtctgaa gaagaacgac gctgtcgtcg cgatcatgaa ggatttgctc gacagcgtga 2400

agtccttcga gaactatatc aaggccttct tcggagaggg caaagagact aatagggacg 2460

agtctttcta cggggatttc gtgctcgctt acgatatcct cctcaaggtg gaccatatct 2520

acgacgccat cagaaactac gtgacccaga agccttacag caaggacaag ttcaagttgt 2580

actttcagaa cccgcagttc atgggcggat gggacaaaga caaagagaca gattacaggg 2640

ccaccatcct caggtacggg tctaagtact acctggccat catggacaag aaatacgcca 2700

agtgcctcca aaagatcgac aaggatgacg tgaacgggaa ctatgagaag atcaactaca 2760

agctccttcc gggaccgaac aagatgcttc ctaaggtgtt cttcagcaag aaatggatgg 2820

cctactacaa cccgtctgag gacatccaga aaatctacaa gaacgggacc ttcaagaaag 2880

gcgacatgtt caacctcaac gactgccaca agctcatcga tttcttcaag gacagcatct 2940

cgcgttaccc gaagtggtct aacgcttacg actttaactt cagcgagaca gaaaagtaca 3000

aggatatcgc cgggttctac cgtgaggttg aggaacaggg ttacaaggtt agcttcgaga 3060

gcgcctccaa gaaagaggtt gacaagttgg tcgaagaggg caagctctac atgttccaga 3120

tctataacaa ggacttctcc gacaagagcc acggaactcc taacctccat acgatgtact 3180

tcaagctgct tttcgacgag aacaaccacg ggcagatcag actttctggt ggtgctgaac 3240

tcttcatgcg tagggcctca ctcaagaaag aagagttggt tgttcacccg gccaactctc 3300

caatcgctaa caagaatcct gacaacccga aaaagaccac cacgctgtct tacgacgtct 3360

acaaggacaa aaggttcagc gaggaccagt acgagcttca tatcccgatc gctatcaaca 3420

agtgcccgaa gaacatcttc aagatcaata ccgaggtgag ggtgctgctc aagcacgatg 3480

ataaccctta cgtgatcgga atcgatcgtg gtgagagaaa cctcctctac atcgttgtgg 3540

tggacggaaa gggaaacatc gtcgagcagt acagcctgaa cgagattatc aacaatttca 3600

acggcatcag gatcaagacc gactaccact cactcctcga taagaaagaa aaagagcgtt 3660

tcgaggccag gcagaactgg acttctatcg aaaacatcaa agagttgaag gccggctaca 3720

tctctcaggt ggtgcataag atctgcgagc tggtggaaaa gtacgatgct gtgatcgctc 3780

ttgaggacct caactctggg ttcaagaaca gtagagtgaa ggttgagaag caggtctacc 3840

aaaagttcga gaagatgctc atcgacaagc tcaactacat ggtggacaaa aagagcaacc 3900

cttgcgctac cggtggtgct cttaagggat accagatcac gaacaagttc gagtccttca 3960

agagcatgag cacccagaac ggcttcatct tctatatccc tgcttggctc accagcaaga 4020

tcgatccttc tactggtttc gtgaacctgc tcaagaccaa gtacacctcg atcgccgaca 4080

gcaagaagtt catctcgtct ttcgacagga tcatgtacgt gccggaagag gatcttttcg 4140

agttcgctct cgactataag aacttcagca ggaccgacgc cgactacatt aagaagtgga 4200

agctctactc ctacggaaac cgtatcagga tcttccgaaa tccgaagaaa aacaacgtgt 4260

tcgactggga agaagtgtgc ctcacctctg cctacaaaga actgttcaac aagtacggca 4320

tcaactacca gcagggtgat atcagggctc ttttgtgcga gcagagcgac aaggcattct 4380

acagctcatt catggccctc atgtctctca tgctccagat gaggaactct atcaccggaa 4440

ggaccgatgt ggacttcctt atctctccgg tcaagaactc tgacgggatc ttctacgaca 4500

gccgtaacta tgaggctcaa gagaacgcta tcctgccgaa gaatgctgat gcaaacgggg 4560

cttacaacat tgcgagaaag gttctctggg ctatcgggca gtttaagaaa gcggaagatg 4620

agaagctcga caaggtgaag atcgccatct ccaacaaaga gtggcttgag tacgctcaga 4680

cctccgttaa gcacaagagg cctgctgcta ctaagaaagc tggccaggcc aaaaagaaga 4740

agtgaggcgc gccgagctcc aggcctccca gctttcgtcc gtatcatcgg tttcgacaac 4800

gttcgtcaag ttcaatgcat cagtttcatt gcccacacac cagaatccta ctaagtttga 4860

gtattatggc attggaaaag ctgttttctt ctatcatttg ttctgcttgt aatttactgt 4920

gttctttcag tttttgtttt cggacatcaa aatgcaaatg gatggataag agttaataaa 4980

tgatatggtc cttttgttca ttctcaaatt attattatct gttgttttta ctttaatggg 5040

ttgaatttaa gtaagaaagg aactaacagt gtgatattaa ggtgcaatgt tagacatata 5100

aaacagtctt tcacctctct ttggttatgt cttgaattgg tttgtttctt cacttatctg 5160

tgtaatcaag tttactatga gtctatgatc aagtaattat gcaatcaagt taagtacagt 5220

ataggctt 5228

<210> 53
<211> 37
<212> DNA
<213> Artificial Sequence

<220>
<223> HH ribozyme encoding sequence

<400> 53
ctgatgagtc cgtgaggacg aaacgagtaa gctcgtc 37

<210> 54
<211> 68
<212> DNA
<213> Hepatitis Delta Virus

<400> 54
ggccggcatg gtcccagcct cctcgctggc gccggctggg caacatgctt cggcatggcg 60

aatgggac 68


<210> 55
<211> 15245
<212> DNA
<213> Artificial Sequence

<220>
<223> pZFNnptII_Cpf1 target vector

<400> 55
tgcaggaatt cgatctggca cgacaggttt cccgactgga aagcgggcag tgagcgcaac 60

gcaattaatg tgagttagct cactcattag gcaccccagg ctttacactt tatgcttccg 120

gctcgtatgt tgtgtggaat tgtgagcgga taacaatttc acacaggaaa cagctatgac 180

atgattacga attcaaaaat tacggatatg aatataggca tatccgtatc cgaattatcc 240

gtttgacagc tagcaacgat tgtacaattg cttctttaaa aaaggaagaa agaaagaaag 300

aaaagaatca acatcagcgt taacaaacgg ccccgttacg gcccaaacgg tcatatagag 360

taacggcgtt aagcgttgaa agactcctat cgaaatacgt aaccgcaaac gtgtcatagt 420

cagatcccct cttccttcac cgcctcaaac acaaaaataa tcttctacag cctatatata 480

caaccccccc ttctatctct cctttctcac aattcatcat ctttctttct ctacccccaa 540

ttttaagaaa tcctctcttc tcctcttcat tttcaaggta aatctctctc tctctctctc 600

tctctgttat tccttgtttt aattaggtat gtattattgc tagtttgtta atctgcttat 660

cttatgtatg ccttatgtga atatctttat cttgttcatc tcatccgttt agaagctata 720

aatttgttga tttgactgtg tatctacacg tggttatgtt tatatctaat cagatatgaa 780

tttcttcata ttgttgcgtt tgtgtgtacc aatccgaaat cgttgatttt tttcatttaa 840

tcgtgtagct aattgtacgt atacatatgg atctacgtat caattgttca tctgtttgtg 900

tttgtatgta tacagatctg aaaacatcac ttctctcatc tgattgtgtt gttacataca 960

tagatataga tctgttatat catttttttt attaattgtg tatatatata tgtgcataga 1020

tctggattac atgattgtga ttatttacat gattttgtta tttacgtatg tatatatgta 1080

gatctggact ttttggagtt gttgacttga ttgtatttgt gtgtgtatat gtgtgttctg 1140

atcttgatat gttatgtatg tgcagcgaat tcggcgcgcc atggctccta agaagaagag 1200

gaaggttagc aagctcgaga agtttaccaa ctgctacagc ctctctaaga ccctcaggtt 1260

caaggctatc cctgtgggaa agacccaaga gaatatcgac aacaagaggc tcctcgtcga 1320

ggatgagaag agagctgaag attacaaggg cgtgaagaag ctcctcgaca ggtactacct 1380

cagcttcatc aacgatgtgc tccacagcat caagctcaag aacctcaaca actacatcag 1440

cctcttccgt aagaaaacca ggaccgagaa agagaacaaa gagcttgaga acctcgagat 1500

caacctccgt aaagagatcg ccaaggcttt caagggaaac gagggataca agagcctctt 1560

caagaaggat attatcgaga caatcctgcc tgagttcctg gacgataagg atgagatcgc 1620

tctcgtgaac agcttcaacg gattcactac tgccttcacc ggattcttcg acaacaggga 1680

aaacatgttc agcgaagagg ccaagagcac ctctatcgct ttcagatgca tcaacgagaa 1740

cctcacgcgt tacatcagca acatggacat cttcgagaag gtggacgcca tcttcgataa 1800

gcacgaggtg caagaaatca aagagaagat cctcaacagc gactacgacg tcgaggactt 1860

ttttgaaggg gagttcttca acttcgttct cacccaagag ggcatcgacg tgtacaacgc 1920

tattatcgga ggattcgtga ccgagtctgg ggagaagatt aagggactca acgagtacat 1980

caacctgtac aaccagaaaa cgaagcagaa gctcccgaag ttcaagccgc tctacaagca 2040

ggttctctct gatcgtgaga gcctctcatt ttacggtgag ggttacacct ctgacgagga 2100

agtgcttgag gttttccgta acaccctcaa caagaacagc gagatcttct cgtccatcaa 2160

gaagttggag aaacttttca agaacttcga cgagtacagc agcgctggga tcttcgttaa 2220

gaacggacct gctatcagca ccatcagcaa ggatattttc ggcgagtgga acgtgatcag 2280

ggacaagtgg aatgctgagt acgatgacat ccacctcaag aagaaggctg tcgtcactga 2340

gaagtacgag gatgacaggc gtaagtcgtt caagaagatc ggctctttca gcctcgagca 2400

gcttcaagaa tacgctgatg ctgatctcag cgtggtcgag aagctcaaag agatcatcat 2460

ccagaaggtc gacgagatct acaaggtgta cgggtcctct gagaagttgt tcgatgctga 2520

tttcgtcctc gagaagagtc tgaagaagaa cgacgctgtc gtcgcgatca tgaaggattt 2580

gctcgacagc gtgaagtcct tcgagaacta tatcaaggcc ttcttcggag agggcaaaga 2640

gactaatagg gacgagtctt tctacgggga tttcgtgctc gcttacgata tcctcctcaa 2700

ggtggaccat atctacgacg ccatcagaaa ctacgtgacc cagaagcctt acagcaagga 2760

caagttcaag ttgtactttc agaacccgca gttcatgggc ggatgggaca aagacaaaga 2820

gacagattac agggccacca tcctcaggta cgggtctaag tactacctgg ccatcatgga 2880

caagaaatac gccaagtgcc tccaaaagat cgacaaggat gacgtgaacg ggaactatga 2940

gaagatcaac tacaagctcc ttccgggacc gaacaagatg cttcctaagg tgttcttcag 3000

caagaaatgg atggcctact acaacccgtc tgaggacatc cagaaaatct acaagaacgg 3060

gaccttcaag aaaggcgaca tgttcaacct caacgactgc cacaagctca tcgatttctt 3120

caaggacagc atctcgcgtt acccgaagtg gtctaacgct tacgacttta acttcagcga 3180

gacagaaaag tacaaggata tcgccgggtt ctaccgtgag gttgaggaac agggttacaa 3240

ggttagcttc gagagcgcct ccaagaaaga ggttgacaag ttggtcgaag agggcaagct 3300

ctacatgttc cagatctata acaaggactt ctccgacaag agccacggaa ctcctaacct 3360

ccatacgatg tacttcaagc tgcttttcga cgagaacaac cacgggcaga tcagactttc 3420

tggtggtgct gaactcttca tgcgtagggc ctcactcaag aaagaagagt tggttgttca 3480

cccggccaac tctccaatcg ctaacaagaa tcctgacaac ccgaaaaaga ccaccacgct 3540

gtcttacgac gtctacaagg acaaaaggtt cagcgaggac cagtacgagc ttcatatccc 3600

gatcgctatc aacaagtgcc cgaagaacat cttcaagatc aataccgagg tgagggtgct 3660

gctcaagcac gatgataacc cttacgtgat cggaatcgat cgtggtgaga gaaacctcct 3720

ctacatcgtt gtggtggacg gaaagggaaa catcgtcgag cagtacagcc tgaacgagat 3780

tatcaacaat ttcaacggca tcaggatcaa gaccgactac cactcactcc tcgataagaa 3840

agaaaaagag cgtttcgagg ccaggcagaa ctggacttct atcgaaaaca tcaaagagtt 3900

gaaggccggc tacatctctc aggtggtgca taagatctgc gagctggtgg aaaagtacga 3960

tgctgtgatc gctcttgagg acctcaactc tgggttcaag aacagtagag tgaaggttga 4020

gaagcaggtc taccaaaagt tcgagaagat gctcatcgac aagctcaact acatggtgga 4080

caaaaagagc aacccttgcg ctaccggtgg tgctcttaag ggataccaga tcacgaacaa 4140

gttcgagtcc ttcaagagca tgagcaccca gaacggcttc atcttctata tccctgcttg 4200

gctcaccagc aagatcgatc cttctactgg tttcgtgaac ctgctcaaga ccaagtacac 4260

ctcgatcgcc gacagcaaga agttcatctc gtctttcgac aggatcatgt acgtgccgga 4320

agaggatctt ttcgagttcg ctctcgacta taagaacttc agcaggaccg acgccgacta 4380

cattaagaag tggaagctct actcctacgg gaaccgtatc aggatcttcc gaaatccgaa 4440

gaaaaacaac gtgttcgact gggaagaagt gtgcctcacc tctgcctaca aagaactgtt 4500

caacaagtac ggcatcaact accagcaggg tgatatcagg gctcttttgt gcgagcagag 4560

cgacaaggca ttctacagct cattcatggc cctcatgtct ctcatgctcc agatgaggaa 4620

ctctatcacc ggaaggaccg atgtggactt ccttatctct ccggtcaaga actctgacgg 4680

gatcttctac gacagccgta actatgaggc tcaagagaac gctatcctgc cgaagaatgc 4740

tgatgcaaac ggggcttaca acattgcgag aaaggttctc tgggctatcg ggcagtttaa 4800

gaaagcggaa gatgagaagc tcgacaaggt gaagatcgcc atctccaaca aagagtggct 4860

tgagtacgct cagacctccg ttaagcacaa gaggcctgct gctactaaga aagctggcca 4920

ggccaaaaag aagaagtgag gcgcgccgag ctccaggcct cccagctttc gtccgtatca 4980

tcggtttcga caacgttcgt caagttcaat gcatcagttt cattgcccac acaccagaat 5040

cctactaagt ttgagtatta tggcattgga aaagctgttt tcttctatca tttgttctgc 5100

ttgtaattta ctgtgttctt tcagtttttg ttttcggaca tcaaaatgca aatggatgga 5160

taagagttaa taaatgatat ggtccttttg ttcattctca aattattatt atctgttgtt 5220

tttactttaa tgggttgaat ttaagtaaga aaggaactaa cagtgtgata ttaaggtgca 5280

atgttagaca tataaaacag tctttcacct ctctttggtt atgtcttgaa ttggtttgtt 5340

tcttcactta tctgtgtaat caagtttact atgagtctat gatcaagtaa ttatgcaatc 5400

aagttaagta cagtataggc ttgagctccc taggatcaag cttatcgatg tcgacaggcc 5460

ttaagggcca gatcttgggc ccggtacccg atcagattgt cgtttcccgc cttcggttta 5520

aactatcagt gtttgacagg atatattggc gggtaaacct aagagaaaag agcgtttatt 5580

agaataatcg gatatttaaa agggcgtgaa aaggtttatc cgttcgtcca tttgtatgtg 5640

catgccaacc acagggttcc cctcgggagt gcttggcatt ccgtgcgata atgacttctg 5700

ttcaaccacc caaacgtcgg aaagcctgac gacggagcag cattccaaaa agatcccttg 5760

gctcgtctgg gtcggctaga aggtcgagtg ggctgctgtg gcttgatccc tcaacgcggt 5820

cgcggacgta gcgcagcgcc gaaaaatcct cgatcgcaaa tccgacgctg tcgaaaagcg 5880

tgatctgctt gtcgctcttt cggccgacgt cctggccagt catcacgcgc caaagttccg 5940

tcacaggatg atctggcgcg agttgctgga tctcgccttc aatccgggtc tgtggcggga 6000

actccacgaa aatatccgaa cgcagcaaga tatcgcggtg catctcggtc ttgcctgggc 6060

agtcgccgcc gacgccgttg atgtggacgc cgaaaaggat ctaggtgaag atcctttttg 6120

ataatctcat gaccaaaatc ccttaacgtg agttttcgtt ccactgagcg tcagaccccg 6180

tagaaaagat caaaggatct tcttgagatc ctttttttct gcgcgtaatc tgctgcttgc 6240

aaacaaaaaa accaccgcta ccagcggtgg tttgtttgcc ggatcaagag ctaccaactc 6300

tttttccgaa ggtaactggc ttcagcagag cgcagatacc aaatactgtt cttctagtgt 6360

agccgtagtt aggccaccac ttcaagaact ctgtagcacc gcctacatac ctcgctctgc 6420

taatcctgtt accagtggct gctgccagtg gcgataagtc gtgtcttacc gggttggact 6480

caagacgata gttaccggat aaggcgcagc ggtcgggctg aacggggggt tcgtgcacac 6540

agcccagctt ggagcgaacg acctacaccg aactgagata cctacagcgt gagctatgag 6600

aaagcgccac gcttcccgaa gggagaaagg cggacaggta tccggtaagc ggcagggtcg 6660

gaacaggaga gcgcacgagg gagcttccag ggggaaacgc ctggtatctt tatagtcctg 6720

tcgggtttcg ccacctctga cttgagcgtc gatttttgtg atgctcgtca ggggggcgga 6780

gcctatggaa aaacgccagc aacgcggcct ttttacggtt cctggccttt tgctggcctt 6840

ttgctcacat gttctttcct gcgttatccc ctgattctgt ggataaccga ttaccgcctt 6900

tgagtgagct gataccgctc gccgcagccg aacgaccgag cgcagcgagt cagtgagcga 6960

ggaagcggaa gagcgcctga tgcggtattt tctccttacg catctgtgcg gtatttcaca 7020

ccgcatatgg tgcactctca gtacaatctg ctctgatgcc gcatagttaa gccagtatac 7080

actccgctat cgctacgtga ctgggtcatg gctgcgcccc gacacccgcc aacacccgct 7140

gacgcgccct gacgggcttg tctgctcccg gcatccgctt acagacaagc tgtgaccgtc 7200

tccgggagct gcatgtgtca gaggttttca ccgtcatcac cgaaacgcgc gaggcagggg 7260

tacgtcgagg tcgatccaac ccctccgctg ctatagtgca gtcggcttct gacgttcagt 7320

gcagccgtcg tctgaaaacg acatgtcgca caagtcctaa gttacgcgac aggctgccgc 7380

cctgcccttt tcctggcgtt ttcttgtcgc gtgttttagt cgcataaagt agaatacttg 7440

cgactagaac cggagacatt acgccatgaa caagagcgcc gccgctggcc tgctgggcta 7500

tgcccgcgtc agcaccgacg accaggactt gaccaaccaa cgggccgaac tgcacgcggc 7560

cggctgcacc aagctgtttt ccgagaagat caccggcacc aggcgcgacc gcccggagct 7620

ggccaggatg cttgaccacc tacgccctgg cgacgttgtg acagtgacca ggctagaccg 7680

cctggcccgc agcacccgcg acctactgga cattgccgag cgcatccagg aggccggcgc 7740

gggcctgcgt agcctggcag agccgtgggc cgacaccacc acgccggccg gccgcatggt 7800

gttgaccgtg ttcgccggca ttgccgagtt cgagcgttcc ctaatcatcg accgcacccg 7860

gagcgggcgc gaggccgcca aggcgcgagg cgtgaagttt ggcccccgcc ctaccctcac 7920

cccggcacag atcgcgcacg cccgcgagct gatcgaccag gaaggccgca ccgtgaaaga 7980

ggcggctgca ctgcttggcg tgcatcgctc gaccctgtac cgcgcacttg agcgcagcga 8040

ggaagtgacg cccaccgagg ccaggcggcg cggtgccttc cgtgaggacg cattgaccga 8100

ggccgacgcc ctggcggccg ccgagaatga acgccaagag gaacaagcat gaaaccgcac 8160

caggacggcc aggacgaacc gtttttcatt accgaagaga tcgaggcgga gatgatcgcg 8220

gccgggtacg tgttcgagcc gcccgcgcac gtctcaaccg tgcggctgca tgaaatcctg 8280

gccggtttgt ctgatgccaa gctcgcggcc tggccggcga gcttggccgc tgaagaaacc 8340

gagcgccgcc gtctaaaaag gtgatgtgta tttgagtaaa acagcttgcg tcatgcggtc 8400

gctgcgtata tgatgcgatg agtaaataaa caaatacgca aggggaacgc atgaaggtta 8460

tcgctgtact taaccagaaa ggcgggtcag gcaagacgac catcgcaacc catctagccc 8520

gcgccctgca actcgccggg gccgatgttc tgttagtcga ttccgatccc cagggcagtg 8580

cccgcgattg ggcggccgtg cgggaagatc aaccgctaac cgttgtcggc atcgaccgcc 8640

cgacgattga ccgcgacgtg aaggccatcg gccggcgcga cttcgtagtg atcgacggag 8700

cgccccaggc ggcggacttg gctgtgtccg cgatcaaggc agccgacttc gtgctgattc 8760

cggtgcagcc aagcccttac gacatatggg ccaccgccga cctggtggag ctggttaagc 8820

agcgcattga ggtcacggat ggaaggctac aagcggcctt tgtcgtgtcg cgggcgatca 8880

aaggcacgcg catcggcggt gaggttgccg aggcgctggc cgggtacgag ctgcccattc 8940

ttgagtcccg tatcacgcag cgcgtgagct acccaggcac tgccgccgcc ggcacaaccg 9000

ttcttgaatc agaacccgag ggcgacgctg cccgcgaggt ccaggcgctg gccgctgaaa 9060

ttaaatcaaa actcatttga gttaatgagg taaagagaaa atgagcaaaa gcacaaacac 9120

gctaagtgcc ggccgtccga gcgcacgcag cagcaaggct gcaacgttgg ccagcctggc 9180

agacacgcca gccatgaagc gggtcaactt tcagttgccg gcggaggatc acaccaagct 9240

gaagatgtac gcggtacgcc aaggcaagac cattaccgag ctgctatctg aatacatcgc 9300

gcagctacca gagtaaatga gcaaatgaat aaatgagtag atgaatttta gcggctaaag 9360

gaggcggcat ggaaaatcaa gaacaaccag gcaccgacgc cgtggaatgc cccatgtgtg 9420

gaggaacggg cggttggcca ggcgtaagcg gctgggttgt ctgccggccc tgcaatggca 9480

ctggaacccc caagcccgag gaatcggcgt gagcggtcgc aaaccatccg gcccggtaca 9540

aatcggcgcg gcgctgggtg atgacctggt ggagaagttg aaggcggcgc aggccgccca 9600

gcggcaacgc atcgaggcag aagcacgccc cggtgaatcg tggcaagcgg ccgctgatcg 9660

aatccgcaaa gaatcccggc aaccgccggc agccggtgcg ccgtcgatta ggaagccgcc 9720

caagggcgac gagcaaccag attttttcgt tccgatgctc tatgacgtgg gcacccgcga 9780

tagtcgcagc atcatggacg tggccgtttt ccgtctgtcg aagcgtgacc gacgagctgg 9840

cgaggtgatc cgctacgagc ttccagacgg gcacgtagag gtttccgcag ggccggccgg 9900

catggcgagt gtgtgggatt acgacctggt actgatggcg gtttcccatc taaccgaatc 9960

catgaaccga taccgggaag ggaagggaga caagcccggc cgcgtgttcc gtccacacgt 10020

tgcggacgta ctcaagttct gccggcgagc cgatggcgga aagcagaaag acgacctggt 10080

agaaacctgc attcggttaa acaccacgca cgttgccatg cagcgtacga agaaggccaa 10140

gaacggccgc ctggtgacgg tatccgaggg tgaagccttg attagccgct acaagatcgt 10200

aaagagcgaa accgggcggc cggagtacat cgagatcgag ctagctgatt ggatgtaccg 10260

cgagatcaca gaaggcaaga acccggacgt gctgacggtt caccccgatt actttttgat 10320

cgatcccggc atcggccgtt ttctctaccg cctggcacgc cgcgccgcag gcaaggcaga 10380

agccagatgg ttgttcaaga cgatctacga acgcagtggc agcgccggag agttcaagaa 10440

gttctgtttc accgtgcgca agctgatcgg gtcaaatgac ctgccggagt acgatttgaa 10500

ggaggaggcg gggcaggctg gcccgatcct agtcatgcgc taccgcaacc tgatcgaggg 10560

cgaagcatcc gccggttcct aatgtacgga gcagatgcta gggcaaattg ccctagcagg 10620

ggaaaaaggt cgaaaaggtc tctttcctgt ggatagcacg tacattggga acccaaagcc 10680

gtacattggg aaccggaacc cgtacattgg gaacccaaag ccgtacattg ggaaccggtc 10740

acacatgtaa gtgactgata taaaagagaa aaaaggcgat ttttccgcct aaaactcttt 10800

aaaacttatt aaaactctta aaacccgcct ggcctgtgca taactgtctg gccagcgcac 10860

agccgaagag ctgcaaaaag cgcctaccct tcggtcgctg cgctccctac gccccgccgc 10920

ttcgcgtcgg cctatcgcgg ccgctggccg ctcaaaaatg gctggcctac ggccaggcaa 10980

tctaccaggg cgcggacaag ccgcgccgtc gccactcgac cgccggcgcc cacatcaagg 11040

caccggtggg tatgcctgac gatgcgtgga gaccgaaacc ttgcgctcgt tcgccagcca 11100

ggacagaaat gcctcgactt cgctgctgcc caaggttgcc gggtgacgca caccgtggaa 11160

acggatgaag gcacgaaccc agtggacata agcctgttcg gttcgtaagc tgtaatgcaa 11220

gtagcgtatg cgctcacgca actggtccag aaccttgacc gaacgcagcg gtggtaacgg 11280

cgcagtggcg gttttcatgg cttgttatga ctgttttttt ggggtacagt ctatgcctcg 11340

ggcatccaag cagcaagcgc gttacgccgt gggtcgatgt ttgatgttat ggagcagcaa 11400

cgatgttacg cagcagggca gtcgccctaa aacaaagtta aacatcatga gggaagcggt 11460

gatcgccgaa gtatcgactc aactatcaga ggtagttggc gtcatcgagc gccatctcga 11520

accgacgttg ctggccgtac atttgtacgg ctccgcagtg gatggcggcc tgaagccaca 11580

cagtgatatt gatttgctgg ttacggtgac cgtaaggctt gatgaaacaa cgcggcgagc 11640

tttgatcaac gaccttttgg aaacttcggc ttcccctgga gagagcgaga ttctccgcgc 11700

tgtagaagtc accattgttg tgcacgacga catcattccg tggcgttatc cagctaagcg 11760

cgaactgcaa tttggagaat ggcagcgcaa tgacattctt gcaggtatct tcgagccagc 11820

cacgatcgac attgatctgg ctatcttgct gacaaaagca agagaacata gcgttgcctt 11880

ggtaggtcca gcggcggagg aactctttga tccggttcct gaacaggatc tatttgaggc 11940

gctaaatgaa accttaacgc tatggaactc gccgcccgac tgggctggcg atgagcgaaa 12000

tgtagtgctt acgttgtccc gcatttggta cagcgcagta accggcaaaa tcgcgccgaa 12060

ggatgtcgct gccgactggg caatggagcg cctgccggcc cagtatcagc ccgtcatact 12120

tgaagctaga caggcttatc ttggacaaga agaagatcgc ttggcctcgc gcgcagatca 12180

gttggaagaa tttgtccact acgtgaaagg cgagatcacc aaggtagtcg gcaaataatg 12240

tctaacaatt cgttcaagcc gacgccgctt cgcggcgcgg cttaactcaa gcgttagatg 12300

cactaagcac ataattgctc acagccaaac tatcaggtca agtctgcttt tattattttt 12360

aagcgtgcat aataagccct acacaaattg ggagatatat catgaaaggc tggctttttc 12420

ttgttatcgc aatagttggc gaagtaatcg caacatagct tgcttggtcg ttccgcgtga 12480

acgtcggctc gattgtacct gcgttcaaat actttgcgat cgtgttgcgc gcctgcccgg 12540

tgcgtcggct gatctcacgg atcgactgct tctctcgcaa cgccatccga cggatgatgt 12600

ttaaaagtcc catgtggatc actccgttgc cccgtcgctc accgtgttgg ggggaaggtg 12660

cacatggctc agttctcaat ggaaattatc tgcctaaccg gctcagttct gcgtagaaac 12720

caacatgcaa gctccaccgg gtgcaaagcg gcagcggcgg caggatatat tcaattgtaa 12780

atggcttcat gtccgggaaa tctacatgga tcagcaatga gtatgatggt caatatggag 12840

aaaaagaaag agtaattacc aatttttttt caattcaaaa atgtagatgt ccgcagcgtt 12900

attataaaat gaaagtacat tttgataaaa cgacaaatta cgatccgtcg tatttatagg 12960

cgaaagcaat aaacaaatta ttctaattcg gaaatcttta tttcgacgtg tctacattca 13020

cgtccaaatg ggggcttaga tgagaaactt cacgatcggc tctagttcga gcgatctagt 13080

aacatagatg acaccgcgcg cgataattta tcctagtttg cgcgctatat tttgttttct 13140

atcgcgtatt aaatgtataa ttgcgggact ctaatcataa aaacccatct cataaataac 13200

gtcatgcatt acatgttaat tattacatgc ttaacgtaat tcaacagaaa ttatatgata 13260

atcatcgcaa gaccggcaac aggattcaat cttaagaaac tttattgcca aatgtttgaa 13320

cgatcgggga aattcgtttc tatgcttcaa ttctgctcta tagatgttct tctagtctta 13380

acttttagaa ggcacaacat ttgaaatgtt ggtaattttt ctgctgggta gtgaaccatt 13440

gtaatttgtg tttgcttata ttatccgttg ggtttctcta tgttatctgt ggggccatat 13500

cccagaactg gttgagtcgg tccaacacct gggtgccaat catgtcgatg gtggggtatg 13560

gtccggattg aaagaaatat agtttaaata tttattgata aaataacaag tcaggtatta 13620

tagtccaagc aaaaacataa atttattgat gcaagtttaa attcagaaat atttcaataa 13680

ctgattatat cagctggtac attgccgtag atgaaagact gagtgcgata ttatgtgtaa 13740

tacataaatt gatgatatag ctagcttagc tcatcgggtc agaagaactc gtcaagaagg 13800

cgatagaagg cgatgcgctg cgaatcggga gcggcgatac cgtaaagcac gaggaagcgg 13860

tcagcccatt cgccgccaag ctcttcagca atatcacggg tagccaacgc tatgtcctga 13920

tagcggtccg ccacacccag ccggccacag tcgatgaatc cagaaaagcg gccattttcc 13980

accatgatat tcggcaagca ggcatcgcca tgagtcacga cgagatcctc gccgtcgggc 14040

atgcgcgcct tgagcctggc gaacagttcg gctggcgcga gcccctgatg ctcttcgtcc 14100

agatcatcct gatcgacaag accggcttcc atccgagtac gtgctcgctc gatgcgatgt 14160

ttcgcttggt ggtcgaatgg gcaggtagcc ggatcaagcg tatgcagccg ccgcattgca 14220

tcagccatga tggatacttt ctcggcagga gcaaggtgag atgacaggag atcctgcccc 14280

ggcacttcgc ccaatagcag ccagtccctt cccgcttcag tgacaacgtc gagcacagct 14340

gcgcaaggaa cgcccgtcgt ggccagccac gatagccgcg ctgcctcgtc ctgaagttca 14400

ttcagggcac cggacaggtc ggtcttgaca aaaagaaccg ggcgcccctg cgctgacagc 14460

cggaacacgg cggcatcaga gcagccgatt gtctgttgtg cccagtcata gccgaatagc 14520

ctctccaccc aagcggccgg agaacctgcg tgcaatccat cttgttcaat ccaagctccc 14580

attttctgtg attttggatg tgtttttgta tggattgaga gtgaatatga gactctaatt 14640

ggataccgag gggaatttat ggaacgtcag tggagcattt ttgacaagaa atatttgcta 14700

gctgatagtg accttaggcg acttttgaac gcgcaataat ggtttctgac gtatgtgctt 14760

agctcattaa actccagaaa cccgcggctg agtggctcct tcaacgttgc ggttctgtca 14820

gttccaaacg taaaacggct tgtcccgcgt catcggcggg ggtcattccg gactgtgggg 14880

ccatatccca gaactggttg agtcggtcca acacctgggt gccaatcatg tcgatggtgg 14940

ggtatggcca attttttttc aattcaaaaa tgtagatgtc cgcagcgtta ttataaaatg 15000

aaagtacatt ttgataaaac gacaaattac gatccgtcgt atttataggc gaaagcaata 15060

aacaaattat tctaattcgg aaatctttat ttcgacgtgt ctacattcac gtccaaatgg 15120

gggcttagat gagaaacttc acgatcggct ctagaggcca tggcggccgc actagtggat 15180

cccccgggat gcatgaattc aagcttatcg atgtcgacag gccttaaggg ccagatcccc 15240

cgggc 15245

<210> 56
<211> 1611
<212> DNA
<213> Artificial Sequence

<220>
<223> crRNA expression cassette

<400> 56
aaaaattacg gatatgaata taggcatatc cgtatccgaa ttatccgttt gacagctagc 60

aacgattgta caattgcttc tttaaaaaag gaagaaagaa agaaagaaaa gaatcaacat 120

cagcgttaac aaacggcccc gttacggccc aaacggtcat atagagtaac ggcgttaagc 180

gttgaaagac tcctatcgaa atacgtaacc gcaaacgtgt catagtcaga tcccctcttc 240

cttcaccgcc tcaaacacaa aaataatctt ctacagccta tatatacaac ccccccttct 300

atctctcctt tctcacaatt catcatcttt ctttctctac ccccaatttt aagaaatcct 360

ctcttctcct cttcattttc aaggtaaatc tctctctctc tctctctctc tgttattcct 420

tgttttaatt aggtatgtat tattgctagt ttgttaatct gcttatctta tgtatgcctt 480

atgtgaatat ctttatcttg ttcatctcat ccgtttagaa gctataaatt tgttgatttg 540

actgtgtatc tacacgtggt tatgtttata tctaatcaga tatgaatttc ttcatattgt 600

tgcgtttgtg tgtaccaatc cgaaatcgtt gatttttttc atttaatcgt gtagctaatt 660

gtacgtatac atatggatct acgtatcaat tgttcatctg tttgtgtttg tatgtataca 720

gatctgaaaa catcacttct ctcatctgat tgtgttgtta catacataga tatagatctg 780

ttatatcatt ttttttatta attgtgtata tatatatgtg catagatctg gattacatga 840

ttgtgattat ttacatgatt ttgttattta cgtatgtata tatgtagatc tggacttttt 900

ggagttgttg acttgattgt atttgtgtgt gtatatgtgt gttctgatct tgatatgtta 960

tgtatgtgca gcggatccaa attactgatg agtccgtgag gacgaaacga gtaagctcgt 1020

ctaatttcta ctaagtgtag atgagtcttc gctcagtctg accgcggaag acctggccgg 1080

catggtccca gcctcctcgc tggcgccggc tgggcaacat gcttcggcat ggcgaatggg 1140

accaggcctc ccagctttcg tccgtatcat cggtttcgac aacgttcgtc aagttcaatg 1200

catcagtttc attgcccaca caccagaatc ctactaagtt tgagtattat ggcattggaa 1260

aagctgtttt cttctatcat ttgttctgct tgtaatttac tgtgttcttt cagtttttgt 1320

tttcggacat caaaatgcaa atggatggat aagagttaat aaatgatatg gtccttttgt 1380

tcattctcaa attattatta tctgttgttt ttactttaat gggttgaatt taagtaagaa 1440

aggaactaac agtgtgatat taaggtgcaa tgttagacat ataaaacagt ctttcacctc 1500

tctttggtta tgtcttgaat tggtttgttt cttcacttat ctgtgtaatc aagtttacta 1560

tgagtctatg atcaagtaat tatgcaatca agttaagtac agtataggct t 1611

<210> 57
<211> 9763
<212> DNA
<213> Artificial Sequence

<220>
<223> pZFNtDTnptII plasmid

<400> 57
agcttatcga tgtcgacagg ccttaagggc cagatcttgg gcccggtacc cgatcagatt 60

gtcgtttccc gccttcggtt taaactatca gtgtttgaca ggatatattg gcgggtaaac 120

ctaagagaaa agagcgttta ttagaataat cggatattta aaagggcgtg aaaaggttta 180

tccgttcgtc catttgtatg tgcatgccaa ccacagggtt cccctcggga gtgcttggca 240

ttccgtgcga taatgacttc tgttcaacca cccaaacgtc ggaaagcctg acgacggagc 300

agcattccaa aaagatccct tggctcgtct gggtcggcta gaaggtcgag tgggctgctg 360

tggcttgatc cctcaacgcg gtcgcggacg tagcgcagcg ccgaaaaatc ctcgatcgca 420

aatccgacgc tgtcgaaaag cgtgatctgc ttgtcgctct ttcggccgac gtcctggcca 480

gtcatcacgc gccaaagttc cgtcacagga tgatctggcg cgagttgctg gatctcgcct 540

tcaatccggg tctgtggcgg gaactccacg aaaatatccg aacgcagcaa gatatcgcgg 600

tgcatctcgg tcttgcctgg gcagtcgccg ccgacgccgt tgatgtggac gccgaaaagg 660

atctaggtga agatcctttt tgataatctc atgaccaaaa tcccttaacg tgagttttcg 720

ttccactgag cgtcagaccc cgtagaaaag atcaaaggat cttcttgaga tccttttttt 780

ctgcgcgtaa tctgctgctt gcaaacaaaa aaaccaccgc taccagcggt ggtttgtttg 840

ccggatcaag agctaccaac tctttttccg aaggtaactg gcttcagcag agcgcagata 900

ccaaatactg ttcttctagt gtagccgtag ttaggccacc acttcaagaa ctctgtagca 960

ccgcctacat acctcgctct gctaatcctg ttaccagtgg ctgctgccag tggcgataag 1020

tcgtgtctta ccgggttgga ctcaagacga tagttaccgg ataaggcgca gcggtcgggc 1080

tgaacggggg gttcgtgcac acagcccagc ttggagcgaa cgacctacac cgaactgaga 1140

tacctacagc gtgagctatg agaaagcgcc acgcttcccg aagggagaaa ggcggacagg 1200

tatccggtaa gcggcagggt cggaacagga gagcgcacga gggagcttcc agggggaaac 1260

gcctggtatc tttatagtcc tgtcgggttt cgccacctct gacttgagcg tcgatttttg 1320

tgatgctcgt caggggggcg gagcctatgg aaaaacgcca gcaacgcggc ctttttacgg 1380

ttcctggcct tttgctggcc ttttgctcac atgttctttc ctgcgttatc ccctgattct 1440

gtggataacc gattaccgcc tttgagtgag ctgataccgc tcgccgcagc cgaacgaccg 1500

agcgcagcga gtcagtgagc gaggaagcgg aagagcgcct gatgcggtat tttctcctta 1560

cgcatctgtg cggtatttca caccgcatat ggtgcactct cagtacaatc tgctctgatg 1620

ccgcatagtt aagccagtat acactccgct atcgctacgt gactgggtca tggctgcgcc 1680

ccgacacccg ccaacacccg ctgacgcgcc ctgacgggct tgtctgctcc cggcatccgc 1740

ttacagacaa gctgtgaccg tctccgggag ctgcatgtgt cagaggtttt caccgtcatc 1800

accgaaacgc gcgaggcagg ggtacgtcga ggtcgatcca acccctccgc tgctatagtg 1860

cagtcggctt ctgacgttca gtgcagccgt cgtctgaaaa cgacatgtcg cacaagtcct 1920

aagttacgcg acaggctgcc gccctgccct tttcctggcg ttttcttgtc gcgtgtttta 1980

gtcgcataaa gtagaatact tgcgactaga accggagaca ttacgccatg aacaagagcg 2040

ccgccgctgg cctgctgggc tatgcccgcg tcagcaccga cgaccaggac ttgaccaacc 2100

aacgggccga actgcacgcg gccggctgca ccaagctgtt ttccgagaag atcaccggca 2160

ccaggcgcga ccgcccggag ctggccagga tgcttgacca cctacgccct ggcgacgttg 2220

tgacagtgac caggctagac cgcctggccc gcagcacccg cgacctactg gacattgccg 2280

agcgcatcca ggaggccggc gcgggcctgc gtagcctggc agagccgtgg gccgacacca 2340

ccacgccggc cggccgcatg gtgttgaccg tgttcgccgg cattgccgag ttcgagcgtt 2400

ccctaatcat cgaccgcacc cggagcgggc gcgaggccgc caaggcgcga ggcgtgaagt 2460

ttggcccccg ccctaccctc accccggcac agatcgcgca cgcccgcgag ctgatcgacc 2520

aggaaggccg caccgtgaaa gaggcggctg cactgcttgg cgtgcatcgc tcgaccctgt 2580

accgcgcact tgagcgcagc gaggaagtga cgcccaccga ggccaggcgg cgcggtgcct 2640

tccgtgagga cgcattgacc gaggccgacg ccctggcggc cgccgagaat gaacgccaag 2700

aggaacaagc atgaaaccgc accaggacgg ccaggacgaa ccgtttttca ttaccgaaga 2760

gatcgaggcg gagatgatcg cggccgggta cgtgttcgag ccgcccgcgc acgtctcaac 2820

cgtgcggctg catgaaatcc tggccggttt gtctgatgcc aagctcgcgg cctggccggc 2880

gagcttggcc gctgaagaaa ccgagcgccg ccgtctaaaa aggtgatgtg tatttgagta 2940

aaacagcttg cgtcatgcgg tcgctgcgta tatgatgcga tgagtaaata aacaaatacg 3000

caaggggaac gcatgaaggt tatcgctgta cttaaccaga aaggcgggtc aggcaagacg 3060

accatcgcaa cccatctagc ccgcgccctg caactcgccg gggccgatgt tctgttagtc 3120

gattccgatc cccagggcag tgcccgcgat tgggcggccg tgcgggaaga tcaaccgcta 3180

accgttgtcg gcatcgaccg cccgacgatt gaccgcgacg tgaaggccat cggccggcgc 3240

gacttcgtag tgatcgacgg agcgccccag gcggcggact tggctgtgtc cgcgatcaag 3300

gcagccgact tcgtgctgat tccggtgcag ccaagccctt acgacatatg ggccaccgcc 3360

gacctggtgg agctggttaa gcagcgcatt gaggtcacgg atggaaggct acaagcggcc 3420

tttgtcgtgt cgcgggcgat caaaggcacg cgcatcggcg gtgaggttgc cgaggcgctg 3480

gccgggtacg agctgcccat tcttgagtcc cgtatcacgc agcgcgtgag ctacccaggc 3540

actgccgccg ccggcacaac cgttcttgaa tcagaacccg agggcgacgc tgcccgcgag 3600

gtccaggcgc tggccgctga aattaaatca aaactcattt gagttaatga ggtaaagaga 3660

aaatgagcaa aagcacaaac acgctaagtg ccggccgtcc gagcgcacgc agcagcaagg 3720

ctgcaacgtt ggccagcctg gcagacacgc cagccatgaa gcgggtcaac tttcagttgc 3780

cggcggagga tcacaccaag ctgaagatgt acgcggtacg ccaaggcaag accattaccg 3840

agctgctatc tgaatacatc gcgcagctac cagagtaaat gagcaaatga ataaatgagt 3900

agatgaattt tagcggctaa aggaggcggc atggaaaatc aagaacaacc aggcaccgac 3960

gccgtggaat gccccatgtg tggaggaacg ggcggttggc caggcgtaag cggctgggtt 4020

gtctgccggc cctgcaatgg cactggaacc cccaagcccg aggaatcggc gtgagcggtc 4080

gcaaaccatc cggcccggta caaatcggcg cggcgctggg tgatgacctg gtggagaagt 4140

tgaaggcggc gcaggccgcc cagcggcaac gcatcgaggc agaagcacgc cccggtgaat 4200

cgtggcaagc ggccgctgat cgaatccgca aagaatcccg gcaaccgccg gcagccggtg 4260

cgccgtcgat taggaagccg cccaagggcg acgagcaacc agattttttc gttccgatgc 4320

tctatgacgt gggcacccgc gatagtcgca gcatcatgga cgtggccgtt ttccgtctgt 4380

cgaagcgtga ccgacgagct ggcgaggtga tccgctacga gcttccagac gggcacgtag 4440

aggtttccgc agggccggcc ggcatggcga gtgtgtggga ttacgacctg gtactgatgg 4500

cggtttccca tctaaccgaa tccatgaacc gataccggga agggaaggga gacaagcccg 4560
gccgcgtgtt ccgtccacac gttgcggacg tactcaagtt ctgccggcga gccgatggcg 4620
gaaagcagaa agacgacctg gtagaaacct gcattcggtt aaacaccacg cacgttgcca 4680
tgcagcgtac gaagaaggcc aagaacggcc gcctggtgac ggtatccgag ggtgaagcct 4740
tgattagccg ctacaagatc gtaaagagcg aaaccgggcg gccggagtac atcgagatcg 4800
agctagctga ttggatgtac cgcgagatca cagaaggcaa gaacccggac gtgctgacgg 4860
ttcaccccga ttactttttg atcgatcccg gcatcggccg ttttctctac cgcctggcac 4920
gccgcgccgc aggcaaggca gaagccagat ggttgttcaa gacgatctac gaacgcagtg 4980
gcagcgccgg agagttcaag aagttctgtt tcaccgtgcg caagctgatc gggtcaaatg 5040
acctgccgga gtacgatttg aaggaggagg cggggcaggc tggcccgatc ctagtcatgc 5100
gctaccgcaa cctgatcgag ggcgaagcat ccgccggttc ctaatgtacg gagcagatgc 5160
tagggcaaat tgccctagca ggggaaaaag gtcgaaaagg tctctttcct gtggatagca 5220
cgtacattgg gaacccaaag ccgtacattg ggaaccggaa cccgtacatt gggaacccaa 5280
agccgtacat tgggaaccgg tcacacatgt aagtgactga tataaaagag aaaaaaggcg 5340
atttttccgc ctaaaactct ttaaaactta ttaaaactct taaaacccgc ctggcctgtg 5400
cataactgtc tggccagcgc acagccgaag agctgcaaaa agcgcctacc cttcggtcgc 5460
tgcgctccct acgccccgcc gcttcgcgtc ggcctatcgc ggccgctggc cgctcaaaaa 5520
tggctggcct acggccaggc aatctaccag ggcgcggaca agccgcgccg tcgccactcg 5580
accgccggcg cccacatcaa ggcaccggtg ggtatgcctg acgatgcgtg gagaccgaaa 5640
ccttgcgctc gttcgccagc caggacagaa atgcctcgac ttcgctgctg cccaaggttg 5700
ccgggtgacg cacaccgtgg aaacggatga aggcacgaac ccagtggaca taagcctgtt 5760
cggttcgtaa gctgtaatgc aagtagcgta tgcgctcacg caactggtcc agaaccttga 5820
ccgaacgcag cggtggtaac ggcgcagtgg cggttttcat ggcttgttat gactgttttt 5880
ttggggtaca gtctatgcct cgggcatcca agcagcaagc gcgttacgcc gtgggtcgat 5940
gtttgatgtt atggagcagc aacgatgtta cgcagcaggg cagtcgccct aaaacaaagt 6000
taaacatcat gagggaagcg gtgatcgccg aagtatcgac tcaactatca gaggtagttg 6060
gcgtcatcga gcgccatctc gaaccgacgt tgctggccgt acatttgtac ggctccgcag 6120
tggatggcgg cctgaagcca cacagtgata ttgatttgct ggttacggtg accgtaaggc 6180
ttgatgaaac aacgcggcga gctttgatca acgacctttt ggaaacttcg gcttcccctg 6240
gagagagcga gattctccgc gctgtagaag tcaccattgt tgtgcacgac gacatcattc 6300
cgtggcgtta tccagctaag cgcgaactgc aatttggaga atggcagcgc aatgacattc 6360
ttgcaggtat cttcgagcca gccacgatcg acattgatct ggctatcttg ctgacaaaag 6420

caagagaaca tagcgttgcc ttggtaggtc cagcggcgga ggaactcttt gatccggttc 6480

ctgaacagga tctatttgag gcgctaaatg aaaccttaac gctatggaac tcgccgcccg 6540

actgggctgg cgatgagcga aatgtagtgc ttacgttgtc ccgcatttgg tacagcgcag 6600

taaccggcaa aatcgcgccg aaggatgtcg ctgccgactg ggcaatggag cgcctgccgg 6660

cccagtatca gcccgtcata cttgaagcta gacaggctta tcttggacaa gaagaagatc 6720

gcttggcctc gcgcgcagat cagttggaag aatttgtcca ctacgtgaaa ggcgagatca 6780

ccaaggtagt cggcaaataa tgtctaacaa ttcgttcaag ccgacgccgc ttcgcggcgc 6840

ggcttaactc aagcgttaga tgcactaagc acataattgc tcacagccaa actatcaggt 6900

caagtctgct tttattattt ttaagcgtgc ataataagcc ctacacaaat tgggagatat 6960

atcatgaaag gctggctttt tcttgttatc gcaatagttg gcgaagtaat cgcaacatag 7020

cttgcttggt cgttccgcgt gaacgtcggc tcgattgtac ctgcgttcaa atactttgcg 7080

atcgtgttgc gcgcctgccc ggtgcgtcgg ctgatctcac ggatcgactg cttctctcgc 7140

aacgccatcc gacggatgat gtttaaaagt cccatgtgga tcactccgtt gccccgtcgc 7200

tcaccgtgtt ggggggaagg tgcacatggc tcagttctca atggaaatta tctgcctaac 7260

cggctcagtt ctgcgtagaa accaacatgc aagctccacc gggtgcaaag cggcagcggc 7320

ggcaggatat attcaattgt aaatggcttc atgtccggga aatctacatg gatcagcaat 7380

gagtatgatg gtcaatatgg agaaaaagaa agagtaatta ccaatttttt ttcaattcaa 7440

aaatgtagat gtccgcagcg ttattataaa atgaaagtac attttgataa aacgacaaat 7500

tacgatccgt cgtatttata ggcgaaagca ataaacaaat tattctaatt cggaaatctt 7560

tatttcgacg tgtctacatt cacgtccaaa tgggggctta gatgagaaac ttcacgatcg 7620

gctctagttc gagcgatcta gtaacataga tgacaccgcg cgcgataatt tatcctagtt 7680

tgcgcgctat attttgtttt ctatcgcgta ttaaatgtat aattgcggga ctctaatcat 7740

aaaaacccat ctcataaata acgtcatgca ttacatgtta attattacat gcttaacgta 7800

attcaacaga aattatatga taatcatcgc aagaccggca acaggattca atcttaagaa 7860

actttattgc caaatgtttg aacgatcggg gaaattcgtt tctatgcttc aattctgctc 7920

tatagatgtt cttctagtct taacttttag aaggcacaac atttgaaatg ttggtaattt 7980

ttctgctggg tagtgaacca ttgtaatttg tgtttgctta tattatccgt tgggtttctc 8040

tatgttatct gtggggccat atcccagaac tggttgagtc ggtccaacac ctgggtgcca 8100

atcatgtcga tggtggggta tggtccggat tgaaagaaat atagtttaaa tatttattga 8160

taaaataaca agtcaggtat tatagtccaa gcaaaaacat aaatttattg atgcaagttt 8220

aaattcagaa atatttcaat aactgattat atcagctggt acattgccgt agatgaaaga 8280

ctgagtgcga tattatgtgt aatacataaa ttgatgatat agctagctta gctcatcggg 8340

tcagaagaac tcgtcaagaa ggcgatagaa ggcgatgcgc tgcgaatcgg gagcggcgat 8400

accgtaaagc acgaggaagc ggtcagccca ttcgccgcca agctcttcag caatatcacg 8460

ggtagccaac gctatgtcct gatagcggtc cgccacaccc agccggccac agtcgatgaa 8520

tccagaaaag cggccatttt ccaccatgat attcggcaag caggcatcgc catgagtcac 8580

gacgagatcc tcgccgtcgg gcatgcgcgc cttgagcctg gcgaacagtt cggctggcgc 8640

gagcccctga tgctcttcgt ccagatcatc ctgatcgaca agaccggctt ccatccgagt 8700

acgtgctcgc tcgatgcgat gtttcgcttg gtggtcgaat gggcaggtag ccggatcaag 8760

cgtatgcagc cgccgcattg catcagccat gatggatact ttctcggcag gagcaaggtg 8820

agatgacagg agatcctgcc ccggcacttc gcccaatagc agccagtccc ttcccgcttc 8880

agtgacaacg tcgagcacag ctgcgcaagg aacgcccgtc gtggccagcc acgatagccg 8940

cgctgcctcg tcctgaagtt cattcagggc accggacagg tcggtcttga caaaaagaac 9000

cgggcgcccc tgcgctgaca gccggaacac ggcggcatca gagcagccga ttgtctgttg 9060

tgcccagtca tagccgaata gcctctccac ccaagcggcc ggagaacctg cgtgcaatcc 9120

atcttgttca atccaagctc ccattttctg tgattttgga tgtgtttttg tatggattga 9180

gagtgaatat gagactctaa ttggataccg aggggaattt atggaacgtc agtggagcat 9240

ttttgacaag aaatatttgc tagctgatag tgaccttagg cgacttttga acgcgcaata 9300

atggtttctg acgtatgtgc ttagctcatt aaactccaga aacccgcggc tgagtggctc 9360

cttcaacgtt gcggttctgt cagttccaaa cgtaaaacgg cttgtcccgc gtcatcggcg 9420

ggggtcattc cggactgtgg ggccatatcc cagaactggt tgagtcggtc caacacctgg 9480

gtgccaatca tgtcgatggt ggggtatggc caattttttt tcaattcaaa aatgtagatg 9540

tccgcagcgt tattataaaa tgaaagtaca ttttgataaa acgacaaatt acgatccgtc 9600

gtatttatag gcgaaagcaa taaacaaatt attctaattc ggaaatcttt atttcgacgt 9660

gtctacattc acgtccaaat gggggcttag atgagaaact tcacgatcgg ctctagaggc 9720

catggcggcc gcactagtgg atcccccggg atgcatgaat tca 9763

<210> 58
<211> 45
<212> DNA
<213> Artificial Sequence

<220>
<223> crRNA_ALS G/T

<400> 58
attaaagatg attcacatct aggtatggtt gtccaatggg aagat 45

<210>  59
<211>  45
<212>  DNA
<213>  Artificial Sequence

<220>
<223>  crRNA_Cyp Del

<400>  59
attaaagatg attcacatct atacgtggtt cggttgatca tatag                    45


<210>  60
<211>  45
<212>  DNA
<213>  Artificial Sequence

<220>
<223>  crRNA_ALS In1

<400>  60
attaaagatg attcacatct aggctatgga tgttgtgttg catca                    45


<210>  61
<211>  45
<212>  DNA
<213>  Artificial Sequence

<220>
<223>  crRNA_ALS In2

<400>  61
attaaagatg attcacatct atacgtggtt cggttgatca tatag                    45


<210>  62
<211>  1603
<212>  DNA
<213>  Artificial Sequence

<220>
<223>  crRNA_ALS_G/T_expression cassette

<400>  62
aaaaattacg gatatgaata taggcatatc cgtatccgaa ttatccgttt gacagctagc      60

aacgattgta caattgcttc tttaaaaaag gaagaaagaa agaaagaaaa gaatcaacat     120

cagcgttaac aaacggcccc gttacggccc aaacggtcat atagagtaac ggcgttaagc     180

gttgaaagac tcctatcgaa atacgtaacc gcaaacgtgt catagtcaga tcccctcttc     240

cttcaccgcc tcaaacacaa aaataatctt ctacagccta tatatacaac ccccccttct     300

atctctcctt tctcacaatt catcatcttt ctttctctac ccccaatttt aagaaatcct     360

ctcttctcct cttcattttc aaggtaaatc tctctctctc tctctctctc tgttattcct     420

tgttttaatt aggtatgtat tattgctagt ttgttaatct gcttatctta tgtatgcctt     480

atgtgaatat ctttatcttg ttcatctcat ccgtttagaa gctataaatt tgttgatttg     540

actgtgtatc tacacgtggt tatgtttata tctaatcaga tatgaatttc ttcatattgt 600

tgcgtttgtg tgtaccaatc cgaaatcgtt gatttttttc atttaatcgt gtagctaatt 660

gtacgtatac atatggatct acgtatcaat tgttcatctg tttgtgtttg tatgtataca 720

gatctgaaaa catcacttct ctcatctgat tgtgttgtta catacataga tatagatctg 780

ttatatcatt ttttttatta attgtgtata tatatatgtg catagatctg gattacatga 840

ttgtgattat ttacatgatt ttgttattta cgtatgtata tatgtagatc tggacttttt 900

ggagttgttg acttgattgt atttgtgtgt gtatatgtgt gttctgatct tgatatgtta 960

tgtatgtgca gcggatccaa attactgatg agtccgtgag gacgaaacga gtaagctcgt 1020

ctaatttcta ctaagtgtag atccatacca acaggttacc cttctaggcc ggcatggtcc 1080

cagcctcctc gctggcgccg gctgggcaac atgcttcggc atggcgaatg ggaccaggcc 1140

tcccagcttt cgtccgtatc atcggtttcg acaacgttcg tcaagttcaa tgcatcagtt 1200

tcattgccca cacaccagaa tcctactaag tttgagtatt atggcattgg aaaagctgtt 1260

ttcttctatc atttgttctg cttgtaattt actgtgttct ttcagttttt gttttcggac 1320

atcaaaatgc aaatggatgg ataagagtta ataaatgata tggtcctttt gttcattctc 1380

aaattattat tatctgttgt ttttacttta atgggttgaa tttaagtaag aaaggaacta 1440

acagtgtgat attaaggtgc aatgttagac atataaaaca gtctttcacc tctctttggt 1500

tatgtcttga attggtttgt ttcttcactt atctgtgtaa tcaagtttac tatgagtcta 1560

tgatcaagta attatgcaat caagttaagt acagtatagg ctt 1603

<210> 63
<211> 1603
<212> DNA
<213> Artificial Sequence

<220>
<223> crRNA_ALS_In1 expression cassette

<400> 63
aaaaattacg gatatgaata taggcatatc cgtatccgaa ttatccgttt gacagctagc 60

aacgattgta caattgcttc tttaaaaaag gaagaaagaa agaaagaaaa gaatcaacat 120

cagcgttaac aaacggcccc gttacggccc aaacggtcat atagagtaac ggcgttaagc 180

gttgaaagac tcctatcgaa atacgtaacc gcaaacgtgt catagtcaga tcccctcttc 240

cttcaccgcc tcaaacacaa aaataatctt ctacagccta tatatacaac ccccccttct 300

atctctcctt tctcacaatt catcatcttt ctttctctac ccccaatttt aagaaatcct 360

ctcttctcct cttcattttc aaggtaaatc tctctctctc tctctctctc tgttattcct 420

tgttttaatt aggtatgtat tattgctagt ttgttaatct gcttatctta tgtatgcctt 480

atgtgaatat ctttatcttg ttcatctcat ccgtttagaa gctataaatt tgttgatttg 540

actgtgtatc tacacgtggt tatgtttata tctaatcaga tatgaatttc ttcatattgt 600

tgcgtttgtg tgtaccaatc cgaaatcgtt gatttttttc atttaatcgt gtagctaatt 660

gtacgtatac atatggatct acgtatcaat tgttcatctg tttgtgtttg tatgtataca 720

gatctgaaaa catcacttct ctcatctgat tgtgttgtta catacataga tatagatctg 780

ttatatcatt ttttttatta attgtgtata tatatatgtg catagatctg gattacatga 840

ttgtgattat ttacatgatt ttgttattta cgtatgtata tatgtagatc tggacttttt 900

ggagttgttg acttgattgt atttgtgtgt gtatatgtgt gttctgatct tgatatgtta 960

tgtatgtgca gcggatccaa attactgatg agtccgtgag gacgaaacga gtaagctcgt 1020

ctaatttcta ctaagtgtag atccgatacc tacaacacaa cgtagtggcc ggcatggtcc 1080

cagcctcctc gctggcgccg gctgggcaac atgcttcggc atggcgaatg ggaccaggcc 1140

tcccagcttt cgtccgtatc atcggtttcg acaacgttcg tcaagttcaa tgcatcagtt 1200

tcattgccca cacaccagaa tcctactaag tttgagtatt atggcattgg aaaagctgtt 1260

ttcttctatc atttgttctg cttgtaattt actgtgttct ttcagttttt gttttcggac 1320

atcaaaatgc aaatggatgg ataagagtta ataaatgata tggtcctttt gttcattctc 1380

aaattattat tatctgttgt ttttacttta atgggttgaa tttaagtaag aaaggaacta 1440

acagtgtgat attaaggtgc aatgttagac atataaaaca gtctttcacc tctctttggt 1500

tatgtcttga attggtttgt ttcttcactt atctgtgtaa tcaagtttac tatgagtcta 1560

tgatcaagta attatgcaat caagttaagt acagtatagg ctt 1603

<210> 64
<211> 1603
<212> DNA
<213> Artificial Sequence

<220>
<223> crRNA_ALS_In2 expression cassette

<400> 64
aaaaattacg gatatgaata taggcatatc cgtatccgaa ttatccgttt gacagctagc 60

aacgattgta caattgcttc tttaaaaaag gaagaaagaa agaaagaaaa gaatcaacat 120

cagcgttaac aaacggcccc gttacggccc aaacggtcat atagagtaac ggcgttaagc 180

gttgaaagac tcctatcgaa atacgtaacc gcaaacgtgt catagtcaga tcccctcttc 240

cttcaccgcc tcaaacacaa aaataatctt ctacagccta tatatacaac ccccccttct 300

atctctcctt tctcacaatt catcatcttt ctttctctac ccccaatttt aagaaatcct 360

ctcttctcct cttcattttc aaggtaaatc tctctctctc tctctctctc tgttattcct 420

tgttttaatt aggtatgtat tattgctagt ttgttaatct gcttatctta tgtatgcctt 480

atgtgaatat ctttatcttg ttcatctcat ccgtttagaa gctataaatt tgttgatttg 540

actgtgtatc tacacgtggt tatgtttata tctaatcaga tatgaatttc ttcatattgt 600

tgcgtttgtg tgtaccaatc cgaaatcgtt gatttttttc atttaatcgt gtagctaatt 660

gtacgtatac atatggatct acgtatcaat tgttcatctg tttgtgtttg tatgtataca 720

gatctgaaaa catcacttct ctcatctgat tgtgttgtta catacataga tatagatctg 780

ttatatcatt ttttttatta attgtgtata tatatatgtg catagatctg gattacatga 840

ttgtgattat ttacatgatt ttgttattta cgtatgtata tatgtagatc tggacttttt 900

ggagttgttg acttgattgt atttgtgtgt gtatatgtgt gttctgatct tgatatgtta 960

tgtatgtgca gcggatccaa attactgatg agtccgtgag gacgaaacga gtaagctcgt 1020

ctaatttcta ctaagtgtag atatgcacca agccaactag tatatcggcc ggcatggtcc 1080

cagcctcctc gctggcgccg gctgggcaac atgcttcggc atggcgaatg ggaccaggcc 1140

tcccagcttt cgtccgtatc atcggtttcg acaacgttcg tcaagttcaa tgcatcagtt 1200

tcattgccca cacaccagaa tcctactaag tttgagtatt atggcattgg aaaagctgtt 1260

ttcttctatc atttgttctg cttgtaattt actgtgttct ttcagttttt gttttcggac 1320

atcaaaatgc aaatggatgg ataagagtta ataaatgata tggtcctttt gttcattctc 1380

aaattattat tatctgttgt ttttacttta atgggttgaa tttaagtaag aaaggaacta 1440

acagtgtgat attaaggtgc aatgttagac atataaaaca gtctttcacc tctctttggt 1500

tatgtcttga attggtttgt ttcttcactt atctgtgtaa tcaagtttac tatgagtcta 1560

tgatcaagta attatgcaat caagttaagt acagtatagg ctt 1603

<210> 65
<211> 1603
<212> DNA
<213> Artificial Sequence

<220>
<223> crRNA_Cyp_Del expression cassette

<400> 65
   aaaaattacg gatatgaata taggcatatc cgtatccgaa ttatccgttt gacagctagc 60
   aacgattgta caattgcttc tttaaaaaag gaagaaagaa agaaagaaaa gaatcaacat 120
   cagcgttaac aaacggcccc gttacggccc aaacggtcat atagagtaac ggcgttaagc 180
   gttgaaagac tcctatcgaa atacgtaacc gcaaacgtgt catagtcaga tcccctcttc 240
   cttcaccgcc tcaaacacaa aaataatctt ctacagccta tatatacaac ccccccttct 300
   atctctcctt tctcacaatt catcatcttt ctttctctac cccaattttt aagaaatcct 360
   ctcttctcct cttcattttc aaggtaaatc tctctctctc tctctctctc tgttattcct 420
   tgttttaatt aggtatgtat tattgctagt ttgttaatct gcttatctta tgtatgcctt 480
   atgtgaatat ctttatcttg ttcatctcat ccgtttagaa gctataaatt tgttgatttg 540
   actgtgtatc tacacgtggt tatgtttata tctaatcaga tatgaatttc ttcatattgt 600
   tgcgtttgtg tgtaccaatc cgaaatcgtt gatttttttc atttaatcgt gtagctaatt 660
   gtacgtatac atatggatct acgtatcaat tgttcatctg tttgtgtttg tatgtataca 720
   gatctgaaaa catcacttct ctcatctgat tgtgttgtta catacataga tatagatctg 780
   ttatatcatt tttttattaa attgtgtata tatatatgtg catagatctg gattacatga 840
   ttgtgattat ttacatgatt ttgttattta cgtatgtata tatgtagatc tggacttttt 900
   ggagttgttg acttgattgt atttgtgtgt gtatatgtgt gttctgatct tgatatgtta 960
   tgtatgtgca gcggatccaa attactgatg agtccgtgag gacgaaacga gtaagctcgt 1020
   ctaatttcta ctaagtgtag atatgcacca agccaactag tatatcggcc ggcatggtcc 1080
   cagcctcctc gctggcgccg gctgggcaac atgcttcggc atggcgaatg ggaccaggcc 1140
   tcccagcttt cgtccgtatc atcggtttcg acaacgttcg tcaagttcaa tgcatcagtt 1200
   tcattgccca cacaccagaa tcctactaag tttgagtatt atggcattgg aaaagctgtt 1260
   ttcttctatc atttgttctg cttgtaattt actgtgttct ttcagttttt gttttcggac 1320
   atcaaaatgc aaatggatgg ataagagtta ataaatgata tggtcctttt gttcattctc 1380
   aaattattat tatctgttgt ttttacttta atgggttgaa tttaagtaag aaaggaacta 1440
   acagtgtgat attaaggtgc aatgttagac atataaaaca gtctttcacc tctctttggt 1500
   tatgtcttga attggtttgt ttcttcactt atctgtgtaa tcaagtttac tatgagtcta 1560
   tgatcaagta attatgcaat caagttaagt acagtatagg ctt 1603

<210> 66
<211> 5816
<212> DNA
<213> Artificial Sequence

<220>
<223> pUbitDTnptII vector

<400> 66
agctttccct atagtgagtc gtattagagc ttggcgtaat catggtcata gctgtttcct 60

gtgtgaaatt gttatccgct cacaattcca cacaacatac gagccggaag cataaagtgt 120

aaagcctggg gtgcctaatg agtgagctaa ctcacattaa ttgcgttgcg ctcactgccc 180

gctttccagt cgggaaacct gtcgtgccag ctgcattaat gaatcggcca acgcgcgggg 240

agaggcggtt tgcgtattgg gcgctcttcc gcttcctcgc tcactgactc gctgcgctcg 300

gtcgttcggc tgcggcgagc ggtatcagct cactcaaagg cggtaatacg gttatccaca 360

gaatcagggg ataacgcagg aaagaacatg tgagcaaaag gccagcaaaa ggccaggaac 420

cgtaaaaagg ccgcgttgct ggcgtttttc cataggctcc gcccccctga cgagcatcac 480

aaaaatcgac gctcaagtca gaggtggcga aacccgacag gactataaag ataccaggcg 540

tttccccctg gaagctccct cgtgcgctct cctgttccga ccctgccgct taccggatac 600

ctgtccgcct ttctcccttc gggaagcgtg gcgctttctc aatgctcacg ctgtaggtat 660

ctcagttcgg tgtaggtcgt tcgctccaag ctgggctgtg tgcacgaacc ccccgttcag 720

cccgaccgct gcgccttatc cggtaactat cgtcttgagt ccaacccggt aagacacgac 780

ttatcgccac tggcagcagc cactggtaac aggattagca gagcgaggta tgtaggcggt 840

gctacagagt tcttgaagtg gtggcctaac tacggctaca ctagaaggac agtatttggt 900

atctgcgctc tgctgaagcc agttaccttc ggaaaaagag ttggtagctc ttgatccggc 960

aaacaaacca ccgctggtag cggtggtttt tttgtttgca agcagcagat tacgcgcaga 1020

aaaaaaggat ctcaagaaga tcctttgatc ttttctacgg ggtctgacgc tcagtggaac 1080

gaaaactcac gttaagggat tttggtcatg agattatcaa aaaggatctt cacctagatc 1140

cttttaaatt aaaaatgaag ttttaaatca atctaaagta tatatgagta aacttggtct 1200

gacagttacc aatgcttaat cagtgaggca cctatctcag cgatctgtct atttcgttca 1260

tccatagttg cctgactccc cgtcgtgtag ataactacga tacgggaggg cttaccatct 1320

ggccccagtg ctgcaatgat accgcgagac ccacgctgac cggctccaga tttatcagca 1380

ataaaccagc cagccggaag ggccgagcgc agaagtggtc ctgcaacttt atccgcctcc 1440

atccagtcta ttaattgttg ccgggaagct agagtaagta gttcgccagt taatagtttg 1500

cgcaacgttg ttgccattgc tacaggcatc gtggtgtcac gctcgtcgtt tggtatggct 1560

tcactcagct ccggttccca acgatcaagg cgagttacat gatcccccat gttgtgcaaa 1620

aaagcggtta gctccttcgg tcctccgatc gttgtcagaa gtaagttggc cgcagtgtta 1680

tcactcatgg ttatggcagc actgcataat tctcttactg tcatgccatc cgtaagatgc 1740

ttttctgtga ctggtgagta ctcaaccaag tcattctgag aatagtgtat gcggcgaccg 1800

agttgctctt gcccggcgtc aatacgggat aataccgcgc cacatagcag aactttaaaa 1860

gtgctcatca ttggaaaacg ttcttcgggg cgaaaactct caaggatctt accgctgttg 1920

agatccagtt cgatgtaacc cactcgtgca cccaactgat cttcagcatc ttttactttc 1980

accagcgttt ctgggtgagc aaaaacagga aggcaaaatg ccgcaaaaaa gggaataagg 2040

gcgacacgga aatgttgaat actcatactc ttcctttttc aatattattg aagcatttat 2100

cagggttatt gtctcatgag cggatacata tttgaatgta tttagaaaaa taaagaaata 2160

ggggttctgc gcacatttcc ccgaaaagtg ccacctgaaa ttgtaaacgt taatgttttg 2220

ttaaatttcg cgttaaatat ttgttaaatc agcttatttt ttaaccagta agcagaaaat 2280

gacaaaaatc cttataaatc aaaagaatag accgagttag ttgtgagtgt tgttccagtt 2340

tggaacaaga gtccactatt aaagaacgtg gactccaacg taaaaccgtc tatcagggcg 2400

atggcccact acgtgaacca tcacccaaat caagtttttg gaggtcgagg tgccgtaaag 2460

cactaaatcg gaaccctaaa gggagccccc gatttagagc ttgacgggga aagccggcga 2520

acgtggcgag aaaggaaggg aagaaagcga aaggagcggg cgctagggcg ctggcaagtg 2580

tagcggtcac gctgcgcgta accaccacac ccgccgcgct taatgcgccg ctactgggcg 2640

cgtcccattc gccattcagg ctgcgcaact gttgggaagg gcgatcggtg cgggcctctt 2700

cgctattacg ccagctggcg aaagggggat gtgctgcaag gcgattaagt tgggtaacgc 2760

cagggttttc ccagtcacga cgttgtaaaa cgacggccag tgaattatcg attaaattaa 2820

ccctcactaa agggagagac gaattcgaat ccaaaaatta cggatatgaa tataggcata 2880

tccgtatccg aattatccgt ttgacagcta gcaacgattg tacaattgct tctttaaaaa 2940

aggaagaaag aaagaaagaa aagaatcaac atcagcgtta acaaacggcc ccgttacggc 3000

ccaaacggtc atatagagta acggcgttaa gcgttgaaag actcctatcg aaatacgtaa 3060

ccgcaaacgt gtcatagtca gatcccctct tccttcaccg cctcaaacac aaaaataatc 3120

ttctacagcc tatatataca accccccctt ctatctctcc tttctcacaa ttcatcatct 3180

ttctttctct acccccaatt ttaagaaatc ctctcttctc ctcttcattt tcaaggtaaa 3240

tctctctctc tctctctctc tctgttattc cttgttttaa ttaggtatgt attattgcta 3300

gtttgttaat ctgcttatct tatgtatgcc ttatgtgaat atctttatct tgttcatctc 3360

atccgtttag aagctataaa tttgttgatt tgactgtgta tctacacgtg gttatgttta 3420

tatctaatca gatatgaatt tcttcatatt gttgcgtttg tgtgtaccaa tccgaaatcg 3480

ttgatttttt tcatttaatc gtgtagctaa ttgtacgtat acatatggat ctacgtatca 3540

attgttcatc tgtttgtgtt tgtatgtata cagatctgaa aacatcactt ctctcatctg 3600

attgtgttgt tacatacata gatatagatc tgttatatca ttttttttat taattgtgta 3660

tatatatatg tgcatagatc tggattacat gattgtgatt atttacatga ttttgttatt 3720

tacgtatgta tatatgtaga tctggacttt ttggagttgt tgacttgatt gtatttgtgt 3780

gtgtatatgt gtgttctgat cttgatatgt tatgtatgtg cagctgaacc ctcgagatcc 3840

tctcaacaca acatatacaa aacaaacgaa tctcaagcaa tcaagcattc tacttctatt 3900

gcagcaattt aaatcatttc ttttaaagca aaagcaattt tctgaaaatt ttcaccattt 3960

acgaacgata gggcgcgatc ccgccaccat ggtgagcaag ggcgaggagg tcatcaaaga 4020

gttcatgcgc ttcaaggtgc gcatggaggg ctccatgaac ggccacgagt tcgagatcga 4080

gggcgagggc gagggccgcc cctacgaggg cacccagacc gccaagctga aggtgaccaa 4140

gggcggcccc ctgcccttcg cctgggacat cctgtccccc cagttcatgt acggctccaa 4200

ggcgtacgtg aagcaccccg ccgacatccc cgattacaag aagctgtcct tccccgaggg 4260

cttcaagtgg gagcgcgtga tgaacttcga ggacggcggt ctggtgaccg tgacccagga 4320

ctcctccctg caggacggca cgctgatcta caaggtgaag atgcgcggca ccaacttccc 4380

ccccgacggc cccgtaatgc agaagaagac catgggctgg gaggcctcca ccgagcgcct 4440

gtacccccgc gacggcgtgc tgaagggcga gatccaccag gccctgaagc tgaaggacgg 4500

cggccactac ctggtggagt tcaagaccat ctacatggcc aagaagcccg tgcaactgcc 4560

cggctactac tacgtggaca ccaagctgga catcacctcc cacaacgagg actacaccat 4620

cgtggaacag tacgagcgct ccgagggccg ccaccacctg ttcctgtacg gcatggacga 4680

gctgtacaag tctagaggta ccggttgttg gcccatggga gcttggattg aacaagatgg 4740

attgcacgca ggttctccgg ccgcttgggt ggagaggcta ttcggctatg actgggcaca 4800

acagacaatc ggctgctctg atgccgccgt gttccggctg tcagcgcagg ggcgcccggt 4860

tctttttgtc aagaccgacc tgtccggtgc cctgaatgaa ctgcaggacg aggcagcgcg 4920

gctatcgtgg ctggccacga cgggcgttcc ttgcgcagct gtgctcgacg ttgtcactga 4980

agcgggaagg gactggctgc tattgggcga agtgccgggg caggatctcc tgtcatctca 5040

ccttgctcct gccgagaaag tatccatcat ggctgatgca atgcggcggc tgcatacgct 5100

tgatccggct acctgcccat tcgaccacca agcgaaacat cgcatcgagc gagcacgtac 5160

tcggatggaa gccggtcttg tcgatcagga tgatctggac gaagagcatc aggggctcgc 5220

gccagccgaa ctgttcgcca ggctcaaggc gcgcatgccc gacggcgagg atctcgtcgt 5280

gacccatggc gatgcctgct tgccgaatat catggtggaa aatggccgct tttctggatt 5340

catcgactgt ggccggctgg gtgtggcgga ccgctatcag gacatagcgt tggctacccg 5400

tgatattgct gaagagcttg gcggcgaatg ggctgaccgc ttcctcgtgc tttacggtat 5460

cgccgctccc gattcgcagc gcatcgcctt ctatcgcctt cttgacgagt tcttctgagc 5520

gggacccaag ctaggccatc gatacgcgta cgtcgcgacc gcggacatgt acagagctcg 5580

agaagtacta gagtccgcaa aaatcaccag tctctctcta caaatctatc tctctctatt 5640

tttctccaga ataatgtgtg agtagttccc agataaggga attagggttc ttatagggtt 5700

tcgctcatgt gttgagcata taagaaaccc ttagtatgta tttgtatttg taaaatactt 5760

ctatcaataa aatttctaat tcctaaaacc aaaatccagt gacctgcagg catgca 5816

<210> 67
<211> 12928
<212> DNA
<213> Artificial Sequence

<220>
<223> pUbitDTnptII_LbCPF1_CrRNA_ALS_G/T vector

<400> 67
gatcctctca acacaacata tacaaaacaa acgaatctca agcaatcaag cattctactt 60

ctattgcagc aatttaaatc atttctttta aagcaaaagc aattttctga aaattttcac 120

catttacgaa cgatagggcg cgatcccgcc accatggtga gcaagggcga ggaggtcatc 180

aaagagttca tgcgcttcaa ggtgcgcatg gagggctcca tgaacggcca cgagttcgag 240

atcgagggcg agggcgaggg ccgcccctac gagggcaccc agaccgccaa gctgaaggtg 300

accaagggcg gccccctgcc cttcgcctgg gacatcctgt ccccccagtt catgtacggc 360

tccaaggcgt acgtgaagca ccccgccgac atccccgatt acaagaagct gtccttcccc 420

gagggcttca agtgggagcg cgtgatgaac ttcgaggacg gcggtctggt gaccgtgacc 480

caggactcct ccctgcagga cggcacgctg atctacaagg tgaagatgcg cggcaccaac 540

ttcccccccg acggccccgt aatgcagaag aagaccatgg gctgggaggc ctccaccgag 600

cgcctgtacc cccgcgacgg cgtgctgaag ggcgagatcc accaggccct gaagctgaag 660

gacggcggcc actacctggt ggagttcaag accatctaca tggccaagaa gcccgtgcaa 720

ctgcccggct actactacgt ggacaccaag ctggacatca cctcccacaa cgaggactac 780

accatcgtgg aacagtacga gcgctccgag ggccgccacc acctgttcct gtacggcatg 840

gacgagctgt acaagtctag aggtaccggt tgttggccca tgggagcttg gattgaacaa 900

gatggattgc acgcaggttc tccggccgct tgggtggaga ggctattcgg ctatgactgg 960

gcacaacaga caatcggctg ctctgatgcc gccgtgttcc ggctgtcagc gcaggggcgc 1020

ccggttcttt ttgtcaagac cgacctgtcc ggtgccctga atgaactgca ggacgaggca 1080

gcgcggctat cgtggctggc cacgacgggc gttccttgcg cagctgtgct cgacgttgtc 1140

actgaagcgg gaagggactg gctgctattg ggcgaagtgc cggggcagga tctcctgtca 1200

tctcaccttg ctcctgccga gaaagtatcc atcatggctg atgcaatgcg gcggctgcat 1260

acgcttgatc cggctacctg cccattcgac caccaagcga aacatcgcat cgagcgagca 1320

cgtactcgga tggaagccgg tcttgtcgat caggatgatc tggacgaaga gcatcagggg 1380

ctcgcgccag ccgaactgtt cgccaggctc aaggcgcgca tgcccgacgg cgaggatctc 1440

gtcgtgaccc atggcgatgc ctgcttgccg aatatcatgg tggaaaatgg ccgcttttct 1500

ggattcatcg actgtggccg gctgggtgtg gcggaccgct atcaggacat agcgttggct 1560

acccgtgata ttgctgaaga gcttggcggc gaatgggctg accgcttcct cgtgctttac 1620

ggtatcgccg ctcccgattc gcagcgcatc gccttctatc gccttcttga cgagttcttc 1680

tgagcgggac ccaagctagg ccatcgatac gcgtacgtcg cgaccgcgga catgtacaga 1740

gctcgagaag tactagagtc cgcaaaaatc accagtctct ctctacaaat ctatctctct 1800

ctatttttct ccagaataat gtgtgagtag ttcccagata agggaattag ggttcttata 1860

gggtttcgct catgtgttga gcatataaga aacccttagt atgtatttgt atttgtaaaa 1920
tacttctatc aataaaattt ctaattccta aaaccaaaat ccagtgacct gcaggcatgc 1980
aagcttatcg atgtcgacag gccttaaggg ccagatcccc cgggctgcag aagcctatac 2040
tgtacttaac ttgattgcat aattacttga tcatagactc atagtaaact tgattacaca 2100
gataagtgaa gaaacaaacc aattcaagac ataaccaaag agaggtgaaa gactgtttta 2160
tatgtctaac attgcacctt aatatcacac tgttagttcc tttcttactt aaattcaacc 2220
cattaaagta aaaacaacag ataataataa tttgagaatg aacaaaagga ccatatcatt 2280
tattaactct tatccatcca tttgcatttt gatgtccgaa aacaaaaact gaaagaacac 2340
agtaaattac aagcagaaca aatgatagaa gaaaacagct tttccaatgc cataatactc 2400
aaacttagta ggattctggt gtgtgggcaa tgaaactgat gcattgaact tgacgaacgt 2460
tgtcgaaacc gatgatacgg acgaaagctg ggaggcctgg tcccattcgc catgccgaag 2520
catgttgccc agccggcgcc agcgaggagg ctgggaccat gccggcctag aagggtaacc 2580
tgttggtatg gatctacact tagtagaaat taatctacac ttagtagaaa ttagacgagc 2640
ttactcgttt cgtcctcacg gactcatcag taatttggat ccgctgcaca tacataacat 2700
atcaagatca gaacacacat atacacacac aaatacaatc aagtcaacaa ctccaaaaag 2760
tccagatcta catatataca tacgtaaata acaaaatcat gtaaataatc acaatcatgt 2820
aatccagatc tatgcacata tatatataca caattaataa aaaaaatgat ataacagatc 2880
tatatctatg tatgtaacaa cacaatcaga tgagagaagt gatgttttca gatctgtata 2940
catacaaaca caaacagatg aacaattgat acgtagatcc atatgtatac gtacaattag 3000
ctacacgatt aaatgaaaaa aatcaacgat ttcggattgg tacacacaaa cgcaacaata 3060
tgaagaaatt catatctgat tagatataaa cataaccacg tgtagataca cagtcaaatc 3120
aacaaattta tagcttctaa acggatgaga tgaacaagat aaagatattc acataaggca 3180
tacataagat aagcagatta acaaactagc aataatacat acctaattaa aacaaggaat 3240
aacagagaga gagagagaga gagagattta ccttgaaaat gaagaggaga agagaggatt 3300
tcttaaaatt gggggtagag aaagaaagat gatgaattgt gagaaaggag agatagaagg 3360
gggggttgta tatataggct gtagaagatt atttttgtgt ttgaggcggt gaaggaagag 3420
gggatctgac tatgacacgt ttgcggttac gtatttcgat aggagtcttt caacgcttaa 3480
cgccgttact ctatatgacc gtttgggccg taacggggcc gtttgttaac gctgatgttg 3540
attcttttct ttctttcttt cttccttttt taaagaagca attgtacaat cgttgctagc 3600
tgtcaaacgg ataattcgga tacggatatg cctatattca tatccgtaat ttttctgcag 3660
gaattcgatc tggcacgaca ggtttcccga ctggaaagcg ggcagtgagc gcaacgcaat 3720
taatgtgagt tagctcactc attaggcacc ccaggcttta cactttatgc ttccggctcg 3780

tatgttgtgt ggaattgtga gcggataaca atttcacaca ggaaacagct atgacatgat 3840

tacgaattca aaaattacgg atatgaatat aggcatatcc gtatccgaat tatccgtttg 3900

acagctagca acgattgtac aattgcttct ttaaaaaagg aagaaagaaa gaaagaaaag 3960

aatcaacatc agcgttaaca aacggccccg ttacggccca aacggtcata tagagtaacg 4020

gcgttaagcg ttgaaagact cctatcgaaa tacgtaaccg caaacgtgtc atagtcagat 4080

cccctcttcc ttcaccgcct caaacacaaa aataatcttc tacagcctat atatacaacc 4140

cccccttcta tctctccttt ctcacaattc atcatctttc tttctctacc cccaatttta 4200

agaaatcctc tcttctcctc ttcattttca aggtaaatct ctctctctct ctctctctct 4260

gttattcctt gttttaatta ggtatgtatt attgctagtt tgttaatctg cttatcttat 4320

gtatgcctta tgtgaatatc tttatcttgt tcatctcatc cgtttagaag ctataaattt 4380

gttgatttga ctgtgtatct acacgtggtt atgtttatat ctaatcagat atgaatttct 4440

tcatattgtt gcgtttgtgt gtaccaatcc gaaatcgttg atttttttca tttaatcgtg 4500

tagctaattg tacgtataca tatggatcta cgtatcaatt gttcatctgt ttgtgtttgt 4560

atgtatacag atctgaaaac atcacttctc tcatctgatt gtgttgttac atacatagat 4620

atagatctgt tatatcattt tttttattaa ttgtgtatat atatatgtgc atagatctgg 4680

attacatgat tgtgattatt tacatgattt tgttatttac gtatgtatat atgtagatct 4740

ggactttttg gagttgttga cttgattgta tttgtgtgtg tatatgtgtg ttctgatctt 4800

gatatgttat gtatgtgcag cgaattcggc gcgccatggc tcctaagaag aagaggaagg 4860

ttagcaagct cgagaagttt accaactgct acagcctctc taagaccctc aggttcaagg 4920

ctatccctgt gggaaagacc caagagaata tcgacaacaa gaggctcctc gtcgaggatg 4980

agaagagagc tgaagattac aagggcgtga agaagctcct cgacaggtac tacctcagct 5040

tcatcaacga tgtgctccac agcatcaagc tcaagaacct caacaactac atcagcctct 5100

tccgtaagaa aaccaggacc gagaaagaga acaaagagct tgagaacctc gagatcaacc 5160

tccgtaaaga gatcgccaag gctttcaagg gaaacgaggg atacaagagc ctcttcaaga 5220

aggatattat cgagacaatc ctgcctgagt tcctggacga taaggatgag atcgctctcg 5280

tgaacagctt caacggattc actactgcct tcaccggatt cttcgacaac agggaaaaca 5340

tgttcagcga agaggccaag agcacctcta tcgctttcag atgcatcaac gagaacctca 5400

cgcgttacat cagcaacatg gacatcttcg agaaggtgga cgccatcttc gataagcacg 5460

aggtgcaaga aatcaaagag aagatcctca acagcgacta cgacgtcgag gacttttttg 5520

aaggggagtt cttcaacttc gttctcaccc aagagggcat cgacgtgtac aacgctatta 5580

tcggaggatt cgtgaccgag tctggggaga agattaaggg actcaacgag tacatcaacc 5640

tgtacaacca gaaaacgaag cagaagctcc cgaagttcaa gccgctctac aagcaggttc 5700

tctctgatcg tgagagcctc tcattttacg gtgagggtta cacctctgac gaggaagtgc 5760

ttgaggtttt ccgtaacacc ctcaacaaga acagcgagat cttctcgtcc atcaagaagt 5820

tggagaaact tttcaagaac ttcgacgagt acagcagcgc tgggatcttc gttaagaacg 5880

gacctgctat cagcaccatc agcaaggata ttttcggcga gtggaacgtg atcagggaca 5940

agtggaatgc tgagtacgat gacatccacc tcaagaagaa ggctgtcgtc actgagaagt 6000

acgaggatga caggcgtaag tcgttcaaga agatcggctc tttcagcctc gagcagcttc 6060

aagaatacgc tgatgctgat ctcagcgtgg tcgagaagct caaagagatc atcatccaga 6120

aggtcgacga gatctacaag gtgtacgggt cctctgagaa gttgttcgat gctgatttcg 6180

tcctcgagaa gagtctgaag aagaacgacg ctgtcgtcgc gatcatgaag gatttgctcg 6240

acagcgtgaa gtccttcgag aactatatca aggccttctt cggagagggc aaagagacta 6300

atagggacga gtctttctac ggggatttcg tgctcgctta cgatatcctc ctcaaggtgg 6360

accatatcta cgacgccatc agaaactacg tgacccagaa gccttacagc aaggacaagt 6420

tcaagttgta ctttcagaac ccgcagttca tgggcggatg ggacaaagac aaagagacag 6480

attacagggc caccatcctc aggtacgggt ctaagtacta cctggccatc atggacaaga 6540

aatacgccaa gtgcctccaa aagatcgaca aggatgacgt gaacggaaac tatgagaaga 6600

tcaactacaa gctccttccg ggaccgaaca agatgcttcc taaggtgttc ttcagcaaga 6660

aatggatggc ctactacaac ccgtctgagg acatccagaa aatctacaag aacgggacct 6720

tcaagaaagg cgacatgttc aacctcaacg actgccacaa gctcatcgat ttcttcaagg 6780

acagcatctc gcgttacccg aagtggtcta acgcttacga ctttaacttc agcgagacag 6840

aaaagtacaa ggatatcgcc gggttctacc gtgaggttga ggaacagggt tacaaggtta 6900

gcttcgagag cgcctccaag aaagaggttg acaagttggt cgaagagggc aagctctaca 6960

tgttccagat ctataacaag gacttctccg acaagagcca cggaactcct aacctccata 7020

cgatgtactt caagctgctt ttcgacgaga acaaccacgg gcagatcaga ctttctggtg 7080

gtgctgaact cttcatgcgt agggcctcac tcaagaaaga agagttggtt gttcacccgg 7140

ccaactctcc aatcgctaac aagaatcctg acaacccgaa aaagaccacc acgctgtctt 7200

acgacgtcta caaggacaaa aggttcagcg aggaccagta cgagcttcat atcccgatcg 7260

ctatcaacaa gtgcccgaag aacatcttca agatcaatac cgaggtgagg gtgctgctca 7320

agcacgatga taacccttac gtgatcggaa tcgatcgtgg tgagagaaac ctcctctaca 7380

tcgttgtggt ggacggaaag ggaaacatcg tcgagcagta cagcctgaac gagattatca 7440

acaatttcaa cggcatcagg atcaagaccg actaccactc actcctcgat aagaaagaaa 7500

aagagcgttt cgaggccagg cagaactgga cttctatcga aaacatcaaa gagttgaagg 7560

ccggctacat ctctcaggtg gtgcataaga tctgcgagct ggtggaaaag tacgatgctg 7620

tgatcgctct tgaggacctc aactctgggt tcaagaacag tagagtgaag gttgagaagc 7680

aggtctacca aaagttcgag aagatgctca tcgacaagct caactacatg gtggacaaaa 7740

agagcaaccc ttgcgctacc ggtggtgctc ttaagggata ccagatcacg aacaagttcg 7800

agtccttcaa gagcatgagc acccagaacg gcttcatctt ctatatccct gcttggctca 7860

ccagcaagat cgatccttct actggtttcg tgaacctgct caagaccaag tacacctcga 7920

tcgccgacag caagaagttc atctcgtctt tcgacaggat catgtacgtg ccggaagagg 7980

atcttttcga gttcgctctc gactataaga acttcagcag gaccgacgcc gactacatta 8040

agaagtggaa gctctactcc tacgggaacc gtatcaggat cttccgaaat ccgaagaaaa 8100

acaacgtgtt cgactgggaa gaagtgtgcc tcacctctgc ctacaaagaa ctgttcaaca 8160

agtacggcat caactaccag cagggtgata tcagggctct tttgtgcgag cagagcgaca 8220

aggcattcta cagctcattc atggccctca tgtctctcat gctccagatg aggaactcta 8280

tcaccggaag gaccgatgtg gacttcctta tctctccggt caagaactct gacgggatct 8340

tctacgacag ccgtaactat gaggctcaag agaacgctat cctgccgaag aatgctgatg 8400

caaacggggc ttacaacatt gcgagaaagg ttctctgggc tatcgggcag tttaagaaag 8460

cggaagatga gaagctcgac aaggtgaaga tcgccatctc caacaaagag tggcttgagt 8520

acgctcagac ctccgttaag cacaagaggc ctgctgctac taagaaagct ggccaggcca 8580

aaaagaagaa gtgaggcgcg ccgagctcca ggcctcccag ctttcgtccg tatcatcggt 8640

ttcgacaacg ttcgtcaagt tcaatgcatc agtttcattg cccacacacc agaatcctac 8700

taagtttgag tattatggca ttggaaaagc tgttttcttc tatcatttgt tctgcttgta 8760

atttactgtg ttctttcagt ttttgttttc ggacatcaaa atgcaaatgg atggataaga 8820

gttaataaat gatatggtcc ttttgttcat tctcaaatta ttattatctg ttgtttttac 8880

tttaatgggt tgaatttaag taagaaagga actaacagtg tgatattaag gtgcaatgtt 8940

agacatataa aacagtcttt cacctctctt tggttatgtc ttgaattggt ttgtttcttc 9000

acttatctgt gtaatcaagt ttactatgag tctatgatca agtaattatg caatcaagtt 9060

aagtacagta taggcttgag ctccctagga tcaagctttc cctatagtga gtcgtattag 9120

agcttggcgt aatcatggtc atagctgttt cctgtgtgaa attgttatcc gctcacaatt 9180

ccacacaaca tacgagccgg aagcataaag tgtaaagcct ggggtgccta atgagtgagc 9240

taactcacat taattgcgtt gcgctcactg cccgctttcc agtcgggaaa cctgtcgtgc 9300

cagctgcatt aatgaatcgg ccaacgcgcg gggagaggcg gtttgcgtat tgggcgctct 9360

tccgcttcct cgctcactga ctcgctgcgc tcggtcgttc ggctgcggcg agcggtatca 9420

gctcactcaa aggcggtaat acggttatcc acagaatcag gggataacgc aggaaagaac 9480

atgtgagcaa aaggccagca aaaggccagg aaccgtaaaa aggccgcgtt gctggcgttt 9540

ttccataggc tccgcccccc tgacgagcat cacaaaaatc gacgctcaag tcagaggtgg 9600

cgaaacccga caggactata aagataccag gcgtttcccc ctggaagctc cctcgtgcgc 9660

tctcctgttc cgaccctgcc gcttaccgga tacctgtccg cctttctccc ttcgggaagc 9720

gtggcgcttt ctcaatgctc acgctgtagg tatctcagtt cggtgtaggt cgttcgctcc 9780

aagctgggct gtgtgcacga accccccgtt cagcccgacc gctgcgcctt atccggtaac 9840

tatcgtcttg agtccaaccc ggtaagacac gacttatcgc cactggcagc agccactggt 9900

aacaggatta gcagagcgag gtatgtaggc ggtgctacag agttcttgaa gtggtggcct 9960

aactacggct acactagaag gacagtattt ggtatctgcg ctctgctgaa gccagttacc 10020

ttcggaaaaa gagttggtag ctcttgatcc ggcaaacaaa ccaccgctgg tagcggtggt 10080

ttttttgttt gcaagcagca gattacgcgc agaaaaaaag gatctcaaga agatcctttg 10140

atcttttcta cggggtctga cgctcagtgg aacgaaaact cacgttaagg gattttggtc 10200

atgagattat caaaaaggat cttcacctag atccttttaa attaaaaatg aagttttaaa 10260

tcaatctaaa gtatatatga gtaaacttgg tctgacagtt accaatgctt aatcagtgag 10320

gcacctatct cagcgatctg tctatttcgt tcatccatag ttgcctgact ccccgtcgtg 10380

tagataacta cgatacggga gggcttacca tctggcccca gtgctgcaat gataccgcga 10440

gacccacgct gaccggctcc agatttatca gcaataaacc agccagccgg aagggccgag 10500

cgcagaagtg gtcctgcaac tttatccgcc tccatccagt ctattaattg ttgccgggaa 10560

gctagagtaa gtagttcgcc agttaatagt ttgcgcaacg ttgttgccat tgctacaggc 10620

atcgtggtgt cacgctcgtc gtttggtatg gcttcactca gctccggttc ccaacgatca 10680

aggcgagtta catgatcccc catgttgtgc aaaaaagcgg ttagctcctt cggtcctccg 10740

atcgttgtca gaagtaagtt ggccgcagtg ttatcactca tggttatggc agcactgcat 10800

aattctctta ctgtcatgcc atccgtaaga tgcttttctg tgactggtga gtactcaacc 10860

aagtcattct gagaatagtg tatgcggcga ccgagttgct cttgcccggc gtcaatacgg 10920

gataataccg cgccacatag cagaacttta aaagtgctca tcattggaaa acgttcttcg 10980

gggcgaaaac tctcaaggat cttaccgctg ttgagatcca gttcgatgta acccactcgt 11040

gcacccaact gatcttcagc atcttttact ttcaccagcg tttctgggtg agcaaaaaca 11100

ggaaggcaaa atgccgcaaa aaagggaata agggcgacac ggaaatgttg aatactcata 11160

ctcttccttt ttcaatatta ttgaagcatt tatcagggtt attgtctcat gagcggatac 11220

atatttgaat gtatttagaa aaataaagaa ataggggttc tgcgcacatt tccccgaaaa 11280

gtgccacctg aaattgtaaa cgttaatgtt ttgttaaatt tcgcgttaaa tatttgttaa 11340

atcagcttat tttttaacca gtaagcagaa aatgacaaaa atccttataa atcaaaagaa 11400

tagaccgagt tagttgtgag tgttgttcca gtttggaaca agagtccact attaaagaac 11460

gtggactcca acgtaaaacc gtctatcagg gcgatggccc actacgtgaa ccatcaccca 11520

aatcaagttt ttggaggtcg aggtgccgta aagcactaaa tcggaaccct aaagggagcc 11580

cccgatttag agcttgacgg ggaaagccgg cgaacgtggc gagaaaggaa gggaagaaag 11640

cgaaaggagc gggcgctagg gcgctggcaa gtgtagcggt cacgctgcgc gtaaccacca 11700

cacccgccgc gcttaatgcg ccgctactgg gcgcgtccca ttcgccattc aggctgcgca 11760

actgttggga agggcgatcg gtgcgggcct cttcgctatt acgccagctg gcgaaagggg 11820

gatgtgctgc aaggcgatta agttgggtaa cgccagggtt ttcccagtca cgacgttgta 11880

aaacgacggc cagtgaatta tcgattaaat taaccctcac taaagggaga gacgaattcg 11940

aatccaaaaa ttacggatat gaatataggc atatccgtat ccgaattatc cgtttgacag 12000

ctagcaacga ttgtacaatt gcttctttaa aaaaggaaga aagaaagaaa gaaaagaatc 12060

aacatcagcg ttaacaaacg gccccgttac ggcccaaacg gtcatataga gtaacggcgt 12120

taagcgttga aagactccta tcgaaatacg taaccgcaaa cgtgtcatag tcagatcccc 12180

tcttccttca ccgcctcaaa cacaaaaata atcttctaca gcctatatat acaacccccc 12240

cttctatctc tcctttctca caattcatca tctttctttc tctaccccca attttaagaa 12300

atcctctctt ctcctcttca ttttcaaggt aaatctctct ctctctctct ctctctgtta 12360

ttccttgttt taattaggta tgtattattg ctagtttgtt aatctgctta tcttatgtat 12420

gccttatgtg aatatcttta tcttgttcat ctcatccgtt tagaagctat aaatttgttg 12480

atttgactgt gtatctacac gtggttatgt ttatatctaa tcagatatga atttcttcat 12540

attgttgcgt ttgtgtgtac caatccgaaa tcgttgattt ttttcattta atcgtgtagc 12600

taattgtacg tatacatatg gatctacgta tcaattgttc atctgtttgt gtttgtatgt 12660

atacagatct gaaaacatca cttctctcat ctgattgtgt tgttacatac atagatatag 12720

atctgttata tcattttttt tattaattgt gtatatatat atgtgcatag atctggatta 12780

catgattgtg attatttaca tgattttgtt atttacgtat gtatatatgt agatctggac 12840

tttttggagt tgttgacttg attgtatttg tgtgtgtata tgtgtgttct gatcttgata 12900

tgttatgtat gtgcagctga accctcga 12928

<210> 68
<211> 12928
<212> DNA
<213> Artificial Sequence

<220>
<223> pUbitDTnptII_LbCpf1_ crRNA _ ALS_In1 vector

<400> 68
   gatcctctca acacaacata tacaaaacaa acgaatctca agcaatcaag cattctactt 60
   ctattgcagc aatttaaatc atttctttta aagcaaaagc aattttctga aaattttcac 120
   catttacgaa cgatagggcg cgatcccgcc accatggtga gcaagggcga ggaggtcatc 180
   aaagagttca tgcgcttcaa ggtgcgcatg gagggctcca tgaacggcca cgagttcgag 240
   atcgagggcg agggcgaggg ccgcccctac gagggcaccc agaccgccaa gctgaaggtg 300
   accaagggcg gccccctgcc cttcgcctgg gacatcctgt ccccccagtt catgtacggc 360
   tccaaggcgt acgtgaagca ccccgccgac atccccgatt acaagaagct gtccttcccc 420
   gagggcttca agtgggagcg cgtgatgaac ttcgaggacg gcggtctggt gaccgtgacc 480
   caggactcct ccctgcagga cggcacgctg atctacaagg tgaagatgcg cggcaccaac 540
   ttcccccccg acggccccgt aatgcagaag aagaccatgg gctgggaggc ctccaccgag 600
   cgcctgtacc cccgcgacgg cgtgctgaag ggcgagatcc accaggccct gaagctgaag 660
   gacggcggcc actacctggt ggagttcaag accatctaca tggccaagaa gcccgtgcaa 720
   ctgcccggct actactacgt ggacaccaag ctggacatca cctcccacaa cgaggactac 780
   accatcgtgg aacagtacga gcgctccgag ggccgccacc acctgttcct gtacggcatg 840
   gacgagctgt acaagtctag aggtaccggt tgttggccca tgggagcttg gattgaacaa 900
   gatggattgc acgcaggttc tccggccgct tgggtggaga ggctattcgg ctatgactgg 960
   gcacaacaga caatcggctg ctctgatgcc gccgtgttcc ggctgtcagc gcaggggcgc 1020
   ccggttcttt ttgtcaagac cgacctgtcc ggtgccctga atgaactgca ggacgaggca 1080
   gcgcggctat cgtggctggc cacgacgggc gttccttgcg cagctgtgct cgacgttgtc 1140
   actgaagcgg gaagggactg gctgctattg ggcgaagtgc cggggcagga tctcctgtca 1200
   tctcaccttg ctcctgccga gaaagtatcc atcatggctg atgcaatgcg gcggctgcat 1260
   acgcttgatc cggctacctg cccattcgac caccaagcga aacatcgcat cgagcgagca 1320
   cgtactcgga tggaagccgg tcttgtcgat caggatgatc tggacgaaga gcatcagggg 1380
   ctcgcgccag ccgaactgtt cgccaggctc aaggcgcgca tgcccgacgg cgaggatctc 1440
   gtcgtgaccc atggcgatgc ctgcttgccg aatatcatgg tggaaaatgg ccgcttttct 1500
   ggattcatcg actgtggccg gctgggtgtg gcggaccgct atcaggacat agcgttggct 1560
   acccgtgata ttgctgaaga gcttggcggc gaatgggctg accgcttcct cgtgctttac 1620
   ggtatcgccg ctcccgattc gcagcgcatc gccttctatc gccttcttga cgagttcttc 1680
   tgagcgggac ccaagctagg ccatcgatac gcgtacgtcg cgaccgcgga catgtacaga 1740
   gctcgagaag tactagagtc cgcaaaaatc accagtctct ctctacaaat ctatctctct 1800
   ctatttttct ccagaataat gtgtgagtag ttcccagata agggaattag ggttcttata 1860
   gggtttcgct catgtgttga gcatataaga aacccttagt atgtatttgt atttgtaaaa 1920
   tacttctatc aataaaattt ctaattccta aaaccaaaat ccagtgacct gcaggcatgc 1980
   aagcttatcg atgtcgacag gccttaaggg ccagatcccc cgggctgcag aagcctatac 2040
   tgtacttaac ttgattgcat aattacttga tcatagactc atagtaaact tgattacaca 2100
   gataagtgaa gaaacaaacc aattcaagac ataaccaaag agaggtgaaa gactgtttta 2160
   tatgtctaac attgcacctt aatatcacac tgttagttcc tttcttactt aaattcaacc 2220
   cattaaagta aaaacaacag ataataataa tttgagaatg aacaaaagga ccatatcatt 2280
   tattaactct tatccatcca tttgcatttt gatgtccgaa aacaaaaact gaaagaacac 2340
   agtaaattac aagcagaaca aatgatagaa gaaaacagct tttccaatgc cataatactc 2400
   aaacttagta ggattctggt gtgtgggcaa tgaaactgat gcattgaact tgacgaacgt 2460
   tgtcgaaacc gatgatacgg acgaaagctg ggaggcctgg tcccattcgc catgccgaag 2520
   catgttgccc agccggcgcc agcgaggagg ctgggaccat gccggccact acgttgtgtt 2580
   gtaggtatcg gatctacact tagtagaaat taatctacac ttagtagaaa ttagacgagc 2640
   ttactcgttt cgtcctcacg gactcatcag taatttggat ccgctgcaca tacataacat 2700
   atcaagatca gaacacacat atacacacac aaatacaatc aagtcaacaa ctccaaaaag 2760
   tccagatcta catatataca tacgtaaata acaaaatcat gtaaataatc acaatcatgt 2820
   aatccagatc tatgcacata tatatataca caattaataa aaaaaatgat ataacagatc 2880
   tatatctatg tatgtaacaa cacaatcaga tgagagaagt gatgttttca gatctgtata 2940
   catacaaaca caaacagatg aacaattgat acgtagatcc atatgtatac gtacaattag 3000
   ctacacgatt aaatgaaaaa aatcaacgat ttcggattgg tacacacaaa cgcaacaata 3060
   tgaagaaatt catatctgat tagatataaa cataaccacg tgtagataca cagtcaaatc 3120
   aacaaattta tagcttctaa acggatgaga tgaacaagat aaagatattc acataaggca 3180
   tacataagat aagcagatta acaaactagc aataatacat acctaattaa aacaaggaat 3240
   aacagagaga gagagagaga gagagattta ccttgaaaat gaagaggaga agagaggatt 3300
   tcttaaaatt gggggtagag aaagaaagat gatgaattgt gagaaaggag agatagaagg 3360
   gggggttgta tatataggct gtagaagatt atttttgtgt ttgaggcggt gaaggaagag 3420
   gggatctgac tatgacacgt ttgcggttac gtatttcgat aggagtcttt caacgcttaa 3480
   cgccgttact ctatatgacc gtttgggccg taacggggcc gtttgttaac gctgatgttg 3540
   attcttttct ttctttcttt cttccttttt taaagaagca attgtacaat cgttgctagc 3600
   tgtcaaacgg ataattcgga tacggatatg cctatattca tatccgtaat ttttctgcag 3660
   gaattcgatc tggcacgaca ggtttcccga ctggaaagcg ggcagtgagc gcaacgcaat 3720
   taatgtgagt tagctcactc attaggcacc ccaggcttta cactttatgc ttccggctcg 3780
   tatgttgtgt ggaattgtga gcggataaca atttcacaca ggaaacagct atgacatgat 3840
   tacgaattca aaaattacgg atatgaatat aggcatatcc gtatccgaat tatccgtttg 3900
   acagctagca acgattgtac aattgcttct ttaaaaaagg aagaaagaaa gaaagaaaag 3960
   aatcaacatc agcgttaaca aacggccccg ttacggccca aacggtcata tagagtaacg 4020
   gcgttaagcg ttgaaagact cctatcgaaa tacgtaaccg caaacgtgtc atagtcagat 4080
   cccctcttcc ttcaccgcct caaacacaaa aataatcttc tacagcctat atatacaacc 4140
   cccccttcta tctctccttt ctcacaattc atcatctttc tttctctacc cccaatttta 4200
   agaaatcctc tcttctcctc ttcattttca aggtaaatct ctctctctct ctctctctct 4260
   gttattcctt gttttaatta ggtatgtatt attgctagtt tgttaatctg cttatcttat 4320
   gtatgcctta tgtgaatatc tttatcttgt tcatctcatc cgtttagaag ctataaattt 4380
   gttgatttga ctgtgtatct acacgtggtt atgtttatat ctaatcagat atgaatttct 4440
   tcatattgtt gcgtttgtgt gtaccaatcc gaaatcgttg atttttttca tttaatcgtg 4500
   tagctaattg tacgtataca tatggatcta cgtatcaatt gttcatctgt ttgtgtttgt 4560
   atgtatacag atctgaaaac atcacttctc tcatctgatt gtgttgttac atacatagat 4620
   atagatctgt tatatcattt tttttattaa ttgtgtatat atatatgtgc atagatctgg 4680
   attacatgat tgtgattatt tacatgattt tgttatttac gtatgtatat atgtagatct 4740
   ggactttttg gagttgttga cttgattgta tttgtgtgtg tatatgtgtg ttctgatctt 4800
   gatatgttat gtatgtgcag cgaattcggc gcgccatggc tcctaagaag aagaggaagg 4860
   ttagcaagct cgagaagttt accaactgct acagcctctc taagaccctc aggttcaagg 4920
   ctatccctgt gggaaagacc caagagaata tcgacaacaa gaggctcctc gtcgaggatg 4980
   agaagagagc tgaagattac aagggcgtga agaagctcct cgacaggtac tacctcagct 5040
   tcatcaacga tgtgctccac agcatcaagc tcaagaacct caacaactac atcagcctct 5100
   tccgtaagaa aaccaggacc gagaaagaga acaaagagct tgagaacctc gagatcaacc 5160
   tccgtaaaga gatcgccaag gctttcaagg gaaacgaggg atacaagagc ctcttcaaga 5220
   aggatattat cgagacaatc ctgcctgagt tcctggacga taaggatgag atcgctctcg 5280
   tgaacagctt caacggattc actactgcct tcaccggatt cttcgacaac agggaaaaca 5340
   tgttcagcga agaggccaag agcacctcta tcgctttcag atgcatcaac gagaacctca 5400
   cgcgttacat cagcaacatg gacatcttcg agaaggtgga cgccatcttc gataagcacg 5460
   aggtgcaaga aatcaaagag aagatcctca acagcgacta cgacgtcgag gacttttttg 5520
   aaggggagtt cttcaacttc gttctcaccc aagagggcat cgacgtgtac aacgctatta 5580
   tcggaggatt cgtgaccgag tctggggaga agattaaggg actcaacgag tacatcaacc 5640
   tgtacaacca gaaaacgaag cagaagctcc cgaagttcaa gccgctctac aagcaggttc 5700
   tctctgatcg tgagagcctc tcattttacg gtgagggtta cacctctgac gaggaagtgc 5760
   ttgaggtttt ccgtaacacc ctcaacaaga acagcgagat cttctcgtcc atcaagaagt 5820
   tggagaaact tttcaagaac ttcgacgagt acagcagcgc tgggatcttc gttaagaacg 5880
   gacctgctat cagcaccatc agcaaggata ttttcggcga gtggaacgtg atcagggaca 5940
   agtggaatgc tgagtacgat gacatccacc tcaagaagaa ggctgtcgtc actgagaagt 6000
   acgaggatga caggcgtaag tcgttcaaga agatcggctc tttcagcctc gagcagcttc 6060
   aagaatacgc tgatgctgat ctcagcgtgg tcgagaagct caaagagatc atcatccaga 6120
   aggtcgacga gatctacaag gtgtacgggt cctctgagaa gttgttcgat gctgatttcg 6180
   tcctcgagaa gagtctgaag aagaacgacg ctgtcgtcgc gatcatgaag gatttgctcg 6240
   acagcgtgaa gtccttcgag aactatatca aggccttctt cggagagggc aaagagacta 6300
   atagggacga gtctttctac ggggatttcg tgctcgctta cgatatcctc ctcaaggtgg 6360
   accatatcta cgacgccatc agaaactacg tgacccagaa gccttacagc aaggacaagt 6420
   tcaagttgta ctttcagaac ccgcagttca tgggcggatg ggacaaagac aaagagacag 6480
   attacagggc caccatcctc aggtacgggt ctaagtacta cctggccatc atggacaaga 6540
   aatacgccaa gtgcctccaa aagatcgaca aggatgacgt gaacgggaac tatgagaaga 6600
   tcaactacaa gctccttccg ggaccgaaca agatgcttcc taaggtgttc ttcagcaaga 6660
   aatggatggc ctactacaac ccgtctgagg acatccagaa aatctacaag aacgggacct 6720
   tcaagaaagg cgacatgttc aacctcaacg actgccacaa gctcatcgat ttcttcaagg 6780
   acagcatctc gcgttacccg aagtggtcta acgcttacga ctttaacttc agcgagacag 6840
   aaaagtacaa ggatatcgcc gggttctacc gtgaggttga ggaacagggt tacaaggtta 6900
   gcttcgagag cgcctccaag aaagaggttg acaagttggt cgaagagggc aagctctaca 6960
   tgttccagat ctataacaag gacttctccg acaagagcca cggaactcct aacctccata 7020
   cgatgtactt caagctgctt ttcgacgaga acaaccacgg gcagatcaga ctttctggtg 7080
   gtgctgaact cttcatgcgt agggcctcac tcaagaaaga agagttggtt gttcacccgg 7140
   ccaactctcc aatcgctaac aagaatcctg acaacccgaa aaagaccacc acgctgtctt 7200
   acgacgtcta caaggacaaa aggttcagcg aggaccagta cgagcttcat atcccgatcg 7260
   ctatcaacaa gtgcccgaag aacatcttca agatcaatac cgaggtgagg gtgctgctca 7320
   agcacgatga taacccttac gtgatcggaa tcgatcgtgg tgagagaaac ctcctctaca 7380
   tcgttgtggt ggacggaaag ggaaacatcg tcgagcagta cagcctgaac gagattatca 7440
   acaatttcaa cggcatcagg atcaagaccg actaccactc actcctcgat aagaaagaaa 7500
   aagagcgttt cgaggccagg cagaactgga cttctatcga aaacatcaaa gagttgaagg 7560
   ccggctacat ctctcaggtg gtgcataaga tctgcgagct ggtggaaaag tacgatgctg 7620
   tgatcgctct tgaggacctc aactctgggt tcaagaacag tagagtgaag gttgagaagc 7680
   aggtctacca aaagttcgag aagatgctca tcgacaagct caactacatg gtggacaaaa 7740
   agagcaaccc ttgcgctacc ggtggtgctc ttaagggata ccagatcacg aacaagttcg 7800
   agtccttcaa gagcatgagc acccagaacg gcttcatctt ctatatccct gcttggctca 7860
   ccagcaagat cgatccttct actggtttcg tgaacctgct caagaccaag tacacctcga 7920
   tcgccgacag caagaagttc atctcgtctt tcgacaggat catgtacgtg ccggaagagg 7980
   atcttttcga gttcgctctc gactataaga acttcagcag gaccgacgcc gactacatta 8040
   agaagtggaa gctctactcc tacgggaacc gtatcaggat cttccgaaat ccgaagaaaa 8100
   acaacgtgtt cgactgggaa gaagtgtgcc tcacctctgc ctacaaagaa ctgttcaaca 8160
   agtacggcat caactaccag cagggtgata tcagggctct tttgtgcgag cagagcgaca 8220
   aggcattcta cagctcattc atggccctca tgtctctcat gctccagatg aggaactcta 8280
   tcaccggaag gaccgatgtg gacttcctta tctctccggt caagaactct gacgggatct 8340
   tctacgacag ccgtaactat gaggctcaag agaacgctat cctgccgaag aatgctgatg 8400
   caaacggggc ttacaacatt gcgagaaagg ttctctgggc tatcggccag tttaagaaag 8460
   cggaagatga gaagctcgac aaggtgaaga tcgccatctc caacaaagag tggcttgagt 8520
   acgctcagac ctccgttaag cacaagaggc ctgctgctac taagaaagct ggccaggcca 8580
   aaaagaagaa gtgaggcgcg ccgagctcca ggcctcccag ctttcgtccg tatcatcggt 8640
   ttcgacaacg ttcgtcaagt tcaatgcatc agtttcattg cccacacacc agaatcctac 8700
   taagtttgag tattatggca ttggaaaagc tgttttcttc tatcatttgt tctgcttgta 8760
   atttactgtg ttctttcagt ttttgttttc ggacatcaaa atgcaaatgg atggataaga 8820
   gttaataaat gatatggtcc ttttgttcat tctcaaatta ttattatctg ttgtttttac 8880
   tttaatgggt tgaatttaag taagaaagga actaacagtg tgatattaag gtgcaatgtt 8940
   agacatataa aacagtcttt cacctctctt tggttatgtc ttgaattggt ttgtttcttc 9000
   acttatctgt gtaatcaagt ttactatgag tctatgatca agtaattatg caatcaagtt 9060
   aagtacagta taggcttgag ctccctagga tcaagctttc cctatagtga gtcgtattag 9120
   agcttggcgt aatcatggtc atagctgttt cctgtgtgaa attgttatcc gctcacaatt 9180
   ccacacaaca tacgagccgg aagcataaag tgtaaagcct ggggtgccta atgagtgagc 9240
   taactcacat taattgcgtt gcgctcactg cccgctttcc agtcgggaaa cctgtcgtgc 9300
   cagctgcatt aatgaatcgg ccaacgcgcg gggagaggcg gtttgcgtat tgggcgctct 9360
   tccgcttcct cgctcactga ctcgctgcgc tcggtcgttc ggctgcggcg agcggtatca 9420
   gctcactcaa aggcggtaat acggttatcc acagaatcag gggataacgc aggaaagaac 9480
   atgtgagcaa aaggccagca aaaggccagg aaccgtaaaa aggccgcgtt gctggcgttt 9540
   ttccataggc tccgcccccc tgacgagcat cacaaaaatc gacgctcaag tcagaggtgg 9600
   cgaaacccga caggactata aagataccag gcgtttcccc ctggaagctc cctcgtgcgc 9660
   tctcctgttc cgaccctgcc gcttaccgga tacctgtccg cctttctccc ttcgggaagc 9720
   gtggcgcttt ctcaatgctc acgctgtagg tatctcagtt cggtgtaggt cgttcgctcc 9780
   aagctgggct gtgtgcacga accccccgtt cagcccgacc gctgcgcctt atccggtaac 9840
   tatcgtcttg agtccaaccc ggtaagacac gacttatcgc cactggcagc agccactggt 9900
   aacaggatta gcagagcgag gtatgtaggc ggtgctacag agttcttgaa gtggtggcct 9960
   aactacggct acactagaag gacagtattt ggtatctgcg ctctgctgaa gccagttacc 10020
   ttcggaaaaa gagttggtag ctcttgatcc ggcaaacaaa ccaccgctgg tagcggtggt 10080
   ttttttgttt gcaagcagca gattacgcgc agaaaaaaag gatctcaaga agatcctttg 10140
   atcttttcta cggggtctga cgctcagtgg aacgaaaact cacgttaagg gattttggtc 10200
   atgagattat caaaaaggat cttcacctag atccttttaa attaaaaatg aagttttaaa 10260
   tcaatctaaa gtatatatga gtaaacttgg tctgacagtt accaatgctt aatcagtgag 10320
   gcacctatct cagcgatctg tctatttcgt tcatccatag ttgcctgact ccccgtcgtg 10380
   tagataacta cgatacggga gggcttacca tctggcccca gtgctgcaat gataccgcga 10440
   gacccacgct gaccggctcc agatttatca gcaataaacc agccagccgg aagggccgag 10500
   cgcagaagtg gtcctgcaac tttatccgcc tccatccagt ctattaattg ttgccgggaa 10560
   gctagagtaa gtagttcgcc agttaatagt ttgcgcaacg ttgttgccat tgctacaggc 10620
   atcgtggtgt cacgctcgtc gtttggtatg gcttcactca gctccggttc ccaacgatca 10680
   aggcgagtta catgatcccc catgttgtgc aaaaaagcgg ttagctcctt cggtcctccg 10740
   atcgttgtca gaagtaagtt ggccgcagtg ttatcactca tggttatggc agcactgcat 10800
   aattctctta ctgtcatgcc atccgtaaga tgcttttctg tgactggtga gtactcaacc 10860
   aagtcattct gagaatagtg tatgcggcga ccgagttgct cttgcccggc gtcaatacgg 10920
   gataataccg cgccacatag cagaacttta aaagtgctca tcattggaaa acgttcttcg 10980
   gggcgaaaac tctcaaggat cttaccgctg ttgagatcca gttcgatgta acccactcgt 11040
   gcacccaact gatcttcagc atcttttact ttcaccagcg tttctgggtg agcaaaaaca 11100
   ggaaggcaaa atgccgcaaa aaagggaata agggcgacac ggaaatgttg aatactcata 11160
   ctcttccttt ttcaatatta ttgaagcatt tatcagggtt attgtctcat gagcggatac 11220
   atatttgaat gtatttagaa aaataaagaa ataggggttc tgcgcacatt tccccgaaaa 11280
   gtgccacctg aaattgtaaa cgttaatgtt ttgttaaatt tcgcgttaaa tatttgttaa 11340
   atcagcttat tttttaacca gtaagcagaa aatgacaaaa atccttataa atcaaaagaa 11400
   tagaccgagt tagttgtgag tgttgttcca gtttggaaca agagtccact attaaagaac 11460
   gtggactcca acgtaaaacc gtctatcagg gcgatggccc actacgtgaa ccatcaccca 11520
   aatcaagttt ttggaggtcg aggtgccgta aagcactaaa tcggaaccct aaagggagcc 11580
   cccgatttag agcttgacgg ggaaagccgg cgaacgtggc gagaaaggaa gggaagaaag 11640
   cgaaaggagc gggcgctagg gcgctggcaa gtgtagcggt cacgctgcgc gtaaccacca 11700
   cacccgccgc gcttaatgcg ccgctactgg gcgcgtccca ttcgccattc aggctgcgca 11760
   actgttggga agggcgatcg gtgcgggcct cttcgctatt acgccagctg gcgaaagggg 11820
   gatgtgctgc aaggcgatta agttgggtaa cgccagggtt ttcccagtca cgacgttgta 11880
   aaacgacggc cagtgaatta tcgattaaat taaccctcac taaagggaga gacgaattcg 11940
   aatccaaaaa ttacggatat gaatataggc atatccgtat ccgaattatc cgtttgacag 12000
   ctagcaacga ttgtacaatt gcttctttaa aaaaggaaga aagaaagaaa gaaaagaatc 12060
   aacatcagcg ttaacaaacg gccccgttac ggcccaaacg gtcatataga gtaacggcgt 12120
   taagcgttga aagactccta tcgaaatacg taaccgcaaa cgtgtcatag tcagatcccc 12180
   tcttccttca ccgcctcaaa cacaaaaata atcttctaca gcctatatat acaacccccc 12240
   cttctatctc tcctttctca caattcatca tctttctttc tctaccccca attttaagaa 12300
   atcctctctt ctcctcttca ttttcaaggt aaatctctct ctctctctct ctctctgtta 12360
   ttccttgttt taattaggta tgtattattg ctagtttgtt aatctgctta tcttatgtat 12420
   gccttatgtg aatatcttta tcttgttcat ctcatccgtt tagaagctat aaatttgttg 12480
   atttgactgt gtatctacac gtggttatgt ttatatctaa tcagatatga atttcttcat 12540
   attgttgcgt ttgtgtgtac caatccgaaa tcgttgattt ttttcattta atcgtgtagc 12600
   taattgtacg tatacatatg gatctacgta tcaattgttc atctgtttgt gtttgtatgt 12660
   atacagatct gaaaacatca cttctctcat ctgattgtgt tgttacatac atagatatag 12720
   atctgttata tcattttttt tattaattgt gtatatatat atgtgcatag atctggatta 12780
   catgattgtg attatttaca tgattttgtt atttacgtat gtatatatgt agatctggac 12840
   tttttggagt tgttgacttg attgtatttg tgtgtgtata tgtgtgttct gatcttgata 12900
   tgttatgtat gtgcagctga accctcga 12928

<210> 69
<211> 12928
<212> DNA
<213> Artificial Sequence

<220>

<223>  pUbitDTnptII_LbCpf1_ crRNA _ ALS_In2 vector

<400>  69
gatcctctca acacaacata tacaaaacaa acgaatctca agcaatcaag cattctactt      60

ctattgcagc aatttaaatc atttctttta aagcaaaagc aattttctga aaattttcac      120

catttacgaa cgatagggcg cgatcccgcc accatggtga gcaagggcga ggaggtcatc      180

aaagagttca tgcgcttcaa ggtgcgcatg gagggctcca tgaacggcca cgagttcgag      240

atcgagggcg agggcgaggg ccgcccctac gagggcaccc agaccgccaa gctgaaggtg      300

accaagggcg gccccctgcc cttcgcctgg gacatcctgt ccccccagtt catgtacggc      360

tccaaggcgt acgtgaagca ccccgccgac atccccgatt acaagaagct gtccttcccc      420

gagggcttca agtgggagcg cgtgatgaac ttcgaggacg gcggtctggt gaccgtgacc      480

caggactcct ccctgcagga cggcacgctg atctacaagg tgaagatgcg cggcaccaac      540

ttcccccccg acggccccgt aatgcagaag aagaccatgg gctgggaggc ctccaccgag      600

cgcctgtacc cccgcgacgg cgtgctgaag ggcgagatcc accaggccct gaagctgaag      660

gacggcggcc actacctggt ggagttcaag accatctaca tggccaagaa gcccgtgcaa      720

ctgcccggct actactacgt ggacaccaag ctggacatca cctcccacaa cgaggactac      780

accatcgtgg aacagtacga gcgctccgag ggccgccacc acctgttcct gtacggcatg      840

gacgagctgt acaagtctag aggtaccggt tgttggccca tgggagcttg gattgaacaa      900

gatggattgc acgcaggttc tccggccgct tgggtggaga ggctattcgg ctatgactgg      960

gcacaacaga caatcggctg ctctgatgcc gccgtgttcc ggctgtcagc gcaggggcgc     1020

ccggttcttt ttgtcaagac cgacctgtcc ggtgccctga atgaactgca ggacgaggca     1080

gcgcggctat cgtggctggc cacgacgggc gttccttgcg cagctgtgct cgacgttgtc     1140

actgaagcgg gaagggactg gctgctattg ggcgaagtgc cggggcagga tctcctgtca     1200

tctcaccttg ctcctgccga gaaagtatcc atcatggctg atgcaatgcg gcggctgcat     1260

acgcttgatc cggctacctg cccattcgac caccaagcga aacatcgcat cgagcgagca     1320

cgtactcgga tggaagccgg tcttgtcgat caggatgatc tggacgaaga gcatcagggg     1380

ctcgcgccag ccgaactgtt cgccaggctc aaggcgcgca tgcccgacgg cgaggatctc     1440

gtcgtgaccc atggcgatgc ctgcttgccg aatatcatgg tggaaaatgg ccgcttttct     1500

ggattcatcg actgtggccg gctgggtgtg gcggaccgct atcaggacat agcgttggct     1560

acccgtgata ttgctgaaga gcttggcggc gaatgggctg accgcttcct cgtgctttac     1620

ggtatcgccg ctcccgattc gcagcgcatc gccttctatc gccttcttga cgagttcttc     1680

tgagcgggac ccaagctagg ccatcgatac gcgtacgtcg cgaccgcgga catgtacaga     1740

gctcgagaag tactagagtc cgcaaaaatc accagtctct ctctacaaat ctatctctct     1800

ctatttttct ccagaataat gtgtgagtag ttcccagata agggaattag ggttcttata 1860
gggtttcgct catgtgttga gcatataaga aacccttagt atgtatttgt atttgtaaaa 1920
tacttctatc aataaaattt ctaattccta aaaccaaaat ccagtgacct gcaggcatgc 1980
aagcttatcg atgtcgacag gccttaaggg ccagatcccc cgggctgcag aagcctatac 2040
tgtacttaac ttgattgcat aattacttga tcatagactc atagtaaact tgattacaca 2100
gataagtgaa gaaacaaacc aattcaagac ataaccaaag agaggtgaaa gactgtttta 2160
tatgtctaac attgcacctt aatatcacac tgttagttcc tttcttactt aaattcaacc 2220
cattaaagta aaaacaacag ataataataa tttgagaatg aacaaaagga ccatatcatt 2280
tattaactct tatccatcca tttgcatttt gatgtccgaa aacaaaaact gaaagaacac 2340
agtaaattac aagcagaaca aatgatagaa gaaaacagct tttccaatgc cataatactc 2400
aaacttagta ggattctggt gtgtgggcaa tgaaactgat gcattgaact tgacgaacgt 2460
tgtcgaaacc gatgatacgg acgaaagctg ggaggcctgg tcccattcgc catgccgaag 2520
catgttgccc agccggcgcc agcgaggagg ctgggaccat gccggccgat atactagttg 2580
gcttggtgca tatctacact tagtagaaat taatctacac ttagtagaaa ttagacgagc 2640
ttactcgttt cgtcctcacg gactcatcag taatttggat ccgctgcaca tacataacat 2700
atcaagatca gaacacacat atacacacac aaatacaatc aagtcaacaa ctccaaaaag 2760
tccagatcta catatataca tacgtaaata acaaaatcat gtaaataatc acaatcatgt 2820
aatccagatc tatgcacata tatatataca caattaataa aaaaaatgat ataacagatc 2880
tatatctatg tatgtaacaa cacaatcaga tgagagaagt gatgttttca gatctgtata 2940
catacaaaca caaacagatg aacaattgat acgtagatcc atatgtatac gtacaattag 3000
ctacacgatt aaatgaaaaa aatcaacgat ttcggattgg tacacacaaa cgcaacaata 3060
tgaagaaatt catatctgat tagatataaa cataaccacg tgtagataca cagtcaaatc 3120
aacaaattta tagcttctaa acggatgaga tgaacaagat aaagatattc acataaggca 3180
tacataagat aagcagatta acaaactagc aataatacat acctaattaa aacaaggaat 3240
aacagagaga gagagagaga gagagattta ccttgaaaat gaagaggaga agagaggatt 3300
tcttaaaatt gggggtagag aaagaaagat gatgaattgt gagaaaggag agatagaagg 3360
gggggttgta tatataggct gtagaagatt atttttgtgt ttgaggcggt gaaggaagag 3420
gggatctgac tatgacacgt ttgcggttac gtatttcgat aggagtcttt caacgcttaa 3480
cgccgttact ctatatgacc gtttgggccg taacggggcc gtttgttaac gctgatgttg 3540
attcttttct ttctttcttt cttccttttt taaagaagca attgtacaat cgttgctagc 3600
tgtcaaacgg ataattcgga tacggatatg cctatattca tatccgtaat ttttctgcag 3660
gaattcgatc tggcacgaca ggtttcccga ctggaaagcg ggcagtgagc gcaacgcaat 3720

taatgtgagt tagctcactc attaggcacc ccaggcttta cactttatgc ttccggctcg 3780

tatgttgtgt ggaattgtga gcggataaca atttcacaca ggaaacagct atgacatgat 3840

tacgaattca aaaattacgg atatgaatat aggcatatcc gtatccgaat tatccgtttg 3900

acagctagca acgattgtac aattgcttct ttaaaaaagg aagaaagaaa gaaagaaaag 3960

aatcaacatc agcgttaaca aacggccccg ttacggccca aacggtcata tagagtaacg 4020

gcgttaagcg ttgaaagact cctatcgaaa tacgtaaccg caaacgtgtc atagtcagat 4080

cccctcttcc ttcaccgcct caaacacaaa aataatcttc tacagcctat atatacaacc 4140

cccccttcta tctctccttt ctcacaattc atcatctttc tttctctacc cccaatttta 4200

agaaatcctc tcttctcctc ttcattttca aggtaaatct ctctctctct ctctctctct 4260

gttattcctt gttttaatta ggtatgtatt attgctagtt tgttaatctg cttatcttat 4320

gtatgcctta tgtgaatatc tttatcttgt tcatctcatc cgtttagaag ctataaattt 4380

gttgatttga ctgtgtatct acacgtggtt atgtttatat ctaatcagat atgaatttct 4440

tcatattgtt gcgtttgtgt gtaccaatcc gaaatcgttg atttttttca tttaatcgtg 4500

tagctaattg tacgtataca tatggatcta cgtatcaatt gttcatctgt ttgtgtttgt 4560

atgtatacag atctgaaaac atcacttctc tcatctgatt gtgttgttac atacatagat 4620

atagatctgt tatatcattt tttttattaa ttgtgtatat atatatgtgc atagatctgg 4680

attacatgat tgtgattatt tacatgattt tgttatttac gtatgtatat atgtagatct 4740

ggactttttg gagttgttga cttgattgta tttgtgtgtg tatatgtgtg ttctgatctt 4800

gatatgttat gtatgtgcag cgaattcggc gcgccatggc tcctaagaag aagaggaagg 4860

ttagcaagct cgagaagttt accaactgct acagcctctc taagaccctc aggttcaagg 4920

ctatccctgt gggaaagacc caagagaata tcgacaacaa gaggctcctc gtcgaggatg 4980

agaagagagc tgaagattac aagggcgtga agaagctcct cgacaggtac tacctcagct 5040

tcatcaacga tgtgctccac agcatcaagc tcaagaacct caacaactac atcagcctct 5100

tccgtaagaa aaccaggacc gagaaagaga acaaagagct tgagaacctc gagatcaacc 5160

tccgtaaaga gatcgccaag gctttcaagg gaaacgaggg atacaagagc ctcttcaaga 5220

aggatattat cgagacaatc ctgcctgagt tcctggacga taaggatgag atcgctctcg 5280

tgaacagctt caacggattc actactgcct tcaccggatt cttcgacaac agggaaaaca 5340

tgttcagcga agaggccaag agcacctcta tcgctttcag atgcatcaac gagaacctca 5400

cgcgttacat cagcaacatg gacatcttcg agaaggtgga cgccatcttc gataagcacg 5460

aggtgcaaga aatcaaagag aagatcctca acagcgacta cgacgtcgag gacttttttg 5520

aaggggagtt cttcaacttc gttctcaccc aagagggcat cgacgtgtac aacgctatta 5580

tcggaggatt cgtgaccgag tctggggaga agattaaggg actcaacgag tacatcaacc 5640

tgtacaacca gaaaacgaag cagaagctcc cgaagttcaa gccgctctac aagcaggttc 5700

tctctgatcg tgagagcctc tcattttacg gtgagggtta cacctctgac gaggaagtgc 5760

ttgaggtttt ccgtaacacc ctcaacaaga acagcgagat cttctcgtcc atcaagaagt 5820

tggagaaact tttcaagaac ttcgacgagt acagcagcgc tgggatcttc gttaagaacg 5880

gacctgctat cagcaccatc agcaaggata ttttcggcga gtggaacgtg atcaggacaa 5940

agtggaatgc tgagtacgat gacatccacc tcaagaagaa ggctgtcgtc actgagaagt 6000

acgaggatga caggcgtaag tcgttcaaga agatcggctc tttcagcctc gagcagcttc 6060

aagaatacgc tgatgctgat ctcagcgtgg tcgagaagct caaagagatc atcatccaga 6120

aggtcgacga gatctacaag gtgtacgggt cctctgagaa gttgttcgat gctgatttcg 6180

tcctcgagaa gagtctgaag aagaacgacg ctgtcgtcgc gatcatgaag gatttgctcg 6240

acagcgtgaa gtccttcgag aactatatca aggccttctt cggagagggc aaagagacta 6300

atagggacga gtctttctac ggggatttcg tgctcgctta cgatatcctc ctcaaggtgg 6360

accatatcta cgacgccatc agaaactacg tgacccagaa gccttacagc aaggacaagt 6420

tcaagttgta ctttcagaac ccgcagttca tgggcggatg ggacaaagac aaagagacag 6480

attacagggc caccatcctc aggtacgggt ctaagtacta cctggccatc atggacaaga 6540

aatacgccaa gtgcctccaa aagatcgaca aggatgacgt gaacgggaac tatgagaaga 6600

tcaactacaa gctccttccg ggaccgaaca agatgcttcc taaggtgttc ttcagcaaga 6660

aatggatggc ctactacaac ccgtctgagg acatccagaa aatctacaag aacggaccct 6720

tcaagaaagg cgacatgttc aacctcaacg actgccacaa gctcatcgat ttcttcaagg 6780

acagcatctc gcgttacccg aagtggtcta acgcttacga ctttaacttc agcgagacag 6840

aaaagtacaa ggatatcgcc gggttctacc gtgaggttga ggaacagggt tacaaggtta 6900

gcttcgagag cgcctccaag aaagaggttg acaagttggt cgaagagggc aagctctaca 6960

tgttccagat ctataacaag gacttctccg acaagagcca cggaactcct aacctccata 7020

cgatgtactt caagctgctt ttcgacgaga acaaccacgg gcagatcaga ctttctggtg 7080

gtgctgaact cttcatgcgt agggcctcac tcaagaaaga agagttggtt gttcacccgg 7140

ccaactctcc aatcgctaac aagaatcctg acaacccgaa aaagaccacc acgctgtctt 7200

acgacgtcta caaggacaaa aggttcagcg aggaccagta cgagcttcat atcccgatcg 7260

ctatcaacaa gtgcccgaag aacatcttca agatcaatac cgaggtgagg gtgctgctca 7320

agcacgatga taacccttac gtgatcggaa tcgatcgtgg tgagagaaac ctcctctaca 7380

tcgttgtggt ggacggaaag ggaaacatcg tcgagcagta cagcctgaac gagattatca 7440

acaatttcaa cggcatcagg atcaagaccg actaccactc actcctcgat aagaaagaaa 7500

aagagcgttt cgaggccagg cagaactgga cttctatcga aaacatcaaa gagttgaagg 7560

ccggctacat ctctcaggtg gtgcataaga tctgcgagct ggtggaaaag tacgatgctg 7620

tgatcgctct tgaggacctc aactctgggt tcaagaacag tagagtgaag gttgagaagc 7680

aggtctacca aaagttcgag aagatgctca tcgacaagct caactacatg gtggacaaaa 7740

agagcaaccc ttgcgctacc ggtggtgctc ttaagggata ccagatcacg aacaagttcg 7800

agtccttcaa gagcatgagc acccagaacg gcttcatctt ctatatccct gcttggctca 7860

ccagcaagat cgatccttct actggtttcg tgaacctgct caagaccaag tacacctcga 7920

tcgccgacag caagaagttc atctcgtctt tcgacaggat catgtacgtg ccggaagagg 7980

atcttttcga gttcgctctc gactataaga acttcagcag gaccgacgcc gactacatta 8040

agaagtggaa gctctactcc tacggaaccc gtatcaggat cttccgaaat ccgaagaaaa 8100

acaacgtgtt cgactgggaa gaagtgtgcc tcacctctgc ctacaaagaa ctgttcaaca 8160

agtacggcat caactaccag cagggtgata tcaggctctt tgtgcgagca cagagcgaca 8220

aggcattcta cagctcattc atggccctca tgtctctcat gctccagatg aggaactcta 8280

tcaccggaag gaccgatgtg gacttcctta tctctccggt caagaactct gacggatctt 8340

tctacgacag ccgtaactat gaggctcaag agaacgctat cctgccgaag aatgctgatg 8400

caaacggggc ttacaacatt gcgagaaagg ttctctgggc tatcgggcag tttaagaaag 8460

cggaagatga gaagctcgac aaggtgaaga tcgccatctc caacaaagag tggcttgagt 8520

acgctcagac ctccgttaag cacaagaggc ctgctgctac taagaaagct ggccaggcca 8580

aaaagaagaa gtgaggcgcg ccgagctcca ggcctcccag ctttcgtccg tatcatcggt 8640

ttcgacaacg ttcgtcaagt tcaatgcatc agtttcattg cccacacacc agaatcctac 8700

taagtttgag tattatggca ttggaaaagc tgttttcttc tatcatttgt tctgcttgta 8760

atttactgtg ttctttcagt ttttgttttc ggacatcaaa atgcaaatgg atggataaga 8820

gttaataaat gatatggtcc ttttgttcat tctcaaatta ttattatctg ttgtttttac 8880

tttaatgggt tgaatttaag taagaaagga actaacagtg tgatattaag gtgcaatgtt 8940

agacatataa aacagtcttt cacctctctt tggttatgtc ttgaattggt ttgtttcttc 9000

acttatctgt gtaatcaagt ttactatgag tctatgatca agtaattatg caatcaagtt 9060

aagtacagta taggcttgag ctccctagga tcaagctttc cctatagtga gtcgtattag 9120

agcttggcgt aatcatggtc atagctgttt cctgtgtgaa attgttatcc gctcacaatt 9180

ccacacaaca tacgagccgg aagcataaag tgtaaagcct ggggtgccta atgagtgagc 9240

taactcacat taattgcgtt gcgctcactg cccgctttcc agtcgggaaa cctgtcgtgc 9300

cagctgcatt aatgaatcgg ccaacgcgcg gggagaggcg gtttgcgtat tgggcgctct 9360

tccgcttcct cgctcactga ctcgctgcgc tcggtcgttc ggctgcggcg agcggtatca 9420

gctcactcaa aggcggtaat acggttatcc acagaatcag gggataacgc aggaaagaac 9480

atgtgagcaa aaggccagca aaaggccagg aaccgtaaaa aggccgcgtt gctggcgttt 9540

ttccataggc tccgcccccc tgacgagcat cacaaaaatc gacgctcaag tcagaggtgg 9600

cgaaacccga caggactata aagataccag gcgtttcccc ctggaagctc cctcgtgcgc 9660

tctcctgttc cgaccctgcc gcttaccgga tacctgtccg cctttctccc ttcgggaagc 9720

gtggcgcttt ctcaatgctc acgctgtagg tatctcagtt cggtgtaggt cgttcgctcc 9780

aagctgggct gtgtgcacga accccccgtt cagcccgacc gctgcgcctt atccggtaac 9840

tatcgtcttg agtccaaccc ggtaagacac gacttatcgc cactggcagc agccactggt 9900

aacaggatta gcagagcgag gtatgtaggc ggtgctacag agttcttgaa gtggtggcct 9960

aactacggct acactagaag gacagtattt ggtatctgcg ctctgctgaa gccagttacc 10020

ttcggaaaaa gagttggtag ctcttgatcc ggcaaacaaa ccaccgctgg tagcggtggt 10080

ttttttgttt gcaagcagca gattacgcgc agaaaaaaag gatctcaaga agatcctttg 10140

atcttttcta cggggtctga cgctcagtgg aacgaaaact cacgttaagg gattttggtc 10200

atgagattat caaaaaggat cttcacctag atccttttaa attaaaaatg aagttttaaa 10260

tcaatctaaa gtatatatga gtaaacttgg tctgacagtt accaatgctt aatcagtgag 10320

gcacctatct cagcgatctg tctatttcgt tcatccatag ttgcctgact ccccgtcgtg 10380

tagataacta cgatacggga gggcttacca tctggcccca gtgctgcaat gataccgcga 10440

gacccacgct gaccggctcc agatttatca gcaataaacc agccagccgg aagggccgag 10500

cgcagaagtg gtcctgcaac tttatccgcc tccatccagt ctattaattg ttgccgggaa 10560

gctagagtaa gtagttcgcc agttaatagt ttgcgcaacg ttgttgccat tgctacaggc 10620

atcgtggtgt cacgctcgtc gtttggtatg gcttcactca gctccggttc ccaacgatca 10680

aggcgagtta catgatcccc catgttgtgc aaaaaagcgg ttagctcctt cggtcctccg 10740

atcgttgtca gaagtaagtt ggccgcagtg ttatcactca tggttatggc agcactgcat 10800

aattctctta ctgtcatgcc atccgtaaga tgcttttctg tgactggtga gtactcaacc 10860

aagtcattct gagaatagtg tatgcggcga ccgagttgct cttgcccggc gtcaatacgg 10920

gataataccg cgccacatag cagaacttta aaagtgctca tcattggaaa acgttcttcg 10980

gggcgaaaac tctcaaggat cttaccgctg ttgagatcca gttcgatgta acccactcgt 11040

gcacccaact gatcttcagc atcttttact ttcaccagcg tttctgggtg agcaaaaaca 11100

ggaaggcaaa atgccgcaaa aaagggaata agggcgacac ggaaatgttg aatactcata 11160

ctcttccttt ttcaatatta ttgaagcatt tatcagggtt attgtctcat gagcggatac 11220

atatttgaat gtatttagaa aaataaagaa ataggggttc tgcgcacatt tccccgaaaa 11280

gtgccacctg aaattgtaaa cgttaatgtt ttgttaaatt tcgcgttaaa tatttgttaa 11340

atcagcttat tttttaacca gtaagcagaa aatgacaaaa atccttataa atcaaaagaa 11400

tagaccgagt tagttgtgag tgttgttcca gtttggaaca agagtccact attaaagaac 11460

gtggactcca acgtaaaacc gtctatcagg gcgatggccc actacgtgaa ccatcaccca 11520

aatcaagttt ttggaggtcg aggtgccgta aagcactaaa tcggaaccct aaagggagcc 11580

cccgatttag agcttgacgg ggaaagccgg cgaacgtggc gagaaaggaa gggaagaaag 11640

cgaaaggagc gggcgctagg gcgctggcaa gtgtagcggt cacgctgcgc gtaaccacca 11700

cacccgccgc gcttaatgcg ccgctactgg gcgcgtccca ttcgccattc aggctgcgca 11760

actgttggga agggcgatcg gtgcgggcct cttcgctatt acgccagctg gcgaaagggg 11820

gatgtgctgc aaggcgatta agttgggtaa cgccagggtt ttcccagtca cgacgttgta 11880

aaacgacggc cagtgaatta tcgattaaat taaccctcac taaagggaga gacgaattcg 11940

aatccaaaaa ttacggatat gaatataggc atatccgtat ccgaattatc cgtttgacag 12000

ctagcaacga ttgtacaatt gcttctttaa aaaaggaaga aagaaagaaa gaaaagaatc 12060

aacatcagcg ttaacaaacg gccccgttac ggcccaaacg gtcatataga gtaacggcgt 12120

taagcgttga aagactccta tcgaaatacg taaccgcaaa cgtgtcatag tcagatcccc 12180

tcttccttca ccgcctcaaa cacaaaaata atcttctaca gcctatatat acaacccccc 12240

cttctatctc tcctttctca caattcatca tctttctttc tctaccccca attttaagaa 12300

atcctctctt ctcctcttca ttttcaaggt aaatctctct ctctctctct ctctctgtta 12360

ttccttgttt taattaggta tgtattattg ctagtttgtt aatctgctta tcttatgtat 12420

gccttatgtg aatatcttta tcttgttcat ctcatccgtt tagaagctat aaatttgttg 12480

atttgactgt gtatctacac gtggttatgt ttatatctaa tcagatatga atttcttcat 12540

attgttgcgt ttgtgtgtac caatccgaaa tcgttgattt ttttcattta atcgtgtagc 12600

taattgtacg tatacatatg gatctacgta tcaattgttc atctgtttgt gtttgtatgt 12660

atacagatct gaaaacatca cttctctcat ctgattgtgt tgttacatac atagatatag 12720

atctgttata tcattttttt tattaattgt gtatatatat atgtgcatag atctggatta 12780

catgattgtg attatttaca tgattttgtt atttacgtat gtatatatgt agatctggac 12840

tttttggagt tgttgacttg attgtatttg tgtgtgtata tgtgtgttct gatcttgata 12900

tgttatgtat gtgcagctga accctcga 12928

<210> 70
<211> 12928
<212> DNA
<213> Artificial Sequence

<220>
<223>  pUbitDTnptII_LbCpf1_ crRNA _ Cyp_Del

<400>  70
gatcctctca acacaacata tacaaaacaa acgaatctca agcaatcaag cattctactt      60

ctattgcagc aatttaaatc atttctttta aagcaaaagc aattttctga aaattttcac      120

catttacgaa cgatagggcg cgatcccgcc accatggtga gcaagggcga ggaggtcatc     180

aaagagttca tgcgcttcaa ggtgcgcatg gagggctcca tgaacggcca cgagttcgag     240

atcgagggcg agggcgaggg ccgcccctac gagggcaccc agaccgccaa gctgaaggtg     300

accaagggcg gccccctgcc cttcgcctgg gacatcctgt ccccccagtt catgtacggc      360

tccaaggcgt acgtgaagca ccccgccgac atccccgatt acaagaagct gtccttcccc     420

gagggcttca agtgggagcg cgtgatgaac ttcgaggacg gcggtctggt gaccgtgacc     480

caggactcct ccctgcagga cggcacgctg atctacaagg tgaagatgcg cggcaccaac     540

ttcccccccg acggccccgt aatgcagaag aagaccatgg gctgggaggc ctccaccgag     600

cgcctgtacc cccgcgacgg cgtgctgaag ggcgagatcc accaggccct gaagctgaag     660

gacggcggcc actacctggt ggagttcaag accatctaca tggccaagaa gcccgtgcaa     720

ctgcccggct actactacgt ggacaccaag ctggacatca cctcccacaa cgaggactac     780

accatcgtgg aacagtacga gcgctccgag ggccgccacc acctgttcct gtacggcatg     840

gacgagctgt acaagtctag aggtaccggt tgttggccca tgggagcttg gattgaacaa     900

gatggattgc acgcaggttc tccggccgct tgggtggaga ggctattcgg ctatgactgg     960

gcacaacaga caatcggctg ctctgatgcc gccgtgttcc ggctgtcagc gcaggggcgc    1020

ccggttcttt ttgtcaagac cgacctgtcc ggtgccctga atgaactgca ggacgaggca    1080

gcgcggctat cgtggctggc cacgacgggc gttccttgcg cagctgtgct cgacgttgtc    1140

actgaagcgg gaagggactg gctgctattg ggcgaagtgc cggggcagga tctcctgtca    1200

tctcaccttg ctcctgccga gaaagtatcc atcatggctg atgcaatgcg gcggctgcat    1260

acgcttgatc cggctacctg cccattcgac caccaagcga aacatcgcat cgagcgagca    1320

cgtactcgga tggaagccgg tcttgtcgat caggatgatc tggacgaaga gcatcagggg    1380

ctcgcgccag ccgaactgtt cgccaggctc aaggcgcgca tgcccgacgg cgaggatctc    1440

gtcgtgaccc atggcgatgc ctgcttgccg aatatcatgg tggaaaatgg ccgcttttct    1500

ggattcatcg actgtggccg gctgggtgtg gcggaccgct atcaggacat agcgttggct    1560

acccgtgata ttgctgaaga gcttggcggc gaatgggctg accgcttcct cgtgctttac    1620

ggtatcgccg ctcccgattc gcagcgcatc gccttctatc gccttcttga cgagttcttc    1680

tgagcgggac ccaagctagg ccatcgatac gcgtacgtcg cgaccgcgga catgtacaga    1740

gctcgagaag tactagagtc cgcaaaaatc accagtctct ctctacaaat ctatctctct    1800

ctatttttct ccagaataat gtgtgagtag ttcccagata agggaattag ggttcttata 1860

gggtttcgct catgtgttga gcatataaga aacccttagt atgtatttgt atttgtaaaa 1920

tacttctatc aataaaattt ctaattccta aaaccaaaat ccagtgacct gcaggcatgc 1980

aagcttatcg atgtcgacag gccttaaggg ccagatcccc cgggctgcag aagcctatac 2040

tgtacttaac ttgattgcat aattacttga tcatagactc atagtaaact tgattacaca 2100

gataagtgaa gaaacaaacc aattcaagac ataaccaaag agaggtgaaa gactgtttta 2160

tatgtctaac attgcacctt aatatcacac tgttagttcc tttcttactt aaattcaacc 2220

cattaaagta aaaacaacag ataataataa tttgagaatg aacaaaagga ccatatcatt 2280

tattaactct tatccatcca tttgcatttt gatgtccgaa aacaaaaact gaaagaacac 2340

agtaaattac aagcagaaca aatgatagaa gaaaacagct tttccaatgc cataatactc 2400

aaacttagta ggattctggt gtgtgggcaa tgaaactgat gcattgaact tgacgaacgt 2460

tgtcgaaacc gatgatacgg acgaaagctg ggaggcctgg tcccattcgc catgccgaag 2520

catgttgccc agccggcgcc agcgaggagg ctgggaccat gccggccgat atactagttg 2580

gcttggtgca tatctacact tagtagaaat taatctacac ttagtagaaa ttagacgagc 2640

ttactcgttt cgtcctcacg gactcatcag taatttggat ccgctgcaca tacataacat 2700

atcaagatca gaacacacat atacacacac aaatacaatc aagtcaacaa ctccaaaaag 2760

tccagatcta catatataca tacgtaaata acaaaatcat gtaaataatc acaatcatgt 2820

aatccagatc tatgcacata tatatataca caattaataa aaaaaatgat ataacagatc 2880

tatatctatg tatgtaacaa cacaatcaga tgagagaagt gatgttttca gatctgtata 2940

catacaaaca caaacagatg aacaattgat acgtagatcc atatgtatac gtacaattag 3000

ctacacgatt aaatgaaaaa aatcaacgat ttcggattgg tacacacaaa cgcaacaata 3060

tgaagaaatt catatctgat tagatataaa cataaccacg tgtagataca cagtcaaatc 3120

aacaaattta tagcttctaa acggatgaga tgaacaagat aaagatattc acataaggca 3180

tacataagat aagcagatta acaaactagc aataatacat acctaattaa aacaaggaat 3240

aacagagaga gagagagaga gagagattta ccttgaaaat gaagaggaga agagaggatt 3300

tcttaaaatt gggggtagag aaagaaagat gatgaattgt gagaaaggag agatagaagg 3360

gggggttgta tatataggct gtagaagatt atttttgtgt ttgaggcggt gaaggaagag 3420

gggatctgac tatgacacgt ttgcggttac gtatttcgat aggagtcttt caacgcttaa 3480

cgccgttact ctatatgacc gtttgggccg taacggggcc gtttgttaac gctgatgttg 3540

attcttttct ttctttcttt cttccttttt taaagaagca attgtacaat cgttgctagc 3600

tgtcaaacgg ataattcgga tacggatatg cctatattca tatccgtaat ttttctgcag 3660

gaattcgatc tggcacgaca ggtttcccga ctggaaagcg ggcagtgagc gcaacgcaat 3720

taatgtgagt tagctcactc attaggcacc ccaggcttta cactttatgc ttccggctcg 3780

tatgttgtgt ggaattgtga gcggataaca atttcacaca ggaaacagct atgacatgat 3840

tacgaattca aaaattacgg atatgaatat aggcatatcc gtatccgaat tatccgtttg 3900

acagctagca acgattgtac aattgcttct ttaaaaaagg aagaaagaaa gaaagaaaag 3960

aatcaacatc agcgttaaca aacggccccg ttacggccca aacggtcata tagagtaacg 4020

gcgttaagcg ttgaaagact cctatcgaaa tacgtaaccg caaacgtgtc atagtcagat 4080

cccctcttcc ttcaccgcct caaacacaaa aataatcttc tacagcctat atatacaacc 4140

cccccttcta tctctccttt ctcacaattc atcatctttc tttctctacc cccaatttta 4200

agaaatcctc tcttctcctc ttcattttca aggtaaatct ctctctctct ctctctctct 4260

gttattcctt gttttaatta ggtatgtatt attgctagtt tgttaatctg cttatcttat 4320

gtatgcctta tgtgaatatc tttatcttgt tcatctcatc cgtttagaag ctataaattt 4380

gttgatttga ctgtgtatct acacgtggtt atgtttatat ctaatcagat atgaatttct 4440

tcatattgtt gcgtttgtgt gtaccaatcc gaaatcgttg atttttttca tttaatcgtg 4500

tagctaattg tacgtataca tatggatcta cgtatcaatt gttcatctgt ttgtgtttgt 4560

atgtatacag atctgaaaac atcacttctc tcatctgatt gtgttgttac atacatagat 4620

atagatctgt tatatcattt tttttattaa ttgtgtatat atatatgtgc atagatctgg 4680

attacatgat tgtgattatt tacatgattt tgttatttac gtatgtatat atgtagatct 4740

ggactttttg gagttgttga cttgattgta tttgtgtgtg tatatgtgtg ttctgatctt 4800

gatatgttat gtatgtgcag cgaattcggc gcgccatggc tcctaagaag aagaggaagg 4860

ttagcaagct cgagaagttt accaactgct acagcctctc taagaccctc aggttcaagg 4920

ctatccctgt gggaaagacc caagagaata tcgacaacaa gaggctcctc gtcgaggatg 4980

agaagagagc tgaagattac aagggcgtga agaagctcct cgacaggtac tacctcagct 5040

tcatcaacga tgtgctccac agcatcaagc tcaagaacct caacaactac atcagcctct 5100

tccgtaagaa aaccaggacc gagaaagaga acaaagagct tgagaacctc gagatcaacc 5160

tccgtaaaga gatcgccaag gctttcaagg gaaacgaggg atacaagagc ctcttcaaga 5220

aggatattat cgagacaatc ctgcctgagt tcctggacga taaggatgag atcgctctcg 5280

tgaacagctt caacggattc actactgcct tcaccggatt cttcgacaac agggaaaaca 5340

tgttcagcga agaggccaag agcacctcta tcgctttcag atgcatcaac gagaacctca 5400

cgcgttacat cagcaacatg gacatcttcg agaaggtgga cgccatcttc gataagcacg 5460

aggtgcaaga aatcaaagag aagatcctca acagcgacta cgacgtcgag gacttttttg 5520

aaggggagtt cttcaacttc gttctcaccc aagagggcat cgacgtgtac aacgctatta 5580

tcggaggatt cgtgaccgag tctggggaga agattaaggg actcaacgag tacatcaacc 5640

tgtacaacca gaaaacgaag cagaagctcc cgaagttcaa gccgctctac aagcaggttc 5700

tctctgatcg tgagagcctc tcattttacg gtgagggtta cacctctgac gaggaagtgc 5760

ttgaggtttt ccgtaacacc ctcaacaaga acagcgagat cttctcgtcc atcaagaagt 5820

tggagaaact tttcaagaac ttcgacgagt acagcagcgc tgggatcttc gttaagaacg 5880

gacctgctat cagcaccatc agcaaggata ttttcggcga gtggaacgtg atcagggaca 5940

agtggaatgc tgagtacgat gacatccacc tcaagaagaa ggctgtcgtc actgagaagt 6000

acgaggatga caggcgtaag tcgttcaaga agatcggctc tttcagcctc gagcagcttc 6060

aagaatacgc tgatgctgat ctcagcgtgg tcgagaagct caaagagatc atcatccaga 6120

aggtcgacga gatctacaag gtgtacgggt cctctgagaa gttgttcgat gctgatttcg 6180

tcctcgagaa gagtctgaag aagaacgacg ctgtcgtcgc gatcatgaag gatttgctcg 6240

acagcgtgaa gtccttcgag aactatatca aggccttctt cggagagggc aaagagacta 6300

atagggacga gtctttctac ggggatttcg tgctcgctta cgatatcctc ctcaaggtgg 6360

accatatcta cgacgccatc agaaactacg tgacccagaa gccttacagc aaggacaagt 6420

tcaagttgta ctttcagaac ccgcagttca tgggcggatg ggacaaagac aaagagacag 6480

attacagggc caccatcctc aggtacgggt ctaagtacta cctggccatc atggacaaga 6540

aatacgccaa gtgcctccaa aagatcgaca aggatgacgt gaacgggaac tatgagaaga 6600

tcaactacaa gctccttccg ggaccgaaca agatgcttcc taaggtgttc ttcagcaaga 6660

aatggatggc ctactacaac ccgtctgagg acatccagaa aatctacaag aacgggacct 6720

tcaagaaagg cgacatgttc aacctcaacg actgccacaa gctcatcgat ttcttcaagg 6780

acagcatctc gcgttacccg aagtggtcta acgcttacga ctttaacttc agcgagacag 6840

aaaagtacaa ggatatcgcc gggttctacc gtgaggttga ggaacagggt tacaaggtta 6900

gcttcgagag cgcctccaag aaagaggttg acaagttggt cgaagagggc aagctctaca 6960

tgttccagat ctataacaag gacttctccg acaagagcca cggaactcct aacctccata 7020

cgatgtactt caagctgctt ttcgacgaga acaaccacgg gcagatcaga ctttctggtg 7080

gtgctgaact cttcatgcgt agggcctcac tcaagaaaga agagttggtt gttcacccgg 7140

ccaactctcc aatcgctaac aagaatcctg acaacccgaa aaagaccacc acgctgtctt 7200

acgacgtcta caaggacaaa aggttcagcg aggaccagta cgagcttcat atcccgatcg 7260

ctatcaacaa gtgcccgaag aacatcttca agatcaatac cgaggtgagg gtgctgctca 7320

agcacgatga taacccttac gtgatcggaa tcgatcgtgg tgagagaaac ctcctctaca 7380

tcgttgtggt ggacggaaag ggaaacatcg tcgagcagta cagcctgaac gagattatca 7440

acaatttcaa cggcatcagg atcaagaccg actaccactc actcctcgat aagaaagaaa 7500
aagagcgttt cgaggccagg cagaactgga cttctatcga aaacatcaaa gagttgaagg 7560
ccggctacat ctctcaggtg gtgcataaga tctgcgagct ggtggaaaag tacgatgctg 7620
tgatcgctct tgaggacctc aactctgggt tcaagaacag tagagtgaag gttgagaagc 7680
aggtctacca aaagttcgag aagatgctca tcgacaagct caactacatg gtggacaaaa 7740
agagcaaccc ttgcgctacc ggtggtgctc ttaagggata ccagatcacg aacaagttcg 7800
agtccttcaa gagcatgagc acccagaacg gcttcatctt ctatatccct gcttggctca 7860
ccagcaagat cgatccttct actggtttcg tgaacctgct caagaccaag tacacctcga 7920
tcgccgacag caagaagttc atctcgtctt tcgacaggat catgtacgtg ccggaagagg 7980
atcttttcga gttcgctctc gactataaga acttcagcag gaccgacgcc gactacatta 8040
agaagtggaa gctctactcc tacgggaacc gtatcaggat cttccgaaat ccgaagaaaa 8100
acaacgtgtt cgactgggaa gaagtgtgcc tcacctctgc ctacaaagaa ctgttcaaca 8160
agtacggcat caactaccag cagggtgata tcagggctct tttgtgcgag cagagcgaca 8220
aggcattcta cagctcattc atggccctca tgtctctcat gctccagatg aggaactcta 8280
tcaccggaag gaccgatgtg gacttcctta tctctccggt caagaactct gacgggatct 8340
tctacgacag ccgtaactat gaggctcaag agaacgctat cctgccgaag aatgctgatg 8400
caaacggggc ttacaacatt gcgagaaagg ttctctgggc tatcgggcag tttaagaaag 8460
cggaagatga gaagctcgac aaggtgaaga tcgccatctc caacaaagag tggcttgagt 8520
acgctcagac ctccgttaag cacaagaggc ctgctgctac taagaaagct ggccaggcca 8580
aaaagaagaa gtgaggcgcg ccgagctcca ggcctcccag ctttcgtccg tatcatcggt 8640
ttcgacaacg ttcgtcaagt tcaatgcatc agtttcattg cccacacacc agaatcctac 8700
taagtttgag tattatggca ttggaaaagc tgttttcttc tatcatttgt tctgcttgta 8760
atttactgtg ttctttcagt ttttgttttc ggacatcaaa atgcaaatgg atggataaga 8820
gttaataaat gatatggtcc ttttgttcat tctcaaatta ttattatctg ttgtttttac 8880
tttaatgggt tgaatttaag taagaaagga actaacagtg tgatattaag gtgcaatgtt 8940
agacatataa aacagtcttt cacctctctt tggttatgtc ttgaattggt ttgtttcttc 9000
acttatctgt gtaatcaagt ttactatgag tctatgatca agtaattatg caatcaagtt 9060
aagtacagta taggcttgag ctccctagga tcaagctttc cctatagtga gtcgtattag 9120
agcttggcgt aatcatggtc atagctgttt cctgtgtgaa attgttatcc gctcacaatt 9180
ccacacaaca tacgagccgg aagcataaag tgtaaagcct ggggtgccta atgagtgagc 9240
taactcacat taattgcgtt gcgctcactg cccgctttcc agtcgggaaa cctgtcgtgc 9300
cagctgcatt aatgaatcgg ccaacgcgcg gggagaggcg gtttgcgtat tgggcgctct 9360

tccgcttcct cgctcactga ctcgctgcgc tcggtcgttc ggctgcggcg agcggtatca 9420

gctcactcaa aggcggtaat acggttatcc acagaatcag gggataacgc aggaaagaac 9480

atgtgagcaa aaggccagca aaaggccagg aaccgtaaaa aggccgcgtt gctggcgttt 9540

ttccataggc tccgcccccc tgacgagcat cacaaaaatc gacgctcaag tcagaggtgg 9600

cgaaacccga caggactata aagataccag gcgtttcccc ctggaagctc cctcgtgcgc 9660

tctcctgttc cgaccctgcc gcttaccgga tacctgtccg cctttctccc ttcgggaagc 9720

gtggcgcttt ctcaatgctc acgctgtagg tatctcagtt cggtgtaggt cgttcgctcc 9780

aagctgggct gtgtgcacga accccccgtt cagcccgacc gctgcgcctt atccggtaac 9840

tatcgtcttg agtccaaccc ggtaagacac gacttatcgc cactggcagc agccactggt 9900

aacaggatta gcagagcgag gtatgtaggc ggtgctacag agttcttgaa gtggtggcct 9960

aactacggct acactagaag gacagtattt ggtatctgcg ctctgctgaa gccagttacc 10020

ttcggaaaaa gagttggtag ctcttgatcc ggcaaacaaa ccaccgctgg tagcggtggt 10080

ttttttgttt gcaagcagca gattacgcgc agaaaaaaag gatctcaaga agatcctttg 10140

atcttttcta cggggtctga cgctcagtgg aacgaaaact cacgttaagg gattttggtc 10200

atgagattat caaaaaggat cttcacctag atccttttaa attaaaaatg aagttttaaa 10260

tcaatctaaa gtatatatga gtaaacttgg tctgacagtt accaatgctt aatcagtgag 10320

gcacctatct cagcgatctg tctatttcgt tcatccatag ttgcctgact ccccgtcgtg 10380

tagataacta cgatacggga gggcttacca tctggcccca gtgctgcaat gataccgcga 10440

gacccacgct gaccggctcc agatttatca gcaataaacc agccagccgg aagggccgag 10500

cgcagaagtg gtcctgcaac tttatccgcc tccatccagt ctattaattg ttgccgggaa 10560

gctagagtaa gtagttcgcc agttaatagt ttgcgcaacg ttgttgccat tgctacaggc 10620

atcgtggtgt cacgctcgtc gtttggtatg gcttcactca gctccggttc ccaacgatca 10680

aggcgagtta catgatcccc catgttgtgc aaaaaagcgg ttagctcctt cggtcctccg 10740

atcgttgtca gaagtaagtt ggccgcagtg ttatcactca tggttatggc agcactgcat 10800

aattctctta ctgtcatgcc atccgtaaga tgcttttctg tgactggtga gtactcaacc 10860

aagtcattct gagaatagtg tatgcggcga ccgagttgct cttgcccggc gtcaatacgg 10920

gataataccg cgccacatag cagaacttta aaagtgctca tcattggaaa acgttcttcg 10980

gggcgaaaac tctcaaggat cttaccgctg ttgagatcca gttcgatgta acccactcgt 11040

gcacccaact gatcttcagc atcttttact ttcaccagcg tttctgggtg agcaaaaaca 11100

ggaaggcaaa atgccgcaaa aaagggaata agggcgacac ggaaatgttg aatactcata 11160

ctcttccttt ttcaatatta ttgaagcatt tatcagggtt attgtctcat gagcggatac 11220

atatttgaat gtatttagaa aaataaagaa ataggggttc tgcgcacatt tccccgaaaa 11280

gtgccacctg aaattgtaaa cgttaatgtt ttgttaaatt tcgcgttaaa tatttgttaa 11340

atcagcttat tttttaacca gtaagcagaa aatgacaaaa atccttataa atcaaaagaa 11400

tagaccgagt tagttgtgag tgttgttcca gtttggaaca agagtccact attaaagaac 11460

gtggactcca acgtaaaacc gtctatcagg gcgatggccc actacgtgaa ccatcaccca 11520

aatcaagttt ttggaggtcg aggtgccgta aagcactaaa tcggaaccct aaagggagcc 11580

cccgatttag agcttgacgg ggaaagccgg cgaacgtggc gagaaaggaa gggaagaaag 11640

cgaaaggagc gggcgctagg gcgctggcaa gtgtagcggt cacgctgcgc gtaaccacca 11700

cacccgccgc gcttaatgcg ccgctactgg gcgcgtccca ttcgccattc aggctgcgca 11760

actgttggga agggcgatcg gtgcgggcct cttcgctatt acgccagctg gcgaaagggg 11820

gatgtgctgc aaggcgatta agttgggtaa cgccagggtt ttcccagtca cgacgttgta 11880

aaacgacggc cagtgaatta tcgattaaat taaccctcac taaagggaga gacgaattcg 11940

aatccaaaaa ttacggatat gaatataggc atatccgtat ccgaattatc cgtttgacag 12000

ctagcaacga ttgtacaatt gcttctttaa aaaaggaaga aagaaagaaa gaaaagaatc 12060

aacatcagcg ttaacaaacg gccccgttac ggcccaaacg gtcatataga gtaacggcgt 12120

taagcgttga aagactccta tcgaaatacg taaccgcaaa cgtgtcatag tcagatcccc 12180

tcttccttca ccgcctcaaa cacaaaaata atcttctaca gcctatatat acaacccccc 12240

cttctatctc tcctttctca caattcatca tctttctttc tctaccccca attttaagaa 12300

atcctctctt ctcctcttca ttttcaaggt aaatctctct ctctctctct ctctctgtta 12360

ttccttgttt taattaggta tgtattattg ctagtttgtt aatctgctta tcttatgtat 12420

gccttatgtg aatatcttta tcttgttcat ctcatccgtt tagaagctat aaatttgttg 12480

atttgactgt gtatctacac gtggttatgt ttatatctaa tcagatatga atttcttcat 12540

attgttgcgt ttgtgtgtac caatccgaaa tcgttgattt ttttcattta atcgtgtagc 12600

taattgtacg tatacatatg gatctacgta tcaattgttc atctgtttgt gtttgtatgt 12660

atacagatct gaaaacatca cttctctcat ctgattgtgt tgttacatac atagatatag 12720

atctgttata tcattttttt tattaattgt gtatatatat atgtgcatag atctggatta 12780

catgattgtg attatttaca tgattttgtt atttacgtat gtatatatgt agatctggac 12840

tttttggagt tgttgacttg attgtatttg tgtgtgtata tgtgtgttct gatcttgata 12900

tgttatgtat gtgcagctga accctcga 12928

<210> 71
<211> 16875
<212> DNA
<213> Artificial Sequence

<220>
<223>  pZFNnptII_LbCpf1_crRNA_ALS_G/T vector

<400>  71
ccggattgaa agaaatatag tttaaatatt tattgataaa ataacaagtc aggtattata      60

gtccaagcaa aaacataaat ttattgatgc aagtttaaat tcagaaatat ttcaataact     120

gattatatca gctggtacat tgccgtagat gaaagactga gtgcgatatt atgtgtaata     180

cataaattga tgatatagct agcttagctc atcgggtcag aagaactcgt caagaaggcg     240

atagaaggcg atgcgctgcg aatcgggagc ggcgataccg taaagcacga ggaagcggtc     300

agcccattcg ccgccaagct cttcagcaat atcacgggta gccaacgcta tgtcctgata     360

gcggtccgcc acacccagcc ggccacagtc gatgaatcca gaaaagcggc cattttccac     420

catgatattc ggcaagcagg catcgccatg agtcacgacg agatcctcgc cgtcgggcat     480

gcgcgccttg agcctggcga acagttcggc tggcgcgagc ccctgatgct cttcgtccag     540

atcatcctga tcgacaagac cggcttccat ccgagtacgt gctcgctcga tgcgatgttt     600

cgcttggtgg tcgaatgggc aggtagccgg atcaagcgta tgcagccgcc gcattgcatc     660

agccatgatg gatactttct cggcaggagc aaggtgagat gacaggagat cctgccccgg     720

cacttcgccc aatagcagcc agtcccttcc cgcttcagtg acaacgtcga gcacagctgc     780

gcaaggaacg cccgtcgtgg ccagccacga tagccgcgct gcctcgtcct gaagttcatt     840

cagggcaccg gacaggtcgg tcttgacaaa aagaaccggg cgcccctgcg ctgacagccg     900

gaacacggcg gcatcagagc agccgattgt ctgttgtgcc cagtcatagc cgaatagcct     960

ctccacccaa gcggccggag aacctgcgtg caatccatct tgttcaatcc aagctcccat    1020

tttctgtgat tttggatgtg tttttgtatg gattgagagt gaatatgaga ctctaattgg    1080

ataccgaggg gaatttatgg aacgtcagtg gagcattttt gacaagaaat atttgctagc    1140

tgatagtgac cttaggcgac ttttgaacgc gcaataatgg tttctgacgt atgtgcttag    1200

ctcattaaac tccagaaacc cgcggctgag tggctccttc aacgttgcgg ttctgtcagt    1260

tccaaacgta aaacggcttg tcccgcgtca tcggcggggg tcattccgga ctgtggggcc    1320

atatcccaga actggttgag tcggtccaac acctgggtgc caatcatgtc gatggtgggg    1380

tatggccaat tttttttcaa ttcaaaaatg tagatgtccg cagcgttatt ataaaatgaa    1440

agtacatttt gataaaacga caaattacga tccgtcgtat ttataggcga aagcaataaa    1500

caaattattc taattcggaa atctttattt cgacgtgtct acattcacgt ccaaatgggg    1560

gcttagatga gaaacttcac gatcggctct agaggccatg gcggccgcac tagtggatcc    1620

cccgggatgc atgaattcaa gcttatcgat gtcgacaggc cttaagggcc agatcccccg    1680

ggctgcagaa gcctatactg tacttaactt gattgcataa ttacttgatc atagactcat    1740

agtaaacttg attacacaga taagtgaaga aacaaaccaa ttcaagacat aaccaaagag 1800

aggtgaaaga ctgttttata tgtctaacat tgcaccttaa tatcacactg ttagttcctt 1860

tcttacttaa attcaaccca ttaaagtaaa aacaacagat aataataatt tgagaatgaa 1920

caaaaggacc atatcattta ttaactctta tccatccatt tgcattttga tgtccgaaaa 1980

caaaaactga aagaacacag taaattacaa gcagaacaaa tgatagaaga aaacagcttt 2040

tccaatgcca taatactcaa acttagtagg attctggtgt gtgggcaatg aaactgatgc 2100

attgaacttg acgaacgttg tcgaaaccga tgatacggac gaaagctggg aggcctggtc 2160

ccattcgcca tgccgaagca tgttgcccag ccggcgccag cgaggaggct gggaccatgc 2220

cggcctagaa gggtaacctg ttggtatgga tctacactta gtagaaatta atctacactt 2280

agtagaaatt agacgagctt actcgtttcg tcctcacgga ctcatcagta atttggatcc 2340

gctgcacata cataacatat caagatcaga acacacatat acacacacaa atacaatcaa 2400

gtcaacaact ccaaaaagtc cagatctaca tatatacata cgtaaataac aaaatcatgt 2460

aaataatcac aatcatgtaa tccagatcta tgcacatata tatatacaca attaataaaa 2520

aaaatgatat aacagatcta tatctatgta tgtaacaaca caatcagatg agagaagtga 2580

tgttttcaga tctgtataca tacaaacaca aacagatgaa caattgatac gtagatccat 2640

atgtatacgt acaattagct acacgattaa atgaaaaaaa tcaacgattt cggattggta 2700

cacacaaacg caacaatatg aagaaattca tatctgatta gatataaaca taaccacgtg 2760

tagatacaca gtcaaatcaa caaatttata gcttctaaac ggatgagatg aacaagataa 2820

agatattcac ataaggcata cataagataa gcagattaac aaactagcaa taatacatac 2880

ctaattaaaa caaggaataa cagagagaga gagagagaga gagatttacc ttgaaaatga 2940

agaggagaag agaggatttc ttaaaattgg gggtagagaa agaaagatga tgaattgtga 3000

gaaaggagag atagaagggg gggttgtata tataggctgt agaagattat ttttgtgttt 3060

gaggcggtga aggaagaggg gatctgacta tgacacgttt gcggttacgt atttcgatag 3120

gagtctttca acgcttaacg ccgttactct atatgaccgt ttgggccgta acggggccgt 3180

ttgttaacgc tgatgttgat tcttttcttt ctttctttct tcctttttta aagaagcaat 3240

tgtacaatcg ttgctagctg tcaaacggat aattcggata cggatatgcc tatattcata 3300

tccgtaattt ttctgcagga attcgatctg gcacgacagg tttcccgact ggaaagcggg 3360

cagtgagcgc aacgcaatta atgtgagtta gctcactcat taggcacccc aggctttaca 3420

ctttatgctt ccggctcgta tgttgtgtgg aattgtgagc ggataacaat ttcacacagg 3480

aaacagctat gacatgatta cgaattcaaa aattacggat atgaatatag gcatatccgt 3540

atccgaatta tccgtttgac agctagcaac gattgtacaa ttgcttcttt aaaaaaggaa 3600

gaaagaaaga aagaaaagaa tcaacatcag cgttaacaaa cggccccgtt acggcccaaa 3660

cggtcatata gagtaacggc gttaagcgtt gaaagactcc tatcgaaata cgtaaccgca 3720

aacgtgtcat agtcagatcc cctcttcctt caccgcctca aacacaaaaa taatcttcta 3780

cagcctatat atacaacccc cccttctatc tctcctttct cacaattcat catctttctt 3840

tctctacccc caattttaag aaatcctctc ttctcctctt cattttcaag gtaaatctct 3900

ctctctctct ctctctctgt tattccttgt tttaattagg tatgtattat tgctagtttg 3960

ttaatctgct tatcttatgt atgccttatg tgaatatctt tatcttgttc atctcatccg 4020

tttagaagct ataaatttgt tgatttgact gtgtatctac acgtggttat gtttatatct 4080

aatcagatat gaatttcttc atattgttgc gtttgtgtgt accaatccga aatcgttgat 4140

ttttttcatt taatcgtgta gctaattgta cgtatacata tggatctacg tatcaattgt 4200

tcatctgttt gtgtttgtat gtatacagat ctgaaaacat cacttctctc atctgattgt 4260

gttgttacat acatagatat agatctgtta tatcattttt tttattaatt gtgtatatat 4320

atatgtgcat agatctggat tacatgattg tgattattta catgattttg ttatttacgt 4380

atgtatatat gtagatctgg actttttgga gttgttgact tgattgtatt tgtgtgtgta 4440

tatgtgtgtt ctgatcttga tatgttatgt atgtgcagcg aattcggcgc gccatggctc 4500

ctaagaagaa gaggaaggtt agcaagctcg agaagtttac caactgctac agcctctcta 4560

agaccctcag gttcaaggct atccctgtgg gaaagaccca agagaatatc gacaacaaga 4620

ggctcctcgt cgaggatgag aagagagctg aagattacaa gggcgtgaag aagctcctcg 4680

acaggtacta cctcagcttc atcaacgatg tgctccacag catcaagctc aagaacctca 4740

acaactacat cagcctcttc cgtaagaaaa ccaggaccga gaaagagaac aaagagcttg 4800

agaacctcga gatcaacctc cgtaaagaga tcgccaaggc tttcaaggga aacgagggat 4860

acaagagcct cttcaagaag gatattatcg agacaatcct gcctgagttc ctggacgata 4920

aggatgagat cgctctcgtg aacagcttca acggattcac tactgccttc accggattct 4980

tcgacaacag ggaaaacatg ttcagcgaag aggccaagag cacctctatc gctttcagat 5040

gcatcaacga gaacctcacg cgttacatca gcaacatgga catcttcgag aaggtggacg 5100

ccatcttcga taagcacgag gtgcaagaaa tcaaagagaa gatcctcaac agcgactacg 5160

acgtcgagga cttttttgaa ggggagttct tcaacttcgt tctcacccaa gagggcatcg 5220

acgtgtacaa cgctattatc ggaggattcg tgaccgagtc tggggagaag attaagggac 5280

tcaacgagta catcaacctg tacaaccaga aaacgaagca gaagctcccg aagttcaagc 5340

cgctctacaa gcaggttctc tctgatcgtg agagcctctc attttacggt gagggttaca 5400

cctctgacga ggaagtgctt gaggttttcc gtaacaccct caacaagaac agcgagatct 5460

tctcgtccat caagaagttg gagaaacttt tcaagaactt cgacgagtac agcagcgctg 5520

ggatcttcgt taagaacgga cctgctatca gcaccatcag caaggatatt ttcggcgagt 5580

ggaacgtgat cagggacaag tggaatgctg agtacgatga catccacctc aagaagaagg 5640

ctgtcgtcac tgagaagtac gaggatgaca ggcgtaagtc gttcaagaag atcggctctt 5700

tcagcctcga gcagcttcaa gaatacgctg atgctgatct cagcgtggtc gagaagctca 5760

aagagatcat catccagaag gtcgacgaga tctacaaggt gtacgggtcc tctgagaagt 5820

tgttcgatgc tgatttcgtc ctcgagaaga gtctgaagaa gaacgacgct gtcgtcgcga 5880

tcatgaagga tttgctcgac agcgtgaagt ccttcgagaa ctatatcaag gccttcttcg 5940

gagagggcaa agagactaat agggacgagt ctttctacgg ggatttcgtg ctcgcttacg 6000

atatcctcct caaggtggac catatctacg acgccatcag aaactacgtg acccagaagc 6060

cttacagcaa ggacaagttc aagttgtact ttcagaaccc gcagttcatg ggcggatggg 6120

acaaagacaa agagacagat tacagggcca ccatcctcag gtacgggtct aagtactacc 6180

tggccatcat ggacaagaaa tacgccaagt gcctccaaaa gatcgacaag gatgacgtga 6240

acgggaacta tgagaagatc aactacaagc tccttccggg accgaacaag atgcttccta 6300

aggtgttctt cagcaagaaa tggatggcct actacaaccc gtctgaggac atccagaaaa 6360

tctacaagaa cgggaccttc aagaaaggcg acatgttcaa cctcaacgac tgccacaagc 6420

tcatcgattt cttcaaggac agcatctcgc gttacccgaa gtggtctaac gcttacgact 6480

ttaacttcag cgagacagaa aagtacaagg atatcgccgg gttctaccgt gaggttgagg 6540

aacagggtta caaggttagc ttcgagagcg cctccaagaa agaggttgac aagttggtcg 6600

aagagggcaa gctctacatg ttccagatct ataacaagga cttctccgac aagagccacg 6660

gaactcctaa cctccatacg atgtacttca agctgctttt cgacgagaac aaccacgggc 6720

agatcagact ttctggtggt gctgaactct tcatgcgtag ggcctcactc aagaaagaag 6780

agttggttgt tcacccggcc aactctccaa tcgctaacaa gaatcctgac aacccgaaaa 6840

agaccaccac gctgtcttac gacgtctaca aggacaaaag gttcagcgag gaccagtacg 6900

agcttcatat cccgatcgct atcaacaagt gcccgaagaa catcttcaag atcaataccg 6960

aggtgagggt gctgctcaag cacgatgata acccttacgt gatcggaatc gatcgtggtg 7020

agagaaacct cctctacatc gttgtggtgg acggaaaggg aaacatcgtc gagcagtaca 7080

gcctgaacga gattatcaac aatttcaacg gcatcaggat caagaccgac taccactcac 7140

tcctcgataa gaaagaaaaa gagcgtttcg aggccaggca gaactggact tctatcgaaa 7200

acatcaaaga gttgaaggcc ggctacatct ctcaggtggt gcataagatc tgcgagctgg 7260

tggaaaagta cgatgctgtg atcgctcttg aggacctcaa ctctgggttc aagaacagta 7320

gagtgaaggt tgagaagcag gtctaccaaa agttcgagaa gatgctcatc gacaagctca 7380

actacatggt ggacaaaaag agcaaccctt gcgctaccgg tggtgctctt aagggatacc 7440

agatcacgaa caagttcgag tccttcaaga gcatgagcac ccagaacggc ttcatcttct 7500

atatccctgc ttggctcacc agcaagatcg atccttctac tggtttcgtg aacctgctca 7560

agaccaagta cacctcgatc gccgacagca agaagttcat ctcgtctttc gacaggatca 7620

tgtacgtgcc ggaagaggat cttttcgagt tcgctctcga ctataagaac ttcagcagga 7680

ccgacgccga ctacattaag aagtggaagc tctactccta cgggaaccgt atcaggatct 7740

tccgaaatcc gaagaaaaac aacgtgttcg actgggaaga agtgtgcctc acctctgcct 7800

acaaagaact gttcaacaag tacggcatca actaccagca gggtgatatc agggctcttt 7860

tgtgcgagca gagcgacaag gcattctaca gctcattcat ggccctcatg tctctcatgc 7920

tccagatgag gaactctatc accggaagga ccgatgtgga cttccttatc tctccggtca 7980

agaactctga cgggatcttc tacgacagcc gtaactatga ggctcaagag aacgctatcc 8040

tgccgaagaa tgctgatgca aacggggctt acaacattgc gagaaaggtt ctctgggcta 8100

tcgggcagtt taagaaagcg gaagatgaga agctcgacaa ggtgaagatc gccatctcca 8160

acaaagagtg gcttgagtac gctcagacct ccgttaagca caagaggcct gctgctacta 8220

agaaagctgg ccaggccaaa aagaagaagt gaggcgcgcc gagctccagg cctcccagct 8280

ttcgtccgta tcatcggttt cgacaacgtt cgtcaagttc aatgcatcag tttcattgcc 8340

cacacaccag aatcctacta agtttgagta ttatggcatt ggaaaagctg ttttcttcta 8400

tcatttgttc tgcttgtaat ttactgtgtt ctttcagttt ttgttttcgg acatcaaaat 8460

gcaaatggat ggataagagt taataaatga tatggtcctt ttgttcattc tcaaattatt 8520

attatctgtt gtttttactt taatgggttg aatttaagta agaaaggaac taacagtgtg 8580

atattaaggt gcaatgttag acatataaaa cagtctttca cctctctttg gttatgtctt 8640

gaattggttt gtttcttcac ttatctgtgt aatcaagttt actatgagtc tatgatcaag 8700

taattatgca atcaagttaa gtacagtata ggcttgagct ccctaggatc aagcttatcg 8760

atgtcgacag gccttaaggg ccagatcttg ggcccggtac ccgatcagat tgtcgtttcc 8820

cgccttcggt ttaaactatc agtgtttgac aggatatatt ggcgggtaaa cctaagagaa 8880

aagagcgttt attagaataa tcggatattt aaaagggcgt gaaaaggttt atccgttcgt 8940

ccatttgtat gtgcatgcca accacagggt tcccctcggg agtgcttggc attccgtgcg 9000

ataatgactt ctgttcaacc acccaaacgt cggaaagcct gacgacggag cagcattcca 9060

aaaagatccc ttggctcgtc tgggtcggct agaaggtcga gtgggctgct gtggcttgat 9120

ccctcaacgc ggtcgcggac gtagcgcagc gccgaaaaat cctcgatcgc aaatccgacg 9180

ctgtcgaaaa gcgtgatctg cttgtcgctc tttcggccga cgtcctggcc agtcatcacg 9240

cgccaaagtt ccgtcacagg atgatctggc gcgagttgct ggatctcgcc ttcaatccgg 9300

gtctgtggcg ggaactccac gaaaatatcc gaacgcagca agatatcgcg gtgcatctcg 9360

gtcttgcctg ggcagtcgcc gccgacgccg ttgatgtgga cgccgaaaag gatctaggtg 9420

aagatccttt ttgataatct catgaccaaa atcccttaac gtgagttttc gttccactga 9480

gcgtcagacc ccgtagaaaa gatcaaagga tcttcttgag atcctttttt tctgcgcgta 9540

atctgctgct tgcaaacaaa aaaaccaccg ctaccagcgg tggtttgttt gccggatcaa 9600

gagctaccaa ctctttttcc gaaggtaact ggcttcagca gagcgcagat accaaatact 9660

gttcttctag tgtagccgta gttaggccac cacttcaaga actctgtagc accgcctaca 9720

tacctcgctc tgctaatcct gttaccagtg gctgctgcca gtggcgataa gtcgtgtctt 9780

accgggttgg actcaagacg atagttaccg gataaggcgc agcggtcggg ctgaacgggg 9840

ggttcgtgca cacagcccag cttggagcga acgacctaca ccgaactgag atacctacag 9900

cgtgagctat gagaaagcgc cacgcttccc gaagggagaa aggcggacag gtatccggta 9960

agcggcaggg tcggaacagg agagcgcacg agggagcttc cagggggaaa cgcctggtat 10020

ctttatagtc ctgtcgggtt tcgccacctc tgacttgagc gtcgattttt gtgatgctcg 10080

tcaggggggc ggagcctatg gaaaaacgcc agcaacgcgg cctttttacg gttcctggcc 10140

ttttgctggc cttttgctca catgttcttt cctgcgttat cccctgattc tgtggataac 10200

cgattaccgc ctttgagtga gctgataccg ctcgccgcag ccgaacgacc gagcgcagcg 10260

agtcagtgag cgaggaagcg gaagagcgcc tgatgcggta ttttctcctt acgcatctgt 10320

gcggtatttc acaccgcata tggtgcactc tcagtacaat ctgctctgat gccgcatagt 10380

taagccagta tacactccgc tatcgctacg tgactgggtc atggctgcgc cccgacaccc 10440

gccaacaccc gctgacgcgc cctgacgggc ttgtctgctc ccggcatccg cttacagaca 10500

agctgtgacc gtctccggga gctgcatgtg tcagaggttt tcaccgtcat caccgaaacg 10560

cgcgaggcag gggtacgtcg aggtcgatcc aacccctccg ctgctatagt gcagtcggct 10620

tctgacgttc agtgcagccg tcgtctgaaa acgacatgtc gcacaagtcc taagttacgc 10680

gacaggctgc cgccctgccc ttttcctggc gttttcttgt cgcgtgtttt agtcgcataa 10740

agtagaatac ttgcgactag aaccggagac attacgccat gaacaagagc gccgccgctg 10800

gcctgctggg ctatgcccgc gtcagcaccg acgaccagga cttgaccaac caacgggccg 10860

aactgcacgc ggccggctgc accaagctgt tttccgagaa gatcaccggc accaggcgcg 10920

accgcccgga gctggccagg atgcttgacc acctacgccc tggcgacgtt gtgacagtga 10980

ccaggctaga ccgcctggcc cgcagcaccc gcgacctact ggacattgcc gagcgcatcc 11040

aggaggccgg cgcgggcctg cgtagcctgg cagagccgtg ggccgacacc accacgccgg 11100

ccggccgcat ggtgttgacc gtgttcgccg gcattgccga gttcgagcgt tccctaatca 11160

tcgaccgcac ccggagcggg cgcgaggccg ccaaggcgcg aggcgtgaag tttggccccc 11220

gccctaccct caccccggca cagatcgcgc acgcccgcga gctgatcgac caggaaggcc 11280

gcaccgtgaa agaggcggct gcactgcttg gcgtgcatcg ctcgaccctg taccgcgcac 11340

ttgagcgcag cgaggaagtg acgcccaccg aggccaggcg gcgcggtgcc ttccgtgagg 11400

acgcattgac cgaggccgac gccctggcgg ccgccgagaa tgaacgccaa gaggaacaag 11460

catgaaaccg caccaggacg gccaggacga accgtttttc attaccgaag agatcgaggc 11520

ggagatgatc gcggccgggt acgtgttcga gccgcccgcg cacgtctcaa ccgtgcggct 11580

gcatgaaatc ctggccggtt tgtctgatgc caagctcgcg gcctggccgg cgagcttggc 11640

cgctgaagaa accgagcgcc gccgtctaaa aaggtgatgt gtatttgagt aaaacagctt 11700

gcgtcatgcg gtcgctgcgt atatgatgcg atgagtaaat aaacaaatac gcaaggggaa 11760

cgcatgaagg ttatcgctgt acttaaccag aaaggcgggt caggcaagac gaccatcgca 11820

acccatctag cccgcgccct gcaactcgcc ggggccgatg ttctgttagt cgattccgat 11880

ccccagggca gtgcccgcga ttgggcggcc gtgcgggaag atcaaccgct aaccgttgtc 11940

ggcatcgacc gcccgacgat tgaccgcgac gtgaaggcca tcggccggcg cgacttcgta 12000

gtgatcgacg gagcgcccca ggcggcggac ttggctgtgt ccgcgatcaa ggcagccgac 12060

ttcgtgctga ttccggtgca gccaagccct tacgacatat gggccaccgc cgacctggtg 12120

gagctggtta agcagcgcat tgaggtcacg gatggaaggc tacaagcggc ctttgtcgtg 12180

tcgcgggcga tcaaaggcac gcgcatcggc ggtgaggttg ccgaggcgct ggccgggtac 12240

gagctgccca ttcttgagtc ccgtatcacg cagcgcgtga gctacccagg cactgccgcc 12300

gccggcacaa ccgttcttga atcagaaccc gagggcgacg ctgcccgcga ggtccaggcg 12360

ctggccgctg aaattaaatc aaaactcatt tgagttaatg aggtaaagag aaaatgagca 12420

aaagcacaaa cacgctaagt gccggccgtc cgagcgcacg cagcagcaag gctgcaacgt 12480

tggccagcct ggcagacacg ccagccatga agcgggtcaa ctttcagttg ccggcggagg 12540

atcacaccaa gctgaagatg tacgcggtac gccaaggcaa gaccattacc gagctgctat 12600

ctgaatacat cgcgcagcta ccagagtaaa tgagcaaatg aataaatgag tagatgaatt 12660

ttagcggcta aaggaggcgg catggaaaat caagaacaac caggcaccga cgccgtggaa 12720

tgccccatgt gtggaggaac gggcggttgg ccaggcgtaa gcggctgggt tgtctgccgg 12780

ccctgcaatg gcactggaac ccccaagccc gaggaatcgg cgtgagcggt cgcaaaccat 12840

ccggcccggt acaaatcggc gcggcgctgg gtgatgacct ggtggagaag ttgaaggcgg 12900

cgcaggccgc ccagcggcaa cgcatcgagg cagaagcacg ccccggtgaa tcgtggcaag 12960

cggccgctga tcgaatccgc aaagaatccc ggcaaccgcc ggcagccggt gcgccgtcga 13020

ttaggaagcc gcccaagggc gacgagcaac cagatttttt cgttccgatg ctctatgacg 13080

tgggcacccg cgatagtcgc agcatcatgg acgtggccgt tttccgtctg tcgaagcgtg 13140

accgacgagc tggcgaggtg atccgctacg agcttccaga cgggcacgta gaggtttccg 13200

cagggccggc cggcatggcg agtgtgtggg attacgacct ggtactgatg gcggtttccc 13260

atctaaccga atccatgaac cgataccggg aagggaaggg agacaagccc ggccgcgtgt 13320

tccgtccaca cgttgcggac gtactcaagt tctgccggcg agccgatggc ggaaagcaga 13380

aagacgacct ggtagaaacc tgcattcggt taaacaccac gcacgttgcc atgcagcgta 13440

cgaagaaggc caagaacggc cgcctggtga cggtatccga gggtgaagcc ttgattagcc 13500

gctacaagat cgtaaagagc gaaaccgggc ggccggagta catcgagatc gagctagctg 13560

attggatgta ccgcgagatc acagaaggca agaacccgga cgtgctgacg gttcaccccg 13620

attacttttt gatcgatccc ggcatcggcc gttttctcta ccgcctggca cgccgcgccg 13680

caggcaaggc agaagccaga tggttgttca agacgatcta cgaacgcagt ggcagcgccg 13740

gagagttcaa gaagttctgt ttcaccgtgc gcaagctgat cgggtcaaat gacctgccgg 13800

agtacgattt gaaggaggag gcggggcagg ctggcccgat cctagtcatg cgctaccgca 13860

acctgatcga gggcgaagca tccgccggtt cctaatgtac ggagcagatg ctagggcaaa 13920

ttgccctagc aggggaaaaa ggtcgaaaag gtctctttcc tgtggatagc acgtacattg 13980

ggaacccaaa gccgtacatt gggaaccgga acccgtacat tgggaaccca aagccgtaca 14040

ttgggaaccg gtcacacatg taagtgactg atataaaaga gaaaaaaggc gatttttccg 14100

cctaaaactc tttaaaactt attaaaactc ttaaaacccg cctggcctgt gcataactgt 14160

ctggccagcg cacagccgaa gagctgcaaa aagcgcctac ccttcggtcg ctgcgctccc 14220

tacgccccgc cgcttcgcgt cggcctatcg cggccgctgg ccgctcaaaa atggctggcc 14280

tacggccagg caatctacca gggcgcggac aagccgcgcc gtcgccactc gaccgccggc 14340

gcccacatca aggcaccggt gggtatgcct gacgatgcgt ggagaccgaa accttgcgct 14400

cgttcgccag ccaggacaga aatgcctcga cttcgctgct gcccaaggtt gccgggtgac 14460

gcacaccgtg gaaacggatg aaggcacgaa cccagtggac ataagcctgt tcggttcgta 14520

agctgtaatg caagtagcgt atgcgctcac gcaactggtc cagaaccttg accgaacgca 14580

gcggtggtaa cggcgcagtg gcggttttca tggcttgtta tgactgtttt tttggggtac 14640

agtctatgcc tcgggcatcc aagcagcaag cgcgttacgc cgtgggtcga tgtttgatgt 14700

tatggagcag caacgatgtt acgcagcagg gcagtcgccc taaaacaaag ttaaacatca 14760

tgagggaagc ggtgatcgcc gaagtatcga ctcaactatc agaggtagtt ggcgtcatcg 14820

agcgccatct cgaaccgacg ttgctggccg tacatttgta cggctccgca gtggatggcg 14880

gcctgaagcc acacagtgat attgatttgc tggttacggt gaccgtaagg cttgatgaaa 14940

caacgcggcg agctttgatc aacgaccttt tggaaacttc ggcttcccct ggagagagcg 15000

agattctccg cgctgtagaa gtcaccattg ttgtgcacga cgacatcatt ccgtggcgtt 15060

atccagctaa gcgcgaactg caatttggag aatggcagcg caatgacatt cttgcaggta 15120

tcttcgagcc agccacgatc gacattgatc tggctatctt gctgacaaaa gcaagagaac 15180

atagcgttgc cttggtaggt ccagcggcgg aggaactctt tgatccggtt cctgaacagg 15240

atctatttga ggcgctaaat gaaaccttaa cgctatggaa ctcgccgccc gactgggctg 15300

gcgatgagcg aaatgtagtg cttacgttgt cccgcatttg gtacagcgca gtaaccggca 15360

aaatcgcgcc gaaggatgtc gctgccgact gggcaatgga gcgcctgccg gcccagtatc 15420

agcccgtcat acttgaagct agacaggctt atcttggaca agaagaagat cgcttggcct 15480

cgcgcgcaga tcagttggaa gaatttgtcc actacgtgaa aggcgagatc accaaggtag 15540

tcggcaaata atgtctaaca attcgttcaa gccgacgccg cttcgcggcg cggcttaact 15600

caagcgttag atgcactaag cacataattg ctcacagcca aactatcagg tcaagtctgc 15660

ttttattatt tttaagcgtg cataataagc cctacacaaa ttgggagata tatcatgaaa 15720

ggctggcttt ttcttgttat cgcaatagtt ggcgaagtaa tcgcaacata gcttgcttgg 15780

tcgttccgcg tgaacgtcgg ctcgattgta cctgcgttca aatactttgc gatcgtgttg 15840

cgcgcctgcc cggtgcgtcg gctgatctca cggatcgact gcttctctcg caacgccatc 15900

cgacggatga tgtttaaaag tcccatgtgg atcactccgt tgccccgtcg ctcaccgtgt 15960

tggggggaag gtgcacatgg ctcagttctc aatggaaatt atctgcctaa ccggctcagt 16020

tctgcgtaga aaccaacatg caagctccac cgggtgcaaa gcggcagcgg cggcaggata 16080

tattcaattg taaatggctt catgtccggg aaatctacat ggatcagcaa tgagtatgat 16140

ggtcaatatg gagaaaaaga aagagtaatt accaattttt tttcaattca aaaatgtaga 16200

tgtccgcagc gttattataa aatgaaagta cattttgata aaacgacaaa ttacgatccg 16260

tcgtatttat aggcgaaagc aataaacaaa ttattctaat tcggaaatct ttatttcgac 16320

gtgtctacat tcacgtccaa atgggggctt agatgagaaa cttcacgatc ggctctagtt 16380

cgagcgatct agtaacatag atgacaccgc gcgcgataat ttatcctagt ttgcgcgcta 16440

tattttgttt tctatcgcgt attaaatgta taattgcggg actctaatca taaaaaccca 16500

tctcataaat aacgtcatgc attacatgtt aattattaca tgcttaacgt aattcaacag 16560

aaattatatg ataatcatcg caagaccggc aacaggattc aatcttaaga aactttattg 16620

ccaaatgttt gaacgatcgg ggaaattcgt ttctatgctt caattctgct ctatagatgt 16680

tcttctagtc ttaactttta gaaggcacaa catttgaaat gttggtaatt tttctgctgg 16740

gtagtgaacc attgtaattt gtgtttgctt atattatccg ttgggtttct ctatgttatc 16800

tgtggggcca tatcccagaa ctggttgagt cggtccaaca cctgggtgcc aatcatgtcg 16860

atggtggggt atggt                                                    16875

<210>  72
<211>  16875
<212>  DNA
<213>  Artificial Sequence

<220>
<223>  pZFNnptII_LbCpf1_crRNA_ALS_In1 vector

<400>  72
ccggattgaa agaaatatag tttaaatatt tattgataaa ataacaagtc aggtattata      60

gtccaagcaa aaacataaat ttattgatgc aagtttaaat tcagaaatat ttcaataact     120

gattatatca gctggtacat tgccgtagat gaaagactga gtgcgatatt atgtgtaata     180

cataaattga tgatatagct agcttagctc atcgggtcag aagaactcgt caagaaggcg     240

atagaaggcg atgcgctgcg aatcgggagc ggcgataccg taaagcacga ggaagcggtc     300

agcccattcg ccgccaagct cttcagcaat atcacgggta gccaacgcta tgtcctgata     360

gcggtccgcc acacccagcc ggccacagtc gatgaatcca gaaaagcggc cattttccac     420

catgatattc ggcaagcagg catcgccatg agtcacgacg agatcctcgc cgtcgggcat     480

gcgcgccttg agcctggcga acagttcggc tggcgcgagc ccctgatgct cttcgtccag     540

atcatcctga tcgacaagac cggcttccat ccgagtacgt gctcgctcga tgcgatgttt     600

cgcttggtgg tcgaatgggc aggtagccgg atcaagcgta tgcagccgcc gcattgcatc     660

agccatgatg gatactttct cggcaggagc aaggtgagat gacaggagat cctgccccgg     720

cacttcgccc aatagcagcc agtcccttcc cgcttcagtg acaacgtcga gcacagctgc     780

gcaaggaacg cccgtcgtgg ccagccacga tagccgcgct gcctcgtcct gaagttcatt     840

cagggcaccg gacaggtcgg tcttgacaaa aagaaccggg cgcccctgcg ctgacagccg     900

gaacacggcg gcatcagagc agccgattgt ctgttgtgcc cagtcatagc cgaatagcct     960

ctccacccaa gcggccggag aacctgcgtg caatccatct tgttcaatcc aagctcccat    1020

tttctgtgat tttggatgtg tttttgtatg gattgagagt gaatatgaga ctctaattgg    1080

ataccgaggg gaatttatgg aacgtcagtg gagcattttt gacaagaaat atttgctagc    1140

tgatagtgac cttaggcgac ttttgaacgc gcaataatgg tttctgacgt atgtgcttag    1200

ctcattaaac tccagaaacc cgcggctgag tggctccttc aacgttgcgg ttctgtcagt    1260

tccaaacgta aaacggcttg tcccgcgtca tcggcggggg tcattccgga ctgtggggcc    1320

atatcccaga actggttgag tcggtccaac acctgggtgc caatcatgtc gatggtgggg    1380

tatggccaat tttttttcaa ttcaaaaatg tagatgtccg cagcgttatt ataaaatgaa    1440

agtacatttt gataaaacga caaattacga tccgtcgtat ttataggcga aagcaataaa    1500

caaattattc taattcggaa atctttattt cgacgtgtct acattcacgt ccaaatgggg    1560

gcttagatga gaaacttcac gatcggctct agaggccatg gcggccgcac tagtggatcc 1620

cccgggatgc atgaattcaa gcttatcgat gtcgacaggc cttaagggcc agatcccccg 1680

ggctgcagaa gcctatactg tacttaactt gattgcataa ttacttgatc atagactcat 1740

agtaaacttg attacacaga taagtgaaga aacaaaccaa ttcaagacat aaccaaagag 1800

aggtgaaaga ctgttttata tgtctaacat tgcaccttaa tatcacactg ttagttcctt 1860

tcttacttaa attcaaccca ttaaagtaaa aacaacagat aataataatt tgagaatgaa 1920

caaaaggacc atatcattta ttaactctta tccatccatt tgcattttga tgtccgaaaa 1980

caaaaactga aagaacacag taaattacaa gcagaacaaa tgatagaaga aaacagcttt 2040

tccaatgcca taatactcaa acttagtagg attctggtgt gtgggcaatg aaactgatgc 2100

attgaacttg acgaacgttg tcgaaaccga tgatacggac gaaagctggg aggcctggtc 2160

ccattcgcca tgccgaagca tgttgcccag ccggcgccag cgaggaggct gggaccatgc 2220

cggccactac gttgtgttgt aggtatcgga tctacactta gtagaaatta atctacactt 2280

agtagaaatt agacgagctt actcgtttcg tcctcacgga ctcatcagta atttggatcc 2340

gctgcacata cataacatat caagatcaga acacacatat acacacacaa atacaatcaa 2400

gtcaacaact ccaaaaagtc cagatctaca tatatacata cgtaaataac aaaatcatgt 2460

aaataatcac aatcatgtaa tccagatcta tgcacatata tatatacaca attaataaaa 2520

aaaatgatat aacagatcta tatctatgta tgtaacaaca caatcagatg agagaagtga 2580

tgttttcaga tctgtataca tacaaacaca aacagatgaa caattgatac gtagatccat 2640

atgtatacgt acaattagct acacgattaa atgaaaaaaa tcaacgattt cggattggta 2700

cacacaaacg caacaatatg aagaaattca tatctgatta gatataaaca taaccacgtg 2760

tagatacaca gtcaaatcaa caaatttata gcttctaaac ggatgagatg aacaagataa 2820

agatattcac ataaggcata cataagataa gcagattaac aaactagcaa taatacatac 2880

ctaattaaaa caaggaataa cagagagaga gagagagaga gagatttacc ttgaaaatga 2940

agaggagaag agaggatttc ttaaaattgg gggtagagaa agaaagatga tgaattgtga 3000

gaaaggagag atagaagggg gggttgtata tataggctgt agaagattat ttttgtgttt 3060

gaggcggtga aggaagaggg gatctgacta tgacacgttt gcggttacgt atttcgatag 3120

gagtctttca acgcttaacg ccgttactct atatgaccgt ttgggccgta acggggccgt 3180

ttgttaacgc tgatgttgat tcttttcttt ctttctttct tcctttttta aagaagcaat 3240

tgtacaatcg ttgctagctg tcaaacggat aattcggata cggatatgcc tatattcata 3300

tccgtaattt ttctgcagga attcgatctg gcacgacagg tttcccgact ggaaagcggg 3360

cagtgagcgc aacgcaatta atgtgagtta gctcactcat taggcacccc aggctttaca 3420

ctttatgctt ccggctcgta tgttgtgtgg aattgtgagc ggataacaat ttcacacagg 3480

aaacagctat gacatgatta cgaattcaaa aattacggat atgaatatag gcatatccgt 3540

atccgaatta tccgtttgac agctagcaac gattgtacaa ttgcttcttt aaaaaaggaa 3600

gaaagaaaga aagaaaagaa tcaacatcag cgttaacaaa cggccccgtt acggcccaaa 3660

cggtcatata gagtaacggc gttaagcgtt gaaagactcc tatcgaaata cgtaaccgca 3720

aacgtgtcat agtcagatcc cctcttcctt caccgcctca aacacaaaaa taatcttcta 3780

cagcctatat atacaacccc cccttctatc tctcctttct cacaattcat catctttctt 3840

tctctacccc caattttaag aaatcctctc ttctcctctt cattttcaag gtaaatctct 3900

ctctctctct ctctctctgt tattccttgt tttaattagg tatgtattat tgctagtttg 3960

ttaatctgct tatcttatgt atgccttatg tgaatatctt tatcttgttc atctcatccg 4020

tttagaagct ataaatttgt tgatttgact gtgtatctac acgtggttat gtttatatct 4080

aatcagatat gaatttcttc atattgttgc gtttgtgtgt accaatccga aatcgttgat 4140

ttttttcatt taatcgtgta gctaattgta cgtatacata tggatctacg tatcaattgt 4200

tcatctgttt gtgtttgtat gtatacagat ctgaaaacat cacttctctc atctgattgt 4260

gttgttacat acatagatat agatctgtta tatcattttt tttattaatt gtgtatatat 4320

atatgtgcat agatctggat tacatgattg tgattattta catgattttg ttatttacgt 4380

atgtatatat gtagatctgg actttttgga gttgttgact tgattgtatt tgtgtgtgta 4440

tatgtgtgtt ctgatcttga tatgttatgt atgtgcagcg aattcggcgc gccatggctc 4500

ctaagaagaa gaggaaggtt agcaagctcg agaagtttac caactgctac agcctctcta 4560

agaccctcag gttcaaggct atccctgtgg gaaagaccca agagaatatc gacaacaaga 4620

ggctcctcgt cgaggatgag aagagagctg aagattacaa gggcgtgaag aagctcctcg 4680

acaggtacta cctcagcttc atcaacgatg tgctccacag catcaagctc aagaacctca 4740

acaactacat cagcctcttc cgtaagaaaa ccaggaccga gaaagagaac aaagagcttg 4800

agaacctcga gatcaacctc cgtaaagaga tcgccaaggc tttcaaggga aacgagggat 4860

acaagagcct cttcaagaag gatattatcg agacaatcct gcctgagttc ctggacgata 4920

aggatgagat cgctctcgtg aacagcttca acggattcac tactgccttc accggattct 4980

tcgacaacag ggaaaacatg ttcagcgaag aggccaagag cacctctatc gctttcagat 5040

gcatcaacga gaacctcacg cgttacatca gcaacatgga catcttcgag aaggtggacg 5100

ccatcttcga taagcacgag gtgcaagaaa tcaaagagaa gatcctcaac agcgactacg 5160

acgtcgagga cttttttgaa ggggagttct tcaacttcgt tctcacccaa gagggcatcg 5220

acgtgtacaa cgctattatc ggaggattcg tgaccgagtc tggggagaag attaagggac 5280

tcaacgagta catcaacctg tacaaccaga aaacgaagca gaagctcccg aagttcaagc 5340

cgctctacaa gcaggttctc tctgatcgtg agagcctctc attttacggt gagggttaca 5400

cctctgacga ggaagtgctt gaggttttcc gtaacaccct caacaagaac agcgagatct 5460

tctcgtccat caagaagttg gagaaacttt tcaagaactt cgacgagtac agcagcgctg 5520

ggatcttcgt taagaacgga cctgctatca gcaccatcag caaggatatt ttcggcgagt 5580

ggaacgtgat cagggacaag tggaatgctg agtacgatga catccacctc aagaagaagg 5640

ctgtcgtcac tgagaagtac gaggatgaca ggcgtaagtc gttcaagaag atcggctctt 5700

tcagcctcga gcagcttcaa gaatacgctg atgctgatct cagcgtggtc gagaagctca 5760

aagagatcat catccagaag gtcgacgaga tctacaaggt gtacgggtcc tctgagaagt 5820

tgttcgatgc tgatttcgtc ctcgagaaga gtctgaagaa gaacgacgct gtcgtcgcga 5880

tcatgaagga tttgctcgac agcgtgaagt ccttcgagaa ctatatcaag gccttcttcg 5940

gagagggcaa agagactaat agggacgagt ctttctacgg ggatttcgtg ctcgcttacg 6000

atatcctcct caaggtggac catatctacg acgccatcag aaactacgtg acccagaagc 6060

cttacagcaa ggacaagttc aagttgtact ttcagaaccc gcagttcatg ggcggatggg 6120

acaaagacaa agagacagat tacagggcca ccatcctcag gtacgggtct aagtactacc 6180

tggccatcat ggacaagaaa tacgccaagt gcctccaaaa gatcgacaag gatgacgtga 6240

acgggaacta tgagaagatc aactacaagc tccttccggg accgaacaag atgcttccta 6300

aggtgttctt cagcaagaaa tggatggcct actacaaccc gtctgaggac atccagaaaa 6360

tctacaagaa cgggaccttc aagaaaggcg acatgttcaa cctcaacgac tgccacaagc 6420

tcatcgattt cttcaaggac agcatctcgc gttacccgaa gtggtctaac gcttacgact 6480

ttaacttcag cgagacagaa aagtacaagg atatcgccgg gttctaccgt gaggttgagg 6540

aacagggtta caaggttagc ttcgagagcg cctccaagaa agaggttgac aagttggtcg 6600

aagagggcaa gctctacatg ttccagatct ataacaagga cttctccgac aagagccacg 6660

gaactcctaa cctccatacg atgtacttca agctgctttt cgacgagaac aaccacgggc 6720

agatcagact ttctggtggt gctgaactct tcatgcgtag ggcctcactc aagaaagaag 6780

agttggttgt tcacccggcc aactctccaa tcgctaacaa gaatcctgac aacccgaaaa 6840

agaccaccac gctgtcttac gacgtctaca aggacaaaag gttcagcgag gaccagtacg 6900

agcttcatat cccgatcgct atcaacaagt gcccgaagaa catcttcaag atcaataccg 6960

aggtgagggt gctgctcaag cacgatgata acccttacgt gatcggaatc gatcgtggtg 7020

agagaaacct cctctacatc gttgtggtgg acggaaaggg aaacatcgtc gagcagtaca 7080

gcctgaacga gattatcaac aatttcaacg gcatcaggat caagaccgac taccactcac 7140

tcctcgataa gaaagaaaaa gagcgtttcg aggccaggca gaactggact tctatcgaaa 7200

acatcaaaga gttgaaggcc ggctacatct ctcaggtggt gcataagatc tgcgagctgg 7260

tggaaaagta cgatgctgtg atcgctcttg aggacctcaa ctctgggttc aagaacagta 7320

gagtgaaggt tgagaagcag gtctaccaaa agttcgagaa gatgctcatc gacaagctca 7380

actacatggt ggacaaaaag agcaaccctt gcgctaccgg tggtgctctt aagggatacc 7440

agatcacgaa caagttcgag tccttcaaga gcatgagcac ccagaacggc ttcatcttct 7500

atatccctgc ttggctcacc agcaagatcg atccttctac tggtttcgtg aacctgctca 7560

agaccaagta cacctcgatc gccgacagca agaagttcat ctcgtctttc gacaggatca 7620

tgtacgtgcc ggaagaggat cttttcgagt tcgctctcga ctataagaac ttcagcagga 7680

ccgacgccga ctacattaag aagtggaagc tctactccta cgggaaccgt atcaggatct 7740

tccgaaatcc gaagaaaaac aacgtgttcg actgggaaga agtgtgcctc acctctgcct 7800

acaaagaact gttcaacaag tacggcatca actaccagca gggtgatatc agggctcttt 7860

tgtgcgagca gagcgacaag gcattctaca gctcattcat ggccctcatg tctctcatgc 7920

tccagatgag gaactctatc accggaagga ccgatgtgga cttccttatc tctccggtca 7980

agaactctga cgggatcttc tacgacagcc gtaactatga ggctcaagag aacgctatcc 8040

tgccgaagaa tgctgatgca aacggggctt acaacattgc gagaaaggtt ctctgggcta 8100

tcgggcagtt taagaaagcg gaagatgaga agctcgacaa ggtgaagatc gccatctcca 8160

acaaagagtg gcttgagtac gctcagacct ccgttaagca caagaggcct gctgctacta 8220

agaaagctgg ccaggccaaa aagaagaagt gaggcgcgcc gagctccagg cctcccagct 8280

ttcgtccgta tcatcggttt cgacaacgtt cgtcaagttc aatgcatcag tttcattgcc 8340

cacacaccag aatcctacta agtttgagta ttatggcatt ggaaaagctg ttttcttcta 8400

tcatttgttc tgcttgtaat ttactgtgtt ctttcagttt ttgttttcgg acatcaaaat 8460

gcaaatggat ggataagagt taataaatga tatggtcctt ttgttcattc tcaaattatt 8520

attatctgtt gtttttactt taatgggttg aatttaagta agaaaggaac taacagtgtg 8580

atattaaggt gcaatgttag acatataaaa cagtctttca cctctctttg gttatgtctt 8640

gaattggttt gtttcttcac ttatctgtgt aatcaagttt actatgagtc tatgatcaag 8700

taattatgca atcaagttaa gtacagtata ggcttgagct ccctaggatc aagcttatcg 8760

atgtcgacag gccttaaggg ccagatcttg ggcccggtac ccgatcagat tgtcgtttcc 8820

cgccttcggt ttaaactatc agtgtttgac aggatatatt ggcgggtaaa cctaagagaa 8880

aagagcgttt attagaataa tcggatattt aaaagggcgt gaaaaggttt atccgttcgt 8940

ccatttgtat gtgcatgcca accacagggt tcccctcggg agtgcttggc attccgtgcg 9000

ataatgactt ctgttcaacc acccaaacgt cggaaagcct gacgacggag cagcattcca 9060

aaaagatccc ttggctcgtc tgggtcggct agaaggtcga gtgggctgct gtggcttgat 9120

ccctcaacgc ggtcgcggac gtagcgcagc gccgaaaaat cctcgatcgc aaatccgacg 9180

ctgtcgaaaa gcgtgatctg cttgtcgctc tttcggccga cgtcctggcc agtcatcacg 9240

cgccaaagtt ccgtcacagg atgatctggc gcgagttgct ggatctcgcc ttcaatccgg 9300

gtctgtggcg ggaactccac gaaaatatcc gaacgcagca agatatcgcg gtgcatctcg 9360

gtcttgcctg ggcagtcgcc gccgacgccg ttgatgtgga cgccgaaaag gatctaggtg 9420

aagatccttt ttgataatct catgaccaaa atcccttaac gtgagttttc gttccactga 9480

gcgtcagacc ccgtagaaaa gatcaaagga tcttcttgag atcctttttt tctgcgcgta 9540

atctgctgct tgcaaacaaa aaaaccaccg ctaccagcgg tggtttgttt gccggatcaa 9600

gagctaccaa ctctttttcc gaaggtaact ggcttcagca gagcgcagat accaaatact 9660

gttcttctag tgtagccgta gttaggccac cacttcaaga actctgtagc accgcctaca 9720

tacctcgctc tgctaatcct gttaccagtg gctgctgcca gtggcgataa gtcgtgtctt 9780

accgggttgg actcaagacg atagttaccg gataaggcgc agcggtcggg ctgaacgggg 9840

ggttcgtgca cacagcccag cttggagcga acgacctaca ccgaactgag atacctacag 9900

cgtgagctat gagaaagcgc cacgcttccc gaagggagaa aggcggacag gtatccggta 9960

agcggcaggg tcggaacagg agagcgcacg agggagcttc cagggggaaa cgcctggtat 10020

ctttatagtc ctgtcgggtt tcgccacctc tgacttgagc gtcgattttt gtgatgctcg 10080

tcaggggggc ggagcctatg gaaaaacgcc agcaacgcgg cctttttacg gttcctggcc 10140

ttttgctggc cttttgctca catgttcttt cctgcgttat cccctgattc tgtggataac 10200

cgattaccgc ctttgagtga gctgataccg ctcgccgcag ccgaacgacc gagcgcagcg 10260

agtcagtgag cgaggaagcg gaagagcgcc tgatgcggta ttttctcctt acgcatctgt 10320

gcggtatttc acaccgcata tggtgcactc tcagtacaat ctgctctgat gccgcatagt 10380

taagccagta tacactccgc tatcgctacg tgactgggtc atggctgcgc cccgacaccc 10440

gccaacaccc gctgacgcgc cctgacgggc ttgtctgctc ccggcatccg cttacagaca 10500

agctgtgacc gtctccggga gctgcatgtg tcagaggttt tcaccgtcat caccgaaacg 10560

cgcgaggcag gggtacgtcg aggtcgatcc aacccctccg ctgctatagt gcagtcggct 10620

tctgacgttc agtgcagccg tcgtctgaaa acgacatgtc gcacaagtcc taagttacgc 10680

gacaggctgc cgccctgccc ttttcctggc gttttcttgt cgcgtgtttt agtcgcataa 10740

agtagaatac ttgcgactag aaccggagac attacgccat gaacaagagc gccgccgctg 10800

gcctgctggg ctatgcccgc gtcagcaccg acgaccagga cttgaccaac caacgggccg 10860

aactgcacgc ggccggctgc accaagctgt tttccgagaa gatcaccggc accaggcgcg 10920

accgcccgga gctggccagg atgcttgacc acctacgccc tggcgacgtt gtgacagtga 10980

ccaggctaga ccgcctggcc cgcagcaccc gcgacctact ggacattgcc gagcgcatcc 11040

aggaggccgg cgcgggcctg cgtagcctgg cagagccgtg ggccgacacc accacgccgg 11100

ccggccgcat ggtgttgacc gtgttcgccg gcattgccga gttcgagcgt tccctaatca 11160

tcgaccgcac ccggagcggg cgcgaggccg ccaaggcgcg aggcgtgaag tttggccccc 11220

gccctaccct caccccggca cagatcgcgc acgcccgcga gctgatcgac caggaaggcc 11280

gcaccgtgaa agaggcggct gcactgcttg gcgtgcatcg ctcgaccctg taccgcgcac 11340

ttgagcgcag cgaggaagtg acgcccaccg aggccaggcg gcgcggtgcc ttccgtgagg 11400

acgcattgac cgaggccgac gccctggcgg ccgccgagaa tgaacgccaa gaggaacaag 11460

catgaaaccg caccaggacg gccaggacga accgtttttc attaccgaag agatcgaggc 11520

ggagatgatc gcggccgggt acgtgttcga gccgcccgcg cacgtctcaa ccgtgcggct 11580

gcatgaaatc ctggccggtt tgtctgatgc caagctcgcg gcctggccgg cgagcttggc 11640

cgctgaagaa accgagcgcc gccgtctaaa aaggtgatgt gtatttgagt aaaacagctt 11700

gcgtcatgcg gtcgctgcgt atatgatgcg atgagtaaat aaacaaatac gcaaggggaa 11760

cgcatgaagg ttatcgctgt acttaaccag aaaggcgggt caggcaagac gaccatcgca 11820

acccatctag cccgcgccct gcaactcgcc ggggccgatg ttctgttagt cgattccgat 11880

ccccagggca gtgcccgcga ttgggcggcc gtgcgggaag atcaaccgct aaccgttgtc 11940

ggcatcgacc gcccgacgat tgaccgcgac gtgaaggcca tcggccggcg cgacttcgta 12000

gtgatcgacg gagcgcccca ggcggcggac ttggctgtgt ccgcgatcaa ggcagccgac 12060

ttcgtgctga ttccggtgca gccaagccct tacgacatat gggccaccgc cgacctggtg 12120

gagctggtta agcagcgcat tgaggtcacg gatggaaggc tacaagcggc ctttgtcgtg 12180

tcgcgggcga tcaaaggcac gcgcatcggc ggtgaggttg ccgaggcgct ggccgggtac 12240

gagctgccca ttcttgagtc ccgtatcacg cagcgcgtga gctacccagg cactgccgcc 12300

gccggcacaa ccgttcttga atcagaaccc gagggcgacg ctgcccgcga ggtccaggcg 12360

ctggccgctg aaattaaatc aaaactcatt tgagttaatg aggtaaagag aaaatgagca 12420

aaagcacaaa cacgctaagt gccggccgtc cgagcgcacg cagcagcaag gctgcaacgt 12480

tggccagcct ggcagacacg ccagccatga agcgggtcaa ctttcagttg ccggcggagg 12540

atcacaccaa gctgaagatg tacgcggtac gccaaggcaa gaccattacc gagctgctat 12600

ctgaatacat cgcgcagcta ccagagtaaa tgagcaaatg aataaatgag tagatgaatt 12660

ttagcggcta aaggaggcgg catggaaaat caagaacaac caggcaccga cgccgtggaa 12720

tgccccatgt gtggaggaac gggcggttgg ccaggcgtaa gcggctgggt tgtctgccgg 12780

ccctgcaatg gcactggaac ccccaagccc gaggaatcgg cgtgagcggt cgcaaaccat 12840

ccggcccggt acaaatcggc gcggcgctgg gtgatgacct ggtggagaag ttgaaggcgg 12900

cgcaggccgc ccagcggcaa cgcatcgagg cagaagcacg ccccggtgaa tcgtggcaag 12960

cggccgctga tcgaatccgc aaagaatccc ggcaaccgcc ggcagccggt gcgccgtcga 13020

ttaggaagcc gcccaagggc gacgagcaac cagatttttt cgttccgatg ctctatgacg 13080

tgggcacccg cgatagtcgc agcatcatgg acgtggccgt tttccgtctg tcgaagcgtg 13140

accgacgagc tggcgaggtg atccgctacg agcttccaga cgggcacgta gaggtttccg 13200

cagggccggc cggcatggcg agtgtgtggg attacgacct ggtactgatg gcggtttccc 13260

atctaaccga atccatgaac cgataccggg aagggaaggg agacaagccc ggccgcgtgt 13320

tccgtccaca cgttgcggac gtactcaagt tctgccggcg agccgatggc ggaaagcaga 13380

aagacgacct ggtagaaacc tgcattcggt taaacaccac gcacgttgcc atgcagcgta 13440

cgaagaaggc caagaacggc cgcctggtga cggtatccga gggtgaagcc ttgattagcc 13500

gctacaagat cgtaaagagc gaaaccgggc ggccggagta catcgagatc gagctagctg 13560

attggatgta ccgcgagatc acagaaggca agaacccgga cgtgctgacg gttcaccccg 13620

attacttttt gatcgatccc ggcatcggcc gttttctcta ccgcctggca cgccgcgccg 13680

caggcaaggc agaagccaga tggttgttca agacgatcta cgaacgcagt ggcagcgccg 13740

gagagttcaa gaagttctgt ttcaccgtgc gcaagctgat cgggtcaaat gacctgccgg 13800

agtacgattt gaaggaggag gcggggcagg ctggcccgat cctagtcatg cgctaccgca 13860

acctgatcga gggcgaagca tccgccggtt cctaatgtac ggagcagatg ctagggcaaa 13920

ttgccctagc aggggaaaaa ggtcgaaaag gtctctttcc tgtggatagc acgtacattg 13980

ggaacccaaa gccgtacatt gggaaccgga acccgtacat tgggaaccca aagccgtaca 14040

ttgggaaccg gtcacacatg taagtgactg atataaaaga gaaaaaaggc gatttttccg 14100

cctaaaactc tttaaaactt attaaaactc ttaaaacccg cctggcctgt gcataactgt 14160

ctggccagcg cacagccgaa gagctgcaaa aagcgcctac ccttcggtcg ctgcgctccc 14220

tacgccccgc cgcttcgcgt cggcctatcg cggccgctgg ccgctcaaaa atggctggcc 14280

tacggccagg caatctacca gggcgcggac aagccgcgcc gtcgccactc gaccgccggc 14340

gcccacatca aggcaccggt gggtatgcct gacgatgcgt ggagaccgaa accttgcgct 14400

cgttcgccag ccaggacaga aatgcctcga cttcgctgct gcccaaggtt gccgggtgac 14460

gcacaccgtg gaaacggatg aaggcacgaa cccagtggac ataagcctgt tcggttcgta 14520

agctgtaatg caagtagcgt atgcgctcac gcaactggtc cagaaccttg accgaacgca 14580

gcggtggtaa cggcgcagtg gcggttttca tggcttgtta tgactgtttt tttggggtac 14640

agtctatgcc tcgggcatcc aagcagcaag cgcgttacgc cgtgggtcga tgtttgatgt 14700

tatggagcag caacgatgtt acgcagcagg gcagtcgccc taaaacaaag ttaaacatca 14760

tgagggaagc ggtgatcgcc gaagtatcga ctcaactatc agaggtagtt ggcgtcatcg 14820

agcgccatct cgaaccgacg ttgctggccg tacatttgta cggctccgca gtggatggcg 14880

gcctgaagcc acacagtgat attgatttgc tggttacggt gaccgtaagg cttgatgaaa 14940

caacgcggcg agctttgatc aacgaccttt tggaaacttc ggcttcccct ggagagagcg 15000

agattctccg cgctgtagaa gtcaccattg ttgtgcacga cgacatcatt ccgtggcgtt 15060

atccagctaa gcgcgaactg caatttggag aatggcagcg caatgacatt cttgcaggta 15120

tcttcgagcc agccacgatc gacattgatc tggctatctt gctgacaaaa gcaagagaac 15180

atagcgttgc cttggtaggt ccagcggcgg aggaactctt tgatccggtt cctgaacagg 15240

atctatttga ggcgctaaat gaaaccttaa cgctatggaa ctcgccgccc gactgggctg 15300

gcgatgagcg aaatgtagtg cttacgttgt cccgcatttg gtacagcgca gtaaccggca 15360

aaatcgcgcc gaaggatgtc gctgccgact gggcaatgga gcgcctgccg gcccagtatc 15420

agcccgtcat acttgaagct agacaggctt atcttggaca agaagaagat cgcttggcct 15480

cgcgcgcaga tcagttggaa gaatttgtcc actacgtgaa aggcgagatc accaaggtag 15540

tcggcaaata atgtctaaca attcgttcaa gccgacgccg cttcgcggcg cggcttaact 15600

caagcgttag atgcactaag cacataattg ctcacagcca aactatcagg tcaagtctgc 15660

ttttattatt tttaagcgtg cataataagc cctacacaaa ttgggagata tatcatgaaa 15720

ggctggcttt ttcttgttat cgcaatagtt ggcgaagtaa tcgcaacata gcttgcttgg 15780

tcgttccgcg tgaacgtcgg ctcgattgta cctgcgttca aatactttgc gatcgtgttg 15840

cgcgcctgcc cggtgcgtcg gctgatctca cggatcgact gcttctctcg caacgccatc 15900

cgacggatga tgtttaaaag tcccatgtgg atcactccgt tgccccgtcg ctcaccgtgt 15960

tggggggaag gtgcacatgg ctcagttctc aatggaaatt atctgcctaa ccggctcagt 16020

tctgcgtaga aaccaacatg caagctccac cgggtgcaaa gcggcagcgg cggcaggata 16080

tattcaattg taaatggctt catgtccggg aaatctacat ggatcagcaa tgagtatgat 16140

ggtcaatatg gagaaaaaga aagagtaatt accaattttt tttcaattca aaaatgtaga 16200

tgtccgcagc gttattataa aatgaaagta cattttgata aaacgacaaa ttacgatccg 16260

tcgtatttat aggcgaaagc aataaacaaa ttattctaat tcggaaatct ttatttcgac 16320

gtgtctacat tcacgtccaa atgggggctt agatgagaaa cttcacgatc ggctctagtt 16380

cgagcgatct agtaacatag atgacaccgc gcgcgataat ttatcctagt ttgcgcgcta 16440

tattttgttt tctatcgcgt attaaatgta taattgcggg actctaatca taaaaaccca 16500

tctcataaat aacgtcatgc attacatgtt aattattaca tgcttaacgt aattcaacag 16560

aaattatatg ataatcatcg caagaccggc aacaggattc aatcttaaga aactttattg 16620

ccaaatgttt gaacgatcgg ggaaattcgt ttctatgctt caattctgct ctatagatgt 16680

tcttctagtc ttaactttta gaaggcacaa catttgaaat gttggtaatt tttctgctgg 16740

gtagtgaacc attgtaattt gtgtttgctt atattatccg ttgggtttct ctatgttatc 16800

tgtgggccca tatcccagaa ctggttgagt cggtccaaca cctgggtgcc aatcatgtcg 16860

atggtggggt atggt 16875

<210> 73
<211> 16875
<212> DNA
<213> Artificial Sequence

<220>
<223> pZFNnptII_LbCpf1_crRNA_ALS_In2 vector

<400> 73
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg gcggccgcac tagtggatcc 1620

cccgggatgc atgaattcaa gcttatcgat gtcgacaggc cttaagggcc agatcccccg 1680

ggctgcagaa gcctatactg tacttaactt gattgcataa ttacttgatc atagactcat 1740

agtaaacttg attacacaga taagtgaaga aacaaaccaa ttcaagacat aaccaaagag 1800

aggtgaaaga ctgttttata tgtctaacat tgcaccttaa tatcacactg ttagttcctt 1860

tcttacttaa attcaaccca ttaaagtaaa aacaacagat aataataatt tgagaatgaa 1920

caaaaggacc atatcattta ttaactctta tccatccatt tgcattttga tgtccgaaaa 1980

caaaaactga aagaacacag taaattacaa gcagaacaaa tgatagaaga aaacagcttt 2040

tccaatgcca taatactcaa acttagtagg attctggtgt gtgggcaatg aaactgatgc 2100

attgaacttg acgaacgttg tcgaaaccga tgatacggac gaaagctggg aggcctggtc 2160

ccattcgcca tgccgaagca tgttgcccag ccggcgccag cgaggaggct gggaccatgc 2220

cggccgatat actagttggc ttggtgcata tctacactta gtagaaatta atctacactt 2280

agtagaaatt agacgagctt actcgtttcg tcctcacgga ctcatcagta atttggatcc 2340

gctgcacata cataacatat caagatcaga acacacatat acacacacaa atacaatcaa 2400

gtcaacaact ccaaaaagtc cagatctaca tatatacata cgtaaataac aaaatcatgt 2460

aaataatcac aatcatgtaa tccagatcta tgcacatata tatatacaca attaataaaa 2520

aaaatgatat aacagatcta tatctatgta tgtaacaaca caatcagatg agagaagtga 2580

tgttttcaga tctgtataca tacaaacaca aacagatgaa caattgatac gtagatccat 2640

atgtatacgt acaattagct acacgattaa atgaaaaaaa tcaacgattt cggattggta 2700

cacacaaacg caacaatatg aagaaattca tatctgatta gatataaaca taaccacgtg 2760

tagatacaca gtcaaatcaa caaatttata gcttctaaac ggatgagatg aacaagataa 2820

agatattcac ataaggcata cataagataa gcagattaac aaactagcaa taatacatac 2880

ctaattaaaa caaggaataa cagagagaga gagagagaga gagatttacc ttgaaaatga 2940

agaggagaag agaggatttc ttaaaattgg gggtagagaa agaaagatga tgaattgtga 3000

gaaaggagag atagaagggg gggttgtata tataggctgt agaagattat ttttgtgttt 3060

gaggcggtga aggaagaggg gatctgacta tgacacgttt gcggttacgt atttcgatag 3120

gagtctttca acgcttaacg ccgttactct atatgaccgt ttgggccgta acggggccgt 3180

ttgttaacgc tgatgttgat tcttttcttt ctttctttct tcctttttta aagaagcaat 3240

tgtacaatcg ttgctagctg tcaaacggat aattcggata cggatatgcc tatattcata 3300

tccgtaattt ttctgcagga attcgatctg gcacgacagg tttcccgact ggaaagcggg 3360

cagtgagcgc aacgcaatta atgtgagtta gctcactcat taggcacccc aggctttaca 3420

ctttatgctt ccggctcgta tgttgtgtgg aattgtgagc ggataacaat ttcacacagg 3480

aaacagctat gacatgatta cgaattcaaa aattacggat atgaatatag gcatatccgt 3540

atccgaatta tccgtttgac agctagcaac gattgtacaa ttgcttcttt aaaaaaggaa 3600

gaaagaaaga aagaaaagaa tcaacatcag cgttaacaaa cggccccgtt acggcccaaa 3660

cggtcatata gagtaacggc gttaagcgtt gaaagactcc tatcgaaata cgtaaccgca 3720

aacgtgtcat agtcagatcc cctcttcctt caccgcctca aacacaaaaa taatcttcta 3780

cagcctatat atacaacccc cccttctatc tctcctttct cacaattcat catctttctt 3840

tctctacccc caattttaag aaatcctctc ttctcctctt cattttcaag gtaaatctct 3900

ctctctctct ctctctctgt tattccttgt tttaattagg tatgtattat tgctagtttg 3960

ttaatctgct tatcttatgt atgccttatg tgaatatctt tatcttgttc atctcatccg 4020

tttagaagct ataaatttgt tgatttgact gtgtatctac acgtggttat gtttatatct 4080

aatcagatat gaatttcttc atattgttgc gtttgtgtgt accaatccga aatcgttgat 4140

ttttttcatt taatcgtgta gctaattgta cgtatacata tggatctacg tatcaattgt 4200

tcatctgttt gtgtttgtat gtatacagat ctgaaaacat cacttctctc atctgattgt 4260

gttgttacat acatagatat agatctgtta tatcattttt tttattaatt gtgtatatat 4320

atatgtgcat agatctggat tacatgattg tgattattta catgattttg ttatttacgt 4380

atgtatatat gtagatctgg actttttgga gttgttgact tgattgtatt tgtgtgtgta 4440

tatgtgtgtt ctgatcttga tatgttatgt atgtgcagcg aattcggcgc gccatggctc 4500

ctaagaagaa gaggaaggtt agcaagctcg agaagtttac caactgctac agcctctcta 4560

agaccctcag gttcaaggct atccctgtgg gaaagaccca agagaatatc gacaacaaga 4620

ggctcctcgt cgaggatgag aagagagctg aagattacaa gggcgtgaag aagctcctcg 4680

acaggtacta cctcagcttc atcaacgatg tgctccacag catcaagctc aagaacctca 4740

acaactacat cagcctcttc cgtaagaaaa ccaggaccga gaaagagaac aaagagcttg 4800

agaacctcga gatcaacctc cgtaaagaga tcgccaaggc tttcaaggga aacgagggat 4860

acaagagcct cttcaagaag gatattatcg agacaatcct gcctgagttc ctggacgata 4920

aggatgagat cgctctcgtg aacagcttca acggattcac tactgccttc accggattct 4980

tcgacaacag ggaaaacatg ttcagcgaag aggccaagag cacctctatc gctttcagat 5040

gcatcaacga gaacctcacg cgttacatca gcaacatgga catcttcgag aaggtggacg 5100

ccatcttcga taagcacgag gtgcaagaaa tcaaagagaa gatcctcaac agcgactacg 5160

acgtcgagga cttttttgaa ggggagttct tcaacttcgt tctcacccaa gagggcatcg 5220

acgtgtacaa cgctattatc ggaggattcg tgaccgagtc tggggagaag attaagggac 5280

tcaacgagta catcaacctg tacaaccaga aaacgaagca gaagctcccg aagttcaagc 5340

cgctctacaa gcaggttctc tctgatcgtg agagcctctc attttacggt gagggttaca 5400

cctctgacga ggaagtgctt gaggttttcc gtaacaccct caacaagaac agcgagatct 5460

tctcgtccat caagaagttg gagaaacttt tcaagaactt cgacgagtac agcagcgctg 5520

ggatcttcgt taagaacgga cctgctatca gcaccatcag caaggatatt ttcggcgagt 5580

ggaacgtgat cagggacaag tggaatgctg agtacgatga catccacctc aagaagaagg 5640

ctgtcgtcac tgagaagtac gaggatgaca ggcgtaagtc gttcaagaag atcggctctt 5700

tcagcctcga gcagcttcaa gaatacgctg atgctgatct cagcgtggtc gagaagctca 5760

aagagatcat catccagaag gtcgacgaga tctacaaggt gtacgggtcc tctgagaagt 5820

tgttcgatgc tgatttcgtc ctcgagaaga gtctgaagaa gaacgacgct gtcgtcgcga 5880

tcatgaagga tttgctcgac agcgtgaagt ccttcgagaa ctatatcaag gccttcttcg 5940

gagagggcaa agagactaat agggacgagt ctttctacgg ggatttcgtg ctcgcttacg 6000

atatcctcct caaggtggac catatctacg acgccatcag aaactacgtg acccagaagc 6060

cttacagcaa ggacaagttc aagttgtact ttcagaaccc gcagttcatg ggcggatggg 6120

acaaagacaa agagacagat tacagggcca ccatcctcag gtacgggtct aagtactacc 6180

tggccatcat ggacaagaaa tacgccaagt gcctccaaaa gatcgacaag gatgacgtga 6240

acgggaacta tgagaagatc aactacaagc tccttccggg accgaacaag atgcttccta 6300

aggtgttctt cagcaagaaa tggatggcct actacaaccc gtctgaggac atccagaaaa 6360

tctacaagaa cgggaccttc aagaaaggcg acatgttcaa cctcaacgac tgccacaagc 6420

tcatcgattt cttcaaggac agcatctcgc gttacccgaa gtggtctaac gcttacgact 6480

ttaacttcag cgagacagaa aagtacaagg atatcgccgg gttctaccgt gaggttgagg 6540

aacagggtta caaggttagc ttcgagagcg cctccaagaa agaggttgac aagttggtcg 6600

aagagggcaa gctctacatg ttccagatct ataacaagga cttctccgac aagagccacg 6660

gaactcctaa cctccatacg atgtacttca agctgctttt cgacgagaac aaccacgggc 6720

agatcagact ttctggtggt gctgaactct tcatgcgtag ggcctcactc aagaaagaag 6780

agttggttgt tcacccggcc aactctccaa tcgctaacaa gaatcctgac aacccgaaaa 6840

agaccaccac gctgtcttac gacgtctaca aggacaaaag gttcagcgag gaccagtacg 6900

agcttcatat cccgatcgct atcaacaagt gcccgaagaa catcttcaag atcaataccg 6960

aggtgagggt gctgctcaag cacgatgata acccttacgt gatcggaatc gatcgtggtg 7020

agagaaacct cctctacatc gttgtggtgg acggaaaggg aaacatcgtc gagcagtaca 7080

gcctgaacga gattatcaac aatttcaacg gcatcaggat caagaccgac taccactcac 7140

tcctcgataa gaaagaaaaa gagcgtttcg aggccaggca gaactggact tctatcgaaa 7200

acatcaaaga gttgaaggcc ggctacatct ctcaggtggt gcataagatc tgcgagctgg 7260

tggaaaagta cgatgctgtg atcgctcttg aggacctcaa ctctgggttc aagaacagta 7320

gagtgaaggt tgagaagcag gtctaccaaa agttcgagaa gatgctcatc gacaagctca 7380

actacatggt ggacaaaaag agcaaccctt gcgctaccgg tggtgctctt aagggatacc 7440

agatcacgaa caagttcgag tccttcaaga gcatgagcac ccagaacggc ttcatcttct 7500

atatccctgc ttggctcacc agcaagatcg atccttctac tggtttcgtg aacctgctca 7560

agaccaagta cacctcgatc gccgacagca agaagttcat ctcgtctttc gacaggatca 7620

tgtacgtgcc ggaagaggat cttttcgagt tcgctctcga ctataagaac ttcagcagga 7680

ccgacgccga ctacattaag aagtggaagc tctactccta cgggaaccgt atcaggatct 7740

tccgaaatcc gaagaaaaac aacgtgttcg actgggaaga agtgtgcctc acctctgcct 7800

acaaagaact gttcaacaag tacggcatca actaccagca gggtgatatc agggctcttt 7860

tgtgcgagca gagcgacaag gcattctaca gctcattcat ggccctcatg tctctcatgc 7920

tccagatgag gaactctatc accggaagga ccgatgtgga cttccttatc tctccggtca 7980

agaactctga cgggatcttc tacgacagcc gtaactatga ggctcaagag aacgctatcc 8040

tgccgaagaa tgctgatgca aacggggctt acaacattgc gagaaaggtt ctctgggcta 8100

tcgggcagtt taagaaagcg gaagatgaga agctcgacaa ggtgaagatc gccatctcca 8160

acaaagagtg gcttgagtac gctcagacct ccgttaagca caagaggcct gctgctacta 8220

agaaagctgg ccaggccaaa aagaagaagt gaggcgcgcc gagctccagg cctcccagct 8280

ttcgtccgta tcatcggttt cgacaacgtt cgtcaagttc aatgcatcag tttcattgcc 8340

cacacaccag aatcctacta agtttgagta ttatggcatt ggaaaagctg ttttcttcta 8400

tcatttgttc tgcttgtaat ttactgtgtt ctttcagttt ttgttttcgg acatcaaaat 8460

gcaaatggat ggataagagt taataaatga tatggtcctt ttgttcattc tcaaattatt 8520

attatctgtt gtttttactt taatgggttg aatttaagta agaaaggaac taacagtgtg 8580

atattaaggt gcaatgttag acatataaaa cagtctttca cctctctttg gttatgtctt 8640

gaattggttt gtttcttcac ttatctgtgt aatcaagttt actatgagtc tatgatcaag 8700

taattatgca atcaagttaa gtacagtata ggcttgagct ccctaggatc aagcttatcg 8760

atgtcgacag gccttaaggg ccagatcttg ggcccggtac ccgatcagat tgtcgtttcc 8820

cgccttcggt ttaaactatc agtgtttgac aggatatatt ggcgggtaaa cctaagagaa 8880

aagagcgttt attagaataa tcggatattt aaaagggcgt gaaaaggttt atccgttcgt 8940

ccatttgtat gtgcatgcca accacagggt tcccctcggg agtgcttggc attccgtgcg 9000

ataatgactt ctgttcaacc acccaaacgt cggaaagcct gacgacggag cagcattcca 9060

aaaagatccc ttggctcgtc tgggtcggct agaaggtcga gtgggctgct gtggcttgat 9120

ccctcaacgc ggtcgcggac gtagcgcagc gccgaaaaat cctcgatcgc aaatccgacg 9180

ctgtcgaaaa gcgtgatctg cttgtcgctc tttcggccga cgtcctggcc agtcatcacg 9240

cgccaaagtt ccgtcacagg atgatctggc gcgagttgct ggatctcgcc ttcaatccgg 9300

gtctgtggcg ggaactccac gaaaatatcc gaacgcagca agatatcgcg gtgcatctcg 9360

gtcttgcctg ggcagtcgcc gccgacgccg ttgatgtgga cgccgaaaag gatctaggtg 9420

aagatccttt ttgataatct catgaccaaa atcccttaac gtgagttttc gttccactga 9480

gcgtcagacc ccgtagaaaa gatcaaagga tcttcttgag atcctttttt tctgcgcgta 9540

atctgctgct tgcaaacaaa aaaaccaccg ctaccagcgg tggtttgttt gccggatcaa 9600

gagctaccaa ctctttttcc gaaggtaact ggcttcagca gagcgcagat accaaatact 9660

gttcttctag tgtagccgta gttaggccac cacttcaaga actctgtagc accgcctaca 9720

tacctcgctc tgctaatcct gttaccagtg gctgctgcca gtggcgataa gtcgtgtctt 9780

accgggttgg actcaagacg atagttaccg gataaggcgc agcggtcggg ctgaacgggg 9840

ggttcgtgca cacagcccag cttggagcga acgacctaca ccgaactgag atacctacag 9900

cgtgagctat gagaaagcgc cacgcttccc gaagggagaa aggcggacag gtatccggta 9960

agcggcaggg tcggaacagg agagcgcacg agggagcttc cagggggaaa cgcctggtat 10020

ctttatagtc ctgtcgggtt tcgccacctc tgacttgagc gtcgattttt gtgatgctcg 10080

tcaggggggc ggagcctatg gaaaaacgcc agcaacgcgg cctttttacg gttcctggcc 10140

ttttgctggc cttttgctca catgttcttt cctgcgttat cccctgattc tgtggataac 10200

cgattaccgc ctttgagtga gctgataccg ctcgccgcag ccgaacgacc gagcgcagcg 10260

agtcagtgag cgaggaagcg gaagagcgcc tgatgcggta ttttctcctt acgcatctgt 10320

gcggtatttc acaccgcata tggtgcactc tcagtacaat ctgctctgat gccgcatagt 10380

taagccagta tacactccgc tatcgctacg tgactgggtc atggctgcgc cccgacaccc 10440

gccaacaccc gctgacgcgc cctgacgggc ttgtctgctc ccggcatccg cttacagaca 10500

agctgtgacc gtctccggga gctgcatgtg tcagaggttt tcaccgtcat caccgaaacg 10560

cgcgaggcag gggtacgtcg aggtcgatcc aacccctccg ctgctatagt gcagtcggct 10620

tctgacgttc agtgcagccg tcgtctgaaa acgacatgtc gcacaagtcc taagttacgc 10680

gacaggctgc cgccctgccc ttttcctggc gttttcttgt cgcgtgtttt agtcgcataa 10740

agtagaatac ttgcgactag aaccggagac attacgccat gaacaagagc gccgccgctg 10800

gcctgctggg ctatgcccgc gtcagcaccg acgaccagga cttgaccaac caacgggccg 10860

aactgcacgc ggccggctgc accaagctgt tttccgagaa gatcaccggc accaggcgcg 10920

accgcccgga gctggccagg atgcttgacc acctacgccc tggcgacgtt gtgacagtga 10980

ccaggctaga ccgcctggcc cgcagcaccc gcgacctact ggacattgcc gagcgcatcc 11040

aggaggccgg cgcgggcctg cgtagcctgg cagagccgtg ggccgacacc accacgccgg 11100

ccggccgcat ggtgttgacc gtgttcgccg gcattgccga gttcgagcgt tccctaatca 11160

tcgaccgcac ccggagcggg cgcgaggccg ccaaggcgcg aggcgtgaag tttggccccc 11220

gccctaccct caccccggca cagatcgcgc acgcccgcga gctgatcgac caggaaggcc 11280

gcaccgtgaa agaggcggct gcactgcttg gcgtgcatcg ctcgaccctg taccgcgcac 11340

ttgagcgcag cgaggaagtg acgcccaccg aggccaggcg gcgcggtgcc ttccgtgagg 11400

acgcattgac cgaggccgac gccctggcgg ccgccgagaa tgaacgccaa gaggaacaag 11460

catgaaaccg caccaggacg gccaggacga accgtttttc attaccgaag agatcgaggc 11520

ggagatgatc gcggccgggt acgtgttcga gccgcccgcg cacgtctcaa ccgtgcggct 11580

gcatgaaatc ctggccggtt tgtctgatgc caagctcgcg gcctggccgg cgagcttggc 11640

cgctgaagaa accgagcgcc gccgtctaaa aaggtgatgt gtatttgagt aaaacagctt 11700

gcgtcatgcg gtcgctgcgt atatgatgcg atgagtaaat aaacaaatac gcaaggggaa 11760

cgcatgaagg ttatcgctgt acttaaccag aaaggcgggt caggcaagac gaccatcgca 11820

acccatctag cccgcgccct gcaactcgcc ggggccgatg ttctgttagt cgattccgat 11880

ccccagggca gtgcccgcga ttgggcggcc gtgcgggaag atcaaccgct aaccgttgtc 11940

ggcatcgacc gcccgacgat tgaccgcgac gtgaaggcca tcggccggcg cgacttcgta 12000

gtgatcgacg gagcgcccca ggcggcggac ttggctgtgt ccgcgatcaa ggcagccgac 12060

ttcgtgctga ttccggtgca gccaagccct tacgacatat gggccaccgc cgacctggtg 12120

gagctggtta agcagcgcat tgaggtcacg gatggaaggc tacaagcggc ctttgtcgtg 12180

tcgcgggcga tcaaaggcac gcgcatcggc ggtgaggttg ccgaggcgct ggccgggtac 12240

gagctgccca ttcttgagtc ccgtatcacg cagcgcgtga gctacccagg cactgccgcc 12300

gccggcacaa ccgttcttga atcagaaccc gagggcgacg ctgcccgcga ggtccaggcg 12360

ctggccgctg aaattaaatc aaaactcatt tgagttaatg aggtaaagag aaaatgagca 12420

aaagcacaaa cacgctaagt gccggccgtc cgagcgcacg cagcagcaag gctgcaacgt 12480

tggccagcct ggcagacacg ccagccatga agcgggtcaa ctttcagttg ccggcggagg 12540

atcacaccaa gctgaagatg tacgcggtac gccaaggcaa gaccattacc gagctgctat 12600

ctgaatacat cgcgcagcta ccagagtaaa tgagcaaatg aataaatgag tagatgaatt 12660

ttagcggcta aaggaggcgg catggaaaat caagaacaac caggcaccga cgccgtggaa 12720

tgccccatgt gtggaggaac gggcggttgg ccaggcgtaa gcggctgggt tgtctgccgg 12780

ccctgcaatg gcactggaac ccccaagccc gaggaatcgg cgtgagcggt cgcaaaccat 12840

ccggcccggt acaaatcggc gcggcgctgg gtgatgacct ggtggagaag ttgaaggcgg 12900

cgcaggccgc ccagcggcaa cgcatcgagg cagaagcacg ccccggtgaa tcgtggcaag 12960

cggccgctga tcgaatccgc aaagaatccc ggcaaccgcc ggcagccggt gcgccgtcga 13020

ttaggaagcc gcccaagggc gacgagcaac cagatttttt cgttccgatg ctctatgacg 13080

tgggcacccg cgatagtcgc agcatcatgg acgtggccgt tttccgtctg tcgaagcgtg 13140

accgacgagc tggcgaggtg atccgctacg agcttccaga cgggcacgta gaggtttccg 13200

cagggccggc cggcatggcg agtgtgtggg attacgacct ggtactgatg gcggtttccc 13260

atctaaccga atccatgaac cgataccggg aagggaaggg agacaagccc ggccgcgtgt 13320

tccgtccaca cgttgcggac gtactcaagt tctgccggcg agccgatggc ggaaagcaga 13380

aagacgacct ggtagaaacc tgcattcggt taaacaccac gcacgttgcc atgcagcgta 13440

cgaagaaggc caagaacggc cgcctggtga cggtatccga gggtgaagcc ttgattagcc 13500

gctacaagat cgtaaagagc gaaaccgggc ggccggagta catcgagatc gagctagctg 13560

attggatgta ccgcgagatc acagaaggca agaacccgga cgtgctgacg gttcaccccg 13620

attacttttt gatcgatccc ggcatcggcc gttttctcta ccgcctggca cgccgcgccg 13680

caggcaaggc agaagccaga tggttgttca agacgatcta cgaacgcagt ggcagcgccg 13740

gagagttcaa gaagttctgt ttcaccgtgc gcaagctgat cgggtcaaat gacctgccgg 13800

agtacgattt gaaggaggag gcggggcagg ctggcccgat cctagtcatg cgctaccgca 13860

acctgatcga gggcgaagca tccgccggtt cctaatgtac ggagcagatg ctagggcaaa 13920

ttgccctagc aggggaaaaa ggtcgaaaag gtctctttcc tgtggatagc acgtacattg 13980

ggaacccaaa gccgtacatt gggaaccgga acccgtacat tgggaaccca aagccgtaca 14040

ttgggaaccg gtcacacatg taagtgactg atataaaaga gaaaaaaggc gatttttccg 14100

cctaaaactc tttaaaactt attaaaactc ttaaaacccg cctggcctgt gcataactgt 14160

ctggccagcg cacagccgaa gagctgcaaa aagcgcctac ccttcggtcg ctgcgctccc 14220

tacgccccgc cgcttcgcgt cggcctatcg cggccgctgg ccgctcaaaa atggctggcc 14280

tacggccagg caatctacca gggcgcggac aagccgcgcc gtcgccactc gaccgccggc 14340

gcccacatca aggcaccggt gggtatgcct gacgatgcgt ggagaccgaa accttgcgct 14400

cgttcgccag ccaggacaga aatgcctcga cttcgctgct gcccaaggtt gccgggtgac 14460

gcacaccgtg gaaacggatg aaggcacgaa cccagtggac ataagcctgt tcggttcgta 14520

agctgtaatg caagtagcgt atgcgctcac gcaactggtc cagaaccttg accgaacgca 14580

gcggtggtaa cggcgcagtg gcggttttca tggcttgtta tgactgtttt tttggggtac 14640

agtctatgcc tcgggcatcc aagcagcaag cgcgttacgc cgtgggtcga tgtttgatgt 14700

tatggagcag caacgatgtt acgcagcagg gcagtcgccc taaaacaaag ttaaacatca 14760

tgagggaagc ggtgatcgcc gaagtatcga ctcaactatc agaggtagtt ggcgtcatcg 14820

agcgccatct cgaaccgacg ttgctggccg tacatttgta cggctccgca gtggatggcg 14880

gcctgaagcc acacagtgat attgatttgc tggttacggt gaccgtaagg cttgatgaaa 14940

caacgcggcg agctttgatc aacgaccttt tggaaacttc ggcttcccct ggagagagcg 15000

agattctccg cgctgtagaa gtcaccattg ttgtgcacga cgacatcatt ccgtggcgtt 15060

atccagctaa gcgcgaactg caatttggag aatggcagcg caatgacatt cttgcaggta 15120

tcttcgagcc agccacgatc gacattgatc tggctatctt gctgacaaaa gcaagagaac 15180

atagcgttgc cttggtaggt ccagcggcgg aggaactctt tgatccggtt cctgaacagg 15240

atctatttga ggcgctaaat gaaaccttaa cgctatggaa ctcgccgccc gactgggctg 15300

gcgatgagcg aaatgtagtg cttacgttgt cccgcatttg gtacagcgca gtaaccggca 15360

aaatcgcgcc gaaggatgtc gctgccgact gggcaatgga gcgcctgccg gcccagtatc 15420

agcccgtcat acttgaagct agacaggctt atcttggaca agaagaagat cgcttggcct 15480

cgcgcgcaga tcagttggaa gaatttgtcc actacgtgaa aggcgagatc accaaggtag 15540

tcggcaaata atgtctaaca attcgttcaa gccgacgccg cttcgcggcg cggcttaact 15600

caagcgttag atgcactaag cacataattg ctcacagcca aactatcagg tcaagtctgc 15660

ttttattatt tttaagcgtg cataataagc cctacacaaa ttgggagata tatcatgaaa 15720

ggctggcttt ttcttgttat cgcaatagtt ggcgaagtaa tcgcaacata gcttgcttgg 15780

tcgttccgcg tgaacgtcgg ctcgattgta cctgcgttca aatactttgc gatcgtgttg 15840

cgcgcctgcc cggtgcgtcg gctgatctca cggatcgact gcttctctcg caacgccatc 15900

cgacggatga tgtttaaaag tcccatgtgg atcactccgt tgccccgtcg ctcaccgtgt 15960

tggggggaag gtgcacatgg ctcagttctc aatggaaatt atctgcctaa ccggctcagt 16020

tctgcgtaga aaccaacatg caagctccac cgggtgcaaa gcggcagcgg cggcaggata 16080

tattcaattg taaatggctt catgtccggg aaatctacat ggatcagcaa tgagtatgat 16140

ggtcaatatg gagaaaaaga aagagtaatt accaattttt tttcaattca aaaatgtaga 16200

tgtccgcagc gttattataa aatgaaagta cattttgata aaacgacaaa ttacgatccg 16260

tcgtatttat aggcgaaagc aataaacaaa ttattctaat tcggaaatct ttatttcgac 16320

gtgtctacat tcacgtccaa atgggggctt agatgagaaa cttcacgatc ggctctagtt 16380

cgagcgatct agtaacatag atgacaccgc gcgcgataat ttatcctagt ttgcgcgcta 16440

tattttgttt tctatcgcgt attaaatgta taattgcggg actctaatca taaaaaccca 16500

tctcataaat aacgtcatgc attacatgtt aattattaca tgcttaacgt aattcaacag 16560

aaattatatg ataatcatcg caagaccggc aacaggattc aatcttaaga aactttattg 16620

ccaaatgttt gaacgatcgg ggaaattcgt ttctatgctt caattctgct ctatagatgt 16680

tcttctagtc ttaactttta gaaggcacaa catttgaaat gttggtaatt tttctgctgg 16740

gtagtgaacc attgtaattt gtgtttgctt atattatccg ttgggtttct ctatgttatc 16800

tgtggggcca tatcccagaa ctggttgagt cggtccaaca cctgggtgcc aatcatgtcg 16860

atggtggggt atggt 16875

<210> 74
<211> 16875
<212> DNA
<213> Artificial Sequence

<220>
<223> pZFNnptII_LbCpf1_crRNA_Cyp_Del vector

<400> 74
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg gcggccgcac tagtggatcc 1620

cccgggatgc atgaattcaa gcttatcgat gtcgacaggc cttaagggcc agatcccccg 1680

ggctgcagaa gcctatactg tacttaactt gattgcataa ttacttgatc atagactcat 1740

agtaaacttg attacacaga taagtgaaga aacaaaccaa ttcaagacat aaccaaagag 1800

aggtgaaaga ctgttttata tgtctaacat tgcaccttaa tatcacactg ttagttcctt 1860

tcttacttaa attcaaccca ttaaagtaaa aacaacagat aataataatt tgagaatgaa 1920

caaaaggacc atatcattta ttaactctta tccatccatt tgcattttga tgtccgaaaa 1980

caaaaactga aagaacacag taaattacaa gcagaacaaa tgatagaaga aaacagcttt 2040

tccaatgcca taatactcaa acttagtagg attctggtgt gtgggcaatg aaactgatgc 2100

attgaacttg acgaacgttg tcgaaaccga tgatacggac gaaagctggg aggcctggtc 2160

ccattcgcca tgccgaagca tgttgcccag ccggcgccag cgaggaggct gggaccatgc 2220

cggccgatat actagttggc ttggtgcata tctacactta gtagaaatta atctacactt 2280

agtagaaatt agacgagctt actcgtttcg tcctcacgga ctcatcagta atttggatcc 2340

gctgcacata cataacatat caagatcaga acacacatat acacacacaa atacaatcaa 2400

gtcaacaact ccaaaaagtc cagatctaca tatatacata cgtaaataac aaaatcatgt 2460

aaataatcac aatcatgtaa tccagatcta tgcacatata tatatacaca attaataaaa 2520

aaaatgatat aacagatcta tatctatgta tgtaacaaca caatcagatg agagaagtga 2580

tgttttcaga tctgtataca tacaaacaca aacagatgaa caattgatac gtagatccat 2640

atgtatacgt acaattagct acacgattaa atgaaaaaaa tcaacgattt cggattggta 2700

cacacaaacg caacaatatg aagaaattca tatctgatta gatataaaca taaccacgtg 2760

tagatacaca gtcaaatcaa caaatttata gcttctaaac ggatgagatg aacaagataa 2820

agatattcac ataaggcata cataagataa gcagattaac aaactagcaa taatacatac 2880

ctaattaaaa caaggaataa cagagagaga gagagagaga gagatttacc ttgaaaatga 2940

agaggagaag agaggatttc ttaaaattgg gggtagagaa agaaagatga tgaattgtga 3000

gaaaggagag atagaagggg gggttgtata tataggctgt agaagattat ttttgtgttt 3060

gaggcggtga aggaagaggg gatctgacta tgacacgttt gcggttacgt atttcgatag 3120

gagtctttca acgcttaacg ccgttactct atatgaccgt ttgggccgta acggggccgt 3180

ttgttaacgc tgatgttgat tcttttcttt ctttctttct tcctttttta aagaagcaat 3240

tgtacaatcg ttgctagctg tcaaacggat aattcggata cggatatgcc tatattcata 3300

tccgtaattt ttctgcagga attcgatctg gcacgacagg tttcccgact ggaaagcggg 3360

cagtgagcgc aacgcaatta atgtgagtta gctcactcat taggcacccc aggctttaca 3420

ctttatgctt ccggctcgta tgttgtgtgg aattgtgagc ggataacaat ttcacacagg 3480

aaacagctat gacatgatta cgaattcaaa aattacggat atgaatatag gcatatccgt 3540

atccgaatta tccgtttgac agctagcaac gattgtacaa ttgcttcttt aaaaaaggaa 3600

gaaagaaaga aagaaaagaa tcaacatcag cgttaacaaa cggccccgtt acggcccaaa 3660

cggtcatata gagtaacggc gttaagcgtt gaaagactcc tatcgaaata cgtaaccgca 3720

aacgtgtcat agtcagatcc cctcttcctt caccgcctca aacacaaaaa taatcttcta 3780

cagcctatat atacaacccc cccttctatc tctcctttct cacaattcat catctttctt 3840

tctctacccc caattttaag aaatcctctc ttctcctctt cattttcaag gtaaatctct 3900

ctctctctct ctctctctgt tattccttgt tttaattagg tatgtattat tgctagtttg 3960

ttaatctgct tatcttatgt atgccttatg tgaatatctt tatcttgttc atctcatccg 4020

tttagaagct ataaatttgt tgatttgact gtgtatctac acgtggttat gtttatatct 4080

aatcagatat gaatttcttc atattgttgc gtttgtgtgt accaatccga aatcgttgat 4140

ttttttcatt taatcgtgta gctaattgta cgtatacata tggatctacg tatcaattgt 4200

tcatctgttt gtgtttgtat gtatacagat ctgaaaacat cacttctctc atctgattgt 4260

gttgttacat acatagatat agatctgtta tatcattttt tttattaatt gtgtatatat 4320

atatgtgcat agatctggat tacatgattg tgattattta catgattttg ttatttacgt 4380

atgtatatat gtagatctgg actttttgga gttgttgact tgattgtatt tgtgtgtgta 4440

tatgtgtgtt ctgatcttga tatgttatgt atgtgcagcg aattcggcgc gccatggctc 4500

ctaagaagaa gaggaaggtt agcaagctcg agaagtttac caactgctac agcctctcta 4560

agaccctcag gttcaaggct atccctgtgg gaaagaccca agagaatatc gacaacaaga 4620

ggctcctcgt cgaggatgag aagagagctg aagattacaa gggcgtgaag aagctcctcg 4680

acaggtacta cctcagcttc atcaacgatg tgctccacag catcaagctc aagaacctca 4740

acaactacat cagcctcttc cgtaagaaaa ccaggaccga gaaagagaac aaagagcttg 4800

agaacctcga gatcaacctc cgtaaagaga tcgccaaggc tttcaaggga aacgagggat 4860

acaagagcct cttcaagaag gatattatcg agacaatcct gcctgagttc ctggacgata 4920

aggatgagat cgctctcgtg aacagcttca acggattcac tactgccttc accggattct 4980

tcgacaacag ggaaaacatg ttcagcgaag aggccaagag cacctctatc gctttcagat 5040

gcatcaacga gaacctcacg cgttacatca gcaacatgga catcttcgag aaggtggacg 5100

ccatcttcga taagcacgag gtgcaagaaa tcaaagagaa gatcctcaac agcgactacg 5160

acgtcgagga cttttttgaa ggggagttct tcaacttcgt tctcacccaa gagggcatcg 5220

acgtgtacaa cgctattatc ggaggattcg tgaccgagtc tggggagaag attaagggac 5280

tcaacgagta catcaacctg tacaaccaga aaacgaagca gaagctcccg aagttcaagc 5340

cgctctacaa gcaggttctc tctgatcgtg agagcctctc attttacggt gagggttaca 5400

cctctgacga ggaagtgctt gaggttttcc gtaacaccct caacaagaac agcgagatct 5460

tctcgtccat caagaagttg gagaaacttt tcaagaactt cgacgagtac agcagcgctg 5520

ggatcttcgt taagaacgga cctgctatca gcaccatcag caaggatatt ttcggcgagt 5580

ggaacgtgat cagggacaag tggaatgctg agtacgatga catccacctc aagaagaagg 5640

ctgtcgtcac tgagaagtac gaggatgaca ggcgtaagtc gttcaagaag atcggctctt 5700

tcagcctcga gcagcttcaa gaatacgctg atgctgatct cagcgtggtc gagaagctca 5760

aagagatcat catccagaag gtcgacgaga tctacaaggt gtacgggtcc tctgagaagt 5820

tgttcgatgc tgatttcgtc ctcgagaaga gtctgaagaa gaacgacgct gtcgtcgcga 5880

tcatgaagga tttgctcgac agcgtgaagt ccttcgagaa ctatatcaag gccttcttcg 5940

gagagggcaa agagactaat agggacgagt ctttctacgg ggatttcgtg ctcgcttacg 6000

atatcctcct caaggtggac catatctacg acgccatcag aaactacgtg acccagaagc 6060

cttacagcaa ggacaagttc aagttgtact ttcagaaccc gcagttcatg ggcggatggg 6120

acaaagacaa agagacagat tacaggccca ccatcctcag gtacgggtct aagtactacc 6180

tggccatcat ggacaagaaa tacgccaagt gcctccaaaa gatcgacaag gatgacgtga 6240

acgggaacta tgagaagatc aactacaagc tccttccggg accgaacaag atgcttccta 6300

aggtgttctt cagcaagaaa tggatggcct actacaaccc gtctgaggac atccagaaaa 6360

tctacaagaa cgggaccttc aagaaaggcg acatgttcaa cctcaacgac tgccacaagc 6420

tcatcgattt cttcaaggac agcatctcgc gttacccgaa gtggtctaac gcttacgact 6480

ttaacttcag cgagacagaa aagtacaagg atatcgccgg gttctaccgt gaggttgagg 6540

aacagggtta caaggttagc ttcgagagcg cctccaagaa agaggttgac aagttggtcg 6600

aagagggcaa gctctacatg ttccagatct ataacaagga cttctccgac aagagccacg 6660

gaactcctaa cctccatacg atgtacttca agctgctttt cgacgagaac aaccacgggc 6720

agatcagact ttctggtggt gctgaactct tcatgcgtag ggcctcactc aagaaagaag 6780

agttggttgt tcacccggcc aactctccaa tcgctaacaa gaatcctgac aacccgaaaa 6840

agaccaccac gctgtcttac gacgtctaca aggacaaaag gttcagcgag gaccagtacg 6900

agcttcatat cccgatcgct atcaacaagt gcccgaagaa catcttcaag atcaataccg 6960

aggtgagggt gctgctcaag cacgatgata acccttacgt gatcggaatc gatcgtggtg 7020

agagaaacct cctctacatc gttgtggtgg acggaaaggg aaacatcgtc gagcagtaca 7080

gcctgaacga gattatcaac aatttcaacg gcatcaggat caagaccgac taccactcac 7140

tcctcgataa gaaagaaaaa gagcgtttcg aggccaggca gaactggact tctatcgaaa 7200

acatcaaaga gttgaaggcc ggctacatct ctcaggtggt gcataagatc tgcgagctgg 7260

tggaaaagta cgatgctgtg atcgctcttg aggacctcaa ctctgggttc aagaacagta 7320

gagtgaaggt tgagaagcag gtctaccaaa agttcgagaa gatgctcatc gacaagctca 7380

actacatggt ggacaaaaag agcaaccctt gcgctaccgg tggtgctctt aagggatacc 7440

agatcacgaa caagttcgag tccttcaaga gcatgagcac ccagaacggc ttcatcttct 7500

atatccctgc ttggctcacc agcaagatcg atccttctac tggtttcgtg aacctgctca 7560

agaccaagta cacctcgatc gccgacagca agaagttcat ctcgtctttc gacaggatca 7620

tgtacgtgcc ggaagaggat cttttcgagt tcgctctcga ctataagaac ttcagcagga 7680

ccgacgccga ctacattaag aagtggaagc tctactccta cgggaaccgt atcaggatct 7740

tccgaaatcc gaagaaaaac aacgtgttcg actgggaaga agtgtgcctc acctctgcct 7800

acaaagaact gttcaacaag tacggcatca actaccagca gggtgatatc agggctcttt 7860

tgtgcgagca gagcgacaag gcattctaca gctcattcat ggccctcatg tctctcatgc 7920

tccagatgag gaactctatc accggaagga ccgatgtgga cttccttatc tctccggtca 7980

agaactctga cgggatcttc tacgacagcc gtaactatga ggctcaagag aacgctatcc 8040

tgccgaagaa tgctgatgca aacggggctt acaacattgc gagaaaggtt ctctgggcta 8100

tcgggcagtt taagaaagcg gaagatgaga agctcgacaa ggtgaagatc gccatctcca 8160

acaaagagtg gcttgagtac gctcagacct ccgttaagca caagaggcct gctgctacta 8220

agaaagctgg ccaggccaaa aagaagaagt gaggcgcgcc gagctccagg cctcccagct 8280

ttcgtccgta tcatcggttt cgacaacgtt cgtcaagttc aatgcatcag tttcattgcc 8340

cacacaccag aatcctacta agtttgagta ttatggcatt ggaaaagctg ttttcttcta 8400

tcatttgttc tgcttgtaat ttactgtgtt ctttcagttt ttgttttcgg acatcaaaat 8460

gcaaatggat ggataagagt taataaatga tatggtcctt ttgttcattc tcaaattatt 8520

attatctgtt gtttttactt taatgggttg aatttaagta agaaaggaac taacagtgtg 8580

atattaaggt gcaatgttag acatataaaa cagtctttca cctctctttg gttatgtctt 8640

gaattggttt gtttcttcac ttatctgtgt aatcaagttt actatgagtc tatgatcaag 8700

taattatgca atcaagttaa gtacagtata ggcttgagct ccctaggatc aagcttatcg 8760

atgtcgacag gccttaaggg ccagatcttg ggcccggtac ccgatcagat tgtcgtttcc 8820

cgccttcggt ttaaactatc agtgtttgac aggatatatt ggcgggtaaa cctaagagaa 8880

aagagcgttt attagaataa tcggatattt aaaagggcgt gaaaaggttt atccgttcgt 8940

ccatttgtat gtgcatgcca accacagggt tcccctcggg agtgcttggc attccgtgcg 9000

ataatgactt ctgttcaacc acccaaacgt cggaaagcct gacgacggag cagcattcca 9060

aaaagatccc ttggctcgtc tgggtcggct agaaggtcga gtgggctgct gtggcttgat 9120

ccctcaacgc ggtcgcggac gtagcgcagc gccgaaaaat cctcgatcgc aaatccgacg 9180

ctgtcgaaaa gcgtgatctg cttgtcgctc tttcggccga cgtcctggcc agtcatcacg 9240

cgccaaagtt ccgtcacagg atgatctggc gcgagttgct ggatctcgcc ttcaatccgg 9300

gtctgtggcg ggaactccac gaaaatatcc gaacgcagca agatatcgcg gtgcatctcg 9360

gtcttgcctg ggcagtcgcc gccgacgccg ttgatgtgga cgccgaaaag gatctaggtg 9420

aagatccttt ttgataatct catgaccaaa atcccttaac gtgagttttc gttccactga 9480

gcgtcagacc ccgtagaaaa gatcaaagga tcttcttgag atcctttttt tctgcgcgta 9540

atctgctgct tgcaaacaaa aaaaccaccg ctaccagcgg tggtttgttt gccggatcaa 9600

gagctaccaa ctctttttcc gaaggtaact ggcttcagca gagcgcagat accaaatact 9660

gttcttctag tgtagccgta gttaggccac cacttcaaga actctgtagc accgcctaca 9720

tacctcgctc tgctaatcct gttaccagtg gctgctgcca gtggcgataa gtcgtgtctt 9780

accgggttgg actcaagacg atagttaccg gataaggcgc agcggtcggg ctgaacgggg 9840

ggttcgtgca cacagcccag cttggagcga acgacctaca ccgaactgag atacctacag 9900

cgtgagctat gagaaagcgc cacgcttccc gaagggagaa aggcggacag gtatccggta 9960

agcggcaggg tcggaacagg agagcgcacg agggagcttc cagggggaaa cgcctggtat 10020

ctttatagtc ctgtcgggtt tcgccacctc tgacttgagc gtcgattttt gtgatgctcg 10080

tcaggggggc ggagcctatg gaaaaacgcc agcaacgcgg cctttttacg gttcctggcc 10140

ttttgctggc cttttgctca catgttcttt cctgcgttat cccctgattc tgtggataac 10200

cgattaccgc ctttgagtga gctgataccg ctcgccgcag ccgaacgacc gagcgcagcg 10260

agtcagtgag cgaggaagcg gaagagcgcc tgatgcggta ttttctcctt acgcatctgt 10320

gcggtatttc acaccgcata tggtgcactc tcagtacaat ctgctctgat gccgcatagt 10380

taagccagta tacactccgc tatcgctacg tgactgggtc atggctgcgc cccgacaccc 10440

gccaacaccc gctgacgcgc cctgacgggc ttgtctgctc ccggcatccg cttacagaca 10500

agctgtgacc gtctccggga gctgcatgtg tcagaggttt tcaccgtcat caccgaaacg 10560

cgcgaggcag gggtacgtcg aggtcgatcc aacccctccg ctgctatagt gcagtcggct 10620
tctgacgttc agtgcagccg tcgtctgaaa acgacatgtc gcacaagtcc taagttacgc 10680
gacaggctgc cgccctgccc ttttcctggc gttttcttgt cgcgtgtttt agtcgcataa 10740
agtagaatac ttgcgactag aaccggagac attacgccat gaacaagagc gccgccgctg 10800
gcctgctggg ctatgcccgc gtcagcaccg acgaccagga cttgaccaac caacgggccg 10860
aactgcacgc ggccggctgc accaagctgt tttccgagaa gatcaccggc accaggcgcg 10920
accgcccgga gctggccagg atgcttgacc acctacgccc tggcgacgtt gtgacagtga 10980
ccaggctaga ccgcctggcc cgcagcaccc gcgacctact ggacattgcc gagcgcatcc 11040
aggaggccgg cgcgggcctg cgtagcctgg cagagccgtg ggccgacacc accacgccgg 11100
ccggccgcat ggtgttgacc gtgttcgccg gcattgccga gttcgagcgt tccctaatca 11160
tcgaccgcac ccggagcggg cgcgaggccg ccaaggcgcg aggcgtgaag tttggccccc 11220
gccctaccct caccccggca cagatcgcgc acgcccgcga gctgatcgac caggaaggcc 11280
gcaccgtgaa agaggcggct gcactgcttg gcgtgcatcg ctcgaccctg taccgcgcac 11340
ttgagcgcag cgaggaagtg acgcccaccg aggccaggcg gcgcggtgcc ttccgtgagg 11400
acgcattgac cgaggccgac gccctggcgg ccgccgagaa tgaacgccaa gaggaacaag 11460
catgaaaccg caccaggacg gccaggacga accgtttttc attaccgaag agatcgaggc 11520
ggagatgatc gcggccgggt acgtgttcga gccgcccgcg cacgtctcaa ccgtgcggct 11580
gcatgaaatc ctggccggtt tgtctgatgc caagctcgcg gcctggccgg cgagcttggc 11640
cgctgaagaa accgagcgcc gccgtctaaa aaggtgatgt gtatttgagt aaaacagctt 11700
gcgtcatgcg gtcgctgcgt atatgatgcg atgagtaaat aaacaaatac gcaaggggaa 11760
cgcatgaagg ttatcgctgt acttaaccag aaaggcgggt caggcaagac gaccatcgca 11820
acccatctag cccgcgccct gcaactcgcc ggggccgatg ttctgttagt cgattccgat 11880
ccccagggca gtgcccgcga ttgggcggcc gtgcgggaag atcaaccgct aaccgttgtc 11940
ggcatcgacc gcccgacgat tgaccgcgac gtgaaggcca tcggccggcg cgacttcgta 12000
gtgatcgacg gagcgcccca ggcggcggac ttggctgtgt ccgcgatcaa ggcagccgac 12060
ttcgtgctga ttccggtgca gccaagccct tacgacatat gggccaccgc cgacctggtg 12120
gagctggtta agcagcgcat tgaggtcacg gatggaaggc tacaagcggc ctttgtcgtg 12180
tcgcgggcga tcaaaggcac gcgcatcggc ggtgaggttg ccgaggcgct ggccgggtac 12240
gagctgccca ttcttgagtc ccgtatcacg cagcgcgtga gctacccagg cactgccgcc 12300
gccggcacaa ccgttcttga atcagaaccc gagggcgacg ctgcccgcga ggtccaggcg 12360
ctggccgctg aaattaaatc aaaactcatt tgagttaatg aggtaaagag aaaatgagca 12420
aaagcacaaa cacgctaagt gccggccgtc cgagcgcacg cagcagcaag gctgcaacgt 12480

tggccagcct ggcagacacg ccagccatga agcgggtcaa ctttcagttg ccggcggagg 12540

atcacaccaa gctgaagatg tacgcggtac gccaaggcaa gaccattacc gagctgctat 12600

ctgaatacat cgcgcagcta ccagagtaaa tgagcaaatg aataaatgag tagatgaatt 12660

ttagcggcta aaggaggcgg catggaaaat caagaacaac caggcaccga cgccgtggaa 12720

tgccccatgt gtggaggaac gggcggttgg ccaggcgtaa gcggctgggt tgtctgccgg 12780

ccctgcaatg gcactggaac ccccaagccc gaggaatcgg cgtgagcggt cgcaaaccat 12840

ccggcccggt acaaatcggc gcggcgctgg gtgatgacct ggtggagaag ttgaaggcgg 12900

cgcaggccgc ccagcggcaa cgcatcgagg cagaagcacg ccccggtgaa tcgtggcaag 12960

cggccgctga tcgaatccgc aaagaatccc ggcaaccgcc ggcagccggt gcgccgtcga 13020

ttaggaagcc gcccaagggc gacgagcaac cagatttttt cgttccgatg ctctatgacg 13080

tgggcacccg cgatagtcgc agcatcatgg acgtggccgt tttccgtctg tcgaagcgtg 13140

accgacgagc tggcgaggtg atccgctacg agcttccaga cgggcacgta gaggtttccg 13200

cagggccggc cggcatggcg agtgtgtggg attacgacct ggtactgatg gcggtttccc 13260

atctaaccga atccatgaac cgataccggg aagggaaggg agacaagccc ggccgcgtgt 13320

tccgtccaca cgttgcggac gtactcaagt tctgccggcg agccgatggc ggaaagcaga 13380

aagacgacct ggtagaaacc tgcattcggt taaacaccac gcacgttgcc atgcagcgta 13440

cgaagaaggc caagaacggc cgcctggtga cggtatccga gggtgaagcc ttgattagcc 13500

gctacaagat cgtaaagagc gaaaccgggc ggccggagta catcgagatc gagctagctg 13560

attggatgta ccgcgagatc acagaaggca agaacccgga cgtgctgacg gttcaccccg 13620

attacttttt gatcgatccc ggcatcggcc gttttctcta ccgcctggca cgccgcgccg 13680

caggcaaggc agaagccaga tggttgttca agacgatcta cgaacgcagt ggcagcgccg 13740

gagagttcaa gaagttctgt ttcaccgtgc gcaagctgat cgggtcaaat gacctgccgg 13800

agtacgattt gaaggaggag gcggggcagg ctggcccgat cctagtcatg cgctaccgca 13860

acctgatcga gggcgaagca tccgccggtt cctaatgtac ggagcagatg ctagggcaaa 13920

ttgccctagc aggggaaaaa ggtcgaaaag gtctctttcc tgtggatagc acgtacattg 13980

ggaacccaaa gccgtacatt gggaaccgga acccgtacat tgggaaccca aagccgtaca 14040

ttgggaaccg gtcacacatg taagtgactg atataaaaga gaaaaaaggc gatttttccg 14100

cctaaaactc tttaaaactt attaaaactc ttaaaacccg cctggcctgt gcataactgt 14160

ctggccagcg cacagccgaa gagctgcaaa aagcgcctac ccttcggtcg ctgcgctccc 14220

tacgccccgc cgcttcgcgt cggcctatcg cggccgctgg ccgctcaaaa atggctggcc 14280

tacggccagg caatctacca gggcgcggac aagccgcgcc gtcgccactc gaccgccggc 14340

gcccacatca aggcaccggt gggtatgcct gacgatgcgt ggagaccgaa accttgcgct 14400

cgttcgccag ccaggacaga aatgcctcga cttcgctgct gcccaaggtt gccgggtgac 14460

gcacaccgtg gaaacggatg aaggcacgaa cccagtggac ataagcctgt tcggttcgta 14520

agctgtaatg caagtagcgt atgcgctcac gcaactggtc cagaaccttg accgaacgca 14580

gcggtggtaa cggcgcagtg gcggttttca tggcttgtta tgactgtttt tttggggtac 14640

agtctatgcc tcgggcatcc aagcagcaag cgcgttacgc cgtgggtcga tgtttgatgt 14700

tatggagcag caacgatgtt acgcagcagg gcagtcgccc taaaacaaag ttaaacatca 14760

tgagggaagc ggtgatcgcc gaagtatcga ctcaactatc agaggtagtt ggcgtcatcg 14820

agcgccatct cgaaccgacg ttgctggccg tacatttgta cggctccgca gtggatggcg 14880

gcctgaagcc acacagtgat attgatttgc tggttacggt gaccgtaagg cttgatgaaa 14940

caacgcggcg agctttgatc aacgaccttt tggaaacttc ggcttcccct ggagagagcg 15000

agattctccg cgctgtagaa gtcaccattg ttgtgcacga cgacatcatt ccgtggcgtt 15060

atccagctaa gcgcgaactg caatttggag aatggcagcg caatgacatt cttgcaggta 15120

tcttcgagcc agccacgatc gacattgatc tggctatctt gctgacaaaa gcaagagaac 15180

atagcgttgc cttggtaggt ccagcggcgg aggaactctt tgatccggtt cctgaacagg 15240

atctatttga ggcgctaaat gaaaccttaa cgctatggaa ctcgccgccc gactgggctg 15300

gcgatgagcg aaatgtagtg cttacgttgt cccgcatttg gtacagcgca gtaaccggca 15360

aaatcgcgcc gaaggatgtc gctgccgact gggcaatgga gcgcctgccg gcccagtatc 15420

agcccgtcat acttgaagct agacaggctt atcttggaca agaagaagat cgcttggcct 15480

cgcgcgcaga tcagttggaa gaatttgtcc actacgtgaa aggcgagatc accaaggtag 15540

tcggcaaata atgtctaaca attcgttcaa gccgacgccg cttcgcggcg cggcttaact 15600

caagcgttag atgcactaag cacataattg ctcacagcca aactatcagg tcaagtctgc 15660

ttttattatt tttaagcgtg cataataagc cctacacaaa ttgggagata tatcatgaaa 15720

ggctggcttt ttcttgttat cgcaatagtt ggcgaagtaa tcgcaacata gcttgcttgg 15780

tcgttccgcg tgaacgtcgg ctcgattgta cctgcgttca aatactttgc gatcgtgttg 15840

cgcgcctgcc cggtgcgtcg gctgatctca cggatcgact gcttctctcg caacgccatc 15900

cgacggatga tgtttaaaag tcccatgtgg atcactccgt tgccccgtcg ctcaccgtgt 15960

tggggggaag gtgcacatgg ctcagttctc aatggaaatt atctgcctaa ccggctcagt 16020

tctgcgtaga aaccaacatg caagctccac cgggtgcaaa gcggcagcgg cggcaggata 16080

tattcaattg taaatggctt catgtccggg aaatctacat ggatcagcaa tgagtatgat 16140

ggtcaatatg gagaaaaaga aagagtaatt accaattttt tttcaattca aaaatgtaga 16200

tgtccgcagc gttattataa aatgaaagta cattttgata aaacgacaaa ttacgatccg 16260

tcgtatttat aggcgaaagc aataaacaaa ttattctaat tcggaaatct ttatttcgac 16320

gtgtctacat tcacgtccaa atgggggctt agatgagaaa cttcacgatc ggctctagtt 16380

cgagcgatct agtaacatag atgacaccgc gcgcgataat ttatcctagt ttgcgcgcta 16440

tattttgttt tctatcgcgt attaaatgta taattgcggg actctaatca taaaaaccca 16500

tctcataaat aacgtcatgc attacatgtt aattattaca tgcttaacgt aattcaacag 16560

aaattatatg ataatcatcg caagaccggc aacaggattc aatcttaaga aactttattg 16620

ccaaatgttt gaacgatcgg ggaaattcgt ttctatgctt caattctgct ctatagatgt 16680

tcttctagtc ttaactttta gaaggcacaa catttgaaat gttggtaatt tttctgctgg 16740

gtagtgaacc attgtaattt gtgtttgctt atattatccg ttgggtttct ctatgttatc 16800

tgtggggcca tatcccagaa ctggttgagt cggtccaaca cctgggtgcc aatcatgtcg 16860

atggtggggt atggt 16875

<210> 75
<211> 4120
<212> DNA
<213> Beta vulgaris

<400> 75
gtatatcgtt gccacatgtg cgttgaattt ttccttttcc tatcctttcc actccatatt 60

ctcctcaaaa gtgtgtaaaa atccgacaca cgagtagaat gggattgaag tgggtcaaga 120

tctgaaacca atgggtcaat gccacaaaat aaggtaaggt ttctcgcagt agcaaaaaaa 180

taaagttaag ttgagagaaa aattatgaat agttgtttct cgtgaagagt tgtatacaaa 240

aaaagtctaa tttgatacat tttcttttac atttataaag gattgaccaa tcatccaaat 300

taccaaatat ttaggatata aatctttcag attacaaccc atatatgata cactaaattt 360

tacatgaggc aatggaggat ttgcatgaat atcgaggaga gaaaaaatta gttacaaaac 420

ttgcataatt tatccaaacc aaatcaagtc aagaaacaac gaacaatatt atcattagta 480

ctataagtat atattatagg cttagagcaa agccctaact accacactgc acacaaatga 540

taactagtaa gagaggaaaa tacaaattta agattcaaca tagcaaatta ttcatgattc 600

atgattcatg attcatgatt catgattcac gaacatcaag aatggtatag ctgataaagg 660

acaatttaaa cataagtgta aagctcgcac atcatcaatt atattcgcat actactagac 720

caatctttac ttagtacatg tgttagtaca tgtgttactt catatcagat gtattgattg 780

ttgccaatga catatcatgt tcacttaatc ttagggccat ttaattataa catggagaat 840

aatacaactt aaaattatgt ggtggctatc atctcatttt ctagataatt aaacctttat 900

tttgtataca tatatattgt ctttacatag caaaacaata ttgaaggtat aacaaccttt 960

cccttttctt ttactacatg tttatgttag agtttttcga tttacgattg tggtaaatta 1020

attgtaattg atcggttgtc ttgtagtcaa gaaatgacgt atgaatcaat ttagggcatg 1080

ttctcttcgg cataaaacag ctgaactgaa ttgaactgaa ctgaaatgaa tagtgatatg 1140

tgagagtaaa agtattgtca agagctgaac tgagctgaac tgaacggatc tgaactgaac 1200

tgatctaatc tgaactaatc tgaactgaac tgaactgaat tgaactgaaa ataagctagg 1260

gaaaacagac ccttactact attatataac ctcgtttaaa tattaggaaa ttaaaaaaat 1320

aattatattt ctttatactt tattaaccta ttgaggtttt tatattgact cccaaatact 1380

attttataga tcatgccatg ttaatgagca aactactttc tcacatcttt ataggagaaa 1440

aagtagatca ctcactagca tatcatgacc agcgaaacca accaacgcta atagttttat 1500

tgcttccatt agagataaga gttaactaat aataccatct ttgtgaaatt ttgatggatt 1560

ttggaacttt agcaatatat ttactgtgtg cactttttgc taccaaaatt ttataccaat 1620

ggctcaagtc ctacttatac acaacataca aactccctcc cggcccacca aggtggccct 1680

tatttggaaa cctccttcaa ctagggccac ttccccaccg cgatttcgcc tcattttgtg 1740

aaaaatatgg gcctttagtc tacataaggc ttggtaatgt ggatgccata accactaatg 1800

atccagaaat catacgtgag atcttagttc gacaagatga tgtatttgcg tctcgtccac 1860

ataccttagc cgcaacccac ttggcttaca atagtggtga tgtggccttg gctccaatgg 1920

gaccaaaatg gaaaagaatg agaaggatat gcatggagca cttgctcaca actagacgac 1980

ttgaactatt tgtgagtcat agggctgatg aggcacgaca tttggtccaa gacgtattaa 2040

ctcgttccca caaagataaa gttgttaatt tgagggaagt gttaggtgca ttttctatga 2100

ataacgtgac tagaatgttg ctagggaagc aatactttgg ggccgggacg gcgggcccac 2160

aagaggctct agagtttatg catataacac atgagttgtt ttggttacta ggcttgattt 2220

acttgggtga ttatttgcct ttttggaggt gggttgatcc atatggatgt gaaaagaaaa 2280

tgagggaagt tgaaaaaagg gtagatgatt tccatcgcaa aattatagag gaacatagga 2340

aggagaagaa aaggaaagaa gaaatgggag tgaatgaggg tgaaatggat tttgtagata 2400

ttttgttggc tttgcctggt gaaaatggaa atgagcatat ggatgatgca gatattaaag 2460

ctctaattca ggtaattcat gtataatttg aatgtgatcg atacaaagtt tgatagaaaa 2520

catatttgca taaatatatg gttgccctac tagacccaat aaaatacata attattgcct 2580

tactagttga aagttgaaac aacctagcta ccattttgtt gtgattatca ttagccaacc 2640

aaaattattt cttgcatcca tatattaatg ttgagatcag agtcggcata tttacaatta 2700

cttgtaacat tttaagcaaa caaattaaaa tattttttgg caagtccatt ttattgaata 2760

atacctatat cttaaaatga attcttggtc atgtacactt gcctttcaag gtaccaatat 2820

ttgaccatat gtaattacta ttaacaaatt tgataaaatc taataatatg taaatataca 2880

ttcacgcaca tattagaaac aaagatcaca aatgataatg caaaataact tatttgaact 2940

aatgttgtga agttaaattt ggaacaaaag gtatatttgt attgccgaat tttaatttat 3000

aaattactta taacaacaac tcaatatgta aaactgttaa gatggagtgt ggatagaatg 3060

agatgagtat acttttacta gttaccactc gaaaatgcat ttcctccttt gtttatagtt 3120

gttctaactt ctattatcat aaataatttt ttggacttat ttcaatgtat atttacaacg 3180

ctaattgttt aattttttaa aaatacataa tgtaaacaag gatttcatgg tcaattatac 3240

acattataaa tattatctta aaaaacttat taatgctcaa ttagtatcca taatatagga 3300

tatgatagca gcagcaacag acacatcagc tgtaaccaac gaatgggcca tggcagaagt 3360

aataaaacac ccacgtgtcc tccacaagat ccaacaagag cttaacacaa tagtaggacc 3420

caatcgaatg gtaacagaat cagatcttcc ccaccttaac tacctacgtt gtgtcgtacg 3480

tgaaacgttc cgaatgcatc cagcaggacc ctttttaatc ccacatgaat cactacgcca 3540

tacaacaatc aacggctatg atatcccatc tgggacacgt gtcttcatca acacacatgg 3600

gttaggacgt aaccttaaag tgtgggacaa catagaggat ttttaccctg aaagacattg 3660

gccgttggat ggaagtagag ttgagattag ccatggatct gattttaaaa tattaccatt 3720

tagtgctggg aagagaagat gtcctggggc cccacttggg gtggtgtttg tgttgatggg 3780

attggctaca ctttttcatg catttgattg gttaccacct gatggaatga aggcagaaga 3840

aattgatact aaggaagttt atgggatgac tatgcctaaa gctcaacctt taatggcttt 3900

ggctaaacct aggcttgctc atttatatct ttgatacatg ttcatattgt ggtgcactta 3960

taagcacaat agacaaatac aagtttgtat cgactctaac atgttgttta gtattagtat 4020

actgcaactc tacaagtatg taatttctat aaactataaa cacaagtcat aacgcatttt 4080

gttttgaaaa aaaagaggtt acattgtctt acaccataaa 4120

<210> 76
<211> 4220
<212> DNA
<213> Beta vulgaris

<400> 76
gtatatcgtt gccacatgtg cgttgaattt ttccttttcc tatcctttcc actccatatt 60

ctcctcaaaa gtgtgtaaaa atccgacaca cgagtagaat gggattgaag tgggtcaaga 120

tctgaaacca atgggtcaat gccacaaaat aaggtaaggt ttctcgcagt agcaaaaaaa 180

taaagttaag ttgagagaaa aattatgaat agttgtttct cgtgaagagt tgtatacaaa 240

aaaagtctaa tttgatacat tttcttttac atttataaag gattgaccaa tcatccaaat 300

taccaaatat ttaggatata aatctttcag attacaaccc atatatgata cactaaattt 360

tacatgaggc aatggaggat ttgcatgaat atcgaggaga gaaaaaatta gttacaaaac 420

ttgcataatt tatccaaacc aaatcaagtc aagaaacaac gaacaatatt atcattagta 480

ctataagtat atattatagg cttagagcaa agccctaact accacactgc acacaaatga 540

taactagtaa gagaggaaaa tacaaattta agattcaaca tagcaaatta ttcatgattc 600

atgattcatg attcatgatt catgattcac gaacatcaag aatggtatag ctgataaagg 660

acaatttaaa cataagtgta aagctcgcac atcatcaatt atattcgcat actactagac 720

caatctttac ttagtacatg tgttagtaca tgtgttactt catatcagat gtattgattg 780

ttgccaatga catatcatgt tcacttaatc ttagggccat ttaattataa catggagaat 840

aatacaactt aaaattatgt ggtggctatc atctcatttt ctagataatt aaacctttat 900

tttgtataca tatatattgt ctttacatag caaaacaata ttgaaggtat aacaaccttt 960

cccttttctt ttactacatg tttatgttag agtttttcga tttacgattg tggtaaatta 1020

attgtaattg atcggttgtc ttgtagtcaa gaaatgacgt atgaatcaat ttagggcatg 1080

ttctcttcgg cataaaacag ctgaactgaa ttgaactgaa ctgaaatgaa tagtgatatg 1140

tgagagtaaa agtattgtca agagctgaac tgagctgaac tgaacggatc tgaactgaac 1200

tgatctaatc tgaactaatc tgaactgaac tgaactgaat tgaactgaaa ataagctagg 1260

gaaaacagac ccttactact attatataac ctcgtttaaa tattaggaaa ttaaaaaaat 1320

aattatattt ctttatactt tattaaccta ttatacaata gtggtgatgt ggccttggct 1380

ccaatgggac caaaatggaa aagaatgaga aggatatgca tggagcactt gctcacaact 1440

agacgacttg aactatttgt gagtcatagg gctgatgagg cacgacattt ggtccaagac 1500

gtattaactc gttcccacaa agataaagtt gttaatttga gggaagtgtt aggtgcattt 1560

tctatgaata acgtgactag aatgttgcta gggaagcaat actttggggc cgggacggcg 1620

ggcccacaag aggctctaga gtttatgcat ataacacatg agttgttttg gttactaggc 1680

ttgatttact tgggtgatta tttgcctttt tggaggtggg ttgatccata tggatgtgaa 1740

aagaaaatga gggaagttga aaaaagggta gatgatttcc atcgcaaaat tatagaggaa 1800

cataggaagg agaagaaaag gaaagaagaa atgggagtga atgagggtga aatggatttt 1860

gtagatattt tgttggcttt gcctggtgaa aatggaaatg agcatatgga tgatgcagat 1920

attaaagctc taattcaggt aattcatgta taatttgaat gtgatacaaa gtttgataga 1980

aaacatattt gcataaatac atggttaccc tactagaccc aataaaatac ataattattg 2040

ccttgctagt tgaaagttga aacaacctag ctaccatttt gttgtgatta tcattagcca 2100

accaaaacta tttcttgcat ccatatatta atgttgagag tcgccatatt tacaattact 2160

tgtaacattt tgagcaaaca aattaaaata ttttttggca agtccatttt attgaatgat 2220

acctatatct taaaatgaat ccttggttat gtacacttgc ctttcaaggt accaatattt 2280

gaccatatgt aattactatt aacaaatttg ataaaatcta ataatatgta aatatacgtt 2340

cacgcacata ttagaaacaa agatcacaaa tgataatgca aaataactta tttgaactaa 2400

tgttgtgaag ttaaatttgg aacaaaaggt atatttgtat tgccgaattt taatttataa 2460

attacttata acaacaactc aatatgtaaa actgttaaga tggagtgtgg atagaatgag 2520

atgagtatac ttttactagt taccactcga aattgcattt cctcctttgt ttatagttgt 2580

tctaacttct attatcataa ataatttttg gacttatttc aatgtatatt tacaacgtta 2640

attgtttaat tttttaaaaa tacataatgt aaacaaggat ttcatggtca attatacaca 2700

ttatagatat tatcttaaaa aacttactaa tgctcaatta gtgtccataa tataggatat 2760

gatagcagca gcaacagaca catcagctgt aaccaacgaa tgggccatgg cagaagtaat 2820

aaaacaccca cgtgtcctcc acaagatcca acaagagctt aacacaatag taggacccaa 2880

tcgaatggta acagaatcag atcttcccca ccttaactac ctacgttgtg tcgtacgcga 2940

aacgtttcgg atgcatccag caggaccctt tttaatccca catgaatcac tacgccatac 3000

aacaatcaac ggctatgata tcccatctgg gacacgtgtc ttcatcaaca cacatgggtt 3060

aggacgtaac cttaaagtgt gggacaacat agaggatttt taccctgaaa gacattggcc 3120

gttggatgga agtagagttg agattagcca tggatctgat tttaaaatat taccatttag 3180

tgctgggaag agaagatgtc ctggggcccc acttggggtg gtgtttgtgt tgatgggatt 3240

ggctacactt tttcatgcat ttgattggtt accacctgat ggaatgaagg cagaagaaat 3300

tgatactaag gaagtttatg ggatgactat gcctaaagct caacctttaa tggctttggc 3360

taaacctagg cttgctcctc atttatatct ttgatacatg ttcatattgt ggtgcactta 3420

taagcacact agctcaaata gacaaataca agcttgtatt gactctaaca tgttatttaa 3480

tattagcata ctgcaactct acaactacaa gtatgtaatc tctataaatt ataaacataa 3540

gtcataacgc atcttgtttt gaaaaaaaaa gctacacttt cttacaccat aaactgttcc 3600

gttactaaat tcttcttggc ttattttaga attaattttt caattttcct atttacttat 3660

aacacaagat aagaaaatcg actaacaata tttcacaaca ttatatcttg atagttcgtt 3720

gaaattaata attttcaata atttcaaaac gcaaaaagct tctttgttcg gataaattca 3780

cgtcattttc aataaaatcg caaaatcttc ccttgccatc aaggaacaaa tttttcacat 3840

caaaactctt cttccaaaaa gtatacttct ccattgatta tgtaaaagag tttgcatgtt 3900

ctaatccatt agtatgtatt tttcggataa gaagttgtat tacaagctta aataaagata 3960

taaacttagt taaatatcga ccaatccaca atatgtatct tcatatgtgt ccatcagcgc 4020

ctaaccttga gatgtagatc atctaaagca cattaacatg gcgtactcct cctgttcaaa 4080

tcagggttgt ttgaaaccaa acttctccaa aatacttcct cagtaatgaa gagaaaaact 4140

cacattctga aaaacatcaa catcatggtt ctatggtcta gtggttatga cactggactc 4200

tgaatccagt aacccgagtt                                                4220

<210>  77
<211>  4211
<212>  DNA
<213>  Artificial Sequence

<220>
<223>  BvCYP703A2_gst including artificial InDel marker allele

<400>  77
gtatatcgtt gccacatgtg cgttgaattt ttccttttcc tatcctttcc actccatatt      60

ctcctcaaaa gtgtgtaaaa atccgacaca cgagtagaat gggattgaag tgggtcaaga     120

tctgaaacca atgggtcaat gccacaaaat aaggtaaggt ttctcgcagt agcaaaaaaa     180

taaagttaag ttgagagaaa aattatgaat agttgtttct cgtgaagagt tgtatacaaa     240

aaaagtctaa tttgatacat tttcttttac atttataaag gattgaccaa tcatccaaat     300

taccaaatat ttaggatata aatctttcag attacaaccc atatatgata cactaaattt     360

tacatgaggc aatggaggat ttgcatgaat atcgaggaga gaaaaaatta gttacaaaac     420

ttgcataatt tatccaaacc aaatcaagtc aagaaacaac gaacaatatt atcattagta     480

ctataagtat atattatagg cttagagcaa agccctaact accacactgc acacaaatga     540

taactagtaa gagaggaaaa tacaaattta agattcaaca tagcaaatta ttcatgattc     600

atgattcatg attcatgatt catgattcac gaacatcaag aatggtatag ctgataaagg     660

acaatttaaa cataagtgta aagctcgcac atcatcaatt atattcgcat actactagac     720

caatctttac ttagtacatg tgttagtaca tgtgttactt catatcagat gtattgattg     780

ttgccaatga catatcatgt tcacttaatc ttagggccat ttaattataa catggagaat     840

aatacaactt aaaattatgt ggtggctatc atctcatttt ctagataatt aaacctttat     900

tttgtataca tatatattgt ctttacatag caaaacaata ttgaaggtat aacaaccttt     960

cccttttctt ttactacatg tttatgttag agtttttcga tttacgattg tggtaaatta    1020

attgtaattg atcggttgtc ttgtagtcaa gaaatgacgt atgaatcaat ttagggcatg    1080

ttctcttcgg cataaaacag ctgaactgaa ttgaactgaa ctgaaatgaa tagtgatatg    1140

tgagagtaaa agtattgtca agagctgaac tgagctgaac tgaacggatc tgaactgaac    1200

tgatctaatc tgaactaatc tgaactgaac tgaactgaat tgaactgaaa ataagctagg    1260

gaaaacagac ccttactact attatataac ctcgtttaaa tattaggaaa ttaaaaaaat    1320

aattatattt ctttatactt tattaaccta ttatacaata gtggtgatgt ggccttggct    1380

ccaatgggac caaaatggaa aagaatgaga aggatatgca tggagcactt gctcacaact    1440

agacgacttg aacttcatag ggctgatgag gcacgacatt tggtccaaga cgtattaact    1500

cgttcccaca aagataaagt tgttaatttg agggaagtgt taggtgcatt ttctatgaat    1560

aacgtgacta gaatgttgct agggaagcaa tactttgggg ccgggacggc gggcccacaa 1620

gaggctctag agtttatgca tataacacat gagttgtttt ggttactagg cttgatttac 1680

ttgggtgatt atttgccttt ttggaggtgg gttgatccat atggatgtga aaagaaaatg 1740

agggaagttg aaaaaagggt agatgatttc catcgcaaaa ttatagagga acataggaag 1800

gagaagaaaa ggaaagaaga aatgggagtg aatgagggtg aaatggattt tgtagatatt 1860

ttgttggctt tgcctggtga aaatggaaat gagcatatgg atgatgcaga tattaaagct 1920

ctaattcagg taattcatgt ataatttgaa tgtgatacaa agtttgatag aaaacatatt 1980

tgcataaata catggttacc ctactagacc caataaaata cataattatt gccttgctag 2040

ttgaaagttg aaacaaccta gctaccattt tgttgtgatt atcattagcc aaccaaaact 2100

atttcttgca tccatatatt aatgttgaga gtcgccatat ttacaattac ttgtaacatt 2160

ttgagcaaac aaattaaaat attttttggc aagtccattt tattgaatga tacctatatc 2220

ttaaaatgaa tccttggtta tgtacacttg cctttcaagg taccaatatt tgaccatatg 2280

taattactat taacaaattt gataaaatct aataatatgt aaatatacgt tcacgcacat 2340

attagaaaca aagatcacaa atgataatgc aaaataactt atttgaacta atgttgtgaa 2400

gttaaatttg gaacaaaagg tatatttgta ttgccgaatt ttaatttata aattacttat 2460

aacaacaact caatatgtaa aactgttaag atggagtgtg gatagaatga gatgagtata 2520

cttttactag ttaccactcg aaattgcatt tcctcctttg tttatagttg ttctaacttc 2580

tattatcata aataattttt ggacttattt caatgtatat ttacaacgtt aattgtttaa 2640

ttttttaaaa atacataatg taaacaagga tttcatggtc aattatacac attatagata 2700

ttatcttaaa aaacttacta atgctcaatt agtgtccata atataggata tgatagcagc 2760

agcaacagac acatcagctg taaccaacga atgggccatg gcagaagtaa taaaacaccc 2820

acgtgtcctc cacaagatcc aacaagagct taacacaata gtaggaccca atcgaatggt 2880

aacagaatca gatcttcccc accttaacta cctacgttgt gtcgtacgcg aaacgtttcg 2940

gatgcatcca gcaggaccct ttttaatccc acatgaatca ctacgccata caacaatcaa 3000

cggctatgat atcccatctg ggacacgtgt cttcatcaac acacatgggt taggacgtaa 3060

ccttaaagtg tgggacaaca tagaggattt ttaccctgaa agacattggc cgttggatgg 3120

aagtagagtt gagattagcc atggatctga ttttaaaata ttaccattta gtgctgggaa 3180

gagaagatgt cctggggccc cacttggggt ggtgtttgtg ttgatgggat tggctacact 3240

ttttcatgca tttgattggt taccacctga tggaatgaag gcagaagaaa ttgatactaa 3300

ggaagtttat gggatgacta tgcctaaagc tcaaccttta atggctttgg ctaaacctag 3360

gcttgctcct catttatatc tttgatacat gttcatattg tggtgcactt ataagcacac 3420

tagctcaaat agacaaatac aagcttgtat tgactctaac atgttattta atattagcat 3480

actgcaactc tacaactaca agtatgtaat ctctataaat tataaacata agtcataacg 3540

catcttgttt tgaaaaaaaa agctacactt tcttacacca taaactgttc cgttactaaa 3600

ttcttcttgg cttattttag aattaatttt tcaattttcc tatttactta taacacaaga 3660

taagaaaatc gactaacaat atttcacaac attatatctt gatagttcgt tgaaattaat 3720

aattttcaat aatttcaaaa cgcaaaaagc ttctttgttc ggataaattc acgtcatttt 3780

caataaaatc gcaaaatctt cccttgccat caaggaacaa atttttcaca tcaaaactct 3840

tcttccaaaa agtatacttc tccattgatt atgtaaaaga gtttgcatgt tctaatccat 3900

tagtatgtat ttttcggata agaagttgta ttacaagctt aaataaagat ataaacttag 3960

ttaaatatcg accaatccac aatatgtatc ttcatatgtg tccatcagcg cctaaccttg 4020

agatgtagat catctaaagc acattaacat ggcgtactcc tcctgttcaa atcagggttg 4080

tttgaaacca aacttctcca aaatacttcc tcagtaatga agagaaaaac tcacattctg 4140

aaaaacatca acatcatggt tctatggtct agtggttatg acactggact ctgaatccag 4200

taacccgagt t 4211

<210> 78
<211> 3252
<212> DNA
<213> Beta vulgaris

<400> 78
atggcggcta ccttcacaaa cccaacattt tccccttcct caactccatt aaccaaaacc 60

ctaaaatccc aatcttccat ctcttcaacc ctcccctttt ccacccctcc caaaacccca 120

actccactct ttcaccgtcc cctccaaatc tcatcctccc aatcccacaa atcatccgcc 180

attaaaacac aaactcaagc accttcttct ccagctattg aagattcatc tttcgtttct 240

cgatttggcc ctgatgaacc cagaaaaggg tccgatgtcc tcgttgaagc tcttgagcgt 300

gaaggtgtta ccaatgtgtt tgcttaccct ggtggtgcat ctatggaaat ccaccaagct 360

ctcacacgct ctaaaaccat ccgcaatgtc ctccctcgcc atgaacaagg cggggttttc 420

gccgccgagg gatatgctag agctactgga aaggttggtg tctgcattgc gacttctggt 480

cctggtgcta ccaacctcgt atcaggtctt gctgacgctc tccttgattc tgtccctctt 540

gttgccatca ctggccaagt tccacgccgt atgattggca ctgatgcttt tcaggagact 600

ccaattgttg aggtgacaag gtctattact aagcataatt atttagtttt ggatgtagag 660

gatattccta gaattgttaa ggaagccttt tttttagcta attctggtag gcctggacct 720

gttttgattg atcttcctaa agatattcag cagcaattgg ttgttcctga ttgggatagg 780

ccttttaagt tgggtgggta tatgtctagg ctgccaaagt ccaagttttc gacgaatgag 840

gttggacttc ttgagcagat tgtgaggttg atgagtgagt cgaagaagcc tgtcttgtat 900
gtgggaggtg ggtgtttgaa ttctagtgag gagttgagga gatttgttga gttgacaggg 960
attccggtgg ctagtacttt gatggggttg gggtcttacc cttgtaatga tgaactgtct 1020
cttcatatgt tggggatgca cgggactgtt tatgccaatt atgcggtgga taaggcggat 1080
ttgttgcttg ctttcggggt taggtttgat gatcgtgtga ccgggaagct cgaggcgttt 1140
gctagccgtg ctaagattgt gcatattgat attgactctg ctgagattgg gaagaacaag 1200
cagccccatg tgtccatttg tgctgatgtt aaattggcat tgcggggtat gaataagatt 1260
ctggagtcta gaatagggaa gctgaatttg gatttctcca agtggagaga agaattaggt 1320
gagcagaaga aggaattccc actgagtttt aagacatttg gggatgcaat tcctccacaa 1380
tatgccattc aggtgcttga tgagttgacc aatggtaatg ctattataag tactggtgtt 1440
gggcagcacc aaatgtgggc tgcgcagcat tacaagtaca gaaaccctcg ccaatggctg 1500
acctctggtg ggttgggggc tatggggttt gggctaccag ccgccattgg agctgcagtt 1560
gctcgaccag atgcagtggt tgtcgatatt gatggggatg gcagttttat tatgaatgtt 1620
caagagttgg ctacaattag ggtggaaaat ctcccagtta agataatgct gctaaacaat 1680
caacatttag gtatggttgt ccaatgggaa gataggttct ataaagctaa ccgggcacat 1740
acataccttg gaaacccttc caaatctgct gatatcttcc ctgatatgct caaattcgct 1800
gaggcatgtg atattccttc tgcccgtgtt agcaacgtgg ctgatttgag ggccgccatt 1860
caaacaatgt tggatactcc agggccgtac ctgctcgatg tgattgtacc gcatcaagag 1920
catgtgttgc ctatgattcc aagtggtgcc ggtttcaagg ataccattac agagggtgat 1980
ggaagaacct cttattgatc ggtttaatga cggttggaac catttaaaga gggtaagcta 2040
tattactgta tgtatattag tatgttcctg gataatttag aagcttttgt ctgttgtctt 2100
ttgcagttta tgaagttagt ttgctgttgt catgttactt gttactttaa aaagcttttt 2160
gtagtttttg agcaactagt atggaatgct cttcctgtat tgcttggaaa attcacaaaa 2220
gtggtttttc ggctatggat gttgtgttgc atcatgcata tatagcttga tatactagtt 2280
ggcttggtgc atctttaaca tatactaatg agactacgac agcaattgcc aattagttgg 2340
cttgacataa ttcttagtct gccactagaa atcttgcttc ttttttttcc ctcatttgtt 2400
gaaagtccct gttgcgacct gacatgggag cattggagat tgtttagcaa tagcagttgc 2460
agttaggtga ctacagtcat cttccaaata tgaatactct ctggagggga ggaggtttta 2520
caaaatatga gtttttaaca catgagaaac ttatattaaa caaggttgag tcaccccata 2580
tttttcaaag ttgacttttg tctgatttgg gtgactatgc ctgttgtatg caataaactc 2640
gatgtacata agacttgtat aatccaatct aacccttcct ggctgattat gaaaccgagt 2700
cggctaattt gttgcttgat cttcatgtgt gagcctgatg ccaggtgacc actagaggag 2760

tacctttcat tgagatatcg attcggttaa ttggttcctc atatgggtct caaaactgaa 2820

tttttctcag gctctcttct aaccagttgt tgaattttat gaaacttcag tccagttaaa 2880

actttgatcc cggatcagaa tttctctgag ttgttcttcc gcctctcagt tccactgttc 2940

cagtgttctt ggtcctcaac ctgtactctg agctatttct gcaaacacct gaagttcctg 3000

ctgcccttgg acaaatacag aatgcagcat tagcatttat ttaagacaag atgaatactt 3060

gctcccctgt tagttatgct aattctgcat tagtattctt taattttaat tagatactgc 3120

aatccgtgaa ctctgcagtt tcttgatctc tctctgtttc aatttctcct atcttatcgc 3180

ccatttcttt aggctttcgt tatctatcgt ctaattcaag agtaggtaac taggtacggg 3240

ataaaacttt tt 3252

<210> 79
<211> 3252
<212> DNA
<213> Artificial Sequence

<220>
<223> BvALS_SU_res: mutated BvALS W569L

<400> 79
atggcggcta ccttcacaaa cccaacattt tccccttcct caactccatt aaccaaaacc 60

ctaaaatccc aatcttccat ctcttcaacc ctcccctttt ccacccctcc caaaacccca 120

actccactct ttcaccgtcc cctccaaatc tcatcctccc aatcccacaa atcatccgcc 180

attaaaacac aaactcaagc accttcttct ccagctattg aagattcatc tttcgtttct 240

cgatttggcc ctgatgaacc cagaaaaggg tccgatgtcc tcgttgaagc tcttgagcgt 300

gaaggtgtta ccaatgtgtt tgcttaccct ggtggtgcat ctatggaaat ccaccaagct 360

ctcacacgct ctaaaaccat ccgcaatgtc ctccctcgcc atgaacaagg cggggttttc 420

gccgccgagg gatatgctag agctactgga aaggttggtg tctgcattgc gacttctggt 480

cctggtgcta ccaacctcgt atcaggtctt gctgacgctc tccttgattc tgtccctctt 540

gttgccatca ctggccaagt tccacgccgt atgattggca ctgatgcttt tcaggagact 600

ccaattgttg aggtgacaag gtctattact aagcataatt atttagtttt ggatgtagag 660

gatattccta gaattgttaa ggaagccttt tttttagcta attctggtag gcctggacct 720

gttttgattg atcttcctaa agatattcag cagcaattgg ttgttcctga ttgggatagg 780

ccttttaagt tgggtgggta tatgtctagg ctgccaaagt ccaagttttc gacgaatgag 840

gttggacttc ttgagcagat tgtgaggttg atgagtgagt cgaagaagcc tgtcttgtat 900

gtgggaggtg ggtgtttgaa ttctagtgag gagttgagga gatttgttga gttgacaggg 960

attccggtgg ctagtacttt gatggggttg gggtcttacc cttgtaatga tgaactgtct 1020

cttcatatgt tggggatgca cgggactgtt tatgccaatt atgcggtgga taaggcggat 1080
ttgttgcttg ctttcggggt taggtttgat gatcgtgtga ccgggaagct cgaggcgttt 1140
gctagccgtg ctaagattgt gcatattgat attgactctg ctgagattgg gaagaacaag 1200
cagccccatg tgtccatttg tgctgatgtt aaattggcat tgcggggtat gaataagatt 1260
ctggagtcta gaatagggaa gctgaatttg gatttctcca agtggagaga agaattaggt 1320
gagcagaaga aggaattccc actgagtttt aagacatttg gggatgcaat tcctccacaa 1380
tatgccattc aggtgcttga tgagttgacc aatggtaatg ctattataag tactggtgtt 1440
gggcagcacc aaatgtgggc tgcgcagcat tacaagtaca gaaaccctcg ccaatggctg 1500
acctctggtg ggttgggggc tatgggtttt gggctaccag ccgccattgg agctgcagtt 1560
gctcgaccag atgcagtggt tgtcgatatt gatggggatg gcagttttat tatgaatgtt 1620
caagagttgg ctacaattag ggtggaaaat ctcccagtta agataatgct gctaaacaat 1680
caacatttag gtatggttgt ccaattggaa gataggttct ataaagctaa ccgggcacat 1740
acataccttg gaaacccttc caaatctgct gatatcttcc ctgatatgct caaattcgct 1800
gaggcatgtg atattccttc tgcccgtgtt agcaacgtgg ctgatttgag ggccgccatt 1860
caaacaatgt tggatactcc agggccgtac ctgctcgatg tgattgtacc gcatcaagag 1920
catgtgttgc ctatgattcc aagtggtgcc ggtttcaagg ataccattac agagggtgat 1980
ggaagaacct cttattgatc ggtttaatga cggttggaac catttaaaga gggtaagcta 2040
tattactgta tgtatattag tatgttcctg gataatttag aagcttttgt ctgttgtctt 2100
ttgcagttta tgaagttagt ttgctgttgt catgttactt gttactttaa aaagcttttt 2160
gtagtttttg agcaactagt atggaatgct cttcctgtat tgcttggaaa attcacaaaa 2220
gtggtttttc ggctatggat gttgtgttgc atcatgcata tatagcttga tatactagtt 2280
ggcttggtgc atctttaaca tatactaatg agactacgac agcaattgcc aattagttgg 2340
cttgacataa ttcttagtct gccactagaa atcttgcttc ttttttttcc ctcatttgtt 2400
gaaagtccct gttgcgacct gacatgggag cattggagat tgtttagcaa tagcagttgc 2460
agttaggtga ctacagtcat cttccaaata tgaatactct ctggagggga ggaggtttta 2520
caaaatatga gtttttaaca catgagaaac ttatattaaa caaggttgag tcaccccata 2580
tttttcaaag ttgacttttg tctgatttgg gtgactatgc ctgttgtatg caataaactc 2640
gatgtacata agacttgtat aatccaatct aacccttcct ggctgattat gaaaccgagt 2700
cggctaattt gttgcttgat cttcatgtgt gagcctgatg ccaggtgacc actagaggag 2760
tacctttcat tgagatatcg attcggttaa ttggttcctc atatgggtct caaaactgaa 2820
tttttctcag gctctcttct aaccagttgt tgaattttat gaaacttcag tccagttaaa 2880
actttgatcc cggatcagaa tttctctgag ttgttcttcc gcctctcagt tccactgttc 2940

cagtgttctt ggtcctcaac ctgtactctg agctatttct gcaaacacct gaagttcctg 3000

ctgcccttgg acaaatacag aatgcagcat tagcatttat ttaagacaag atgaatactt 3060

gctcccctgt tagttatgct aattctgcat tagtattctt taattttaat tagatactgc 3120

aatccgtgaa ctctgcagtt tcttgatctc tctctgtttc aatttctcct atcttatcgc 3180

ccatttcttt aggctttcgt tatctatcgt ctaattcaag agtaggtaac taggtacggg 3240

ataaaacttt tt 3252

<210> 80
<211> 3261
<212> DNA
<213> Artificial Sequence

<220>
<223> BvALS_SU_res including artifical InDel marker allele

<400> 80
atggcggcta ccttcacaaa cccaacattt tccccttcct caactccatt aaccaaaacc 60

ctaaaatccc aatcttccat ctcttcaacc ctcccctttt ccacccctcc caaaacccca 120

actccactct ttcaccgtcc cctccaaatc tcatcctccc aatcccacaa atcatccgcc 180

attaaaacac aaactcaagc accttcttct ccagctattg aagattcatc tttcgtttct 240

cgatttggcc ctgatgaacc cagaaaaggg tccgatgtcc tcgttgaagc tcttgagcgt 300

gaaggtgtta ccaatgtgtt tgcttaccct ggtggtgcat ctatggaaat ccaccaagct 360

ctcacacgct ctaaaaccat ccgcaatgtc ctccctcgcc atgaacaagg cggggttttc 420

gccgccgagg gatatgctag agctactgga aaggttggtg tctgcattgc gacttctggt 480

cctggtgcta ccaacctcgt atcaggtctt gctgacgctc tccttgattc tgtccctctt 540

gttgccatca ctggccaagt tccacgccgt atgattggca ctgatgcttt tcaggagact 600

ccaattgttg aggtgacaag gtctattact aagcataatt atttagtttt ggatgtagag 660

gatattccta gaattgttaa ggaagccttt tttttagcta attctggtag gcctggacct 720

gttttgattg atcttcctaa agatattcag cagcaattgg ttgttcctga ttgggatagg 780

ccttttaagt tgggtgggta tatgtctagg ctgccaaagt ccaagttttc gacgaatgag 840

gttggacttc ttgagcagat tgtgaggttg atgagtgagt cgaagaagcc tgtcttgtat 900

gtgggaggtg ggtgtttgaa ttctagtgag gagttgagga gatttgttga gttgacaggg 960

attccggtgg ctagtacttt gatggggttg ggtcttaccc cttgtaatga tgaactgtct 1020

cttcatatgt tggggatgca cgggactgtt tatgccaatt atgcggtgga taaggcggat 1080

ttgttgcttg ctttcggggt taggtttgat gatcgtgtga ccgggaagct cgaggcgttt 1140

gctagccgtg ctaagattgt gcatattgat attgactctg ctgagattgg gaagaacaag 1200

cagccccatg tgtccatttg tgctgatgtt aaattggcat tgcggggtat gaataagatt 1260
ctggagtcta gaatagggaa gctgaatttg gatttctcca agtggagaga agaattaggt 1320
gagcagaaga aggaattccc actgagtttt aagacatttg gggatgcaat tcctccacaa 1380
tatgccattc aggtgcttga tgagttgacc aatggtaatg ctattataag tactggtgtt 1440
gggcagcacc aaatgtgggc tgcgcagcat tacaagtaca gaaaccctcg ccaatggctg 1500
acctctggtg ggttgggggc tatgggtttt gggctaccag ccgccattgg agctgcagtt 1560
gctcgaccag atgcagtggt tgtcgatatt gatggggatg gcagttttat tatgaatgtt 1620
caagagttgg ctacaattag ggtggaaaat ctcccagtta agataatgct gctaaacaat 1680
caacatttag gtatggttgt ccaattggaa gataggttct ataaagctaa ccgggcacat 1740
acataccttg gaaacccttc caaatctgct gatatcttcc ctgatatgct caaattcgct 1800
gaggcatgtg atattccttc tgcccgtgtt agcaacgtgg ctgatttgag ggccgccatt 1860
caaacaatgt tggatactcc agggccgtac ctgctcgatg tgattgtacc gcatcaagag 1920
catgtgttgc ctatgattcc aagtggtgcc ggtttcaagg ataccattac agagggtgat 1980
ggaagaacct cttattgatc ggtttaatga cggttggaac catttaaaga gggtaagcta 2040
tattactgta tgtatattag tatgttcctg gataatttag aagcttttgt ctgttgtctt 2100
ttgcagttta tgaagttagt ttgctgttgt catgttactt gttactttaa aaagcttttt 2160
gtagtttttg agcaactagt atggaatgct cttcctgtat tgcttggaaa attcacaaaa 2220
gtggtttttc ggctatggat gttgtgttgc atcatgcata tatagcttga tatactagtt 2280
ggctgcacta tcgtggtgca tctttaacat atactaatga gactacgaca gcaattgcca 2340
attagttggc ttgacataat tcttagtctg ccactagaaa tcttgcttct tttttttccc 2400
tcatttgttg aaagtccctg ttgcgacctg acatgggagc attggagatt gtttagcaat 2460
agcagttgca gttaggtgac tacagtcatc ttccaaatat gaatactctc tggaggggag 2520
gaggttttac aaaatatgag tttttaacac atgagaaact tatattaaac aaggttgagt 2580
caccccatat ttttcaaagt tgacttttgt ctgatttggg tgactatgcc tgttgtatgc 2640
aataaactcg atgtacataa gacttgtata atccaatcta acccttcctg gctgattatg 2700
aaaccgagtc ggctaatttg ttgcttgatc ttcatgtgtg agcctgatgc caggtgacca 2760
ctagaggagt acctttcatt gagatatcga ttcggttaat tggttcctca tatgggtctc 2820
aaaactgaat ttttctcagg ctctcttcta accagttgtt gaattttatg aaacttcagt 2880
ccagttaaaa ctttgatccc ggatcagaat ttctctgagt tgttcttccg cctctcagtt 2940
ccactgttcc agtgttcttg gtcctcaacc tgtactctga gctatttctg caaacacctg 3000
aagttcctgc tgcccttgga caaatacaga atgcagcatt agcatttatt taagacaaga 3060
tgaatacttg ctcccctgtt agttatgcta attctgcatt agtattcttt aattttaatt 3120

agatactgca atccgtgaac tctgcagttt cttgatctct ctctgtttca atttctccta 3180

tcttatcgcc catttcttta ggctttcgtt atctatcgtc taattcaaga gtaggtaact 3240

aggtacggga taaaactttt t 3261

## Description

### TECHNICAL FIELD

This invention relates to the field of biotechnology. More specifically, the invention relates to a method for making an artificial marker allele for the identification of a nucleic acid of interest in an organism. The invention also relates to determining the presence of a nucleic acid of interest in a mixed population and a method for introgressing a nucleic acid of interest into a population. The invention also relates to organisms, particularly plants and seeds, comprising an artificial marker allele and to various uses for the artificial marker allele.

### BACKGROUND

Plant breeding has made remarkable progress in increasing crop yields for over a century. Nevertheless, plant breeders constantly face new challenges. Changes in agricultural practices create the need for developing genotypes with new agronomic characteristics. New fungal and insect pests continually evolve and overcome existing host-plant resistance. New land areas are regularly being used for farming, exposing plants to altered growing conditions. Finally, a rising global population will require increased crop for food production. Thus, the task of increasing crop yields represents an unprecedented challenge for plant breeders and agricultural scientists.

Plant breeding will play a key role in the coordinated effort for providing solutions to the above problems. Given the context of current yield trends, predicted population growth and pressure on the environment, traits relating to yield stability and sustainability are a major focus of plant breeding efforts. These traits include durable disease resistance, abiotic stress tolerance and nutrient- and water-use efficiency.

Despite optimism about continued yield improvement from conventional breeding, new biotechnological solutions will be needed to maximize the probability of success. One area of biotechnology, namely DNA marker technology, derived from research in molecular genetics and genomics, offers great promise for plant breeding. Owing to genetic linkage, DNA markers can be used to detect the presence of allelic variation in the genes underlying a desired trait. By using DNA markers to assist in plant breeding, efficiency and precision could be greatly increased. The use of DNA markers in plant breeding is called marker-assisted selection (MAS) and is a component of the new discipline of 'molecular breeding'.

Over the last two decades, the use of DNA marker technology in plant breeding has dramatically increased. However, the use of marker assisted breeding and the identification of suitable markers is a laborious and time-consuming process. Furthermore, marker assisted breeding is limited because plant genomes are richly dispersed with repetitive sequences which significantly obstruct the possibility of the development and use of diagnostic markers. Especially for crops with large genome sizes, the identification of low copy number DNA segments can be highly challenging. Furthermore, in many cases only DNA polymorphisms with extremely tight linkage to the trait gene or even the causal polymorphism itself can be exploited to be converted into useful DNA markers.

EP 2 342 337 B1 describes a method of introducing unique, artificial and selectable markers at targeted regions instead of identifying and exploiting naturally occurring polymorphisms. The strategy described is based on identifying and selecting a section of DNA that is closely linked to the trait(s) of interest and converting this section into a selectable marker by inserting a single nucleotide polymorphism (SNP) into a substantially conserved nucleotide composition of this DNA section. The method described in EP 2 342 337 B1 however suffers from the drawbacks of being time-consuming and laborious and generating a marker of low sensitivity and reliability, making the resulting markers unsuitable for quality control purposes, for example.

It would therefore be advantageous to be able to provide artificial marker alleles and methods for the production of the same which overcome the aforementioned problems.

### SUMMARY OF THE INVENTION

The present invention overcomes these problems by providing artificial InDel marker alleles having increased sensitivity and reliability that can be used in particular for quality control applications.

According to a first aspect of the present invention, there is provided a method for making an artificial marker allele for the identification of a nucleic acid of interest, preferably encoding a polypeptide conferring a trait of interest, in an organism, the method comprising:
(a) identifying at least one genomic locus in the genome of the organism, which is genetically linked to the nucleic acid of interest, and
(b) introducing at least one InDel into the at least one genomic locus,
thereby making a marker allele which is inheritable to subsequent generations of the organism along with the nucleic acid of interest.

According to a second aspect of the present invention, there is provided a method for determining the presence of a nucleic acid of interest, preferably encoding a polypeptide conferring a trait of interest, in a mixed population of individuals comprising the nucleic acid of interest and individuals not comprising the nucleic acid of interest, said method comprising detection of an artificial marker allele as defined in the first aspect of the invention using at least one molecular marker specific for the artificial marker allele and/or at least one molecular marker specific for the wild type genomic locus.

According to a third aspect of the present invention, there is provided a method for assessing the homogeneity of a population of individuals comprising a nucleic acid of interest, preferably encoding a polypeptide conferring a trait of interest, said method comprising detection of an artificial marker allele as defined in the first aspect of the invention and determining homogeneity in the population by using at least one molecular marker specific for the artificial marker allele and/or at least one molecular marker specific for the wild type genomic locus, wherein the detection of the wild type genomic locus indicates heterogenous distribution of individuals comprising the nucleic acid of interest in the population.

According to a fourth aspect of the present invention, there is provided a method for introgressing a nucleic acid of interest, preferably encoding a polypeptide conferring a trait of interest, to a population of individuals, comprising the steps of:
(i) making an artificial marker allele according to the first aspect of the invention in a donor organism comprising the nucleic acid of interest;
(ii) crossing said donor organism with a recipient organism of the same species not comprising the nucleic acid of interest to generate progeny of heterogenous genetic composition;
(iii) backcrossing/selfing and selection for the presence of the artificial marker allele to obtain progeny of homozygous genetic composition, which comprise the nucleic acid of interest in the background of the recipient organism,
(iv) optionally, repeating step (iii) at least once, preferably several times.

Step (iii) of the method is based on detection using at least one molecular marker specific for detection of the presence of the artificial marker allele in the progeny and/or at least one molecular marker specific for detection of the absence of the artificial marker allele in the progeny.

The recipient organism may be a plant, an animal, a microorganism or a fungus, preferably a plant, more preferably a plant of an elite line, a wild type plant, a mutant plant, a gene-edited or a base-edited plant or a transgenic plant.

According to a fifth aspect of the present invention, there is provided a method for making an artificial marker allele comprising designing one or more genotype-specific InDels and introducing said InDels into a genomic locus in the genome of an organism, wherein the genomic locus is genetically linked to a nucleic acid of interest, preferably encoding a polypeptide conferring a trait of interest. Also provided is an artificial marker allele comprising at least one genotype-specific InDel obtainable by such method.

According to a sixth aspect of the present invention, there is provided use of an artificial marker allele according to the fifth aspect or use of an artificial marker allele obtainable by a method according to the first aspect of the present invention in marker assisted breeding.

Also provided is the use of a programmable nuclease for the generation of an artificial marker allele according to the first aspect of the present invention for the identification of a nucleic acid of interest in the genome of an organism. The programmable nuclease may be selected from CRISPR nuclease systems, zinc finger nucleases, TALENs, meganucleases, or base editors.

According to a seventh aspect of the present invention, there is provided an organism, preferably a plant or a seed thereof, comprising an artificial marker allele obtainable by a method according to the first aspect or comprising an artificial marker allele according to the fifth aspect.

### DETAILED DESCRIPTION

The first aspect of the present invention provides a method for making an artificial marker allele for the identification of a nucleic acid of interest, preferably encoding a polypeptide conferring a trait of interest, in an organism, the method comprising:
(a) identifying at least one genomic locus in the genome of the organism, which is genetically linked to the nucleic acid of interest, and
(b) introducing at least one InDel into the at least one genomic locus,
thereby making a marker allele which is inheritable to subsequent generations of the organism along with the nucleic acid of interest.

The nucleic acid of interest preferably encodes a polypeptide encoding a trait of interest. The trait may be a phenotypic trait and may be observable phenotypically, e.g., by the naked eye or by other means, such as microscopy, through biochemical analysis, genomic analysis, transcriptional profiling etc. The phenotype may be attributed to a single gene or genetic locus or may result from the action of several genes. Typical traits in the genome of a plant of economic importance include yield-related traits, including lodging resistance, flowering time, shattering resistance, seed color, endosperm composition, nutritional content, herbicide resistance, including resistance to glyphosate, glufosinate/phosphinotricin, hygromycin (hyg), protoporphyrinogen oxidase (PPO) inhibitors, ALS inhibitors, and Dicamba, disease resistance, including viral resistance, fungal resistance, bacterial resistance, or insect resistance, resistance or tolerance to abiotic stress, including drought stress, osmotic stress, heat stress, cold stress, oxidative stress, heavy metal stress, nitrogen deficiency, phosphate deficiency, salt stress or waterlogging and nutrient- and water-use efficiency, male sterility. In a preferred embodiment of the invention, a trait of interest may be artificially introduced into a nucleic acid of interest by means of gene-editing (GE) based or base editor based gene modification based on gene-editing (GE) by means of a programmable nuclease or nickase, based on base editing by means of a base editor or based on a combination thereof.

An "allele" as used herein refers to a variant form of a nucleic acid sequence or gene at a particular genomic locus and the term "artificial marker allele" as used herein is taken to mean an artificially created unique allele generally not found in nature in an organism in question. The "artificial marker allele" in the context of the present invention is genetically linked to a nucleic acid of interest which is associated with a desired trait. The "artificial marker allele" as used herein therefore refers to a nucleotide polymorphism which can be used for the identification of a nucleic acid of interest associated with a trait of interest in the genome of an organism.

The first step of the method for making an artificial marker allele comprises identifying at least one genomic locus in the genome of the organism that is genetically linked to the nucleic acid of interest. Such a genomic locus is one which is unique within the genome of an organism and highly conserved across different genotypes of the organism. A skilled person in the field of animal or plant breeding will appreciate what is meant by the term "highly conserved" in the context of the present invention. In particular, the term "highly conserved" as used herein refers to a genomic sequence, preferably between 100 and 200 bp in length, which shares at least 90%, 90,5%, 91%, 91,5%, 92%, 92,5%, 93%, 93,5%, 94%, 94,5% 95%, 95,5%, 96%, 96,5%, 97%, 97,5%, 98%, 98,5%, 99%, 99,5% or 100% sequence identity across different genotypes of the organism. As used herein, "genotype" refers to the genetic constitution of an individual or group of individuals at one or more genetic loci. The genotype of an individual or a group of individuals is the sum of all genes and determines its phenotype. When referring to conservation across different genotypes of an organism, this may be conservation across different individuals in a population of a given species, cultivars or races of the organism. "Cultivar" and "variety" are used interchangeably herein to mean a group of plants within a species, for example *B. vulgaris,* that share certain genetic traits resulting in the same phenotype that separate them from other possible varieties within that species. Cultivars can be inbreds or hybrids, as applicable for the crop in question.

To identify a highly conserved genomic locus across different genotypes, detailed sequence analysis in a group of individuals is carried out to identify a region of approximately 100 to 200 base pairs (bp). The identified region must be unique within the target genome to allow specific insertion of the InDel by genome editing or base editing. The highly conserved genomic locus then allows general usage of the marker in the broadest possible range of genotypes and genetic background.

The genomic locus is ideally positioned outside of any coding region (exceptionally there may be reason to select a coding region), splicing signal or regulatory element of the nucleic acid of interest, 3'UTRs, 5'UTRs, introns, miRNAs, non-coding RNAs and any other possible features. These precautions are taken because genomic interaction cannot be excluded. The promoter region of a gene is usually not very well characterized, therefore a location in 3' direction of the target gene is preferred. However, where location is in the 5' region of a gene is favored, a promoter length of 1000 bp is assumed and will not be selected for introduction of the at least one InDel.

Furthermore, the genomic locus should preferably be in the physical vicinity and complete linkage disequilibrium (LD) to the nucleic acid of interest to avoid separation of the artificial marker allele from the nucleic acid of interest in the course of recombination. The term "linkage disequilibrium" (LD) refers to a non-random segregation of genetic loci or traits (or both) and implies that the relevant loci are within sufficient physical and/or genetic proximity along a length of a chromosome so that they segregate together with greater than random (i.e., non-random) frequency.

The genomic locus is closely linked to the nucleic acid of interest such that when an InDel is introduced into the genomic locus, so as to create an artificial marker allele, the marker allele is inheritable to subsequent generations of the organism along with the nucleic acid of interest. The genomic locus is ideally positioned in a region flanking the nucleic acid of interest and is preferably located at the 3' end of the nucleic acid of interest. The region flanking the nucleic acid of interest is preferably at a distance of at least 2 cM, 1 cM, 0.5 cM, 0.1 cM, 0.09 cM, 0.08 cM, 0.07 cM, 0.06 cM, 0.05 cM, 0.04 cM, 0.03 cM, 0.02 cM 0.01 cM, 0.009 cM, 0.008 cM, 0.007 cM, 0.006 cM, 0.005 cM, 0.004 cM, 0.003 cM, 0.002 cM 0.001 cM, 0.0009 cM, 0.0008 cM, 0.0007 cM, 0.0006 cM, 0.0005 cM, 0.0004 cM, 0.0003 cM, 0.0002 cM or 0.0001 cM from the nucleic acid of interest or at a distance anywhere in between the above values. "cM" as used herein defines the distance between two loci on a chromosome and is a measurement of recombination frequency well known in the art.

The terms "flanking region ..." or "region flanking ..." are used interchangeably herein and refer to a nucleic acid sequence of a predetermined genomic locus which is genetically linked to a nucleic acid of interest into which the at least on InDel is inserted to generate an artificial InDel marker allele.

Alternatively, the genomic locus may be within the nucleic acid of interest itself. When the genomic locus is located within the nucleic acid of interest, the genomic locus should preferably be positioned outside of any coding region, splicing signal or regulatory element of the nucleic acid of interest, 3'UTRs, 5'UTRs, introns, miRNAs, non-coding RNAs and the like, so that when the at least one InDel is introduced into the genomic locus it does not cause a loss of function.

The nucleotide sequence of the genomic locus obtained after insertion of the at least one InDel, i.e. the obtained artificial marker allele, is unique within the genome of the organism, as far as can be determined, meaning that it does not occur or only very rarely occurs in the germplasm of the organism in question. The resulting organism thus contains a specifically introduced alteration in its genetic sequence that is closely linked to the nucleic acid of interest, which preferably encodes a polypeptide conferring a trait of interest. This specifically introduced InDel (which creates an artificial marker allele) can now be used and assayed in any conventional way in marker-assisted breeding, and as further described herein.

The term "germplasm", as used herein, refers to genetic material with a specific molecular makeup that provides for some or all of the hereditary qualities of an organism or cell culture and collections of that material. Breeders use the term "germplasm" to indicate their collection of genetic material from e.g. wild type species, elite or domestic breeding lines from which they can draw to create varieties or races. As used herein, "germplasm" may be any living genetic resource including but not limited to cells, seeds or tissues from which new plants may be grown, or plant parts, such as leaves, stems, pollen, ovules, or cells that can be cultured into a whole plant.

The "organism" is preferably a plant, but may also be an animal, fungus or microorganism. The term "plant" as used herein refers to whole plants, ancestors and progeny thereof and to plant parts. Plant parts may include seeds, tissues, cells, organs, leaves, stems, roots, emerged radicles, flowers, flower parts, petals, fruits, pollen, pollen tubes, anther filaments, ovules, embryo sacs, egg cells, ovaries, zygotes, embryos, zygotic embryos, somatic embryos, apical meristems, vascular bundles, pericycles, gametophytes, spores and cuttings. The term "plant" as used herein also comprises germplasm of a plant which can be cultured into whole plants or plant parts. Progeny and ancestor plants can be from any filial generation, e.g. P, F1, F2, F3 and so on and any plant resulting from backcrossing therefrom.

The plant may be any plant and may, for example, be selected from *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Astragalus sinicus, Lotus japonicas, Torenia fournieri, Allium cepa, Allium fistulosum, Allium sativum,* and *Allium tuberosum.*

The second step in the method for making an artificial marker allele comprises introducing at least one InDel into the at least one genomic locus.

An "InDel" or "InDel marker" as defined herein is taken to mean at least one nucleotide insertion and/or at least one nucleotide deletion in the genomic locus within the genome of an organism. The at least one nucleotide insertion is also referred to herein as an "insertion marker" and the at least one nucleotide deletion is also referred to herein as a "deletion marker".

An "InDel" in the context of the present invention refers to an insertion and/or deletion of at least one nucleotide in the nucleotide sequence of a predetermined genomic locus, thereby altering the length of the nucleotide sequence of the genomic locus by at least one nucleotide. An "InDel" in the context of the present invention therefore refers to the incorporation of at least one additional nucleotide into an endogenous nucleotide sequence or the removal of at least one nucleotide from an endogenous nucleotide sequence. In contrast to an InDel, a "single nucleotide polymorphism" (SNP) means a sequence variation that occurs when a single nucleotide (A, C, T or G) in the genomic sequence is altered. A SNP is a substitution or replacement of a single nucleotide within a given nucleotide sequence, which leaves the length of the nucleotide sequence unchanged.

In a preferred embodiment of the invention, the at least one InDel comprises more than one nucleotide insertion and/or more than one nucleotide deletion. The at least one InDel may comprise an insertion of between 1 and 60 base pairs of a sequence which is non-homologous to the genome of the organism in which the at least one InDel is to be introduced. Optionally, the InDel may comprise an insertion of more than 60 base pairs of a sequence which is non-homologous to the genome of the organism in which the at least one InDel is to be introduced. The insertion may optionally comprise or consist of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 of a sequence which is non-homologous to the genome of the organism in which said at least one InDel is introduced. Preferably, the insertion comprises or consists of a nucleotide sequence of at least 9, 10, 11, 12, 13, 14, 15, 16, 17, 18, 19 or 20 base pairs of a sequence which is non-homologous to the genome of the organism in which said at least one InDel is introduced. The term "non-homologous" in this context means that the insertion is unique and does not share homology to a nucleic acid sequence in the genome of the organism which might potentially result in the incorporation of the insertion into an undesired genomic location due to homology-directed repair. The term "non-homologous" in the present context further means that the insertion when introduced into the genomic locus results in an artificial marker allele which is unique within the genome of the organism in question.

In the context of the method of making an artificial marker allele, the insertion and its flanking region in the predetermined genomic locus need to be evaluated and selected for optimal assay design, meaning that they must be singular and non-repetitive in the genome of a given organism. Furthermore, the insertion and its flanking region should exhibit one or more of the following characteristics: approximately 50% GC content, balanced distribution between G/C and A/T bases, reduced chance of secondary structures. The insertion of at least one nucleotide in the flanking region of a predetermined genomic locus should result in an insertion marker allele which is monomorphic, i.e. unique, across different genotypes of the organism. This analysis is carried out through iterative and repeated analysis of short sequences using standard bioinformatic tools and sequencing approaches.

Furthermore, the flanking region should be monomorphic in the gene pool of the organism meaning that it is highly conserved between different genotypes of the organism.

In a further embodiment of the present invention, the at least one InDel may additionally or alternatively comprise or consist of a deletion of between 1 and 60 base pairs of a sequence in the genomic locus in the genome of the organism. Optionally, the deletion is of more than 60 base pairs of a sequence in the genomic locus in the genome of the organism. The deletion may be of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 base pairs. Preferably, the deletion comprises or consists of a nucleotide sequence of at least 9, 10, 11, 12, 13, 14, 15, 16, 17, 18, 19 or 20 base pairs.

The deletion selected is one which does not result in a loss of function in the gene or genomic region. For example, the deletion marker is preferably located outside a gene associated with the desired trait of interest and/or is located in a non-coding region so as to avoid any loss-of-function. One skilled in the art would readily appreciate which genomic regions to avoid when designing a suitable deletion marker.

Furthermore, the deletion and its flanking region in the predetermined genomic locus need to be selected for optimal assay design, meaning that they must be singular and non-repetitive in the genome of a given organism and exhibit one or more of the following characteristics: approximately 50% GC content, balanced distribution between G/C and A/T bases, reduced chance of secondary DNA structures. The deletion of at least one nucleotide in the flanking region in the predetermined genomic locus should result in a deletion marker allele which is monomorphic across different genotypes of the organism. This analysis is carried out through iterative and repeated analysis of short sequences using standard bioinformatic tools.

A "balanced distribution between G/C and A/T bases" refers to a content of 40%-55% GC and respective A/T, i.e. 60%-45% depending on the actual GC content. The distribution may be 40% G/C and 60% A/T, 41% G/C and 59% A/T, 42% G/C and 58% A/T, 43% G/C and 57% A/T, 44% G/C and 56% A/T, 45% G/C and 55% A/T, 46% G/C and 54% A/T, 47% G/C and 53% A/T, 48% G/C and 52% A/T, 49% G/C and 51% A/T, 50% G/C and 50% A/T, 51% G/C and 49% A/T, 52% G/C and 48% A/T, 53% G/C and 47% A/T, 54% G/C and 46% A/T, and/or 55% G/C and 45% A/T. A balanced distribution between G/C and A/T bases effects the creation of secondary structures in the DNA at or adjacent to the predetermined locus, whereby such secondary structures influence the annealing of molecular markers. Ones skilled in the art is well-aware of this fact and is able to predict computational the suitability of a certain sequence for an optimal assay design.

Furthermore, the flanking region should be monomorphic, i.e. highly conserved in the gene pool of the organism meaning that it is highly conserved between different genotypes of the organism.

The insertion and/or deletion size can vary depending on the marker assays to be developed.

"Introducing" in the meaning of the present invention includes stable or transient integration by means of transformation including Agrobacterium-mediated transformation, transfection, microinjection, biolistic bombardment, insertion using gene editing technology like CRISPR systems (e.g. CRISPR/Cas, in particular CRISPR/Cas9 or CRISPR/Cpf1), CRISPR/CasX, or CRISPR/CasY), TALENs, zinc finger nucleases or meganucleases, homologous recombination optionally by means of one of the below mentioned gene editing technology including preferably a repair template, modification of a genomic locus using random or targeted mutagenesis like TILLING or mentioned gene editing technology, etc.

Preferably the at least one InDel may be introduced into the genomic locus using any known suitable mutagenesis methods for the introduction of nucleotide insertion(s) and/or deletion(s).

For example, the at least one InDel may be introduced using a programable nuclease or nickase. The programmable nuclease or nickase may be selected from any known gene editing (GE) tools, such as site-directed nucleases (SDNs), including CRISPR nuclease system, including a CRISPR/Cas9 system, a CRISPR/Cfp1 system, a CRISPR/CasX system, a CRISPR/CasY system, zinc-finger nucleases, TALENs, meganucleases and/or any combination, variant or catalytically active fragment thereof.

Site directed nucleases (SDNs) or nickases use a DNA cutting enzyme (nuclease) for the generation of the targeted (or site directed) DNA break. Variants of SDN applications are often categorized as SDN-1 (absence of a repair template), SDN-2 (gene editing by using DNA repair template) and SDN-3 (introduction of larger insertions/deletions by using DNA repair template) depending on the outcome of the DNA double strand break repair or the DNA single strand break repair.

Any programable nuclease or nickase may be used for the introduction of point mutations, insertions or deletions into the genome of an organism. The skilled person would readily be able to select a suitable technique based on the genomic sequence and the desired efficiency.

For example, point mutations may be generated by a classic SDN-1 approach (i.e. non-homologous end joining (NHEJ) to randomly insert/delete one or more bases to cause a point mutation). At the position where the point mutation is to be generated (or in close proximity thereto), the double strand is cleaved. The NHEJ pathway then repairs the double strand break, thereby randomly generating the desired point mutation. The selection of one particular point mutation would be difficult as a large number of plants would need to be screened, therefore, a SDN-2 approach (as detailed below) is preferred for introducing specific point mutations at a predetermined genomic location (i.e. homology directed repair (HDR) with repair template).

For the SDN-2 approach the DNA double strand is cleaved at a predetermined genomic location (or in close proximity thereto) where the point mutation is to be introduced. By adding a "repair template" with homologous flanking regions upstream and downstream of the cleavage site, the desired point mutation can be introduced by HDR. This increases the probability of obtaining the desired mutation.

In general, the approaches described for the generation of point mutations also work for the generation of deletions. In addition to the above approaches, it is possible to delete a desired sequence by generating two double strand breaks upstream and downstream of the sequence to be deleted. In the selection step it is then important to ensure that a precise cleavage has occurred.

The approaches described for the generation of point mutations also work for the generation of insertions. The SDN-2 approach is preferred for the generation of insertions, although the SDN-1 approach may also be useful for in certain circumstances.

A CRISPR nuclease system in this context describes a molecular complex comprising at least one small and individual guide RNA in combination with a Cas nuclease or another CRISPR nuclease like a Cpf1 nuclease (Zetsche et al. (2015); "Cpf1 is a single RNA-guided endonuclease of a class 2 CRISPR-Cas system". Cell 163(3): 759-771) which can produce a specific DNA double-stranded break. The terms "CRISPR polypeptide", "CRISPR endonuclease", "CRISPR nuclease", "CRISPR protein", "CRISPR effector" or "CRISPR enzyme" are used interchangeably herein and refer to any naturally occurring or artificial amino acid sequence, or the nucleic acid sequence encoding the same, acting as site-specific DNA nuclease or nickase, wherein the "CRISPR polypeptide" is derived from a CRISPR system of any organism, which can be cloned and used for targeted genome engineering. The terms "CRISPR nuclease" or "CRISPR polypeptide" also comprise mutants or catalytically active fragments or fusions of a naturally occurring CRISPR effector sequence, or the respective sequences encoding the same. A "CRISPR nuclease" or "CRISPR polypeptide" may thus, for example, also refer to a CRISPR nickase or even a nuclease-deficient variant of a CRISPR polypeptide having endonucleolytic function in its natural environment.

The terms "guide RNA", "gRNA", "single guide RNA", or "sgRNA" are used interchangeably herein and either refer to a synthetic fusion of a CRISPR RNA (crRNA) and a trans-activating crRNA (tracrRNA), or the term refers to a single RNA molecule consisting only of a crRNA and/or a tracrRNA, or the term refers to a gRNA individually comprising a crRNA or a tracrRNA moiety. A tracr and a crRNA moiety, if present as required by the respective CRISPR polypeptide, thus do not necessarily have to be present on one covalently attached RNA molecule, yet they can also be comprised by two individual RNA molecules, which can associate or can be associated by non-covalent or covalent interaction to provide a gRNA according to the present disclosure. In the case of single RNA-guided endonucleases like Cpf1 (see Zetsche et al., 2015, *supra*)*,* for example, a crRNA as a single guide nucleic acid sequence might be sufficient for mediating DNA targeting.

The term "zinc finger nuclease," as used herein, refers to a nuclease comprising a nucleic acid cleavage domain conjugated to a binding domain that comprises a zinc finger array. The cleavage domain may be the cleavage domain of the type II restriction endonuclease Fokl. Zinc finger nucleases can be designed to target virtually any desired sequence in a given nucleic acid molecule for cleavage, and the possibility to the design zinc finger binding domains to bind unique sites in the context of complex genomes allows for targeted cleavage of a single genomic site in living cells. Targeting a double- strand break to a desired genomic locus can be used to introduce InDels into the nucleotide sequence of a desired genomic locus. Zinc finger nucleases can be generated to target a site of interest by methods well known to those of skill in the art. For example, zinc finger binding domains with a desired specificity can be designed by combining individual zinc finger motifs of known specificity. The structure of the zinc finger protein Zif268 bound to DNA has informed much of the work in this field and the concept of obtaining zinc fingers for each of the 64 possible base pair triplets and then mixing and matching these modular zinc fingers to design proteins with any desired sequence specificity has been described (Pavletich NP, Pabo CO (1991); "Zinc finger-DNA recognition: crystal structure of a Zif268-DNA complex at 2.1 A". Science 252 (5007): 809-17).

The term "TAL effector nucleases" (TALENs) as used herein refer to sequence-specific nucleases or nucleic acids encoding the same. TAL effectors are proteins of plant pathogenic bacteria that are injected by the pathogen into the plant cell, where they travel to the nucleus and function as transcription factors to turn on specific plant genes. The primary amino acid sequence of a TAL effector dictates the nucleotide sequence to which it binds. Thus, target sites can be predicted for TAL effectors, and TAL effectors can also be engineered and generated for the purpose of binding to particular nucleotide sequences. Specificity depends on an effector-variable number of imperfect, typically 34 amino acid repeats (Schornack et al. (2006) J. Plant Physiol. 163:256). Polymorphisms are primarily at repeat positions 12 and 13, which are referred to herein as the repeat variable-diresidue (RVD). RVDs of TAL effectors correspond to the nucleotides in their target sites in a direct, linear fashion, one RVD to one nucleotide, with some degeneracy and no apparent context dependence. This finding represents a valuable mechanism for protein-DNA recognition that enables target site prediction for new target specific TAL effector. TAL effectors *perse* do not comprise a nuclease domain. TAL effector nucleases or TALENs therefor represent fusion construct in which the TAL effector-encoding nucleic acid sequences is fused to a sequence encoding a nuclease or a portion of a nuclease, typically a nonspecific cleavage domain from a type II restriction endonuclease such as FokI (Kim et al. (1996) Proc. Natl. Acad. Sci. USA 93:1156-1160). Other useful endonucleases which can be fused to the effector domain may include, for example, HhaI, HindIII, Nod, BbvCI, EcoRI, BglI, and AlwI. The fact that some endonucleases (e.g., FokI) only function as dimers can be capitalized upon to enhance the target specificity of the TAL effector. For example, in some cases each FokI monomer can be fused to a TAL effector sequence that recognizes a different DNA target sequence, and only when the two recognition sites are in close proximity do the inactive monomers come together to create a functional enzyme. By requiring DNA binding to activate the nuclease, a highly site-specific restriction enzyme can be created.

As used herein, the term "meganuclease" refers to an endonuclease that binds double-stranded DNA at a recognition sequence that is greater than 12 base pairs. Naturally-occurring meganucleases can be monomeric (e.g., I-SceI) or dimeric (e.g., I-CreI). The term meganuclease, as used herein, can be used to refer to monomeric meganucleases, dimeric meganucleases, or to the monomers which associate to form a dimeric meganuclease. The term "homing endonuclease" is synonymous with the term "meganuclease. Due to the large recognition site of meganucleases, this site generally occurs only once in any given genome. Meganucleases can therefore be used to achieve very high levels of gene targeting efficiencies in mammalian cells and plants (Rouet et al., MoI. Cell. Biol., 1994, 14, 8096-106; Choulika et al., MoI. Cell. Biol., 1995, 15, 1968-73). Among meganucleases, the LAGLIDADG family of homing endonucleases has become a valuable tool for the study of genomes and over the past years. The term "LAGLIDADG meganuclease" refers either to meganucleases including a single LAGLIDADG motif, which are naturally dimeric, or to meganucleases including two LAGLIDADG motifs, which are naturally monomeric.

For example, the at least one InDel may also be introduced using a programable base editor, optionally in combination with a programable nuclease. The programable "base editor" as used herein refers to a protein or a fragment thereof having the same catalytical activity as the protein it is derived from, which protein or fragment thereof, alone or when provided as molecular complex, referred to as base editing complex herein, has the capacity to mediate a targeted base modification, i.e., the conversion of a base of interest resulting in a point mutation of interest. Preferably, the at least one base editor in the context of the present invention is temporarily or permanently linked to at least one site-specific, programable effector, or optionally to a component of at least one site-specific, programable effector complex. The linkage can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al ((2017). Programmable base editing of A•T to G•C in genomic DNA without DNA cleavage. Nature, 551(7681), 464.) described adenine base editors (ABEs) that mediate the conversion of A•T to G•C in genomic DNA.

Any base editing complex according to the present invention can thus comprise at least one cytidine deaminase, or a catalytically active fragment thereof. The at least one base editing complex can comprise the cytidine deaminase, or a domain thereof in the form of a catalytically active fragment, as base editor.

In one embodiment of the present invention, a donor plant comprising a desired trait may be modified, for example, by using a programmable nuclease to introduce an InDel into a suitable genomic locus as described herein to generate an artificial InDel marker allele. In the case where the the artificial InDel marker allele comprises a deletion, primers specific for the deleted sequence are designed. A person skilled in the art will readily be able to design suitable primers.

A "primer" as used herein refers to an oligonucleotide (synthetic or occurring naturally), which is capable of acting as a point of initiation of nucleic acid synthesis or replication along a complementary strand when placed under conditions in which synthesis of a complementary strand is catalysed by a polymerase. Typically, primers are about 10 to 30 nucleotides in length, but may be longer or shorter. Primers may be provided in double-stranded form, though the single-stranded form is more typically used. A primer can further contain a detectable label, for example a 5' end label.

After crossing the donor plant with a wildtype plant, progeny samples are analyzed for the presence or absence of the deletion marker allele by, for example, (q)PCR, or other suitable techniques. "Wild type" plant/organism as defined herein is taken to mean an unmodified plant of the same species or variety as the donor plant into which the at least one InDel has been introduced.

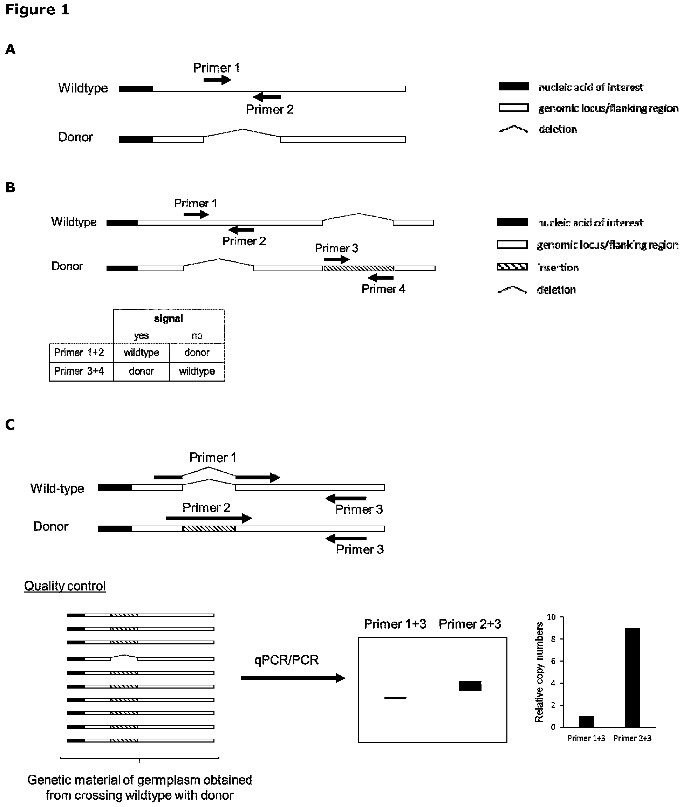

Signals obtained for primers specific for the deleted sequence indicate that the progeny plant has the wildtype genotype, lacking the donor trait of interest or at least that the trait of interest was not inherited in a homozygous way by the progeny plant. Conversely, no signal for primers 1+2 (see Figure 1A) could suggest homogenous multiplication of the donor trait. It however remains uncertain whether no signal is definitively due to the absence of the donor trait or whether other factors are responsible (e.g. insufficient primer annealing etc.). In order to increase primer specificity, the deletion is preferably at least approximately 10bp in length, preferably approximately 20bp in length.

Where the artificial InDel marker allele comprises a combination of insertions and deletions, insertions and deletions linked to the desired trait may be inserted using a programable nuclease, as defined herein, at a predetermined genomic locus within a flanking region of the nucleic acid of interest, which preferably encodes a polypeptide conferring a trait of interest. Primers specific for the insertion (see primers 3+4 in the illustration below) can be used for the detection of the donor trait. Since the insertion is absent in the wildtype, a positive signal reliably indicates the presence of the donor trait in the progeny plant making the assessment of the presence or absence of a desired trait highly specific and more accurate. Furthermore, primers specific for the deletion marker can be used for the identification of progeny plants which do contain the desired trait (no signal for primers 1+2).

The combination of an insertion with a deletion is thus more reliable when determining the presence or absence of a desired trait, since the presence/absence is determined by a positive PCR signal. This approach allows for the assessment whether a desired trait is present in the genome of progeny samples obtained from crossing a donor plant and a wild type plant and whether the donor trait was multiplied in a homozygous or heterozygous manner.

According to a preferred embodiment, the method of the invention therefore comprises introduction of an InDel comprising an insertion and a deletion. The insertion and/or deletion is preferably at least 10, 11, 12, 13, 14, 15, 16, 17, 18, 19 or 20 base pairs.

As illustrated in Figure 1B, in such preferred embodiment, primers 1+2 may be located completely in the region of deletion so that only the wildtype genotype is detected. Even if one of the primers is located outside the deletion (or both primers partially), the marker system remains specific, since PCR products will only be obtained for the wildtype genotype. Specificity of the marker system is thus assured as long as most of the primer(s) (e.g. 10bp) is located in the region of deletion.

In addition to primers 1+2 specific for the deletion, additional primers may be used which are specific for the insertion. For example, primers 3+4 may be located completely in the region of the insertion so that only the donor genotype is detected. Even if one of the primers is located outside the insertion (or both primers partially), the marker system remains specific, since PCR products will only be obtained for the donor genotype provided that at least one primer is located in the region of insertion.

The combination of an insertion and a deletion in the herein described methods of the invention and the use of primers specific for the inserted InDels thus provide a reliable strategy for determining the presence or absence of a desired trait, since the presence/absence is determined by a positive PCR signal which significantly reduces the chance of "false positive" or "false negatives".

In a further embodiment of the herein described methods of the invention, the at least one InDel may advantageously be introduced into the genomic locus in the genome of a donor plant (comprising the nucleic acid of interest, preferably encoding a polypeptide conferring the trait of interest) at the beginning of the breeding process, i.e. before the donor is crossed with a desired elite line. An "elite line" means any line that has resulted from breeding and selection for superior agronomic performance. Numerous elite lines are available and known to those of skill in the art.

The abovementioned approach ensures that all elite lines which are crossed with the donor can be readily screened for the InDel marker allele. By designing a screening assay based on the InDel marker allele generated in the genomic background of a given donor, it is possible to use one established screening system for different elite lines to assess whether the desired trait has been inherited by such edited line. This approach avoids laborious and time-consuming development of marker assays designed for the genetic background of a given elite line into which the InDel marker allele has been inserted by crossing the elite line with the donor line. With the above described method, it is therefore possible to assess whether different elite lines contain the desired trait of the donor by applying one established screening method which was designed for the genomic background of the trait donor. Furthermore, side effects (e.g. pleiotropic effects) on phenotype due to the genome editing can be tested in parallel.

Furthermore, if InDel marker alleles are already used in a breeding process, one or several elite donors may be edited to generate a second donor generation suitable for the concept of marker-assisted breeding and quality control (see Figure 1C).

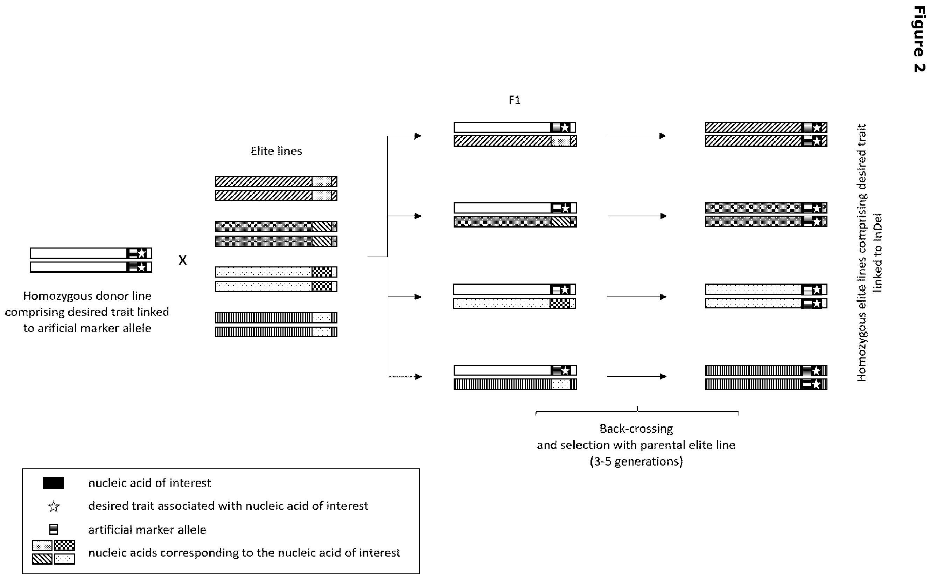

Figure 2 illustrates exemplary the above-mentioned breeding process. A homozygous donor comprising a desired trait (asterisk) is linked to an artificial InDel marker allele (grey filled). The InDel polymorphism has been introduced into a genomic locus of a suitable flanking region of a nucleic acid of interest associated with a desired trait (black filled) via a programmable nuclease. In a common breeding process, the homozygous donor is crossed with several elite lines to obtain (after backcrossing/selfing and selection) homozygous elite lines comprising the nucleic acid of interest associated with the desired trait. Due to the development of an InDel marker allele specific for the genomic background of the donor line, the elite lines can be screened for the InDel marker allele associated with the desired trait by using one single screening assay designed specifically for the flanking region of the InDel marker allele of the donor genotype. Based on this approach, there is thus no need to develop screening assays specific for the different genotypic flanking regions of the different elite lines. The insertion of the at least one InDel into a genomic locus genetically linked to the donor trait at the very beginning of the breeding process (introgression process) into the elite line therefore provides a method to screen different elite lines for the insertion of a desired trait independently of their respective genomic background.

The term "backcrossing" as referred to herein is a process in which a progeny plant is repeatedly crossed back to one of its parents. The "donor" comprises the nucleic acid sequence of interest associated with the desired trait linked to the InDel marker allele and which is to be introgressed into the recipient line. The "recipient" may be an elite line or any other plant into which the nucleic acid of interest is to be introgressed. "Introgression" as defined herein refers to the transmission of a desired allele of a genetic locus from one genetic background to another. The initial cross gives rise to the F1 generation. As shown in Figure 2, a backcross is performed repeatedly across several generations (with a progeny individual of each successive backcross generation being itself backcrossed to the same parental genotype) until a homozygous elite line comprising the trait of interest linked to the InDel marker allele is obtained.

As used herein, "selecting" or "selection" in the context of marker-assisted selection or breeding refers to the act of picking or choosing desired individuals, normally from a population, based on certain pre-determined criteria. Suitable selection techniques are commonly known and are a routine part of an experimental setup for any skilled person in the field of plant breeding.

The InDel introduction into the genomic locus results in the creation of an artificial marker allele which is inheritable to subsequent generations of the organism along with the nucleic acid of interest. The artificial marker allele (the InDel once introduced into the genomic region) may be detectable and distinguishable on the basis of its polynucleotide length and/or sequence.

The artificial marker allele may therefore be detected using any available method for the detection of polymorphisms in genomic DNA samples, such detection tools and methods are referred to herein as "molecular markers". The genomic DNA sample may be genomic DNA isolated directly from a plant, cloned genomic DNA, or amplified genomic DNA.

PCR-based methods are preferred for the detection of the artificial marker allele, however any of various hybridization techniques with specific probes including Southern blotting, in-situ hybridization and comparative genomic hybridization may alternatively be used. Furthermore, DNA digestion and high-solution capillary electrophoresis can be used to detect artificial marker alleles. Other suitable detection methods include microarrays, mass spectrometry-based methods, and/or nucleic acid sequencing methods.

In a preferred embodiment of the invention, the molecular marker is defined as a pair of primers specific for the artificial marker allele, i.e. the predetermined genomic locus comprising the at least one InDel, or the wild type genomic locus. The primers are preferably at least 10, 11, 12, 13, 14, 15, 16, 17, 18, 19 or 20 base pairs in length.

Marker assays for target genes are also mostly available. However, these assays are not always fully diagnostic/unique. The fully diagnostic marker allele is in every case the functional polymorphism. However, due to the characteristics of the flanking regions of the nucleic acid/trait of interest, it is not always possible to design suitable marker assays for marker-assisted selection. In addition, in case of a functional SNP marker allele, it is not possible to develop highly sensitive assays that would be suitable for reliable quality control assays for new traits in the breeding process. An InDel marker allele, like the one described herein, can be applied in marker-assisted selection of the target trait and would be applicable in highly sensitive quality control assays. For example, the inventive marker alleles can be used to assure purity of seed multiplications regarding the respective target trait and to avoid contaminations of seeds containing an undesired trait or which lack the desired trait of interest. Although, sensitive assays can in principle be developed based on SNP polymorphisms, the sensitivity of SNP detection is technically limited and significantly lower compared to the herein described artificial InDel marker alleles, since the detection of the polymorphism is based on only one single base pair mismatch, which can easily result in the detection of false positives or false negatives. In case of the InDel polymorphisms described herein it is possible to detect one (undesired) allele among several thousand samples, whereas a SNP polymorphism would allow detection of an (undesired) allele only within a few dozen samples.

According to a second aspect of the present invention, there is provided a method for determining the presence of a nucleic acid of interest, preferably encoding a polypeptide conferring a trait of interest, in a mixed population of individuals comprising the nucleic acid of interest and individuals not comprising the nucleic acid of interest, said method comprising detection of an artificial marker allele as defined in the first aspect of the invention using at least one molecular marker specific for the artificial marker allele and/or at least one molecular marker specific for the wild type genomic locus.

The at least one molecular marker is as defined herein in the first aspect of the invention and is preferably a pair of primers annealing to the wild type genomic locus or the artificial marker allele. Preferably the primers allow the detection of the artificial marker allele comprising an insertion and deletion marker. The primers may be specific to the inserted or deleted sequences in the genomic locus. The primers are preferably at least 10, 11, 12, 13, 14, 15, 16, 17, 18, 19 or 20 base pairs in length.

A "population" of plants means a set comprising any number of physical individuals or samples or data taken therefrom for evaluation.

According to a third aspect of the present invention, there is provided a method for assessing the homogeneity of a population of individuals comprising a nucleic acid of interest, preferably encoding a polypeptide conferring a trait of interest, said method comprising detection of an artificial marker allele as defined in the first aspect of the invention and determining homogeneity in the population by using at least one molecular marker specific for the artificial marker allele and/or at least one molecular marker specific for the wild type genomic locus, wherein the detection of the wild type genomic locus indicates heterogenous distribution of individuals comprising the nucleic acid of interest in the population.

Preferably the at least one molecular marker is a pair of primers annealing to the wild type genomic locus or the artificial marker allele. Preferably the primers allow the detection of the artificial marker allele comprising an insertion and deletion marker. The primers may be specific to the inserted or deleted sequences in the genomic locus. The primers are preferably at least 10, 11, 12, 13, 14, 15, 16, 17, 18, 19 or 20 base pairs in length.

According to a fourth aspect of the present invention, there is provided a method for introgressing a nucleic acid of interest, preferably encoding a polypeptide conferring a trait of interest, to a population of individuals, comprising the steps of:
(i) making an artificial marker allele according to the first aspect of the invention in a donor organism comprising the nucleic acid of interest;
(ii) crossing said donor organism with a recipient organism of the same species not comprising the nucleic acid of interest to generate progeny of heterogenous genetic composition;
(iii) backcrossing/selfing and selection for the presence of the artificial marker allele to obtain progeny of homozygous genetic composition, which comprise the nucleic acid of interest in the background of the recipient organism,
(iv) optionally, repeating step (iii) at least once, preferably several times.

Step (iii) of the method is based on detection using at least one molecular marker specific for detection of the presence of the artificial marker allele in the progeny and/or at least one molecular marker specific for detection of the absence of the artificial marker allele in the progeny. Preferably, the at least one molecular marker is a pair of primers annealing to the wild type genomic locus or the artificial marker allele. Preferably the primers allow the detection of the artificial marker allele comprising an insertion and deletion marker. The primers may be specific to the inserted or deleted sequences in the genomic locus. The primers are preferably at least 10, 11, 12, 13, 14, 15, 16, 17, 18, 19 or 20 base pairs in length.

The recipient organism may be an elite line, a wild type organism or a transgenic organism. The terms "wild type" and "elite" are as defined herein. The term "transgenic" refers to organisms into which a gene or genetic material has been transferred (typically by any of a number of genetic engineering techniques) from one organism to another or from the same organism but where the genetic material is not at its natural locus in the genome.

According to a fifth aspect of the present invention, there is provided a method for making an artificial marker allele specific for a nucleic acid of interest comprising designing one or more genotype-specific InDels and introducing said InDels into a genomic locus in the genome of an organism, wherein the genomic locus is linked to the nucleic acid of interest. The organism may comprise at least 1, 2, 3, 4, 5, 6, 7, 8, 9, 10, 11, 12, 13, 14, 15, 16, 17, 18, 19, 20 or more separate InDels, which create a fingerprint of sorts for detection and tracking purposes.

Also provided is an artificial marker allele comprising at least one genotype-specific InDel obtainable by the aforementioned method.

According to a sixth aspect of the present invention, there is provided use of an artificial marker allele according to the fifth aspect or use of an artificial marker allele obtainable by a method according to the first aspect of the present invention in marker assisted breeding.

A further aspect of the invention relates to the use of the InDel marker allele in combination with the modification of an endogenous gene of interest. Modification of a gene of interest can be achieved by commonly known gene editing approaches (e.g. site-directed nucleases, including CRISPR nuclease systems, Zinc-finger nucleases, TALENs, meganucleases and the like) to generate an "artificial trait" of interest. The combined use of GE based gene modification and the herein described artificial InDel marker alleles readily allow the direct and reliable detection of regenerated modified plants (from gene edited plant material) or modified progenies thereof.

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial increase or reduction of the transgene expression and/or substantial increase or reduction of expression of the endogenous gene. The isolated gene may be isolated from an organism or may be manmade, for example by chemical synthesis.

Also provided herein is the use of a programmable nuclease for the generation of an artificial marker allele for the identification of a nucleic acid of interest in the genome of an organism. The programmable nuclease may be selected from CRISPR nuclease systems, zinc finger nucleases, TALENs, or meganucleases as described herein.

According to a seventh aspect of the present invention, there is provided a plant or seed comprising an artificial marker allele obtainable by a method according to the first aspect or comprising an artificial marker allele according to the fifth aspect of the present invention. The plant may be any plant and may for example be a plant selected from *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Astragalus sinicus, Lotus japonicas, Torenia fournieri, Allium cepa, Allium fistulosum, Allium sativum,* and *Allium tuberosum.*

Throughout the description and claims of this specification, the words "comprise" and "contain" and variations of the words, for example "comprising" and "comprises", mean "including but not limited to", and do not exclude other components, integers or steps. Moreover, the singular encompasses the plural unless the context otherwise requires: in particular, where the indefinite article is used, the specification is to be understood as contemplating plurality as well as singularity, unless the context requires otherwise.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥ 3, ≥4, ≥5, ≥6 or ≥7 etc. of said members, and up to all said members.

Preferred features of each aspect of the invention may be as described in connection with any of the other aspects. Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible.

### BRIEF DESCRIPTION OF THE DRAWINGS

One or more embodiments of the invention will now be described, by way of example only, with reference to the accompanying drawing, in which:
**Figure 1A-C** shows a schematic representation of marker-assisted analyses and a quality control assay in which the purity of multiplied seeds having a desired trait can be assured, by using the InDel approach of the present invention.
**Figure 2** shows a schematic representation of a breeding process in which an InDel is introduced into the genomic locus of a donor plant (comprising the nucleic acid of interest, preferably encoding a polypeptide conferring the trait of interest) at the beginning of the breeding process, i.e. before the donor is crossed with a desired elite line.
**Figure 3** shows InDel marker-assisted selection of a gene encoding a mutated cytochrome P450 oxidase conferring male sterility.
**Figure 4** shows InDel marker-assisted selection of a gene encoding a point-mutated acetolactate synthase conferring herbicide resistance.

### EXAMPLES

### Example 1: Deletion Marker Allele

### Introduction of a deletion by genome editing for marker-assisted selection

This example demonstrates the use of a deletion marker for the detection of a desired trait, which would be otherwise difficult to identify due to the characteristics of the genomic regions flanking the causative polymorphism.

The Beta vulgaris mutant BvCYP703A2_gst as disclosed in DE 10 2016 106 656.7 comprises a deletion in the gene encoding for a cytochrome P450 oxidase which confers to the mutant a male sterile phenotype (wildtype (WT) BvCYP703 = BvCYP703_WT (SEQ ID NO: 75)). This phenotype can be used e.g. to improve breeding programs and for the production of hybrid seeds.

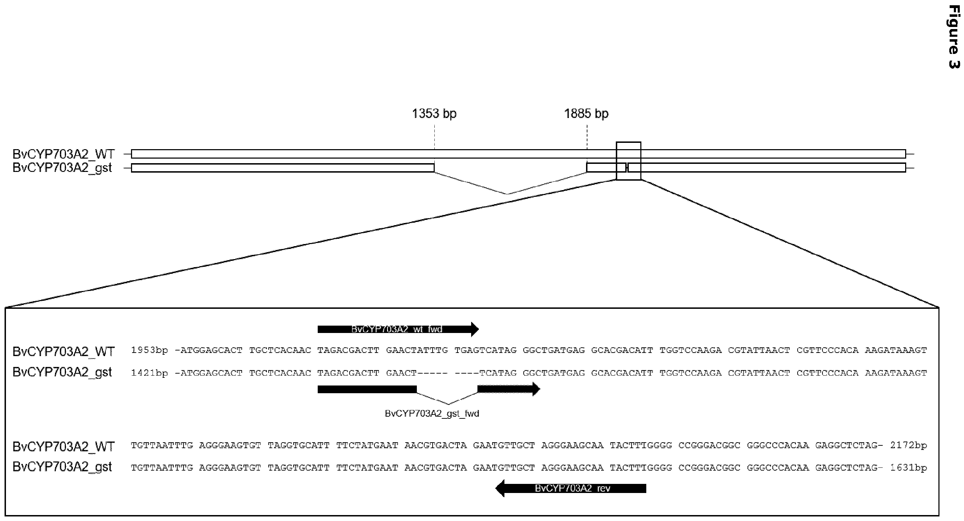

The mutant BvCYP703A2_gst (SEQ ID NO: 76) comprises a large deletion between position 1560 and 2100 (see Figure 3). However, due to the characteristics of the genomic regions flanking the deletion (i.e. highly repetitive and high AT content) it is difficult (if not impossible) to design suitable primers and assays that would allow direct detection of the causative polymorphism itself. It would therefore not be possible to screen progeny plants, which were obtained by crossing a wild-type plant with the donor, for the desired genotype by direct detection of the deletion due to the lack of suitable detection assays.

The inventors have therefore identified a region in the flanking region of the gene encoding a cytochrome P450 oxidase which is suitable for InDel marker-assisted selection of the desired genotype. The naturally occurring deletion causing the trait (male sterility) is located between positions -200 and +333 of the BvCYP703A2 gene (numbering starts at the translation initiation site). Since the deletion causes a disruption of the gene, there is no doubt that remaining gene features (e.g. exons) are unfunctional and additional manipulation within the remaining exons does not cause pleiotropic effects. Therefore, parts of remaining exon 1, spanning region +334 to +500 were chosen as target site for an artificial InDel marker allele. The maximum distance from the deletion position +334 to the end of the region of interest (+500) is 166 bp corresponding to a genetic distance of 0.00096 cM. Blast analysis of the 166 bp fragment did not reveal unspecific hits in the sugar beet genome. Further sequence analysis (repetitivity, GC content, base distribution) led to definition of region +434 to +443 as target site for an artificial deletion, with an InDel specific primer set between positions +420 to +449.

A deletion is inserted into this target site via genomic editing as described herein (SEQ ID NO: 77). Suitable primers are designed specific to the flanking region of the deletion marker (see above). Due to its tight linkage to the desired genotype, this deletion can then be used to identify progeny plants conferring male sterility. For homo/heterogenous detection of the deletion two PCR reactions should be performed.

Possible primers which can be used for the detection of the donor and/or wild type strain may be:
BvCYP703A2_WT_fwd: 5'-TAGACGACTTGAACTATTTGTGAG-3' (SEQ ID NO: 45)
BvCYP703A2_gst_fwd: 5'-TAGACGACTTGAACTTCATAGGGC-3' (SEQ ID NO: 46)
BvCYP703A2_rev: 5'-AAAGTATTGCTTCCCTAGCAACA-3' (SEQ ID NO: 47)

### Example 2: Insertion Marker Allele

### Introduction of an insertion by genome editing for marker-assisted selection

This example demonstrates that a desired trait, which is difficult to detect because its causal link is a single nucleotide polymorphism (SNP), can be reliably identified by using the herein described InDel marker approach.

In this example, a single point mutation at position +1706 in the gene encoding for the enzyme acetolactate synthase confers resistance to sulfonyl urea herbicides in a Beta vulgaris plant (as disclosed in WO 2012/049268; wildtype (WT) BvALS = BvALS_WT (SEQ ID NO: 78; point-mutated BvALS = BvALS_SU_res (SEQ ID NO: 79)). This single nucleotide polymorphism is difficult to detect because primers designed specifically for screening plants having the donor trait would differ in only one single nucleotide in comparison to the wild-type sequence, thereby increasing the likelihood of false-positives and/or false negatives which limits the quality of the screen.

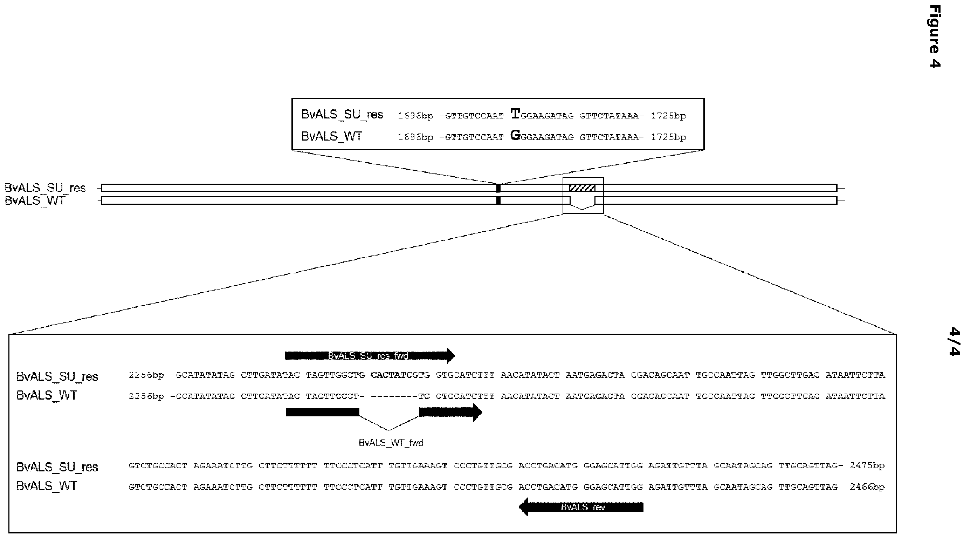

This drawback can be overcome by introducing an InDel marker into the flanking region of the mutated gene encoding for acetolactate synthase (see Figure 4).

The inventors have identified a morphogenic flanking region of the mutated gene suitable for the design of an artificial marker allele. The SNP causing the trait (SU resistance, W569L) is located at position +1706 of the BvALS gene (numbering starts at the translation initiation site). The annotated 3'UTR region of the gene ends at position +2252. The inventors were unable to localize a genomic feature starting from position +2253 to +4000. The maximum distance from the SNP position +1706 to the end of the region of interest (+4000) is 2294 bp corresponding to a genetic distance of 0.00036 cM. Blast analysis of the 2294 bp fragment did not reveal unspecific hits in the sugar beet genome. Iterative sequence analysis (blast, alignments) led to selection of region +2274 to +2445 suitable for artificial InDel placement. Further sequence analysis (repetitively, GC content, base distribution) led to definition of region +2285 to +2293 as artificial target site, with an InDel specific primer set between positions +2274 to +2303 (see Figure 4).

Into this target site a 9 bp long insertion can be inserted which is non-homologous and unique to the genomic pool of the donor line (SEQ ID NO: 80). Suitable primers are designed for the flanking regions of the insertion as described herein. For homo/heterogenous detection of the insertion marker two PCR reactions are required.

Based on this approach it is then possible to screen progeny plants, obtained from crossing the donor with the wild-type plant, for the insertion of the desired mutation conferring herbicide resistance without the need to rely on the causative polymorphism itself.

Possible primers which can be used for the detection of the donor and/or wild type strain may be:
BvALS_WT_fwd: 5'-ACTAGTTGGCTTGGTGCATCT-3' (SEQ ID NO: 48)
BvALS_SU_res_fwd: 5'-ACTAGTTGGCTGCACTATCGTGC-3' (SEQ ID NO: 49)
BvALS_rev: 5'-CCAATGCTCCCATGTCAGGT-3' (SEQ ID NO: 50)

### Example 3: Quality control assay to assure purity of seed multiplications for a respective trait

This example illustrates how purity of multiplied seeds having a desired trait can be assured by using the herein described InDel approach.

In this example, the donor line comprising a desired trait is modified by introducing a nucleotide sequence (GCACTATCG) into its genome to generate an artificial insertion marker allele which is tightly linked to the desired trait.

After crossing the donor comprising the insertion marker allele with a wildtype plant, which does not contain the artificial marker allele, F1 progeny plants are obtained which are heterogenous in their genetic composition. Backcrossing and subsequent selection result in plants which contain the trait of interest within the genetic background of the wildtype plant. In order to ensure homogeneity and purity of seed multiplication of plants comprising the desired trait, seed samples are analyzed by using primer pairs specific for the wildtype (primers 1+3) and/or the donor (primer 2+3). Analysis of the seed samples by e.g. (q)PCR then readily allows assessment of the degree of purity (see Figure 1C).

Based on this quality control assay, it is thus possible to reliably assess whether the tested seed samples are homozygous for the desired trait or whether the seeds are "contaminated" with the wildtype gene/trait corresponding to the desired donor trait. Such quality control would not be possible, if the polymorphism linked to the desired trait is a single nucleotide polymorphism, since a single nucleotide mismatch does not offer sufficient resolution and specificity to ensure a reliable quality assessment by (q)PCR.

### Example 4: GE based technology for the generation of artificial InDel marker alleles which are linked to a desired trait.

This example provides a technical description on how to
(a) generate a deletion marker allele via GE based gene modification into a donor genome having a large deletion in the gene encoding a cytochrome P450 oxidase causing male sterility (Example 1),
(b) generate an insertion marker allele via GE based gene modification into a donor genome having a point mutation in the gene encoding for the enzyme acetate lactate synthase conferring herbicide resistance in a Beta vulgaris plant (Example 2), and
(c) modify an endogenous gene encoding the enzyme acetate lactate synthase by introducing a specific point mutation (G→T) via GE, thereby conferring herbicide resistance in a Beta vulgaris plant and generating an insertion marker allele linked to the artificially generated trait of interest.

### Design and selection of crRNA:

Suitable crRNAs for Cpf1-induced induction of double strand breaks were designed by using the CRISPR RGEN Tools (http://www.rgenome.net/cas-designer/ [Park J., Bae S., and Kim J.-S. Cas-Designer: A web-based tool for choice of CRISPR-Cas9 target sites. Bioinformatics 31, 4014-4016 (2015). and Bae S., Park J., and Kim J.-S. Cas-OFFinder: A fast and versatile algorithm that searches for potential off-target sites of Cas9 RNA-guided endonucleases. Bioinformatics 30, 1473-1475 (2014).). Therefore, suitable protospacers within the genomic DNA sequence were identified and selected. To ensure functionality of Cpf1 endonuclease from *Lachnospiraceae bacterium* ND2006 (Lb) (SEQ ID NO: 51), protospacers with a length of 24 nucleotides were selected, wherein their genomic binding sequence at the 5' end was flanked with an essential protospacer adjacent motif (PAM) having the sequence 5'-TTTV-3' (V is G, C or A). Suitable protospacers were selected based on the prescribed quality criteria of the tool and analyzed for potential off-targets with an internal reference genome of B. vulgaris.

For further experiments crRNAs were selected, which in addition to the actual target sequence had at most 15 identical bases with a functional PAM. Since the first 18 nucleotides of the protospacer are essential for recognizing and cleaving the target sequence, it was thereby possible to avoid unwanted cleavage within other genomic sequences [Tang, X., L. G. Lowder, T. Zhang, A. A. Malzahn, X. Zheng, D. F. Voytas, Z. Zhong, Y. Chen, Q. Ren, Q. Li, E. R. Kirkland, Y. Zhang and Y. Qi (2017). "A CRISPR-Cpfl system for efficient genome editing and transcriptional repression in plants." Nat Plants 3: 17018.]. Based on this approach, the following potential crRNAs specific for various positions were identified (see Table A).

**Table A: Selected target sequences. PAM sequences are underlined.**

| Name of crRNA | Genomic target sequence with 5'-flanking PAM (underlined) | Binding on +/- strand | Function |
|---|---|---|---|
| crRNA_ALS_G/T | TTTAggtatggttgtccaatgggaagat (SEQ ID NO: 1) | - | generating G→T point mutation in ALS |
| crRNA_ALS_In1 | TTTCggctatggatgttgtgttgcatca (SEQ ID NO: 2) | - | candidate 1 for generating an insertion marker for MAS in ALS |
| crRNA_ALS_In2 | TTTCtacgtggttcggttgatcatatag (SEQ ID NO: 3) | + | Candidate 2 for generating an insertion for MAS in ALS |
| crRNA_CYP_Del | TTTGtacgtggttcggttgatcatatag (SEQ ID NO: 4) | + | Generating a deletion marker for MAS in CYP |

### Cloning of genetic elements:

For the cloning of Cpf1- and crRNA expression cassettes, a hindering recognition sequence of the restriction enzyme BbsI was removed from the target vector pZFNnptII by introducing a point mutation (T→G). The mutagenesis was performed with a commercially available mutagenesis kit according to the manufacturer's instructions by using two mutagenesis primers (see Table B).

**Table B: Mutagenesis primers used for the introduction of the point mutation (G→T, underlined) for removal of the BbsI recognition sequence**

| Name | Sequence 5'→3' |
|---|---|
| Mutagenesis primer 1 | CAGTGCAGCCGTCGTCTGAAAACGACA (SEQ ID NO: 5) |
| Mutagenesis primer 2 | AACGTCAGAAGCCGACTGCACTATAG (SEQ ID NO: 6) |

For the expression of the Lbcpf1 gene in *B. vulgaris* a DNA fragment comprising a DNA sequence, codon-optimized for *A. thaliana,* was synthesized wherein the DNA sequence had a 5' flanking PcUbi promoter sequence from *Petroselinum crispum* and a 3' flanking 3A terminator sequence from *Pea sp.* (SEQ ID NO: 52). Restriction cleavage sites within the coding sequence of Lbcpf1 which are relevant for cloning, were removed by introducing silent mutations (i.e. nucleotide exchange without effecting the amino acid sequence). Codon-optimization was performed based on the GeneArt algorithm from ThermoScientific. To allow the transport of cpfl into the nucleus of the cell, the coding sequence of cpfl was linked to a nuclear localization signal (NLS) from SV40 at the 5' end and a NLS from Nucleoplasmin at the 3' end. For the ligation with the binary target vector pZFNnptII the expression cassette was flanked by two HindIII restriction cleavage sites. For the cloning of the crRNA-expression cassette an additional PstI cleavage site was inserted between the 5' flanking HindIII cleavage site and the PcUbi promoter sequence. Ligation of pZFNnptII_LbCpf1 was done by following a standard protocol. Successful insertion of the PcUbi: :Cpf1: :TPea expression cassette (SEQ ID NO: 52) was confirmed via sequencing, wherein the used primers were designed to specifically bind to a region spanning the flanking region of the vector as well as parts of the expression cassette (see Table C).

**Table C: Primers used for sequencing of the PcUbi::Cpf1::TPea expression cassette integrated into pZFNnptII vector**

| Name | Sequence 5'→3' |
|---|---|
| pSeq_LbCpf1_F1 | AGCGCAACGCAATTAATGTG (SEQ ID NO: 7) |
| pSeq_LbCpf1_R1 | GATGAAGCTGAGGTAGTACC (SEQ ID NO:8) |
| pSeq_LbCpf1_F2 | AGGAAGGTTAGCAAGCTCGAG (SEQ ID NO: 9) |
| pSeq_LbCpf1_R2 | TCTCGTCGACCTTCTGGATG (SEQ ID NO: 10) |
| pSeq_LbCpf1_F3 | ATGCTGAGTACGATGACATCC (SEQ ID NO: 11) |
| pSeq_LbCpf1_R3 | TAGACCTGCTTCTCAACCTTCA (SEQ ID NO: 12) |
| pSeq_LbCpf1_F4 | ACCACTCACTCCTCGATAAG (SEQ ID NO: 13) |
| pSeq_LbCpf1_R4 | AACGACAATCTGATCGGGTAC (SEQ ID NO: 14) |

After transcription in a plant cell, crRNAs were intended to be cleaved by two flanking ribozymes. Therefore, the precursor crRNAs were flanked by the coding sequences of a Hammerhead ribozyme (SEQ ID NO: 53) and a HDV ribozyme (SEQ ID NO: 54) [Tang, X., L. G. Lowder, T. Zhang, A. A. Malzahn, X. Zheng, D. F. Voytas, Z. Zhong, Y. Chen, Q. Ren, Q. Li, E. R. Kirkland, Y. Zhang and Y. Qi (2017). "A CRISPR-Cpfl system for efficient genome editing and transcriptional repression in plants." Nat Plants 3: 17018.]. Other approaches exist for the transcription of crRNA, e.g. via PolII promoters, Cpf1 cleavage from mRNA, other ribozymes etc. For a seamless ligation of the single protospacer to the sequence of the crRNA repeats, two BbsI recognition sequences were integrated between the crRNA repeat and the HDV ribozyme, wherein the overhangs used for cloning were adjusted accordingly.

To ensure an identical expression strength of cpfl and crRNAs, the crRNA ribozyme cassette was flanked by a PcUbi promoter sequence at the 5' end and a 3A terminator sequence at the 3' end. The crRNA expression cassette was flanked by two PstI cleavage sites for the later ligation into the pZFNnptII_Cpf1 target vector (SEQ ID NO: 55). The crRNA expression cassette (SEQ ID NO: 56) was commercially obtained as a synthetic DNA fragment. Ligation was performed by following a standard protocol. The correct insertion of the expression cassette was confirmed by multiple rounds of sequencing. The protospacer were ordered as complementary oligonucleotides and annealed according to standard protocols. The 24bp long DNA fragments generated in this way were flanked by 4nt overhangs relevant for the ligation step (see Table D).

**Table D: Sequences of oligonucleotides used for the generation of 24bp short protospacer. 4nt overhangs used for ligation are underlined**

| Name crRNA | Sequence 5'→3' |
|---|---|
| crRNA_ALS_G/T | AGATGGTATGGTTGTCCAATGGGAAGAT (SEQ ID NO: 15) |
| | GGCCATCTTCCCATTGGACAACCATACC (SEQ ID NO: 16) |
| crRNA_ALS_In1 | AGATGGCTATGGATGTTGTGTTGCATCA (SEQ ID NO: 17) |
| | GGCCTGATGCAACACAACATCCATAGCC (SEQ ID NO: 18) |
| crRNA_ALS_In2 | AGATTACGTGGTTCGGTTGATCATATAG (SEQ ID NO: 19) |
| | GGCCCTATATGATCAACCGAACCACGTA (SEQ ID NO: 20) |
| crRNA-CYP-Del | AGATTACGTGGTTCGGTTGATCATATAG (SEQ ID NO: 21) |
| | GGCCCTATATGATCAACCGAACCACGTA (SEQ ID NO: 22) |

The efficiency of the 4 crRNAs were tested via Agrobacterium induced gene transfer in leaves of *B*. *vulgaris.* The pZFNtDTnptII plasmid (SEQ ID NO: 57) was co-transformed to verify the transformation efficiency. Transformation of the leaf explants were done by vacuum infiltration following a standard protocol. The fluorescence of tDT was measured after six days by fluorescence microscopy. Explants with a heterogenous fluorescence were discarded. Leaf explants were shock-frozen in liquid nitrogen ten days after infiltration, ground and genomic DNA was isolated via the CTAB protocol. The efficiency of the single crRNAs was validated via NGS (external service provider) based on the number of inserted edits (e.g. number of insertions, deletions or nucleotide exchanges) relative to non-edited sequences in the genomic DNA.

Since all tested crRNAs showed activity, the crRNAs crRNA_ALS_G/T (SEQ ID NO: 58), crRNA-CYP-Del (SEQ ID NO: 59), crRNA_ALS_In1 (SEQ ID NO: 60) (most efficient) and crRNA_ALS_In2 (SEQ ID NO: 61), with the above described ribozyme, promoter and terminator sequences as reverse-oriented expression cassettes were ordered as synthetic DNA constructs (in total 4 constructs; for each crRNAs one construct (SEQ ID NO: 62, 63, 64, 65)). The DNA constructs were each flanked by two PstI restriction cleavage sites for cloning into the target vector pZFNnptII_LbCpf1 (SEQ ID NO: 55). After insertion of crRNAs, LbCpf1 and crRNA expression cassettes were ligated via HindIII from the pZFNnptII_LbCpf1_crRNA vector (SEQ ID NO: 23, 71, 72, 73, 74) into the pUbitDTnptII vector (SEQ ID NO: 66, 67, 68, 69, 70).

### Generation and use of repair templates for HD-repair

### ALS G→T mutation

In order to generate the G→T point mutation, the repair template was designed to comprise 1000 bp upstream and downstream of the point mutation. The whole DNA template was ordered as a 2001 bp long synthetic DNA fragment (SEQ ID NO: 24) and directly used for transformation in the vector backbone of the provider. The repair template plasmid and the pUbitDTnptII_LbCpf1_crRNA plasmid (SEQ ID NO: 67) were introduced into *B. vulgaris* callus culture via biolistic co-bombardment using a gene gun according to an optimized delivery protocol. The transformation efficiency was validated based on the transient tDT fluorescence via fluorescence microscopy one day after transformation. The callus culture was cultivated in shoot induction medium in the absence of selective pressure (i.e. without Kanamycin). The regenerated shoots were subsequently tested for the site-directed mutation (in principle, if point mutation results in increased ALS resistance, such increase can be used for selection of the desired event). Therefore, genomic DNA was isolated via CTAB. Point mutations were amplified via two PCRs and the use of primers 5'ALS_G/T and ALS_G/T_Rv, as well as ALS_G/T_Fw and 3'ALS_G/T. Afterwards, PCR products were sequenced in each case with both primers. Here, it is important that binding of the first primer occurs within the homology region of the repair template and binding of the second primer outside of the 5' and 3' flanking homology regions of the repair template (see Table E).

**Table E: Primers used for the detection of point mutations**

| Name | Sequence 5'→3' | Size of PCR product | Binding |
|---|---|---|---|
| 5'ALS_G/T | GTT TTG GAT GTA GAG GAT ATT CCT AGA (SEQ ID NO: 25) | 1138 bp | 5' outside the repair template |
| ALS_G/T_Rv | CAG GGA AGA TAT CAG CAG ATT TG (SEQ ID NO: 26) | | Within the repair template |
| ALS_G/T_Fw | CTA CAA TTA GGG TGG AAA ATC TC (SEQ ID NO: 27) | 1127 bp | Within the repair template |
| 3'ALS_G/T | CTC TAG TGG TCA CCT GGC ATC (SEQ ID NO: 28) | | 3' outside the repair template |

In addition to the detection of the successful point mutation in the genome of *B. vulgaris,* the undesired integration of plasmid DNA was also analyzed. Therefore, genomic DNA, for which the successful integration of a point mutation at the desired locus had been confirmed, was analyzed for the presence of plasmid DNA via PCR. Sequence regions within the cpfl, the crRNA ribozyme cassette and the tDT were amplified using the primers listed in Table F below.

**Table F: Primers used for the detection of stable integrated plasmid-specific sequences in the genome of B. vulgaris shoots**

| Name | Sequence 5'→3' | Size of PCR product | Binding |
|---|---|---|---|
| pSeq_LbCpf1_F4 | ACCACTCACTCCTCGATAAG (SEQ ID NO: 29) | 214 | Cpf1 |
| pSeq_LbCpf1_R3 | TAGACCTGCTTCTCAACCTTCA (SEQ ID NO: 30) | | |
| pSeq_Ribozyme_F | TGCAGCGGATCCAAATTACTG (SEQ ID NO: 31) | 172 | crRNA-ribozyme cassette |
| pSeq_Ribozyme_R | CCTGGTCCCATTCGCCAT (SEQ ID NO: 32) | | |
| pSeq_tDT_F | TTACAAGAAGCTGTCCTTCC (SEQ ID NO: 33) | 400 | *tDT* |
| pSeq_tDT_R | GTACTGTTCCACGATGGTGT (SEQ ID NO: 34) | | |

### ALS 9bp insertion:

For the ALS 9bp insertion an analogous approach was used as described for ALS G→T mutations above. The 9bp insertion GCACTATCG was flanked upstream and downstream with a 1000bp homologous sequence (SEQ ID NO: 35).

**Table G: Primers used for the detection of the insertion**

| Name | sequence 5'→3' | Size of the PCR product | Binding |
|---|---|---|---|
| 5'ALS_Insertion | GTG CTG ATG TTA AAT TGG CAT TGC (SEQ ID NO: 36) | 1148 +9 bp | 5' outside the repair template |
| ALS_Insertion_Rv | CTA GTG GCA GAC TAA GAA TTA TG (SEQ ID NO: 37) | | Within the repair template |
| ALS_Insertion_Fw | GAA TGC TCT TCC TGT ATT GCT TG (SEQ ID NO: 38) | 1165 + 9 bp | Within the repair template |
| 3'ALS_Insertion | CAG TTC AAC ACA AAA GAA GTT GTC (SEQ ID NO: 39) | | 3' outside the repair template |

### Combined insertion of the G→T point mutation and the 9bp insertion in ALS:

In general, an analogous procedure is applied as for both approaches described above. In this case, however, the repair template is only flanked by 250bp homologous sequences upstream and downstream, since homologues flanking sequences with 1000bp upstream and downstream of the respective repair templates would overlap. In this setup, the plasmids for crRNA_ALS_G/T and crRNA_ALS_In1, as well as both repair templates were transformed using biolistic co-bombardment. Detection of the point mutation and the 9bp insertion was done as described above.

### CYP deletion marker:

The deletion can also be generated and detected using one of the above described approaches. For the generation of a deletion marker, it is important that the repair template must contain the 9bp deletion (ATTTGTGAG). This is then also flanked 1000bp homologous sequences upstream and downstream of the repair template and used for the construct (SEQ ID NO: 40).

**Table H: Primers used for the detection of the deletion**

| Name | Sequentes 5'→3' | Size of the PCR product | Binding |
|---|---|---|---|
| 5'CYP_Deletion | GTC TTT ACA TAG CAA AAC AAT ATT GAA G (SEQ ID NO: 41) | 1167 - 9 bp | 5' outside the repair template |
| CYP_Deletion_Rv | CTA ACA CTT CCC TCA AAT TAA CAA C (SEQ ID NO: 42) | | Within the repair template |
| CYP_Deletion_Fw | CAA TAG TGG TGA TGT GGC CTT GG (SEQ ID NO: 43) | 1198 - 9 bp | Within the repair template |
| 3'CYP_Deletion | GGT AAC TAG TAA AAG TAT ACT CAT C (SEQ ID NO: 44) | | 3' outside the repair template |

## Claims

1. A method for making an artificial marker allele for the identification of a nucleic acid of interest, preferably encoding a polypeptide conferring a trait of interest, in an organism, said method comprising:
(a) identifying at least one genomic locus in the genome of said organism, which is genetically linked to said nucleic acid of interest, and
(b) introducing at least one InDel into said at least one genomic locus,
thereby making a marker allele which is inheritable to subsequent generations of said organism along with said nucleic acid of interest.

2. The method according to claim 1, wherein said at least one InDel comprises at least one nucleotide insertion and/or at least one nucleotide deletion.

3. The method according to any preceding claim, wherein said genomic locus is unique within the genome of said organism and highly conserved across different genotypes of said organism and/or wherein the nucleotide sequence of the genomic locus obtained after insertion of the at least one artificial InDel is unique within the genome of said organism.

4. The method according to any preceding claim, wherein said genomic locus is positioned outside of any coding region, splicing signal or regulatory element of the nucleic acid of interest and/or is positioned in a region flanking the nucleic acid of interest or within the nucleic acid of interest.

5. The method according to claim 4, wherein the region flanking the nucleic acid of interest is located at the 3' end of the nucleic acid of interest.

6. The method according to claim 5, wherein the region flanking the nucleic acid of interest is a distance of at least 2 cM or 1 cM or 0.5 cM or 0.1 cM from said nucleic acid of interest.

7. The method according to any preceding claim, wherein said at least one InDel comprises an insertion and wherein said insertion comprises a nucleotide sequence in the range of between 1 and 60 contiguous base pairs and which sequence is non-homologous to the genome of the organism in which said at least one InDel is introduced, preferably said insertion comprises a nucleotide sequence of at least 10 or at least 20 contiguous base pairs.

8. The method according to any preceding claim, wherein said at least one InDel comprises a deletion and wherein said deletion is in the range of between 1 and 60 contiguous base pairs, preferably at least 10 or at least 20 contiguous base pairs, relative to the corresponding wild type sequence of the genomic locus in which said at least one InDel is introduced.

9. The method according to any preceding claim, wherein said at least one InDel is introduced by a programmable nuclease, preferably said programmable nuclease is selected from CRISPR nuclease and guide RNA systems, zinc finger nucleases, TALENs, or meganucleases.

10. The method according to any preceding claim, wherein said nucleic acid of interest may be an endogenous gene, a heterologous gene, a mutated gene, a transgenic gene or a modified gene introduced or generated by gene editing or base editing.

11. A Method for determining the presence of a nucleic acid of interest, preferably encoding a polypeptide conferring a trait of interest, in a mixed population of individuals comprising the nucleic acid of interest and individuals not comprising the nucleic acid of interest, said method comprising detection of an artificial marker allele as defined in any of claims 1 to 10 using at least one molecular marker specific for the artificial marker allele and/or at least one molecular marker specific for the wild type genomic locus.

12. A Method for assessing the homogeneity of a population of individuals comprising a nucleic acid of interest, preferably encoding a polypeptide conferring a trait of interest, said method comprising detection of an artificial marker allele as defined in any of claims 1 to 10 and determining homogeneity in the population by using at least one molecular marker specific for the artificial marker allele and/or at least one molecular marker specific for the wild type genomic locus, wherein the detection of the wild type genomic locus indicates heterogenous distribution of individuals comprising the nucleic acid of interest in the population.

13. A method for making an artificial marker allele for the detection of a nucleic acid of interest comprising designing one or more genotype-specific InDels and introducing said InDels into a genomic locus in the genome of an organism, wherein the genomic locus is genetically linked to the nucleic acid of interest.

14. Use of an artificial marker allele obtainable by any one of claims 1 to 10 in marker assisted selection.

15. Plant or seed comprising an artificial marker allele obtainable by a method according to any of claims 1 to 10.
